# EUROPEAN PATENT SPECIFICATION

(11) **EP 2 742 040 B1**
(45) Date of publication and mention of the grant of the patent: **06.04.2016**
(21) Application number: 12750354.8
(22) Date of filing: 10.08.2012
(51) Int. Cl.: C07D 403/12, C07D 403/14, C07D 405/14, C07D 409/14, C07D 413/14, C07D 417/14, C07D 471/04, A61K 31/416, A61K 31/4439, A61P 37/00

(54) **INDAZOLE COMPOUNDS, COMPOSITIONS AND METHODS OF USE**
INDAZOL-VERBINDUNGEN, PHARMAZEUTISCHE ZUSAMMENSETZUNGEN UND VERFAHREN ZU IHRER VERWENDUNG
COMPOSES D'INDAZOLE, COMPOSITIONS PHARMACEUTIQUES ET PROCEDES D'UTILISATION DE CEUX-CI

(30) Priority: 12.08.2011 US 201161523036 P
(43) Date of publication of application: 18.06.2014
(73) Proprietor: F.Hoffmann-La Roche AG, 4070 Basel (CH)
(72) Inventor: BURCH, Jason, South San Francisco, CA 94080 (US); GOLDSMITH, Richard, A., South San Francisco, CA 94080 (US); ORTWINE, Daniel, Fred, South San Francisco, CA 94080 (US); PASTOR, Richard, South San Francisco, CA 94080 (US); PEI, Zhonghua, South San Francisco, CA 94080 (US)
(74) Representative: Sauer, Frank
(86) International application number: PCT/EP2012/065656
(87) International publication number: WO 2013/024011

(56) References cited:
- PAUL G WYATT ET AL: "Identification of N-(4-Piperidinyl)-4-(2,6-dichlorobenzoylam ino)-1H-pyrazole-3-carboxamide (AT7519), a Novel Cyclin Dependent Kinase Inhibitor Using Fragment-Based X-Ray Crystallography and Structure Based Drug Design", JOURNAL OF MEDICINAL CHEMISTRY, AMERICAN CHEMICAL SOCIETY, US , vol. 51, no. 16 1 January 2008 (2008-01-01), pages 4986-4999, XP002674967, ISSN: 0022-2623, DOI: 10.1021/JM800382H Retrieved from the Internet: URL:http://pubs.acs.org/doi/abs/10.1021/jm 800382h [retrieved on 2008-07-26]
- JIN XIE ET AL: "Aminopyridinecarboxamide-based inhaled IKK-2 inhibitors for asthma and COPD: Structureactivity relationship", BIOORGANIC & MEDICINAL CHEMISTRY, PERGAMON, GB, vol. 19, no. 3, 13 December 2010 (2010-12-13), pages 1242-1255, XP028133990, ISSN: 0968-0896, DOI: 10.1016/J.BMC.2010.12.027 [retrieved on 2010-12-23]

## Description

### FIELD OF THE INVENTION

Compounds of formula I, which are inhibitors of ITK kinase, as well as compositions containing these compounds, and methods of use including, but not limited to, *in vitro, in situ* and *in vivo* diagnosis or treatment of mammalian cells.

### BACKGROUND OF THE INVENTION

ITK is a Tec family kinase that is expressed in T cells, NKT cells, NK cells, and mast cells. ITK is activated downstream of antigen engagement of the T cell receptor (TCR) and mediates TCR signals through the phosphorylation and activation of PLCg. Mice in which ITK is deleted showed defective differentiation of T cells towards to the Th2 subset, but not the Th1 subset. Additional studies indicate that Th2 cytokine production, but not early Th2 lineage commitment, is defective in ITK-deficient mouse T cells. Th2 cells promote allergic inflammation, and ITK knock-out mice have reduced lung inflammation, mucus production, and airway hyperreactivity in models of allergic asthma. The reduction in lung pathology in ITK knock-out asthma models is not rescued by a kinase-deficient ITK transgene, indicating that the kinase activity of ITK is necessary for asthma pathology. Human patients with immunological and inflammatory disorders, such as the allergic disease atopic dermatitis, express higher levels of ITK in peripheral blood T cells.

There exists a need for inhibitors of ITK kinase and treatments of diseases and disorders mediated by ITK kinase.

### SUMMARY OF THE INVENTION

An aspect includes a compound of formula I: 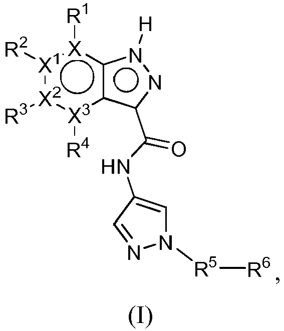 stereoisomers or a pharmaceutically acceptable salt thereof, wherein X, X¹, X², X³, R¹, R², R³, R⁴, R⁵ and R⁶ are described herein.

Another aspect includes a pharmaceutical composition comprising a compound of formula I, stereoisomers or a pharmaceutically acceptable salt thereof and a therapeutically inert carrier, diluent or excipient.

Another aspect includes a method of treating a disease responsive to the inhibition of ITK kinase in a patient, comprising administering an effective amount of a compound of formula I, stereoisomers or a pharmaceutically acceptable salt thereof

Another aspect includes a method of treating an immunological or inflammatory disease in a patient, comprising administering an effective amount of a compound of formula I, stereoisomers or a pharmaceutically acceptable salt thereof

Another aspect includes the use of a compound of formula I, stereoisomers or a pharmaceutically acceptable salt thereof in therapy.

Another aspect includes the use of a compound of formula I, stereoisomers or a pharmaceutically acceptable salt thereof in the treatment of a disease responsive to the inhibition of ITK kinase.

Another aspect includes the use of a compound of formula I, stereoisomers or a pharmaceutically acceptable salt thereof in the treatment of an immunological or inflammatory disease.

Another aspect includes the use of a compound of formula I, stereoisomers or a pharmaceutically acceptable salt thereof for the preparation of medicament for the treatment treatment of an immunological or inflammatory disease.

Another aspect includes a method of manufacturing a compound of formula I, comprising reacting a comopund of formula 1-3 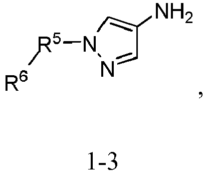 or a salt thereof, with a comopund of formula 1-4 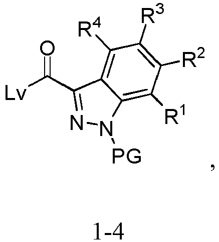 or a salt thereof, wherein PG is an amino protecting group and Lv is a leaving group, to form a compound of formula I.

### DETAILED DESCRIPTION OF THE INVENTION

### DEFINITIONS

"Acyl" means a carbonyl containing substituent represented by the formula -C(O)-R in which R is hydrogen, alkyl, a cycloalkyl, a heterocyclyl, cycloalkyl-substituted alkyl or heterocyclyl-substituted alkyl wherein the alkyl, alkoxy, cycloalkyl and heterocyclyl are as defined herein. Acyl groups include alkanoyl (e.g. acetyl), aroyl (e.g. benzoyl), and heteroaroyl (e.g. pyridinoyl).

The term "alkyl" refers to a saturated linear or branched-chain monovalent hydrocarbon radical, wherein the alkyl radical may be optionally substituted independently with one or more substituents described herein. In one example, the alkyl radical is one to eighteen carbon atoms (C₁-C₁₈). In other examples, the alkyl radical is C₀-C₆, C₀-C₅, C₀-C₃, C₁-C₁₂, C₁-C₁₀, C₁-C₈, C₁-C₆, C₁-C₅, C₁-C₄ or C₁-C₃. C₀ alkyl refers to a bond. Examples of alkyl groups include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl,-C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl and 1-octyl.

The term "alkenyl" refers to linear or branched-chain monovalent hydrocarbon radical with at least one site of unsaturation, i.e., a carbon-carbon double bond, wherein the alkenyl radical may be optionally substituted independently with one or more substituents described herein, and includes radicals having "cis" and "trans" orientations, or alternatively, "E" and "Z" orientations. In one example, the alkenyl radical is two to eighteen carbon atoms (C₂-C₁₈). In other examples, the alkenyl radical is C₂-C₁₂, C₂-C₁₀, C₂-C₈, C₂-C₆ or C₂-C₃. Examples include, but are not limited to, ethenyl or vinyl (-CH=CH₂), prop-1-enyl (-CH=CHCH₃), prop-2-enyl (-CH₂CH=CH₂), 2-methylprop-1-enyl, but-1-enyl, but-2-enyl, but-3-enyl, buta-1,3-dienyl, 2-methylbuta-1,3-diene, hex-1-enyl, hex-2-enyl, hex-3-enyl, hex-4-enyl and hexa-1,3-dienyl.

The term "alkoxy" refers to a linear or branched monovalent radical represented by the formula -OR in which R is alkyl, alkenyl, alkynyl or cycloalkyl, which can be further optionally substituted as defined herein. Alkoxy groups include methoxy, ethoxy, propoxy, isopropoxy, mono-, di- and tri-fluoromethoxy and cyclopropoxy.

The term "alkynyl" refers to a linear or branched monovalent hydrocarbon radical with at least one site of unsaturation, i.e., a carbon-carbon, triple bond, wherein the alkynyl radical may be optionally substituted independently with one or more substituents described herein. In one example, the alkynyl radical is two to eighteen carbon atoms (C₂-C₁₈). In other examples, the alkynyl radical is C₂-C₁₂, C₂-C₁₀, C₂-C₈, C₂-C₆ or C₂-C₃. Examples include, but are not limited to, ethynyl (-C≡CH), prop-1-ynyl (-C≡CCH₃), prop-2-ynyl (propargyl, -CH₂C≡CH), but-1-ynyl, but-2-ynyl and but-3-ynyl.

"Alkylene" refers to a saturated, branched or straight chain hydrocarbon group having two monovalent radical centers derived by the removal of two hydrogen atoms from the same or two different carbon atoms of a parent alkane. In one example, the divalent alkylene group is one to eighteen carbon atoms (C₁-C₁₈). In other examples, the divalent alkylene group is C₀-C₆, C₀-C₅, C₀-C₃, C₁-C₁₂, C₁-C₁₀, C₁-C₈, C₁-C₆, C₁-C₅, C₁-C₄, or C₁-C₃. The group C₀ alkylene refers to a bond. Example alkylene groups include methylene (-CH₂-), 1,1-ethyl (-CH(CH₃)-), (1,2-ethyl (-CH₂CH₂-), 1,1-propyl (-CH(CH₂CH₃)-), 2,2-propyl (-C(CH₃)₂-), 1,2-propyl (-CH(CH₃)CH₂-), 1,3-propyl (-CH₂CH₂CH₂-), 1,1-dimethyleth-1,2-yl (-C(CH₃)₂CH₂-), 1,4-butyl (-CH₂CH₂CH₂CH₂-), and the like.

"Alkenylene" refers to an unsaturated, branched or straight chain hydrocarbon group having two monovalent radical centers derived by the removal of two hydrogen atoms from the same or two different carbon atoms of a parent alkene. In one example, the alkenylene group is two to eighteen carbon atoms (C₂-C₁₈). In other examples, the alkenylene group is C₂-C₁₂, C₂-C₁₀, C₂-C₈, C₂-C₆ or C₂-C₃. Example alkenylene groups include: 1,2-ethylene (-CH=CH-).

"Alkynylene" refers to an unsaturated, branched or straight chain hydrocarbon group having two monovalent radical centers derived by the removal of two hydrogen atoms from the same or two different carbon atoms of a parent alkyne. In one example, the alkynylene radical is two to eighteen carbon atoms (C₂-C₁₈). In other examples, the alkynylene radical is C₂-C₁₂, C₂-C₁₀, C₂-C₈, C₂-C₆ or C₂-C₃. Example alkynylene radicals include: acetylene (-C≡C-), propargyl (-CH₂C≡C-), and 4-pentynyl (-CH₂CH₂CH₂C≡C-).

"Amidine" means the group -C(NH)-NHR in which R is hydrogen, alkyl, a cycloalkyl, a heterocyclyl, cycloalkyl-substituted alkyl or heterocyclyl-substituted alkyl wherein the alkyl, alkoxy, cycloalkyl and heterocyclyl are as defined herein. A particular amidine is the group-NH-C(NH)-NH₂.

"Amino" means primary (i.e., -NH₂), secondary (i.e., -NRH) and tertiary (i.e., -NRR) amines, that are optionally substituted, in which R is alkyl, alkoxy, a cycloalkyl, a heterocyclyl, cycloalkyl-substituted alkyl or heterocyclyl-substituted alkyl wherein the alkyl, alkoxy, cycloalkyl and heterocyclyl are as defined herein Particular secondary and tertiary amines are alkylamine, dialkylamine, arylamine, diarylamine, aralkylamine and diaralkylamine wherein the alkyl is as herein defined and optionally substituted. Particular secondary and tertiary amines are methylamine, ethylamine, propylamine, isopropylamine, phenylamine, benzylamine dimethylamine, diethylamine, dipropylamine and diisopropylamine.

"Amino-protecting group" as used herein refers to a derivative of the groups commonly employed to block or protect an amino group while reactions are carried out on other functional groups on the compound. Examples of such protecting groups include carbamates, amides, alkyl and aryl groups, imines, as well as many N-heteroatom derivatives which can be removed to regenerate the desired amine group. Particular amino protecting groups are Pmb (p-Methoxybenzyl), Boc (tert-Butyloxycarbonyl), Fmoc (9-Fluorenylmethyloxycarbonyl) and Cbz (Carbobenzyloxy). Further examples of these groups are found in T. W. Greene and P. G. M. Wuts, "Protective Groups in Organic Synthesis", 2nd ed., John Wiley & Sons, Inc., New York, NY, 1991, chapter 7; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected amino" refers to an amino group substituted with one of the above amino-protecting groups.

"Aryl" when used alone, or as part of another term, means a carbocyclic aromatic group, whether or not fused to one or more groups, having the number of carbon atoms designated, or if no number is designated, up to 14 carbon atoms. One example includes aryl groups having 6-14 carbon atoms. Another example inlcudes aryl groups having 6-10 carbon atoms. Examples of aryl groups include phenyl, naphthyl, biphenyl, phenanthrenyl, naphthacenyl, 1,2,3,4-tetrahydronaphthalenyl, 1H-indenyl, 2,3-dihydro-1H-indenyl, and the like (see e.g. Lang's Handbook of Chemistry (Dean, J. A., ed) 13th ed. Table 7-2 [1985]). A particular aryl is phenyl. Substituted phenyl or substituted aryl means a phenyl group or aryl group substituted with one, two, three, four or five, for example 1-2, 1-3 or 1-4 substituents chosen from groups specified herein. In one example, optional substituents on aryl are selected from halogen (F, Cl, Br, I), hydroxy, protected hydroxy, cyano, nitro, alkyl (for example C₁-C₆ alkyl), alkoxy (for example C₁-C₆ alkoxy), benzyloxy, carboxy, protected carboxy, carboxymethyl, protected carboxymethyl, hydroxymethyl, protected hydroxymethyl, aminomethyl, protected aminomethyl, trifluoromethyl, alkylsulfonylamino, alkylsulfonylaminoalkyl, arylsulfonylamino, arylsulfonylaminoalkyl, heterocyclylsulfonylamino, heterocyclylsulfonylaminoalkyl, heterocyclyl, aryl, or other groups specified. One or more methyne (CH) and/or methylene (CH₂) groups in these substituents may in turn be substituted with a similar group as those denoted above. Examples of the term "substituted phenyl" include a mono- or di(halo)phenyl group such as 2-chlorophenyl, 2-bromophenyl, 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(isopropyl)phenyl, 4-ethylphenyl, 3-(n-propyl)phenyl and the like; a mono or di(alkoxy)phenyl group, for example, 3,4-dimethoxyphenyl, 3-methoxy-4-benzyloxyphenyl, 3-ethoxyphenyl, 4-(isopropoxy)phenyl, 4-(t-butoxy)phenyl, 3-ethoxy-4-methoxyphenyl and the like; 3- or 4-trifluoromethylphenyl; a mono- or dicarboxyphenyl or (protected carboxy)phenyl group such 4-carboxyphenyl, a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-(N-methylsulfonylamino))phenyl. Also, the term "substituted phenyl" represents disubstituted phenyl groups where the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl, and the like, as well as trisubstituted phenyl groups where the substituents are different, for example 3-methoxy-4-benzyloxy-6-methyl sulfonylamino, 3-methoxy-4-benzyloxy-6-phenyl sulfonylamino, and tetrasubstituted phenyl groups where the substituents are different such as 3-methoxy-4-benzyloxy-5-methyl-6-phenyl sulfonylamino. Particular substituted phenyl groups include the 2-chlorophenyl, 2-aminophenyl, 2-bromophenyl, 3-methoxyphenyl, 3-ethoxy-phenyl, 4-benzyloxyphenyl, 4-methoxyphenyl, 3-ethoxy-4-benzyloxyphenyl, 3,4-diethoxyphenyl, 3-methoxy-4-benzyloxyphenyl, 3-methoxy-4-(1-chloromethyl)benzyloxy-6-methyl sulfonyl aminophenyl groups. Fused aryl rings may also be substituted with any, for example 1, 2 or 3, of the substituents specified herein in the same manner as substituted alkyl groups.

The terms "cancer" and "cancerous", "neoplasm", "tumor" refer to or describe the physiological condition in mammals that is typically characterized by unregulated cell growth. A "tumor" comprises one or more cancerous cells. Examples of cancer include carcinoma, lymphoma, blastoma, sarcoma, and leukemia or lymphoid malignancies. More particular examples of such cancers include squamous cell cancer (*e.g.,* epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, multiple myeloma and B-cell lymphoma, brain, as well as head and neck cancer, and associated metastases.

A "chemotherapeutic agent" is an agent useful in the treatment of a given disorder, for example, cancer or inflammatory disorders. Examples of chemotherapeutic agents include NSAIDs; hormones such as glucocorticoids; corticosteroids such as hydrocortisone, hydrocortisone acetate, cortisone acetate, tixocortol pivalate, prednisolone, methylprednisolone, prednisone, triamcinolone acetonide, triamcinolone alcohol, mometasone, amcinonide, budesonide, desonide, fluocinonide, fluocinolone acetonide, halcinonide, betamethasone, betamethasone sodium phosphate, dexamethasone, dexamethasone sodium phosphate, fluocortolone, hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate and fluprednidene acetate; immune selective anti-inflammatory peptides (ImSAIDs) such as phenylalanine-glutamine-glycine (FEG) and its D-isomeric form (feG) (IMULAN BioTherapeutics, LLC); anti-rheumatic drugs such as azathioprine, ciclosporin (cyclosporine A), D-penicillamine, gold salts, hydroxychloroquine, leflunomide, methotrexate (MTX), minocycline, sulfasalazine, cyclophosphamide, tumor necrosis factor alpha (TNFα) blockers such as etanercept (Enbrel), infliximab (Remicade), adalimumab (Humira), certolizumab pegol (Cimzia), golimumab (Simponi), Interleukin 1 (IL-1) blockers such as anakinra (Kineret), monoclonal antibodies against B cells such as rituximab (RITUXAN®), T cell costimulation blockers such as abatacept (Orencia), Interleukin 6 (IL-6) blockers such as tocilizumab (ACTEMERA®); Interleukin 13 (IL-13) blockers such as lebrikizumab; Interferon alpha (IFN) blockers such as Rontalizumab; Beta 7 integrin blockers such as rhuMAb Beta7; IgE pathway blockers such as Anti-M1 prime; Secreted homotrimeric LTa3 and membrane bound heterotrimer LTa1/β2 blockers such as Anti-lymphotoxin alpha (LTa); hormone antagonists, such as tamoxifen, finasteride or LHRH antagonists; radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); miscellaneous investigational agents such as thioplatin, PS-341, phenylbutyrate, ET-18- OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; autophagy inhibitors such as chloroquine; alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gammall and calicheamicin omegaI1 (see, e.g., Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as fenretinide, retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (*e.g.,* LURTOTECAN®); rmRH (*e.g.,* ABARELIX®); BAY439006 (sorafenib; Bayer); SU-11248 (sunitinib, SUTENT®, Pfizer); perifosine, COX-2 inhibitor (*e.g.* celecoxib or etoricoxib), proteosome inhibitor (*e.g.* PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (see definition below); farnesyltransferase inhibitors such as lonafarnib (SCH 6636, SARASAR^{™}); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN^{™}) combined with 5-FU and leucovorin.

Additional chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide.

Additional chemotherapeutic agents include therapeutic antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG®, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth). Additional humanized monoclonal antibodies with therapeutic potential as agents in combination with the compounds of the invention include: apolizumab, aselizumab, atlizumab, bapineuzumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, tucotuzumab celmoleukin, tucusituzumab, umavizumab, urtoxazumab, ustekinumab, visilizumab, and the anti-interleukin-12 (ABT-874/J695, Wyeth Research and Abbott Laboratories) which is a recombinant exclusively human-sequence, full-length IgG₁ λ antibody genetically modified to recognize interleukin-12 p40 protein.

Chemotherapeutic agents also include "EGFR inhibitors," which refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX^{®}) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (see WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6. 3 and E7.6. 3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (see, e.g., EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA^{®} Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSAJ) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]-6[5[[[2methylsulfonyl)ethyl]amino]methyl]-2-furanyl]-4-quinazolinamine).

Chemotherapeutic agents also include "tyrosine kinase inhibitors" including the EGFR-targeted drugs noted in the preceding paragraph; small molecule HER2 tyrosine kinase inhibitor such as TAK165 available from Takeda; CP-724,714, an oral selective inhibitor of the ErbB2 receptor tyrosine kinase (Pfizer and OSI); dual-HER inhibitors such as EKB-569 (available from Wyeth) which preferentially binds EGFR but inhibits both HER2 and EGFR-overexpressing cells; lapatinib (GSK572016; available from Glaxo-SmithKline), an oral HER2 and EGFR tyrosine kinase inhibitor; PKI-166 (available from Novartis); pan-HER inhibitors such as canertinib (CI-1033; Pharmacia); Rat-1 inhibitors such as antisense agent ISIS-5132 available from ISIS Pharmaceuticals which inhibit Raf-1 signaling; non-HER targeted TK inhibitors such as imatinib mesylate (GLEEVECJ, available from Glaxo SmithKline); multi-targeted tyrosine kinase inhibitors such as sunitinib (SUTENT®, available from Pfizer); VEGF receptor tyrosine kinase inhibitors such as vatalanib (PTK787/ZK222584, available from Novartis/Schering AG); MAPK extracellular regulated kinase I inhibitor CI-1040 (available from Pharmacia); quinazolines, such as PD 153035,4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo[2,3-d] pyrimidines; curcumin (diferuloyl methane, 4,5-bis (4-fluoroanilino)phthalimide); tyrphostines containing nitrothiophene moieties; PD-0183805 (Warner-Lamber); antisense molecules (*e*.*g*. those that bind to HER-encoding nucleic acid); quinoxalines (US Patent No. 5,804,396); tryphostins (US Patent No. 5,804,396); ZD6474 (Astra Zeneca); PTK-787 (Novartis/Schering AG); pan-HER inhibitors such as CI-1033 (Pfizer); Affinitac (ISIS 3521; Isis/Lilly); imatinib mesylate (GLEEVECJ); PKI 166 (Novartis); GW2016 (Glaxo SmithKline); CI-1033 (Pfizer); EKB-569 (Wyeth); Semaxinib (Pfizer); ZD6474 (AstraZeneca); PTK-787 (Novartis/Schering AG); INC-1C11 (Imclone), rapamycin (sirolimus, RAPAMUNE®); or as described in any of the following patent publications: US Patent No. 5,804,396; WO 1999/09016 (American Cyanamid); WO 1998/43960 (American Cyanamid); WO 1997/38983 (Warner Lambert); WO 1999/06378 (Warner Lambert); WO 1999/06396 (Warner Lambert); WO 1996/30347 (Pfizer, Inc); WO 1996/33978 (Zeneca); WO 1996/3397 (Zeneca) and WO 1996/33980 (Zeneca).

Chemotherapeutic agents also include asthma treatment agents, including inhaled corticosteroids such as fluticasone, budesonide, mometasone, flunisolide and beclomethasone; leukotriene modifiers, such as montelukast, zafirlukast and zileuton; long-acting beta agonists, such as salmeterol and formoterol; combinations of the above such as combinations of fluticasone and salmeterol, and combinations of budesonide and formoterol; theophylline; short-acting beta agonists, such as albuterol, levalbuterol and pirbuterol; ipratropium; oral and intravenous corticosteroids, such as prednisone and methylprednisolone; omalizumab; lebrikizumab; antihistamines; and decongestants; cromolyn; and ipratropium.

The term "NSAID" and the terms "non-steroidal anti-inflammatory drug" refer to therapeutic agents with analgesic, antipyretic and anti-inflammatory effects. NSAIDs include non-selective inhibitors of the enzyme cyclooxygenase. Specific examples of NSAIDs include aspirin, propionic acid derivatives such as ibuprofen, fenoprofen, ketoprofen, flurbiprofen, oxaprozin and naproxen, acetic acid derivatives such as indomethacin, sulindac, etodolac, diclofenac, enolic acid derivatives such as piroxicam, meloxicam, tenoxicam, droxicam, lornoxicam and isoxicam, fenamic acid derivatives such as mefenamic acid, meclofenamic acid, flufenamic acid, tolfenamic acid, and COX-2 inhibitors such as celecoxib, etoricoxib, lumiracoxib, parecoxib, rofecoxib, rofecoxib, and valdecoxib. NSAIDs can be indicated for the symptomatic relief of conditions such as rheumatoid arthritis, osteoarthritis, inflammatory arthropathies, ankylosing spondylitis, psoriatic arthritis, Reiter's syndrome, acute gout, dysmenorrhoea, metastatic bone pain, headache and migraine, postoperative pain, mild-to-moderate pain due to inflammation and tissue injury, pyrexia, ileus, and renal colic.

Additionally, chemotherapeutic agents include pharmaceutically acceptable salts, acids or derivatives of any of chemotherapeutic agents, described herein, as well as combinations of two or more of them.

"Cycloalkyl" refers to a non-aromatic, saturated or partially unsaturated hydrocarbon ring group wherein the cycloalkyl group may be optionally substituted independently with one or more substituents described herein. In one example, the cycloalkyl group is 3 to 12 carbon atoms (C₃-C₁₂). In other examples, cycloalkyl is C₃-C₈, C₃-C₁₀ or C₅-C₁₀. In other examples, the cycloalkyl group, as a monocycle, is C₃-C₈, C₃-C₆ or C₅-C₆. In another example, the cycloalkyl group, as a bicycle, is C₇-C₁₂. In another example, the cycloalkyl group, as a spiro system, is C₅-C₁₂. Examples of monocyclic cycloalkyl include cyclopropyl, cyclobutyl, cyclopentyl, 1-cyclopent-1-enyl, 1-cyclopent-2-enyl, 1-cyclopent-3-enyl, cyclohexyl, perdeuteriocyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl and cyclododecyl. Exemplary arrangements of bicyclic cycloalkyls having 7 to 12 ring atoms include, but are not limited to, [4,4], [4,5], [5,5], [5,6] or [6,6] ring systems. Exemplary bridged bicyclic cycloalkyls include, but are not limited to, bicyclo[2.2.1]heptane, bicyclo[2.2.2]octane and bicyclo[3.2.2]nonane. Examples of spiro cycloalkyl include, spiro[2.2]pentane, spiro[2.3]hexane, spiro[2.4]heptane, spiro[2.5]octane and spiro[4.5]decane.

"Carboxy-protecting group" as used herein refers to those groups that are stable to the conditions of subsequent reaction(s) at other positions of the molecule, which may be removed at the appropriate point without disrupting the remainder of the molecule, to give the unprotected carboxy-group. Examples of carboxy protecting groups include, ester groups and heterocyclyl groups. Ester derivatives of the carboxylic acid group may be employed to block or protect the carboxylic acid group while reactions are carried out on other functional groups on the compound. Examples of such ester groups include substituted arylalkyl, including substituted benzyls, such as 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, alkyl or substituted alkyl esters such as methyl, ethyl, t-butyl allyl or t-amyl, triphenylmethyl (trityl), 4-methoxytrityl, 4,4'-dimethoxytrityl, 4,4',4"-trimethoxytrityl, 2-phenylprop-2-yl, thioesters such as t-butyl thioester, silyl esters such as trimethylsilyl, t-butyldimethylsilyl esters, phenacyl, 2,2,2-trichloroethyl, beta-(trimethylsilyl)ethyl, beta-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. Another example of carboxy-protecting groups are heterocyclyl groups such as 1,3-oxazolinyl. Further examples of these groups are found in T. W. Greene and P. G. M. Wuts, "Protective Groups in Organic Synthesis", 2nd ed., John Wiley & Sons, Inc., New York, N.Y., 1991, chapter 5; E. Haslam, "Protective Groups in Organic Chemistry", J. G. W. McOmie, Ed., Plenum Press, New York, N.Y., 1973, Chapter 5, and T.W. Greene, "Protective Groups in Organic Synthesis", John Wiley and Sons, New York, NY, 1981, Chapter 5. The term "protected carboxy" refers to a carboxy group substituted with one of the above carboxy-protecting groups.

"Guanidine" means the group -NH-C(NH)-NHR in which R is hydrogen, alkyl, alkoxy, a cycloalkyl, a heterocyclyl, cycloalkyl -substituted alkyl or heterocyclyl-substituted alkyl wherein the alkyl, alkoxy, cycloalkyl and heterocyclyl are as defined herein. A particular guanidine is the group -NH-C(NH)-NH₂.

"Hydroxy-protecting group" as used herein refers to a derivative of the hydroxy group commonly employed to block or protect the hydroxy group while reactions are carried out on other functional groups on the compound. Examples of such protecting groups include tetrahydropyranyloxy, benzoyl, acetoxy, carbamoyloxy, benzyl, and silylethers (e.g. TBS, TBDPS) groups. Further examples of these groups are found in T. W. Greene and P. G. M. Wuts, "Protective Groups in Organic Synthesis", 2nd ed., John Wiley & Sons, Inc., New York, NY, 1991, chapters 2-3; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected hydroxy" refers to a hydroxy group substituted with one of the above hydroxy-protecting groups.

"Heterocyclic group", "heterocyclic", "heterocycle", "heterocyclyl", or "heterocyclo" alone, and when used as a moiety in a complex group such as a heterocycloalkyl group, are used interchangeably and refer to any mono-, bi-, tricyclic or spiro, saturated or unsaturated, aromatic (heteroaryl) or non-aromatic, ring system, having 3 to 20 ring atoms, where the ring atoms are carbon, and at least one atom in the ring or ring system is a heteroatom selected from nitrogen, sulfur or oxygen. In one example, heterocyclyl includes 3-12 ring atoms and includes monocycles, bicycles, tricycles and spiro ring systems, wherein the ring atoms are carbon, and at least one atom in the ring or ring system is a heteroatom selected from nitrogen, sulfur or oxygen. In one example, heterocyclyl includes 1 to 4 heteroatoms. In another example, heterocyclyl includes 3- to 7-membered monocycles having one or more heteroatoms selected from nitrogen, sulfur or oxygen. In another example, heterocyclyl includes 4- to 6-membered monocycles having one or more heteroatoms selected from nitrogen, sulfur or oxygen. In another example, heterocyclyl includes 3-membered monocycles. In another example, heterocyclyl includes 4-membered monocycles. In another example, heterocyclyl includes 5-6-membered monocycles. In one example, the heterocyclyl group includes 0 to 3 double bonds. Any nitrogen or sulfur heteroatom may optionally be oxidized (e.g. NO, SO, SO₂), and any nitrogen heteroatom may optionally be quaternized (e.g. [NR₄]⁺Cl⁻, [NR₄]⁺OH⁻). Example heterocycles are oxiranyl, aziridinyl, thiiranyl, azetidinyl, oxetanyl, thietanyl, 1,2-dithietanyl, 1,3-dithietanyl, pyrrolidinyl, dihydro-1H-pyrrolyl, dihydrofuranyl, tetrahydrofuranyl, dihydrothienyl, tetrahydrothienyl, imidazolidinyl, piperidinyl, piperazinyl, morpholinyl, thiomorpholinyl, 1,1-dioxo-thiomorpholinyl, dihydropyranyl, tetrahydropyranyl, hexahydrothiopyranyl, hexahydropyrimidinyl, oxazinanyl, thiazinanyl, thioxanyl, homopiperazinyl, homopiperidinyl, azepanyl, oxepanyl, thiepanyl, oxazepinyl, oxazepanyl, diazepanyl, 1,4-diazepanyl, diazepinyl, thiazepinyl, thiazepanyl, tetrahydrothiopyranyl, oxazolidinyl, thiazolidinyl, isothiazolidinyl, 1,1-dioxoisothiazolidinonyl, oxazolidinonyl, imidazolidinonyl, 4,5,6,7-tetrahydro[2H]indazolyl, tetrahydrobenzoimidazolyl, 4,5,6,7-tetrahydrobenzo[d]imidazolyl, 1,6-dihydroimidazol[4,5-d]pyrrolo[2,3-b]pyridinyl, thiazinyl, oxazinyl, thiadiazinyl, oxadiazinyl, dithiazinyl, dioxazinyl, oxathiazinyl, thiatriazinyl, oxatriazinyl, dithiadiazinyl, imidazolinyl, dihydropyrimidyl, tetrahydropyrimidyl, 1-pyrrolinyl, 2-pyrrolinyl, 3-pyrrolinyl, indolinyl, thiapyranyl, 2H-pyranyl, 4H-pyranyl, dioxanyl, 1,3-dioxolanyl, pyrazolinyl, pyrazolidinyl, dithianyl, dithiolanyl, pyrimidinonyl, pyrimidindionyl, pyrimidin-2,4-dionyl, piperazinonyl, piperazindionyl, pyrazolidinylimidazolinyl, 3-azabicyclo[3.1.0]hexanyl, 3,6-diazabicyclo[3.1.1]heptanyl, 6-azabicyclo[3.1.1]heptanyl, 3-azabicyclo[3.1.1]heptanyl, 3-azabicyclo[4.1.0]heptanyl, azabicyclo[2.2.2]hexanyl, 2-azabicyclo[3.2.1]octanyl, 8-azabicyclo[3.2.1]octanyl, 2-azabicyclo[2.2.2]octanyl, 8-azabicyclo[2.2.2]octanyl, 7-oxabicyclo[2.2.1]heptane, azaspiro[3.5]nonanyl, azaspiro[2.5]octanyl, azaspiro[4.5]decanyl, 1-azaspiro[4.5]decan-2-only, azaspiro[5.5]undecanyl, tetrahydroindolyl, octahydroindolyl, tetrahydroisoindolyl, tetrahydroindazolyl, 1,1-dioxohexahydrothiopyranyl. Examples of 5-membered heterocycles containing a sulfur or oxygen atom and one to three nitrogen atoms are thiazolyl, including thiazol-2-yl and thiazol-2-yl N-oxide, thiadiazolyl, including 1,3,4-thiadiazol-5-yl and 1,2,4-thiadiazol-5-yl, oxazolyl, for example oxazol-2-yl, and oxadiazolyl, such as 1,3,4-oxadiazol-5-yl, and 1,2,4-oxadiazol-5-yl. Example 5-membered ring heterocycles containing 2 to 4 nitrogen atoms include imidazolyl, such as imidazol-2-yl; triazolyl, such as 1,3,4-triazol-5-yl; 1,2,3-triazol-5-yl, 1,2,4-triazol-5-yl, and tetrazolyl, such as 1H-tetrazol-5-yl. Example benzo-fused 5-membered heterocycles are benzoxazol-2-yl, benzthiazol-2-yl and benzimidazol-2-yl. Example 6-membered heterocycles contain one to three nitrogen atoms and optionally a sulfur or oxygen atom, for example pyridyl, such as pyrid-2-yl, pyrid-3-yl, and pyrid-4-yl; pyrimidyl, such as pyrimid-2-yl and pyrimid-4-yl; triazinyl, such as 1,3,4-triazin-2-yl and 1,3,5-triazin-4-yl; pyridazinyl, in particular pyridazin-3-yl, and pyrazinyl. The pyridine N-oxides and pyridazine N-oxides and the pyridyl, pyrimid-2-yl, pyrimid-4-yl, pyridazinyl and the 1,3,4-triazin-2-yl groups, are other example heterocycle groups. Substituents for "optionally substituted heterocycles" include hydroxyl, alkyl, alkoxy, acyl, halogen, mercapto, oxo, carboxyl, halo-substituted alkyl, amino, cyano, nitro, amidino, guanidino.

"Heteroaryl" alone and when used as a moiety in a complex group such as a heteroaralkyl group, refers to any mono-, bi-, or tricyclic ring system where at least one ring is a 5- or, 6-membered aromatic ring containing from 1 to 4 heteroatoms selected from nitrogen, oxygen, and sulfur, and in an example embodiment, at least one heteroatom is nitrogen. See, for example, *Lang's Handbook of Chemistry, supra.* Included in the definition are any bicyclic groups where any of the above heteroaryl rings are fused to an aryl ring. In one embodiment, heteroaryl includes 4-6 membered monocyclic aromatic groups where one or more ring atoms is nitrogen, sulfur or oxygen. In another embodiment, heteroaryl includes 5-6 membered monocyclic aromatic groups where one or more ring atoms is nitrogen, sulfur or oxygen. Example heteroaryl groups (whether substituted or unsubstituted) include thienyl, furyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, triazinyl, tetrazinyl, tetrazolo[1,5-b]pyridazinyl, imidazol[1,2-a]pyrimidinyl and purinyl, as well as benzo-fused derivatives, for example benzoxazolyl, benzofuryl, benzothiazolyl, benzothiadiazolyl, benzotriazolyl, benzoimidazolyl and indolyl. Additional examples of "heteroaryl" groups are: 1,3-thiazol-2-yl, 4-(carboxymethyl)-5-methyl-1,3-thiazol-2-yl, 4-(carboxymethyl)-5-methyl-1,3-thiazol-2-yl sodium salt, 1,2,4-thiadiazol-5-yl, 3-methyl-1,2,4-thiadiazol-5-yl, 1,3,4-triazol-5-yl, 2-methyl-1,3,4-triazol-5-yl, 2-hydroxy-1,3,4-triazol-5-yl, 2-carboxy-4-methyl-1,3,4-triazol-5-yl sodium salt, 2-carboxy-4-methyl-1,3,4-triazol-5-yl, 1,3-oxazol-2-yl, 1,3,4-oxadiazol-5-yl, 2-methyl-1,3,4-oxadiazol-5-yl, 2-(hydroxymethyl)-1,3,4-oxadiazol-5-yl, 1,2,4-oxadiazol-5-yl, 1,3,4-thiadiazol-5-yl, 2-thiol-1,3,4-thiadiazol-5-yl, 2-(methylthio)-1,3,4-thiadiazol-5-yl, 2-amino-1,3,4-thiadiazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 2-methyl-1H-tetrazol-5-yl, 1,2,3-triazol-5-yl, 1-methyl-1,2,3-triazol-5-yl, 2-methyl-1,2,3-triazol-5-yl, 4-methyl-1,2,3-triazol-5-yl, pyrid-2-yl N-oxide, 6-methoxy-2-(n-oxide)-pyridaz-3-yl, 6-hydroxypyridaz-3-yl, 1-methylpyrid-2-yl, 1-methylpyrid-4-yl, 2-hydroxypyrimid-4-yl, 1,4,5,6-tetrahydro-5,6-dioxo-4-methyl-as-triazin-3-yl, 1,4,5,6-tetrahydro-4-(formylmethyl)-5,6-dioxo-as-triazin-3-yl, 2,5-dihydro-5-oxo-6-hydroxy-astriazin-3-yl, 2,5-dihydro-5-oxo-6-hydroxy-as-triazin-3-yl sodium salt, 2,5-dihydro-5-oxo-6-hydroxy-2-methyl-astriazin-3-yl sodium salt, 2,5-dihydro-5-oxo-6-hydroxy-2-methyl-as-triazin-3-yl, 2,5-dihydro-5-oxo-6-methoxy-2-methyl-as-triazin-3-yl, 2,5-dihydro-5-oxo-as-triazin-3-yl, 2,5-dihydro-5-oxo-2-methyl-as-triazin-3-yl, 2,5-dihydro-5-oxo-2,6-dimethyl-as-triazin-3-yl, tetrazolo[1,5-b]pyridazin-6-yl and 8-aminotetrazolo[1,5-b]-pyridazin-6-yl. Heteroaryl groups are optionally substituted as described for heterocycles.

In particular embodiments, a heterocyclyl group is attached at a carbon atom of the heterocyclyl group. By way of example, carbon bonded heterocyclyl groups include bonding arrangements at position 2, 3, 4, 5, or 6 of a pyridine ring, position 3, 4, 5, or 6 of a pyridazine, position 2, 4, 5, or 6 of a pyrimidine ring, position 2, 3, 5, or 6 of a pyrazine ring, position 2, 3, 4, or 5 of a furan, tetrahydrofuran, thiofuran, thiophene, pyrrole or tetrahydropyrrole ring, position 2, 4, or 5 of an oxazole, imidazole or thiazole ring, position 3, 4, or 5 of an isoxazole, pyrazole, or isothiazole ring, position 2 or 3 of an aziridine ring, position 2, 3, or 4 of an azetidine ring, position 2, 3, 4, 5, 6, 7, or 8 of a quinoline ring or position 1, 3, 4, 5, 6, 7, or 8 of an isoquinoline ring.

In certain embodiments, the heterocyclyl group is N-attached. By way of example, the nitrogen bonded heterocyclyl or heteroaryl group include bonding arrangements at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline.

"Leaving group" refers to a portion of a first reactant in a chemical reaction that is displaced from the first reactant in the chemical reaction. Examples of leaving groups include, but are not limited to, halogen atoms, alkoxy and sulfonyloxy groups. Example sulfonyloxy groups include, but are not limited to, alkylsulfonyloxy groups (for example methyl sulfonyloxy (mesylate group) and trifluoromethylsulfonyloxy (triflate group)) and arylsulfonyloxy groups (for example *p*-toluenesulfonyloxy (tosylate group) and *p*-nitrosulfonyloxy (nosylate group)).

"Optionally substituted" unless otherwise specified means that a group may be unsubstituted or substituted by one or more (e.g. 0, 1, 2, 3 or 4) of the substituents listed for that group in which said substituents may be the same or different. In an embodiment an optionally substituted group has 1 substituent. In another embodiment an optionally substituted group has 2 substituents. In another embodiment an optionally substituted group has 3 substituents.

In certain embodiments, divalent groups are described generically without specific bonding configurations, for example in the group -CH₂C(O)-. It is understood that the generic description is meant to include both bonding configurations, unless specified otherwise. For example, in the group R¹-R²-R³, if the group R² is described as -CH₂C(O)-, then it is understood that this group can be bonded both as R¹-CH₂C(O)-R³, and as R¹-C(O)CH₂-R³, unless specified otherwise.

"Package insert" is used to refer to instructions customarily included in commercial packages of therapeutic products that contain information about the indications, usage, dosage, administration, contraindications and/or warnings concerning the use of such therapeutic products.

"Pharmaceutically acceptable salts" include both acid and base addition salts. "Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, benzenesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particularly base addition salts are the ammonium, potassium, sodium, calcium and magnesium salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, tromethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly organic non-toxic bases are isopropylamine, diethylamine, ethanolamine, tromethamine, dicyclohexylamine, choline, and caffeine.

A "sterile" formulation is aseptic or free from all living microorganisms and their spores.

"Stereoisomers" refers to compounds which have identical chemical constitution, but differ with regard to the arrangement of the atoms or groups in space. Stereoisomers include diastereomers, enantiomers, conformers and the like.

"Chiral" refers to molecules which have the property of non-superimposability of the mirror image partner, while the term "achiral" refers to molecules which are superimposable on their mirror image partner.

"Diastereomer" refers to a stereoisomer with two or more centers of chirality and whose molecules are not mirror images of one another. Diastereomers have different physical properties, e.g. melting points, boiling points, spectral properties or biological activities. Mixtures of diastereomers may separate under high resolution analytical procedures such as electrophoresis and chromatography such as HPLC.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds exist in optically active forms, i.e., they have the ability to rotate the plane of plane-polarized light. In describing an optically active compound, the prefixes D and L, or *R* and *S,* are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

A "solvate" refers to an association or complex of one or more solvent molecules and a compound of the present invention. Examples of solvents that form solvates include water, isopropanol, ethanol, methanol, DMSO, ethyl acetate, acetic acid, and ethanolamine. The term "hydrate" refers to the complex where the solvent molecule is water.

A "subject," "individual," or "patient" is a vertebrate. In certain embodiments, the vertebrate is a mammal. Mammals include, but are not limited to, farm animals (such as cows), sport animals, pets (such as cats, dogs, and horses), primates, mice and rats. In certain embodiments, a mammal is a human.

"Therapeutically effective amount" means an amount of a compound of the present invention that (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR). In the case of inflammatory or immunological disorders, the therapeutic effective amount is an amount sufficient to decrease or alleviate an allergic disorder, the symptoms of an autoimmune and/or inflammatory disease, or the symptoms of an acute inflammatory reaction (e.g. asthma). In some embodiments, a therapeutically effective amount is an amount of a chemical entity described herein sufficient to significantly decrease the activity, expression or number of Th2 cytokines or B-cells.

"Treatment" (and variations such as "treat" or "treating") refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, stabilized (*i.e*., not worsening) state of disease, preventing metastasis, decreasing the rate of disease progression, amelioration or palliation of the disease state, prolonging survival as compared to expected survival if not receiving treatment and remission or improved prognosis. In some embodiments, compounds of the invention are used to delay development of a disease or disorder or to slow the progression of a disease or disorder. Those in need of treatment include those already with the condition or disorder as well as those prone to have the condition or disorder, (for example, through a genetic mutation) or those in which the condition or disorder is to be prevented.

The terms "compound(s) of this invention," and "compound(s) of the present invention", unless otherwise indicated, include compounds of formula I and stereoisomers, tautomers, solvates, metabolites, isotopes, salts (e.g., pharmaceutically acceptable salts), and prodrugs thereof.

### INHIBITORS OF ITK

One aspect includes a compound of formula I: 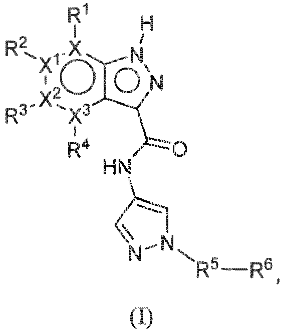 stereoisomers or a pharmaceutically acceptable salt thereof, wherein:
X, X¹, X² are C and X³ is C or N;
R¹, R², R³ and R⁴ are independently do not exist, hydrogen, C₁-C₁₂ alkyl, C₂-C₁₂ alkenyl, C₂-C₁₂ alkynyl, halogen, -CN, -OR⁷, -SR⁷, -NR⁷R⁸, -CF₃, -OCF₃, -NO₂, -C(O)R⁷, -C(O)OR, -C(O)NR⁷R⁸, -NR⁷C(O)R⁸, -S(O)₁₋₂R⁷, -NR⁷S(O)₁₋₂R⁸, -S(O)₁₋₂NR⁷R⁸, C₃-C₆ cycloalkyl, 3-10-membered heterocyclyl or 6-10 membered aryl, wherein R¹, R², R³ and R⁴ are independently optionally substituted by R⁹;
R⁵ is does not exist, C₁-C₆ alkylene, C₂-C₆ alkenylene, C₂-C₆ alkynylene, wherein said alkylene, alkenylene and alkynylene are independently optionally substituted by halogen, oxo, C₁-C₁₂ alkyl, C₂-C₁₂ alkenyl, C₂-C₁₂ alkynyl, -OR¹⁶, -SR¹⁶, -NR¹⁶R¹⁷, -CN, -C(O)R¹⁶,-C(O)OR¹⁶, -NR¹⁶C(O)R¹⁷, -NR¹⁶S(O)₁₋₂R¹⁷, -CF₃, -OCF₃, 3-10-membered heterocyclyl or 6-10 membered aryl, and wherein said alkyl, alkenyl, alkynyl, heterocyclyl and phenyl are independently optionally substituted with R⁹;
R⁶ is hydrogen, C₃-C₁₀ cycloalkyl, 3-10-membered heterocyclyl or 6-10-membered aryl, wherein R⁶ is independently optionally substituted by R⁹;
each R⁷ and R⁸ are independently hydrogen, C₁-C₆ alkyl, C₃-C₆ cycloalkyl, 3-6-membered heterocyclyl or phenyl, wherein said alkyl, cycloalkyl, heterocyclyl and phenyl are independently optionally substituted by halogen, -CN, -CF₃, -OCF₃, oxo or C₁-C₆ alkyl optionally substituted by halogen or oxo; or
R⁷ and R⁸ are independently taken together with the atom to which they are attached to form a 3-6 membered heterocyclyl optionally substituted by halogen, oxo or C₁-C₆ alkyl optionally substituted by halogen or oxo.
each R⁹ is independently hydrogen, oxo, C₁-C₁₂ alkyl, C₂-C₁₂ alkenyl, C₂-C₁₂ alkynyl, halogen, -(C₀-C₆ alkylene)CN, -(C₀-C₆ alkylene)OR¹⁰, -(C₀-C₆ alkylene)SR¹⁰, -(C₀-C₆ alkylene)NR¹⁰R¹¹, -(C₀-C₆ alkylene)CF₃, -(C₀-C₆ alkylene)NO₂, -(C₀-C₆ alkylene)C(O)R¹⁰,-(C₀-C₆ alkylene)C(O)OR¹⁰, -(C₀-C₆ alkylene)C(O)NR¹⁰R¹¹, -(C₀-C₆ alkylene)NR¹⁰C(O)R¹¹,-(C₀-C₆ alkylene)S(O)₁₋₂R¹⁰, -(C₀-C₆ alkylene)NR¹⁰S(O)₁₋₂R¹¹, -(C₀-C₆ alkylene)S(O)₁₋₂NR¹⁰R¹¹, -(C₀-C₆ alkylene)(C₃-C₆ cycloalkyl), -(C₀-C₆ alkylene)(3-10-membered heterocyclyl), -(C₀-C₆ alkylene)C(O)(3-10-membered heterocyclyl), or -(C₀-C₆ alkylene)(6-10 membered aryl), wherein each R⁹ is independently optionally substituted by halogen, oxo, -CF₃, -CN, -OR¹²,-SR¹², -NR¹²R¹³, -C(O)R¹², -S(O)₁₋₂R¹², C₁-C₆ alkyl optionally substituted by oxo or halogen, C₂-C₆ alkenyl optionally substituted by oxo or halogen, or C₂-C₆ alkynyl optionally substituted by oxo or halogen;
each R¹⁰ and R¹¹ are independently hydrogen, C₁-C₆ alkyl, C₂-C₆ alkenyl, C₂-C₆ alkynyl, 3-6-membered heterocyclyl, phenyl or C₃-C₆ cycloalkyl, wherein said alkyl, alkenyl, alkynyl, heterocyclyl, phenyl and cycloalkyl are independently optionally substituted by halogen, oxo,-CF₃, -OCF₃, -OR¹⁴, -SR¹⁴, -NR¹⁴R¹⁵, -CN, 3-6-membered heterocyclyl, phenyl, C₃-C₆ cycloalkyl or C₁-C₆ alkyl optionally substituted by halogen or oxo; or
R¹⁰ and R¹¹ are independently taken together with the atom to which they are attached to form a 3-6 membered heterocyclyl optionally substituted by halogen, oxo or C₁-C₆ alkyl optionally substituted by halogen or oxo;
each R¹² and R¹³ are independently hydrogen or C₁-C₆ alkyl optionally substituted by halogen or oxo; or
R¹² and R¹³ are independently taken together with the atom to which they are attached to form a 3-6 membered heterocyclyl optionally substituted by halogen, oxo or C₁-C₆ alkyl optionally substituted by halogen;
each R¹⁴ and R¹⁵ are independently hydrogen or C₁-C₆ alkyl optionally substituted by halogen or oxo; or
R¹⁴ and R¹⁵ are independently taken together with the atom to which they are attached to form a 3-6 membered heterocyclyl optionally substituted by halogen, oxo or C₁-C₆ alkyl optionally substituted by halogen;
each R¹⁶ and R¹⁷ are independently hydrogen, C₁-C₆ alkyl, C₂-C₆ alkenyl, C₂-C₆ alkynyl, 3-6-membered heterocyclyl, phenyl or C₃-C₆ cycloalkyl, wherein said alkyl, alkenyl, alkynyl, heterocyclyl, phenyl and cycloalkyl are independently optionally substituted by halogen, oxo,-CF₃, -OCF₃, -OR¹⁸, -SR¹⁸, -NR¹⁸R¹⁹, -CN, 3-6-membered heterocyclyl, phenyl, C₃-C₆ cycloalkyl or C₁-C₆ alkyl optionally substituted by halogen, -OR²⁰, -NR²⁰R²¹, or oxo; or
R¹⁶ and R¹⁷ are independently taken together with the atom to which they are attached to form a 3-6 membered heterocyclyl optionally substituted by halogen, oxo or C₁-C₆ alkyl optionally substituted by halogen or oxo;
each R¹⁸ and R¹⁹ are independently hydrogen or C₁-C₆ alkyl optionally substituted by halogen or oxo; or
R¹⁸ and R¹⁹ are independently taken together with the atom to which they are attached to form a 3-6 membered heterocyclyl optionally substituted by halogen, oxo or C₁-C₆ alkyl optionally substituted by halogen; and
each R²⁰ and R²¹ are independently hydrogen or C₁-C₆ alkyl optionally substituted by halogen or oxo.

Certain embodiments include a compound of formula I, stereoisomers or a pharmaceutically acceptable salt thereof, wherein:
X, X¹, X² are C and X³ is C or N;
R¹, R², R³ and R⁴ are independently do not exist, hydrogen, C₁-C₁₂ alkyl, C₂-C₁₂ alkenyl, C₂-C₁₂ alkynyl, halogen, -CN, -OR⁷, -SR⁷, -NR⁷R⁸, -CF₃, -OCF₃, -NO₂, -C(O)R⁷, -C(O)OR⁷, -C(O)NR⁷R⁸, -NR⁷C(O)R⁸, -S(O)₁₋₂R⁷, -NR⁷S(O)₁₋₂R⁸, -S(O)₁₋₂NR⁷R⁸, C₃-C₆ cycloalkyl, 3-10-membered heterocyclyl or 6-10 membered aryl, wherein R¹, R², R³ and R⁴ are independently optionally substituted by R⁹;
R⁵ is C₁-C₆ alkylene, C₂-C₆ alkenylene, C₂-C₆ alkynylene, wherein said alkylene, alkenylene and alkynylene are independently optionally substituted by halogen, oxo, C₁-C₁₂ alkyl, C₂-C₁₂ alkenyl, C₂-C₁₂ alkynyl, -OR¹⁶, -SR¹⁶, -NR¹⁶R¹⁷, -CN, -CF₃, -OCF₃, and wherein said alkyl, alkenyl and alkynyl are independently optionally substituted with oxo or halogen;
R⁶ is hydrogen, C₃-C₁₀ cycloalkyl, 3-10-membered heterocyclyl or 6-10-membered aryl, wherein R⁶ is independently optionally substituted by R⁹;
each R⁷ and R⁸ are independently hydrogen, C₁-C₆ alkyl, C₃-C₆ cycloalkyl, 3-6-membered heterocyclyl or phenyl, wherein said alkyl, cycloalkyl, heterocyclyl and phenyl are independently optionally substituted by halogen, -CN, -CF₃, -OCF₃ or oxo; or
R⁷ and R⁸ are independently taken together with the atom to which they are attached to form a 3-6 membered heterocyclyl optionally substituted by halogen, oxo or C₁-C₆ alkyl optionally substituted by halogen or oxo;
each R⁹ is independently hydrogen, oxo, C₁-C₁₂ alkyl, C₂-C₁₂ alkenyl, C₂-C₁₂ alkynyl, halogen, -(C₀-C₆ alkylene)CN, -(C₀-C₆ alkylene)OR¹⁰, -(C₀-C₆ alkylene)SR¹⁰, -(C₀-C₆ alkylene)NR¹⁰R¹¹, -(C₀-C₆ alkylene)CF₃, -(C₀-C₆ alkylene)NO₂, -(C₀-C₆ alkylene)C(O)R¹⁰,-(C₀-C₆ alkylene)C(O)OR¹⁰, -(C₀-C₆ alkylene)C(O)NR¹⁰R¹¹, -(C₀-C₆ alkylene)NR¹⁰C(O)R¹¹,-(C₀-C₆ alkylene)S(O)₁₋₂R¹⁰, -(C₀-C₆ alkylene)NR IOS(O)₁₋₂R II, -(C₀-C₆ alkylene)S(O)₁₋₂NR¹⁰R¹¹, -(C₀-C₆ alkylene)(C₃-C₆ cycloalkyl), -(C₀-C₆ alkylene)(3-10-membered heterocyclyl), -(C₀-C₆ alkylene)C(O)(3-10-membered heterocyclyl), or -(C₀-C₆ alkylene)(6-10 membered aryl), wherein each R⁹ is independently optionally substituted by halogen, oxo, -CF₃, -CN, -OR¹²,-SR¹², -NR¹²R¹³, -C(O)R¹², -S(O)₁₋₂R¹², C₁-C₆ alkyl optionally substituted by oxo or halogen, C₂-C₆ alkenyl optionally substituted by oxo or halogen, or C₂-C₆ alkynyl optionally substituted by oxo or halogen;
each R¹⁰ and R¹¹ are independently hydrogen, C₁-C₆ alkyl, C₂-C₆ alkenyl, C₂-C₆ alkynyl, 3-6-membered heterocyclyl, phenyl or C₃-C₆ cycloalkyl, wherein said alkyl, alkenyl, alkynyl, heterocyclyl, phenyl and cycloalkyl are independently optionally substituted by halogen, oxo,-CF₃, -OCF₃, -OR¹⁴, -SR¹⁴, -NR¹⁴R¹⁵, -CN, 3-6-membered heterocyclyl, phenyl, C₂-C₆ cycloalkyl or C₁-C₆ alkyl optionally substituted by halogen or oxo; or
R¹⁰ and R¹¹ are independently taken together with the atom to which they are attached to form a 3-6 membered heterocyclyl optionally substituted by halogen, oxo or C₁-C₆ alkyl optionally substituted by halogen or oxo;
each R¹² and R¹³ are independently hydrogen or C₁-C₆ alkyl optionally substituted by halogen or oxo; or
R¹² and R¹³ are independently taken together with the atom to which they are attached to form a 3-6 membered heterocyclyl optionally substituted by halogen, oxo or C₁-C₆ alkyl optionally substituted by halogen;
each R¹⁴ and R¹⁵ are independently hydrogen or C₁-C₆ alkyl optionally substituted by halogen or oxo; or
R¹⁴ and R¹⁵ are independently taken together with the atom to which they are attached to form a 3-6 membered heterocyclyl optionally substituted by halogen, oxo or C₁-C₆ alkyl optionally substituted by halogen;
each R¹⁶ and R¹⁷ are independently hydrogen, C₁-C₆ alkyl, C₂-C₆ alkenyl, C₂-C₆ alkynyl, 3-6-membered heterocyclyl, phenyl or C₃-C₆ cycloalkyl, wherein said alkyl, alkenyl, alkynyl, heterocyclyl, phenyl and cycloalkyl are independently optionally substituted by halogen, oxo,-CF₃, -OCF₃, -OR¹⁸, -SR¹⁸, -NR¹⁸R¹⁹, -CN, 3-6-membered heterocyclyl, phenyl, C₂-C₆ cycloalkyl or C₁-C₆ alkyl optionally substituted by halogen or oxo; or
R¹⁶ and R¹⁷ are independently taken together with the atom to which they are attached to form a 3-6 membered heterocyclyl optionally substituted by halogen, oxo or C₁-C₆ alkyl optionally substituted by halogen or oxo; and
each R¹⁸ and R¹⁹ are independently hydrogen or C₁-C₆ alkyl optionally substituted by halogen or oxo; or
R¹⁸ and R¹⁹ are independently taken together with the atom to which they are attached to form a 3-6 membered heterocyclyl optionally substituted by halogen, oxo or C₁-C₆ alkyl optionally substituted by halogen.

In certain embodiments, compounds of formula I are other than:
N-(1-ethyl-1H-pyrazol-4-yl)-1H-indazole-3-carboxamide;
5-amino-N-(1-ethyl-1H-pyrazol-4-yl)-1H-indazole-3-carboxamide; 5-amino-N-(1-methyl-1H-pyrazol-4-yl)-1H-1ndazole-3-carboxamide;
N-[1-(imidazo[1,2-a]pyridin-2-ylmethyl)-1H-pyrazol-4-yl]-1H-Indazole-3-carboxamide;
N-[1-[2-(diethylamino)ethyl]-1H-pyrazol-4-yl]-1H-Indazole-3-carboxamide;
N-(1-methyl-1H-pyrazol-4-yl)-5-nitro-1H-Indazole-3-carboxamide;
N-[1-[2-(3,4-dimethoxyphenyl)ethyl]-1H-pyrazol-4-yl]-1H-Indazole-3-carboxamide;
4-[(2H-indazol-3-ylcarbonyl)amino]-1H-Pyrazoie-1-acetic acid;
N-[1-[2-(phenylmethoxy)ethyl]-1H-pyrazol-4-yl]-1H-Indazole-3-carboxamide;
N-[1-(4-cyanobutyl)-1H-pyrazol-4-yl]-1H-Indazole-3-carboxamide; or
N-[1-[(3-cyanophenyl)methyl]-1H-pyrazol-4-yl]-1H-Indazole-3-carboxamide.
The excluded compounds are known from chemical catalogs prior of the filing date of the present application. The kind of use is unknown.

In certain embodiments, X, X¹, X² and X³ are C.

In certain embodiments, R¹ and R⁴ are independently hydrogen, halogen or -OR⁷.

In certain embodiments, X³ is N; R⁴ does not exist; and X, X¹, and X² are C.

In certain embodiments, R¹ is does not exist, hydrogen, halogen or -OR⁷, wherein R¹ is optionally substituted by R⁹. In certain embodiments, R¹ is hydrogen or -OCH₃. In certain embodiments, R¹ is hydrogen, F or -OCH₃.

In certain embodiments, R² is does not exist, hydrogen, halogen, -OR⁷ or 5-6-membered heterocyclyl wherein R² is optionally substituted by R⁹. In certain embodiments, R² is hydrogen, F, -OCH₃, pyrazolyl or pyridinyl.

In certain embodiments, R² is hydrogen, halogen, CF₃, CN or -NR⁷R⁸.

In certain embodiments, R² is 3-10 membered heterocyclyl optionally substituted by R⁹.

In certain embodiments, R² is dihydro-2H-pyrano[2,3-b]pyridinyl, pyridzainyl, oxazolyl, thiazolyl, pyrazinyl, pyrrolidinyl, azetidinyl, piperazinyl, 3,6-dihydropyridinyl, pyrazolyl, piperidinyl, 2,3-dihydropyrido[3,2-b][1,4]oxazin or pyrimidinyl, wherein R² is optionally substituted by R⁹.

In certain embodiments, R² is hydrogen, halogen, -OR⁷, CF₃, CN or -NR⁷R⁸, dihydro-2H-pyrano[2,3-b]pyridinyl, pyridzainyl, oxazolyl, thiazolyl, pyrazinyl, pyrrolidinyl, azetidinyl, piperazinyl, 3,6-dihydropyridinyl, pyrazolyl, piperidinyl, 2,3-dihydropyrido[3,2-b][1,4]oxazin or pyrimidinyl, wherein R¹ is optionally substituted by R⁹.

In certain embodiments, R³ is does not exist, hydrogen, halogen, -OR⁷, -NR⁷R⁸, C₁-C₁₂ alkyl, 3-10-membered heterocyclyl or 6-10 membered aryl, wherein R³ is optionally substituted by R⁹. In certain embodiments, R³ is hydrogen, methyl, ethyl, Cl, F, -CH₃, -OCH₃, phenyl, pyrazolyl, pyridinyl, pyrimidinyl, pyrrolidinyl or imidizolyl, wherein R³ is optionally substituted by R⁹.

In certain embodiments, R³ is hydrogen, halogen, C₁-C₆ alkyl, cycloalkyl, or -NR⁷R⁸, wherein said alkyl is optionally substituted by halogen or -OR¹⁰.

In certain embodiments, R³ is hydrogen, halogen, -OR⁷, -NR⁷R⁸, C₁-C₁₂ alkyl, 3-10-membered heterocyclyl or 6-10 membered aryl, wherein R³ is optionally substituted by R⁹.

In certain embodiments, R⁴ is does not exist, hydrogen or -OR⁷, wherein R⁴ is optionally substituted by R⁹. In certain embodiments, R⁴ hydrogen or -OCH₃.

In certain embodiments, X, X¹, X² and X³ are C; and R¹ and R⁴ are hydrogen.

In certain embodiments, X, X¹, X² and X³ are C; and R¹, R², R³ and R⁴ are independently hydrogen, C₁-C₁₂ alkyl, C₂-C₁₂ alkenyl, C₂-C₁₂ alkynyl, halogen, -CN, -OR⁷, -SR⁷, -NR⁷R⁸,-CF₃, -OCF₃, -NO₂, -C(O)R⁷, -C(O)OR⁷, -C(O)NR⁷R⁸, -NR⁷C(O)R⁸, -S(O)₁₋₂R⁷, -NR⁷S(O)₁₋₂R⁸, -S(O)₁₋₂NR⁷R⁸, C₃-C₆ cycloalkyl, 3-10-membered heterocyclyl or 6-10 membered aryl, wherein R¹, R², R³ and R⁴ are independently optionally substituted by R⁹.

In certain embodiments, R¹, R², R³ and R⁴ are independently do not exist, hydrogen, C₁-C₁₂ alkyl, halogen, -OR⁷, 5-6-membered heterocyclyl or phenyl, wherein R¹, R², R³ and R⁴ are independently optionally substituted by R⁹. In certain embodiments, R¹, R², R³ and R⁴ are independently hydrogen, C₁-C₁₂ alkyl, halogen, -OR⁷, 5-6-membered heterocyclyl or phenyl, wherein R¹, R², R³ and R⁴ are independently optionally substituted by R⁹.

In certain embodiments, R¹, R², R³ and R⁴ are independently do not exist, hydrogen, methyl, 2-hydroxyethyl, F, Cl, -OCH₃, pyrazolyl, pyridinyl, pyrimidinyl or phenyl, wherein R¹, R², R³ and R⁴ are independently optionally substituted by R⁹. In certain embodiments, R¹, R², R³ and R⁴ are independently hydrogen, methyl, 2-hydroxyethyl, F, Cl, -OCH₃, pyrazolyl, pyridinyl, pyrimidinyl or phenyl, wherein R¹, R², R³ and R⁴ are independently optionally substituted by R⁹.

In certain embodiments, R¹, R³ and R⁴ are independently do not exist, hydrogen, halogen or C₁-C₁₂ alkyl, and R² is hydrogen, C₁-C₁₂ alkyl, halogen, -OR⁷, 5-6-membered heterocyclyl or phenyl, wherein R¹, R², R³ and R⁴ are independently optionally substituted by R⁹. In certain embodiments, R¹, R³ and R⁴ are independently hydrogen, halogen or C₁-C₁₂ alkyl, and R² is hydrogen, C₁-C₁₂ alkyl, halogen, -OR⁷, 5-6-membered heterocyclyl or phenyl, wherein R¹, R², R³ and R⁴ are independently optionally substituted by R⁹.

In certain embodiments, R¹, R³ and R⁴ are independently do not exist or hydrogen, and R² is does not exist, hydrogen, F, -OCH₃ or 5-6-membered heterocyclyl, wherein R² is optionally substituted by R⁹. In certain embodiments, R¹, R³ and R⁴ are hydrogen, and R² is hydrogen, F, -OCH₃ or 5-6-membered heterocyclyl, wherein R² is optionally substituted by R⁹.

In certain embodiments, R¹, R³ and R⁴ are independently do not exist or hydrogen, and R² is pyrazolyl or pyridinyl optionally substituted by R⁹.

In certain embodiments, R¹, R² and R⁴ are independently do not exist, hydrogen, halogen or C₁-C₁₂ alkyl, and R³ is hydrogen, C₁-C₁₂ alkyl, halogen, -OR⁷, 5-6-membered heterocyclyl or phenyl, wherein R¹, R², R³ and R⁴ are independently optionally substituted by R⁹.

In certain embodiments, R¹, R² and R⁴ are independently do not exist or hydrogen and R³ is hydrogen, C₁-C₁₂ alkyl, halogen, -OR⁷, 5-6-membered heterocyclyl or phenyl, wherein R³ is independently optionally substituted by R⁹.

In certain embodiments, R¹, R² and R⁴ are independently do not exist or hydrogen and R³ is C₁-C₁₂ alkyl, halogen, -OR⁷, 5-6-membered heterocyclyl or phenyl, wherein R³ is independently optionally substituted by R⁹.

In certain embodiments, R¹, R² and R⁴ are independently do not exist or hydrogen and R³ is 5-membered heterocyclyl optionally substituted by R⁹. In certain embodiments, R¹, R² and R⁴ are hydrogen and R³ is 5-membered heterocyclyl optionally substituted by R⁹.

In certain embodiments, R¹, R² and R⁴ are independently do not exist or hydrogen and R³ is pyrazolyl optionally substituted by R⁹. In certain embodiments, R¹, R² and R⁴ are hydrogen and R³ is pyrazolyl optionally substituted by R⁹.

In certain embodiments, R¹, R² and R⁴ are independently do not exist or hydrogen and R³ is 6-membered heterocyclyl optionally substituted by R⁹. In certain embodiments, R¹, R² and R⁴ are hydrogen and R³ is 6-membered heterocyclyl optionally substituted by R⁹.

In certain embodiments, R¹, R² and R⁴ are independently do not exist or hydrogen and R³ is pyridinyl or pyrimidinyl optionally substituted by R⁹. In certain embodiments, R¹, R² and R⁴ are hydrogen and R³ is pyridinyl or pyrimidinyl optionally substituted by R⁹.

In certain embodiments, R⁵ is C₁-C₆ alkylene optionally substituted by halogen, oxo, C₁-C₁₂ alkyl, C₂-C₁₂ alkenyl, C₂-C₁₂ alkynyl, -OR¹⁶, -SR¹⁶, -NR¹⁶R¹⁷, -CN, -CF₃, -OCF₃, - C(O)R¹⁶, -C(O)OR¹⁶, -NR¹⁶C(O)R¹⁷, -NR¹⁶S(O)₁₋₂R¹⁷, 3-10-membered heterocyclyl or 6-10 membered aryl, and wherein said alkyl, alkenyl, alkynyl, heterocyclyl and phenyl are independently optionally substituted with R⁹.

In certain embodiments, R⁵ is C₁-C₆ alkylene optionally substituted by halogen, oxo, C₁-C₁₂ alkyl, C₂-C₁₂ alkenyl, C₂-C₁₂ alkynyl, -OR¹⁶, -SR¹⁶, -NR¹⁶R¹⁷, -CN, -CF₃, -OCF₃, wherein said alkyl, alkenyl and alkynyl are independently optionally substituted with oxo or halogen;

In certain embodiments, R⁵ does not exist, and R⁶ is hydrogen, C₃-C₁₀ cycloalkyl, 3-10-membered heterocyclyl or 6-10-membered aryl, wherein R⁶ is independently optionally substituted by R⁹.

In certain embodiments, R⁵ does not exist and R⁶ is hydrogen so that -R⁵-R⁶ taken together is hydrogen.

In certain embodiments, R⁵ is -CH₂-, -CH₂CH₂-, -CH₂CH₂CH₂-, -CH₂(CH₃)CH₂-, - CH₂CH(CH₃)₂-, -CH₂CH₂CH₂CH₂-, -CH₂CH(CH₂CH₃)CH₂CH₂-, -CH(CH₃)-, (*R*)-CH(CH₃)-, (*S*)-CH(CH₃)-, (*R*)-CH(CH(CH₃)₂)-, (*S*)-CH(CH(CH₃)₂)-, -CH(CH₂CH₃)-, -CH(CH₂CH₃)-, (*R*)-CH(CH₂CH₃)-, (*S*)-CH(CH₂CH₃)- or -C(CH₃)₂-, wherein R⁵ is independently optionally substituted by halogen, oxo, C₁-C₁₂ alkyl, C₂-C₁₂ alkenyl, C₂-C₁₂ alkynyl, -OR¹⁶, -SR¹⁶, - NR¹⁶R¹⁷, -CN, -CF₃, -OCF₃, -C(O)R¹⁶, -C(O)OR¹⁶, -NR¹⁶C(O)R¹⁷, -NR¹⁶S(O)₁₋₂R¹⁷, 3-10-membered heterocyclyl or 6-10 membered aryl, and wherein said alkyl, alkenyl, alkynyl, heterocyclyl and phenyl are independently optionally substituted with R⁹.

In certain embodiments, R⁵ is C₁-C₆ alkylene optionally substituted by -OH or -N(CH₃)₂.

In certain embodiments, R⁶ is hydrogen.

In certain embodiments, R⁶ is C₃-C₁₀ cycloalkyl, wherein R⁶ is independently optionally substituted by R⁹. In certain embodiments, R⁶ is cyclohexyl, cyclobutyl or norbornyl, wherein R⁶ is independently optionally substituted by R⁹.

In certain embodiments, R⁶ is 5-10-membered heterocyclyl or phenyl, wherein R⁶ is independently optionally substituted by R⁹. In certain embodiments, R⁶ is imidazolyl, pyrrolidinyl, azetidinyl, pyridazinyl, chromanyl, pyrimidinyl, tetrahydrofuranyl, tetrahydropyranyl, dioxanyl, morpholinyl, oxetanyl, phenyl, pyrazolyl, benzisoxazolyl, furanyl, isoxazolyl, benzothiazolyl, thiazolyl, thienyl, pyridinyl, piperidinyl, imidazo[1,2-a]pyridinyl or quinolinyl, wherein R⁶ is independently optionally substituted by R⁹.

In certain embodiments, R⁶ is hydrogen, cyclohexyl, cyclobutyl, norbornyl, imidazolyl, pyrrolidinyl, azetidinyl, pyridazinyl, chromanyl, pyrimidinyl, tetrahydrofuranyl, tetrahydropyranyl, dioxanyl, morpholinyl, oxetanyl, phenyl, pyrazolyl, benzisoxazolyl, furanyl, isoxazolyl, benzothiazolyl, thiazolyl, thienyl, pyridinyl, piperidinyl, imidazo[1,2-a]pyridinyl or quinolinyl, wherein R⁶ is independently optionally substituted by R⁹.

In certain embodiments, R⁶ is phenyl, pyrazolyl, benzisoxazolyl, furanyl, isoxazolyl, benzothiazolyl, thiazolyl, thienyl, pyridinyl, piperidinyl, imidazo[1,2-a]pyridinyl or quinolinyl, wherein R⁶ is independently optionally substituted by R⁹.

In certain embodiments, R⁷ and R⁸ are independently hydrogen or C₁-C₆ alkyl, wherein said alkyl is independently optionally substituted by halogen, -CN, -CF₃, -OCF₃, oxo or C₁-C₆ alkyl optionally substituted by halogen or oxo; or

R⁷ and R⁸ are independently taken together with the atom to which they are attached to form a 3-6 membered heterocyclyl optionally substituted by halogen, oxo or C₁-C₆ alkyl optionally substituted by halogen or oxo.

In certain embodiments, each R⁷ and R⁸ are independently hydrogen or methyl.

In certain embodiments, R⁸ is independently 3-6-membered heterocyclyl independently optionally substituted by halogen, -CN, -CF₃, -OCF₃, oxo or C₁-C₆ alkyl optionally substituted by halogen or oxo. In certain embodiments, R⁸ is independently piperidinyl, pyrrolidinyl, tetrahydropyranyl, wherein R⁸ is independently optionally substituted by halogen, -CN, -CF₃, - OCF₃, oxo or C₁-C₆ alkyl optionally substituted by halogen or oxo. In certain embodiments, each R⁹ is independently hydrogen, C₁-C₁₂ alkyl, C₂-C₁₂ alkynyl, halogen, -CN, -(C₀-C₆ alkylene)OR¹⁰, -(C₀-C₆ alkylene)NR¹⁰R¹¹, -CF₃, -(C₀-C₆ alkylene)C(O)OR¹⁰, -(C₀-C₆ alkylene)C(O)NR¹⁰R¹¹, -(C₀-C₆ alkylene)(5-6-membered heterocyclyl), -(C₀-C₆ alkylene)C(O)(5-6-membered heterocyclyl) or phenyl, wherein each R⁹ is independently optionally substituted by halogen, oxo, -CF₃, -CN, -OR¹², -SR¹², -NR¹²R¹³, -C(O)R¹², -S(O)₁₋₂R¹², C₁-C₆ alkyl optionally substituted by oxo or halogen, C₂-C₆ alkenyl optionally substituted by oxo or halogen, or C₂-C₆ alkynyl optionally substituted by oxo or halogen.

In certain embodiments, each R⁹ is independently hydrogen, methyl, ethyl, propyl, -CN, -OCH₃, -OH, -C(O)OCH₃, -C(O)OH, -C(O)NH₂, -CF₃, -OCF₃, Br, Cl, F, -CH₃, -C(CH₃)₂OH, -NH₂, -CH₂N(CH₃)₂, -NH(CH₃), -N(CH₃)CH₂OH, -CH₂OH, morpholinyl, -C(O)piperidinyl, ethynyl, piperazinyl, pyridinyl, tetrazolyl, phenyl, -C(O)NH(CH₃), -CH₂morpholinyl, isopropyl, thienyl,

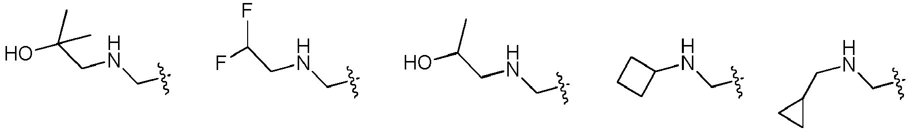

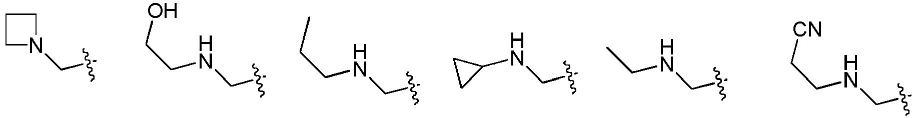

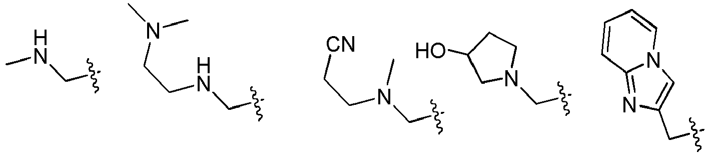

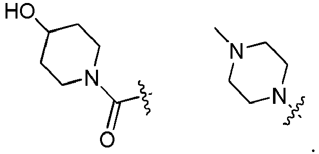

In certain embodiments, each R¹⁰ and R¹¹ is independently hydrogen or C₁-C₆ alkyl optionally substituted by halogen or oxo, wherein said alkyl is independently optionally substituted by halogen or oxo; or

R¹⁰ and R¹¹ are independently taken together with the atom to which they are attached to form a 3-6 membered heterocyclyl optionally substituted by halogen, oxo or C₁-C₆ alkyl optionally substituted by halogen or oxo.

In certain embodiments, each R¹⁰ and R¹¹ is independently hydrogen or C₁-C₆ alkyl.

In certain embodiments, each R¹² and R¹³ is independently hydrogen or methyl.

In certain embodiments, each R¹⁴ and R¹⁵ is independently hydrogen or methyl.

In certain embodiments, each R¹⁶ and R¹⁷ are independently hydrogen or C₁-C₆ alkyl, wherein said alkyl is independently optionally substituted by halogen or oxo; or

R¹⁶ and R¹⁷ are independently taken together with the atom to which they are attached to form a 3-6 membered heterocyclyl optionally substituted by halogen, oxo or C₁-C₆ alkyl optionally substituted by halogen or oxo.

In certain embodiments, each R¹⁶ and R¹⁷ are independently hydrogen or C₁-C₆ alkyl.

In certain embodiments, each R¹⁸ and R¹⁹ is independently hydrogen or methyl.

In certain embodiments, each R²⁰ and R²¹ are independently hydrogen or methyl.

In certain embodiments, X, X¹, X² and X³ are C, R¹ and R⁴ are hydrogen, R⁵ is C₁-C₆ alkylene, wherein said alkylene, alkenylene and alkynylene are independently optionally substituted by halogen, oxo, C₁-C₁₂ alkyl, C₂-C₁₂ alkenyl, C₂-C₁₂ alkynyl, -OR¹⁶, -SR¹⁶, - NR¹⁶R¹⁷, -CN, -C(O)R¹⁶, -C(O)OR¹⁶, -NR¹⁶C(O)R¹⁷, -NR¹⁶S(O)₁₋₂R¹⁷, -CF₃, -OCF₃, 3-10-membered heterocyclyl or 6-10 membered aryl, and wherein said alkyl, alkenyl, alkynyl, heterocyclyl and phenyl are independently optionally substituted with R⁹; and R² is C₁-C₁₂ alkyl, C₂-C₁₂ alkenyl, C₂-C₁₂ alkynyl, halogen, -CN, -OR⁷, -SR⁷, -NR⁷R⁸, -CF₃, -OCF₃, -NO₂, - C(O)R⁷, -C(O)OR⁷, -C(O)NR⁷R⁸, -NR⁷C(O)R⁸, -S(O)₁₋₂R⁷, -NR⁷S(O)₁₋₂R⁸, -S(O)₁₋₂NR⁷R⁸, C₃-C₆ cycloalkyl, 3-10-membered heterocyclyl or 6-10 membered aryl, wherein R² and R³ are independently optionally substituted by R⁹.

In certain embodiments each R¹²⁻²¹ is independently hydrogen or methyl.

Another aspect includes a compound selected from Examples 1-140, stereoisomers or a pharmaceutically acceptable salt thereof.

Another aspect includes a compound selected from Examples 1-279, stereoisomers or a pharmaceutically acceptable salt thereof.

Another aspect includes tautomeric forms of compounds of formula I. The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Another aspect includes a prodrug of formula I. A prodrug is a compound that may be converted under physiological conditions or by solvolysis to the specified compound or to a salt of such compound. Prodrugs include compounds wherein an amino acid residue, or a polypeptide chain of two or more (e.g., two, three or four) amino acid residues, is covalently joined through an amide or ester bond to a free amino, hydroxy or carboxylic acid group of a compound of the present invention. The amino acid residues include but are not limited to the 20 naturally occurring amino acids commonly designated by three letter symbols and also includes phosphoserine, phosphothreonine, phosphotyrosine, 4-hydroxyproline, hydroxylysine, demosine, isodemosine, gamma-carboxyglutamate, hippuric acid, octahydroindole-2-carboxylic acid, statine, 1,2,3,4-tetrahydroisoquinoline-3-carboxylic acid, penicillamine, ornithine, 3-methylhistidine, norvaline, beta-alanine, gamma-aminobutyric acid, cirtulline, homocysteine, homoserine, methyl-alanine, para-benzoylphenylalanine, phenylglycine, propargylglycine, sarcosine, methionine sulfone and tert-butylglycine.

Additional types of prodrugs are also encompassed. For instance, a free carboxyl group of a compound of Formula I can be derivatized as an amide or alkyl ester. As another example, compounds of this invention comprising free hydroxy groups may be derivatized as prodrugs by converting the hydroxy group into a group such as, but not limited to, a phosphate ester, hemisuccinate, dimethylaminoacetate, or phosphoryloxymethyloxycarbonyl group, as outlined in Advanced Drug Delivery Reviews, 1996, 19, 115. Carbamate prodrugs of hydroxy and amino groups are also included, as are carbonate prodrugs, sulfonate esters and sulfate esters of hydroxy groups. Derivatization of hydroxy groups as (acyloxy)methyl and (acyloxy)ethyl ethers, wherein the acyl group may be an alkyl ester optionally substituted with groups including, but not limited to, ether, amine and carboxylic acid functionalities, or where the acyl group is an amino acid ester as described above, are also encompassed. Prodrugs of this type are described in J. Med. Chem., 1996, 39, 10. More specific examples include replacement of the hydrogen atom of the alcohol group with a group such as (C₁-C₆)alkanoyloxymethyl, 1-((C₁-C₆)alkanoyloxy)ethyl,
1-methyl-1-((C₁-C₆)alkanoyloxy)ethyl, (C₁-C₆)alkoxycarbonyloxymethyl, N-(C₁-C₆)alkoxy-carbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

Another aspect includes isotopically-labeled compounds of formula I, which are structurally identical to those recited herein, but for the fact that one or more atoms are replaced by an atom having an atomic mass or mass number different from the atomic mass or mass number usually found in nature. All isotopes of any particular atom or element as specified are contemplated within the scope of the compounds of the invention, and their uses. Exemplary isotopes that can be incorporated into compounds of the invention include isotopes of hydrogen, carbon, nitrogen, oxygen, phosphorus, sulfur, fluorine, chlorine and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. Certain isotopically-labeled compounds of the present invention (e.g., those labeled with ³H and ¹⁴C) are useful in compound and/or substrate tissue distribution assays. Tritiated (i.e., ³H) and carbon-14 (i.e., ¹⁴C) isotopes are useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements) and hence may be preferred in some circumstances. Positron emitting isotopes such as ¹⁵O, ¹³N, ¹¹C and ¹⁸F are useful for positron emission tomography (PET) studies to examine substrate receptor occupancy. Isotopically labeled compounds of the present invention can generally be prepared by following procedures analogous to those disclosed in the Schemes and/or in the Examples herein below, by substituting an isotopically labeled reagent for a non-isotopically labeled reagent.

Another aspect includes salts of compounds of the present invention. Examples of salts include those salts prepared by reaction of a compound of formula I with a mineral or organic acid or an inorganic base, such salts including, but not limited to, sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyn-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycollates, tartrates, methanesulfonates, propanesulfonates, naphthalene-1-sulfonates, naphthalene-2-sulfonates, and mandelates. Since a single compound of the present invention may include more than one acidic or basic moiety, the compounds of the present invention may include mono, di or tri-salts in a single compound. In one example, the salt is a pharmaceutically acceptable acid addition salt. In another example, the salt is a pharmaceutically acceptable base addition salt.

The compounds of formula I also include other salts of such compounds which are not necessarily pharmaceutically acceptable salts, and which may be useful as intermediates for preparing and/or purifying compounds of formula I and/or for separating enantiomers of compounds of formula I.

Another aspect includes the in vivo metabolic products of compounds of formula I described herein. A "metabolite" is a pharmacologically active product produced through metabolism in the body of a specified compound or salt thereof. Such products may result, for example, from the oxidation, reduction, hydrolysis, amidation, deamidation, esterification, deesterification, enzymatic cleavage, glucuronidation, and the like, of the administered compound. Accordingly, another aspect includes metabolites of compounds of formula I, including compounds produced by a process comprising contacting a compound of this invention with a mammal for a period of time sufficient to yield a metabolic product thereof.

Metabolites are identified, for example, by preparing a radiolabelled (e.g., ¹⁴C or ³H) isotope of a compound of the invention, administering it parenterally in a detectable dose (e.g., greater than about 0.5 mg/kg) to an animal such as rat, mouse, guinea pig, monkey, or to a human, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g., by MS, LC/MS or NMR analysis. In general, analysis of metabolites is done in the same way as conventional drug metabolism studies well known to those skilled in the art. The metabolites, so long as they are not otherwise found in vivo, are useful in diagnostic assays for therapeutic dosing of the compounds of the invention.

### SYNTHESIS OF COMPOUNDS OF FORMULA I

Compounds of this invention may be synthesized by synthetic routes that include processes analogous to those well known in the chemical arts, particularly in light of the description contained herein. The starting materials are generally available from commercial sources such as Aldrich Chemicals (Milwaukee, WI) or are readily prepared using methods well known to those skilled in the art (e.g., prepared by methods generally described in Louis F. Fieser and Mary Fieser, Reagents for Organic Synthesis, v. 1-19, Wiley, N.Y. (1967-1999 ed.), or Beilsteins Handbuch der organischen Chemise, 4, Aufl. ed. Springer-Verlag, Berlin, including supplements).

Compounds of formula I may be prepared singly or as compound libraries comprising 2 or more compounds, for example 5 to 1,000 compounds, or 10 to 100 compounds. Libraries of compounds of formula I may be prepared by a combinatorial 'split and mix' approach or by multiple parallel syntheses using either solution phase or solid phase chemistry, by procedures known to those skilled in the art. Thus according to a further aspect of the invention there is provided a compound library comprising at least 2 compounds of formula I.

For illustrative purposes, Schemes 1-2 show a general method for preparing the compounds of the present invention as well as key intermediates. For a more detailed description of the individual reaction steps, see the Examples section below. Those skilled in the art will appreciate that other synthetic routes may be used to synthesize the compounds described herein. Although specific starting materials and reagents are depicted in the Schemes and discussed below, other starting materials and reagents can be easily substituted to provide a variety of derivatives and/or reaction conditions. In addition, many of the compounds prepared by the methods described below can be further modified in light of this disclosure using chemistry known to those skilled in the art.

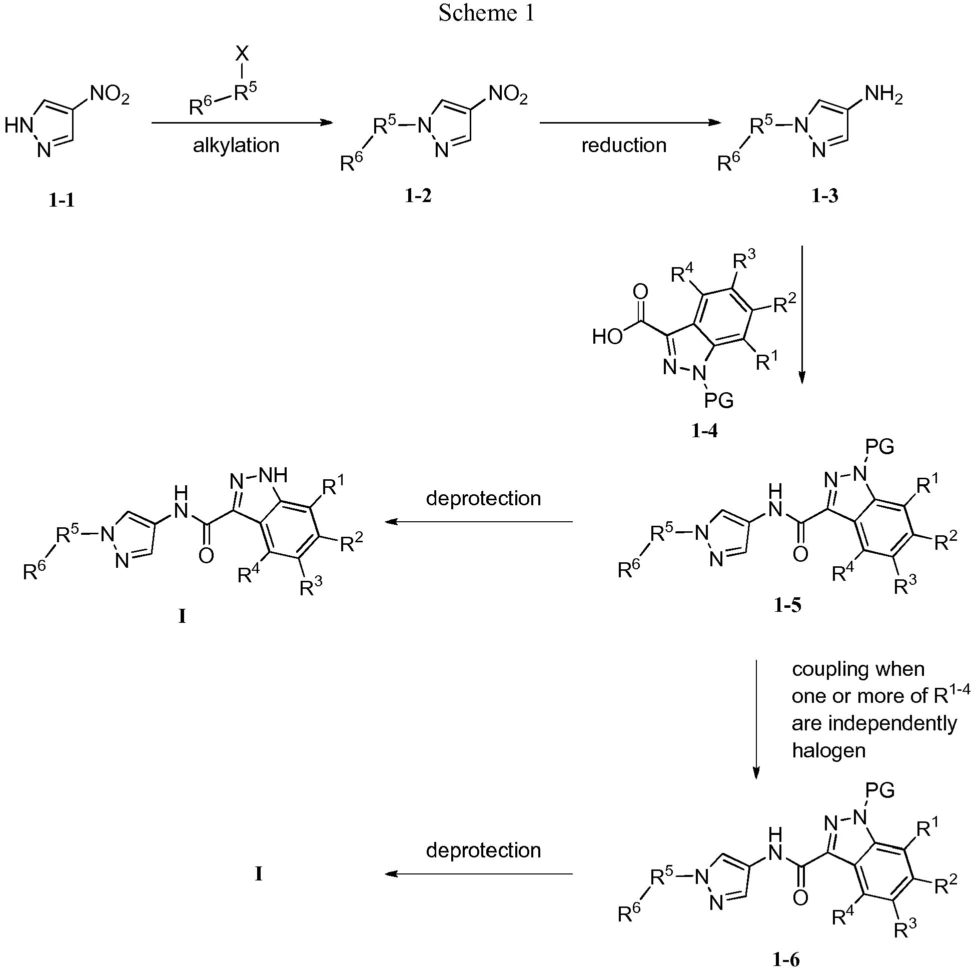

Scheme 1 shows a method of preparing compounds of formula I, wherein R¹-R⁶ are defined herein for formula I. Pyrazoles 1-1 can be alkylated to give alkylated pyrazoles 1-2, using alkylating agents such as alkylhalides or substituted alkylhalides (for example ethylbromide, bromomethylbenzene or 1-bromomethyl-3-chlorobenzene), or using Mitsunobu type alkylating conditions using triphenylphosphine, a diamide, such as DEAD, and a hydroxyalkyl or substituted hydroxyalkyl group (for example 1-phenylethanol or 3-(dimethylamino)-1-phenylpropan-1-ol). Alkylated pyrazoles 1-2 can be reduced to give the amino pyrazoles 1-3 using conditions such as transition metal catalyzed hydrogenation or reductions, such as palladium and hydrogen gas or tin halide salts in acidic solutions. Amino pyrazoles 1-3 can be coupled with imidazo carboxcylic acids 1-4 to form amides 1-5 using amide coupling conditions. Amides 1-5 can be further derivatized by coupling reactions when one or more of R¹, R², R³ and R⁴ are independently halogen (as exemplified in Scheme 2), to form derivatized amides 1-6.

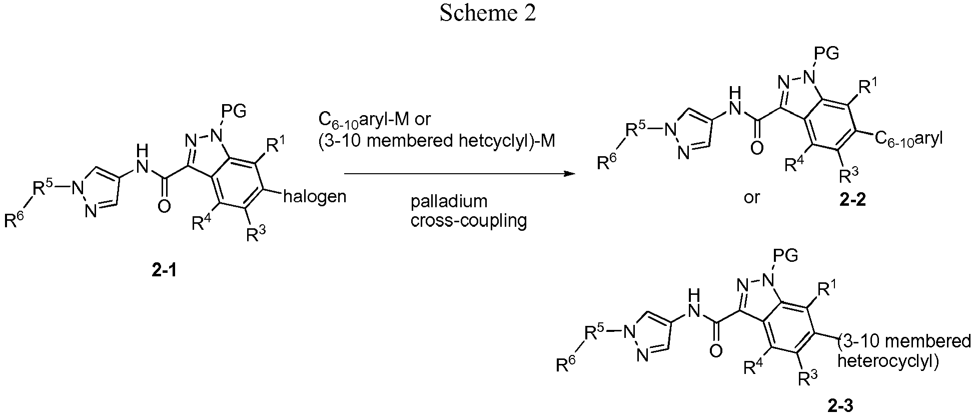

Scheme 2 shows an example method of cross coupling amides 2-1 to form derivatized compounds 2-2 or 2-3. Halogenated-amide 2-1 is cross-coupled using a transition metal catalyst, for example palladium, with an aryl- or heterocyclyl-substituted substrate (wherein M is for example a boronic acid or ester, or a zinc, copper, tin or magnesium organometallic).

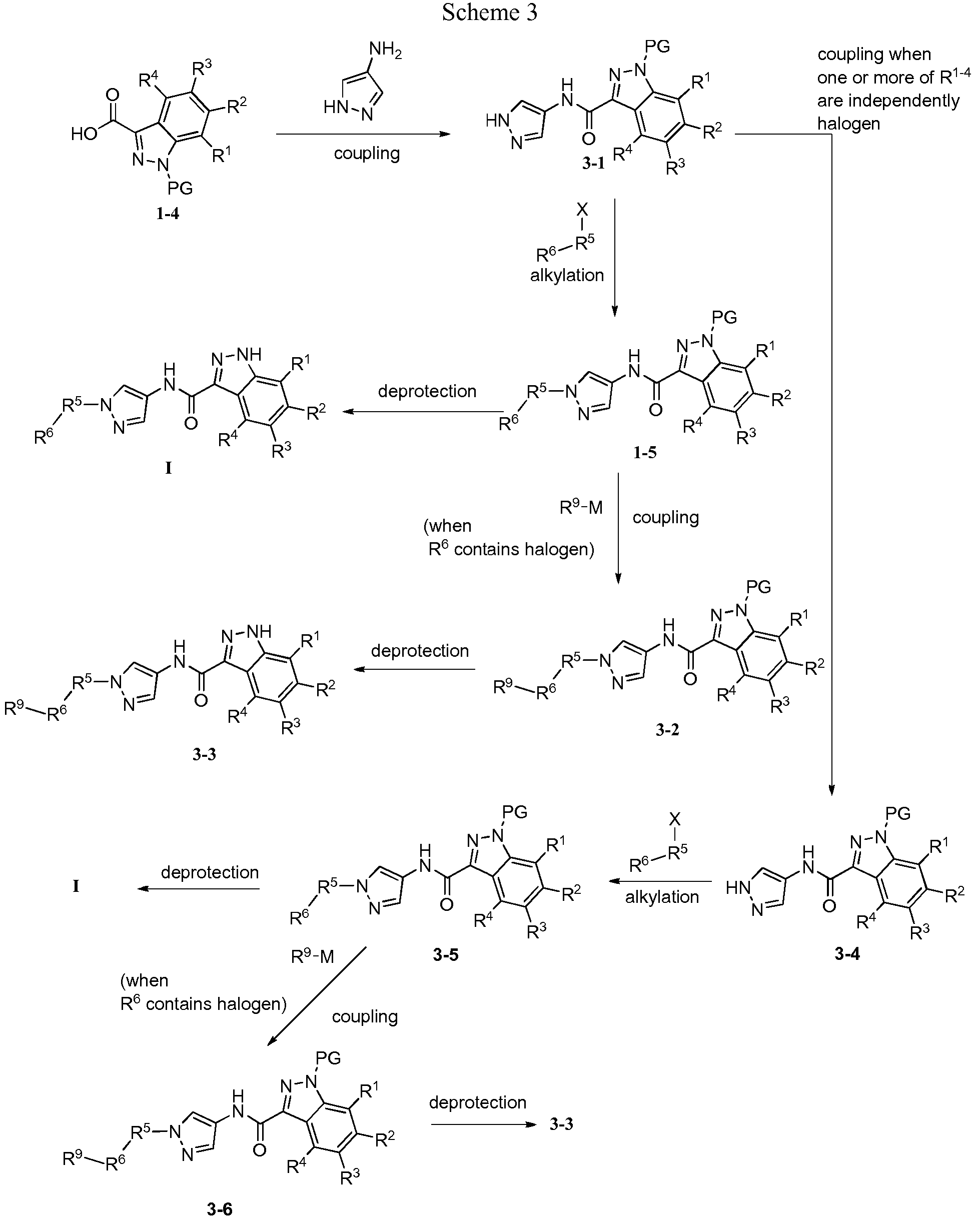

Scheme 3 shows a method of preparing compounds of formula I, wherein R¹-R⁶ are defined herein for formula I. Acids 1-4 can be coupled with amino pyrazoles to form amides 3-1. Amides 3-1 can be alkylated to form alkylated amides 1-5. Alkylated amides can then be deprotected to form compounds of formula I. Additionally, amides 3-1 can be derivatized when one or more of R¹-R⁴ are indepenedently halogen to form derivatized amides 3-4, which can be alkylated to form compounds 3-5 and deprotected to form compounds 3-3. When compounds 3-5 contain halogen in R⁶, then further coupling reactions can lead to derivatized compounds 3-6, which can be deprotected to form compounds 3-3.

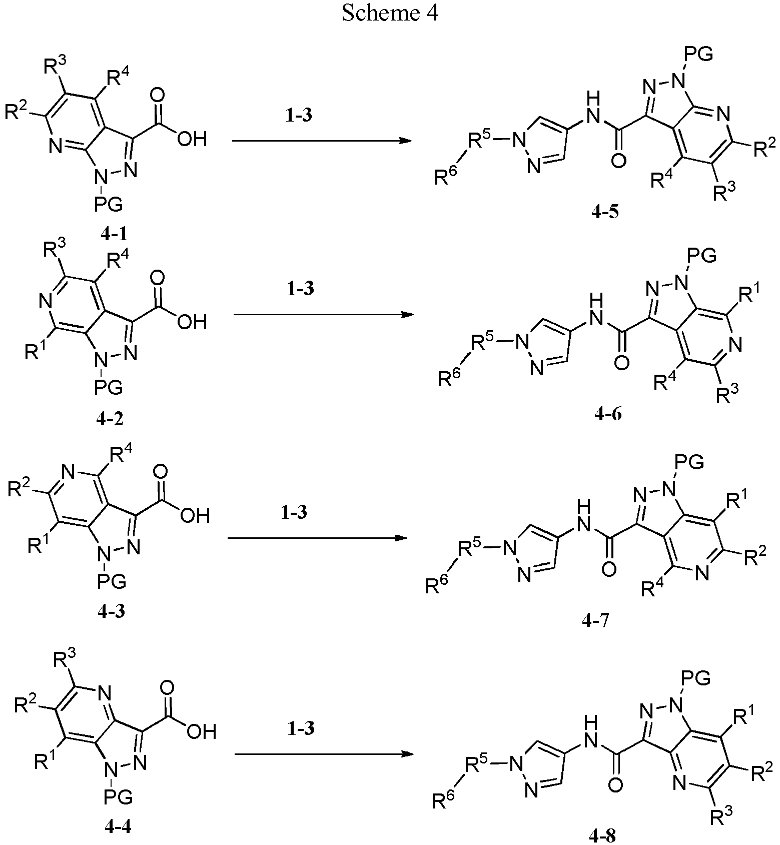

Scheme 4 shows a method of preparing compounds of formula I, wherein R¹-R⁶ are defined herein for formula I. Similarly to Scheme 1, acids 4-1, 4-2, 4-3 or 4-4 can be coupled with compound 1-3 to form the amides 4-5, 4-6, 4-7 or 4-8. The amides can be further derivatized according to similar reactions as shown in Scheme 1, to form compounds of formula I. The compound 1H-pyrazolo[3,4-b]pyridine-3-carboxylic acid can be made according to Lynch et al. Can. J. Chem. 1988, 66, 420, and 1H-pyrazolo[3,4-c]pyridine-3-carboxylic acid and 1H-pyrazolo[4,3-c]pyridine-3-carboxylic acid can be made according to U.S. Pat. Appl. Publication No. US 2007-78147 A1.

### PHARMACEUTICAL COMPOSITIONS AND ADMINISTRATION

Another embodiment provides pharmaceutical compositions or medicaments containing the compounds of formula I and a therapeutically inert carrier, diluent or excipient, as well as methods of using the compounds of the invention to prepare such compositions and medicaments. In one example, compounds of formula I may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula I is formulated in an acetate buffer, at pH 5. In another embodiment, the compounds of formula I are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to inhibit ITK kinase activity in a cell. For example, such amount may be below the amount that is toxic to normal cells, or the mammal as a whole.

In one example, the pharmaceutically effective amount of the compound of the invention administered parenterally per dose will be in the range of about 0.01-100 mg/kg, alternatively about 0.1 to 20 mg/kg of patient body weight per day, with the typical initial range of compound used being 0.3 to 15 mg/kg/day. In another embodiment, oral unit dosage forms, such as tablets and capsules, preferably contain from about 25-100 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.
A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 25mg, 50mg, 100mg, 250mg, or 500mg of the compound of the invention compounded with about 30-90 mg anhydrous lactose, about 5-40 mg sodium croscarmellose, about 5-30 mg polyvinylpyrrolidone (PVP) K30, and about 1-10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5-400 mg, of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

One aspect, therefore, includes a pharmaceutical composition comprising a compound of Formula I, or a stereoisomer or pharmaceutically acceptable salt thereof. In a further embodiment includes a pharmaceutical composition comprising a compound of Formula I, or a stereoisomer or pharmaceutically acceptable salt thereof, together with a pharmaceutically acceptable carrier or excipient.

Another embodiment includes a pharmaceutical composition comprising a compound of formula I and a therapeutically inert carrier, diluent or excipient.

Another embodiment includes a pharmaceutical composition comprising a compound of Formula I for use in the treatment of a disease responsive to the inhibition of ITK kinase. Another embodiment includes a pharmaceutical composition comprising a compound of Formula I for use in the treatment of a immunological or inflammatory disease. Another embodiment includes a pharmaceutical composition comprising a compound of Formula I for use in the treatment of asthma or atopic dermatitis.

### INDICATIONS AND METHODS OF TREATMENT

ITK is activated downstream of antigen engagement of the T cell receptor (TCR) and mediates TCR signals through the phosphorylation and activation of PLCg. Mice in which ITK is deleted showed defective differentiation of T cells towards to the Th2 subset, but not the Th1 subset. Additional studies indicate that Th2 cytokine production, but not early Th2 lineage commitment, is defective in ITK-deficient mouse T cells. Th2 cells promote allergic inflammation, and ITK knock-out mice have reduced lung inflammation, mucus production, and airway hyperreactivity in models of allergic asthma.

The compounds of the invention inhibit the activity of ITK kinase. Accordingly, the compounds of the invention are useful for the treatment of inflammation and immunological diseases. Inflammatory diseases which can be treated according to the methods of this invention include, but are not limited to, asthma, allergic rhinitis, atopic dermatitis, rheumatoid arthritis, psoriasis, contact dermatitis, and delayed hypersensitivity reactions.

An embodiment includes a method of treating or preventing a disease responsive to the inhibition of ITK kinase in a mammal in need of such treatment, wherein the method comprises administering to said mammal a therapeutically effective amount of a compound of Formula I, a stereoisomer or pharmaceutically acceptable salt thereof.

An embodiment includes use of a compound of Formula I, a stereoisomer or pharmaceutically acceptable salt thereof in therapy.

Another embodiment includes use of a compound of Formula I, a stereoisomer or pharmaceutically acceptable salt thereof in treating or preventing a disease responsive to the inhibition of ITK kinase.

Another embodiment includes use of a compound of Formula I, a stereoisomer or pharmaceutically acceptable salt thereof in treating or preventing an inflammatory disease.

Another embodiment includes use of a compound of Formula I, a stereoisomer or pharmaceutically acceptable salt thereof in treating asthma, allergic rhinitis, atopic dermatitis, rheumatoid arthritis, psoriasis, contact dermatitis, and delayed hypersensitivity reactions.

Another embodiment includes use of a compound of Formula I, a stereoisomer or pharmaceutically acceptable salt thereof in the manufacture of a medicament for the treatment of an inflammatory disease.

Another embodiment includes use of a compound of Formula I, a stereoisomer or pharmaceutically acceptable salt thereof in the manufacture of a medicament for the treatment of asthma, allergic rhinitis, atopic dermatitis, rheumatoid arthritis, psoriasis, contact dermatitis, and delayed hypersensitivity reactions.

Another embodiment includes the use of a compound of Formula I, a stereoisomer or pharmaceutically acceptable salt thereof for the preparation of a medicament for treating or preventing an inflammatory disease.

Another embodiment includes the use of a compound of Formula I, a stereoisomer or pharmaceutically acceptable salt thereof for the preparation of a medicament for treating asthma, allergic rhinitis, atopic dermatitis, rheumatoid arthritis, psoriasis, contact dermatitis, and delayed hypersensitivity reactions.

Another embodiment includes a compound of Formula I, a stereoisomer or pharmaceutically acceptable salt thereof for treating or preventing an inflammatory disease.

Another embodiment includes a compound of Formula I, a stereoisomer or pharmaceutically acceptable salt thereof for treating asthma, allergic rhinitis, atopic dermatitis, rheumatoid arthritis, psoriasis, contact dermatitis, and delayed hypersensitivity reactions.

Another embodiment includes a compound of Formula I, a stereoisomer or pharmaceutically acceptable salt thereof for use in treating or preventing an inflammatory disease.

Another embodiment includes a compound of Formula I, a stereoisomer or pharmaceutically acceptable salt thereof for use in treating asthma, allergic rhinitis, atopic dermatitis, rheumatoid arthritis, psoriasis, contact dermatitis, and delayed hypersensitivity reactions.
Another embodiment includes the use of a compound of Formula I in therapy.
Another embodiment includes the use of a compound of Formula I in the treatment of an immunological or inflammatory disease.
Another embodiment includes the use of a compound of Formula I for the preparation of medicament for the treatment treatment of an immunological or inflammatory disease.
Another embodiment includes a compound of Formula I for use in the treatment of an immunological or inflammatory disease

Another embodiment includes a method of treating a disease responsive to the inhibition of ITK kinase in a patient, comprising administering an effective amount of a compound of Formula I, stereoisomers or a pharmaceutically acceptable salt thereof

Compounds of the invention are also useful for reducing inflammation in cells that overexpress ITK. Alternatively, compounds of the invention are useful for reducing inflammation in cells that have aberrant or overactive antigen engagement of the T cell receptor. Alternatively, compounds of the invention are useful for reducing inflammation in cells that have over-activation or phosphorylation of PLCg. Additionally, the compounds can be used for the treatment of inflammation or immunological disorders in cells that overexpress Th2 cytokine. Another embodiment includes a method of treating or preventing cancer in a mammal in need of such treatment, wherein the method comprises administering to said mammal a therapeutically effective amount of a compound of Formula I, a stereoisomer or pharmaceutically acceptable salt thereof.

### COMBINATION THERAPY

The compounds of formula I may be employed alone or in combination with other chemotherapeutic agents for treatment. The compounds of the present invention can be used in combination with one or more additional drugs, for example an anti-hyperproliferative, anticancer, cytostatic, cytotoxic, anti-inflammatory or chemotherapeutic agent. The second compound of the pharmaceutical combination formulation or dosing regimen preferably has complementary activities to the compound of this invention such that they do not adversely affect each other. Such agents are suitably present in combination in amounts that are effective for the purpose intended. The compounds may be administered together in a unitary pharmaceutical composition or separately and, when administered separately this may occur simultaneously or sequentially. Such sequential administration may be close or remote in time. In one embodiment, compounds of the present invention are coadministered with a cytostatic compound selected from the group consisting of cisplatin, doxorubicin, taxol, taxotere and mitomycin C. In another embodiment, the cytostatic compound is doxorubicin. In another embodiment, compounds of the present invention are coadministered with an anti-inflammatory agent selected from a NSAID and corticosteroid. In one embodiment, compounds of the present invention are coadministered with any of anti-asthmtic agents, including but not limited to beta2-adrenergic agonists, inhaled and oral corticosteroids, leukotriene receptor antagonist, and omalizumab. In another embodiment, compounds of the present invention are coadministered with an anti-asthmtic agent selected from a NSAID, combinations of fluticasone and salmeterol, combinations of budesonide and formoterol, omalizumab, lebrikizumab and corticosteroid selected from fluticasone, budesonide, mometasone, flunisolide and beclomethasone. In another embodiment, compounds of the present invention are coadministered with an anti-rheumatoid agent, in one example, RITUXAN®. In another embodiment, compounds of the present invention are coadministered with a chemotherapeutic agent selected from etanercept (Enbrel), infliximab (Remicade), adalimumab (Humira), certolizumab pegol (Cimzia), golimumab (Simponi), Interleukin 1 (IL-1) blockers such as anakinra (Kineret), monoclonal antibodies against B cells such as rituximab (RITUXAN®), T cell costimulation blockers such as abatacept (Orencia), Interleukin 6 (IL-6) blockers such as tocilizumab (ACTEMERA®); Interleukin 13 (IL-13) blockers such as lebrikizumab; Interferon alpha (IFN) blockers such as Rontalizumab; Beta 7 integrin blockers such as rhuMAb Beta7; IgE pathway blockers such as Anti-M1 prime; Secreted homotrimeric LTa3 and membrane bound heterotrimer LTa1/β2 blockers such as Anti-lymphotoxin alpha (LTa).

The compounds of the present invention can be also used in combination with radiation therapy. The phrase "radiation therapy" refers to the use of electromagnetic or particulate radiation in the treatment of neoplasia. Radiation therapy delivers doses of radiation sufficiently high to a target area to cause death of reproducing cells, in both tumor and normal tissues. The radiation dosage regimen is generally defined in terms of radiation absorbed dose (rad), time and fractionation, and must be carefully defined by the oncologist. The amount of radiation a patient receives will depend on various considerations but two of the most important considerations are the location of the tumor in relation to other critical structures or organs of the body, and the extent to which the tumor has spread. Examples of radiotherapeutic agents are provided in Hellman, Principles of Radiation Therapy, Cancer, in Principles I and Practice of Oncology, 24875 (Devita et al., 4th ed., vol 1, 1993). Alternative forms of radiation therapy include three-dimensional conformal external beam radiation, intensity modulated radiation therapy (IMRT), stereotactic radiosurgery and brachytherapy (interstitial radiation therapy), the latter placing the source of radiation directly into the tumor as implanted "seeds". These alternative treatment modalities deliver greater doses of radiation to the tumor, which accounts for their increased effectiveness when compared to standard external beam radiation therapy.

### ARTICLES OF MANUFACTURE

Another embodiment includes a method of manufacturing a compound of formula I, comprising reacting a comopund of formula 1-3 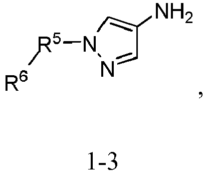 or a salt thereof, with a comopund of formula 1-4 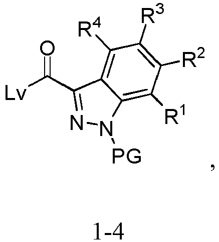 or a salt thereof, wherein PG is an amino protecting group and Lv is a leaving group, to form a compound of formula I.

In certain embodiments, PG is a BOC group. In certain embodiments, Lv is OH.

Another embodiment includes a kit for treating a disease or disorder responsive to the inhibition of ITK kinase. The kit includes:
(a) a first pharmaceutical composition comprising a compound of formula I; and
(b) instructions for use.

In another embodiment, the kit further includes:
(c) a second pharmaceutical composition, which includes a chemotherapeutic agent.

In one embodiment, the instructions describe the simultaneous, sequential or separate administration of said first and second pharmaceutical compositions to a patient in need thereof.

In one embodiment, the first and second compositions are contained in separate containers.

In one embodiment, the first and second compositions are contained in the same container.

Containers for use include, for example, bottles, vials, syringes, blister pack, etc. The containers may be formed from a variety of materials such as glass or plastic. The container includes a compound of formula I or formulation thereof which is effective for treating the condition and may have a sterile access port (for example the container may be an intravenous solution bag or a vial having a stopper pierceable by a hypodermic injection needle). The container includes a composition comprising at least one compound of formula I. The label or package insert indicates that the composition is used for treating the condition of choice, such as cancer. In one embodiment, the label or package inserts indicates that the composition comprising the compound of formula I can be used to treat a disorder. In addition, the label or package insert may indicate that the patient to be treated is one having a disorder characterized by overactive or irregular kinase activity. The label or package insert may also indicate that the composition can be used to treat other disorders.

The article of manufacture may comprise (a) a first container with a compound of formula I contained therein; and (b) a second container with a second pharmaceutical formulation contained therein, wherein the second pharmaceutical formulation comprises a chemotherapeutic agent. The article of manufacture in this embodiment of the invention may further comprise a package insert indicating that the first and second compounds can be used to treat patients at risk of stroke, thrombus or thrombosis disorder. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In order to illustrate the invention, the following examples are included. However, it is to be understood that these examples do not limit the invention and are only meant to suggest a method of practicing the invention. Persons skilled in the art will recognize that the chemical reactions described may be readily adapted to prepare other compounds of formula I, and alternative methods for preparing the compounds of formula I are within the scope of this invention. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds of the invention.

### EXAMPLES

The invention will be more fully understood by reference to the following examples. They should not, however, be construed as limiting the scope of the invention.

### Intermediate Examples

### Example A

### Synthesis of 6-Bromo-1-(2-trimethylsilanyl-ethoxymethyl)-1H-indazole-3-carboxylic acid methyl ester

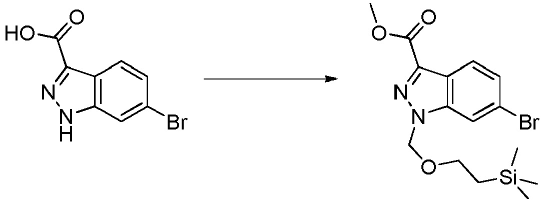

To a suspension of 6-bromo-1H-indazole-3-carboxylic acid (3.00 g, 12 mmol) in methanol (50 mL, 1 mol) was added Sulfuric acid (1.50 mL, 28 mmol), and the mixture was heated to 90 °C for 4 hours. After cooling to rt, the mixture was diluted with 200 mL EtOAc, and washed with 150 mL sat. NaHCO₂(aq) and 150 mL brine. The organic extracts were dried (Na₂SO₄) and concentrated in vacuo to provide 2.42 g (76%) of pure 6-bromo-1H-indazole-3-carboxylic acid methyl ester as a yellow solid. This material was diluted in tetrahydrofuran (50 mL), cooled to 0 °C, then sodium hydride (0.417 g, 10.4 mmol) was added. The mixture was stirred at 0 °C for 30 minutes, then [β-(trimethylsilyl)ethoxy]methyl chloride (1.85 mL, 10.4 mmol) was added dropwise. The mixture was allowed to slowly warm to room temperature over 90 minutes, then MeOH was added to quench excess hydride, and the mixture was concentrated in vacuo. The residue was diluted with 200 mL EtOAc, then washed with 200 mL brine. The aqueous layer was further extracted with 50 mL EtOAc, then the combined organic extracts were dried (Na₂SO₄) and concentrated in vacuo. Purification by CombiFlash (40 g column; load with CH₂Cl₂; 100:0 to 50:50 heptane:EtOAc over 30 minutes) provided 3.22 g (88%) of the title compound as a yellow oil.

### Example B

### Synthesis of 6-Bromo-1-(2-trimethylsilanyl-ethoxymethyl)-1H-indazole-3-carboxylic acid

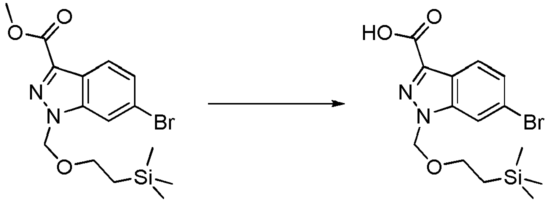

To a solution of 6-Bromo-1-(2-trimethylsilanyl-ethoxymethyl)-1H-indazole-3-carboxylic acid methyl ester (3.22 g, 8.36 mmol) in tetrahydrofuran (30 mL) was added a suspension of lithium hydroxide (0.800 g, 33.4 mmol) in water (10 mL), and the mixture was stirred vigorously at room temperature overnight. The mixture was poured into 40 mL 1 N HCl(aq) and diluted with 50 mL H₂O and 100 mL EtOAc. The layers were separated, then the organic phase was further washed with 100 mL brine. The organic extracts were dried (Na₂SO₄) and concentrated in vacuo to provide 2.51 g (81%) of the title compound as a yellow solid.

### Example C

### Synthesis of 5-Bromo-1-((2-(trimethylsilyl)ethoxy)methyl)-1H-indazole-3-carboxylic acid

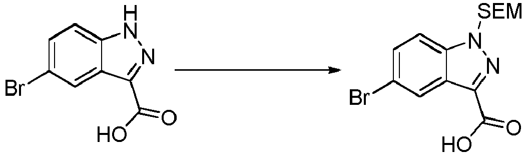

Prepared in an analogous manner to 6-Bromo-1-(2-trimethylsilanyl-ethoxymethyl)-1H-indazole-3-carboxylic acid, substituting 5-bromo-1H-indazole-3-carboxylic acid for 6-bromo-1H-indazole-3-carboxylic acid in the first step.

### Example D

### Synthesis of 5-Phenyl-1H-indazole-3-carboxylic acid

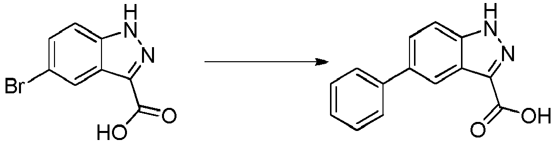

5-Bromo-1*H*-indazole-3-carboxylic acid (500 mg, 2.1 mmol) and phenylboronic acid (400 mg, 3.1 mmol) in dioxane/H₂O (1 mL/1 mL) and Cs₂CO₃ (1.3 g, 4.2 mmol) were combined in a microwave reaction tube. After the mixture was purged by bubbling with N₂ for a few minutes, Pd(dppf)Cl₂ (150 mg, 0.2 mmol) was added. The mixture was heated at 120 °C for 20 min under the microwave irradiation. It was filtered, and the solvent was removed under reduced pressure. The residue was purified by silica gel column chromatography to give the titled compound with a purity of 50% (600 mg, 40%).

### Example E

### Synthesis of 6-(pyrrolidin-1-yl)-1H-indazole-3-carboxylic acid

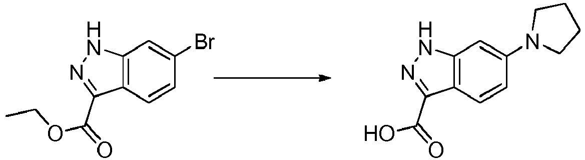

A vial was charged with ethyl 6-bromo-1H-indazole-3-carboxylate (500.00 mg, 1.8581 mmol), pyrrolidine (170.6 µL, 2.044 mmol), RuPhos Palladium(II) Phenethylamine Chloride (81.24 mg, 0.1115 mmol) and 2-Dicyclohexylphosphino-2',6'-diisopropoxybiphenyl (52.02 mg, 0.1115 mmol) in Tetrahydrofuran (11 mL). Next, 1.0 M of Lithium hexamethyldisilazide in Tetrahydrofuran (5.6 mL, 5.6 mmol) was added. The reaction vial was sealed and the reaction mixture was purged with a stream of N₂ via needle inlet/outlet for several minutes. The mixture was heated at 60°C for 3 hours. The reaction mixture was diluted with EtOAc and washed with water, saline and concentrated to dryness. The crude material was purified by CombiFlash (dry load) on a 12 G silica column, eluting with 10 - 90% EtOAc / heptanes to give 0.288 mg ethyl 6-(pyrrolidin-1-yl)-1H-indazole-3-carboxylate. To a solution of this material in Tetrahydrofuran (8 mL) was added Lithium hydroxide (0.133 g, 5.55 mmol). The reaction mixture was heated at 60 °C overnight. The reaction mixture was then concentrated in vacuo and acidified with 2N HCl to pH = 3 with precipitation of a white powder. This white powder was collected by vac. Filtration and taken into EtOAc and concentrated to give 250 mg (95%, 2 steps) of the title compound as a crystalline white powder.

### Example F

### Synthesis of 6-Bromo-1-(2-trimethylsilanyl-ethoxymethyl)-1H-indazole-3-carboxylic acid (1H-pyrazol-4-yl)-amide

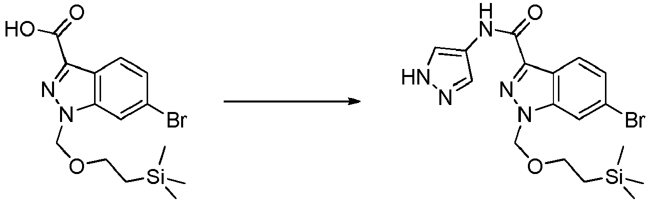

To a stirred mixture of 6-Bromo-1-(2-trimethylsilanyl-ethoxymethyl)-1H-indazole-3-carboxylic acid (57.2 g, 0.154 mol), HATU (64.4 g, 0.169 mol), and diisopropylethylamine (69.7 g, 0.539 mol) in CH₂Cl₂ (1500mL) was added a solution of 1H-pyrazole-4-amine (22.4 g, 0.270 mol) in DMF (500 mL) dropwise. The mixture was stirred at room temperature for 18 h. The reaction mixture was quenched with water. MTBE was added to the mixture, and after separation, the aqueous layer was extracted with MTBE three times. The combined organic layers were washed by water three times and saturated brine once, dried over Na₂SO₄. After concentration, the residue was triturated with MTBE to afford 55 g of desired product as an off white solid. (Yield: 82%).

### Example G

### Synthesis of N-(1H-pyrazol-4-yl)-1-trityl-1H-indazole-3-carboxamide

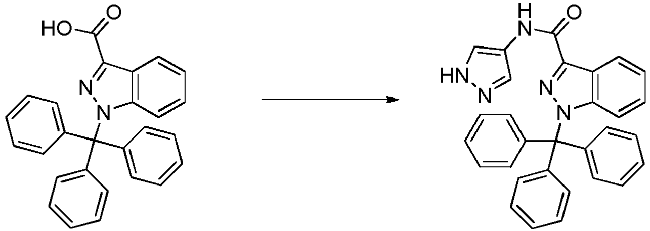

Prepared in an analogous manner to 6-Bromo-1-(2-trimethylsilanyl-ethoxymethyl)-1H-indazole-3-carboxylic acid (1H-pyrazol-4-yl)-amide, replacing 6-Bromo-1-(2-trimethylsilanyl-ethoxymethyl)-1H-indazole-3-carboxylic acid with 1-trityl-1H-indazole-3-carboxylic acid.

### Example H

### Synthesis of 3-((4-nitro-1H-pyrazol-1-yl)methyl)benzonitrile

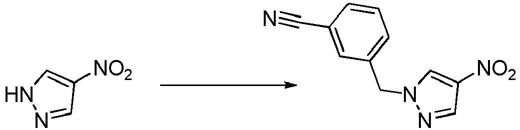

To a solution of 4-Nitro-1H-pyrazole (4.00 g, 35.4 mmol) in N,N-Dimethylformamide (200 mL) was added K₂CO₃ (5.867 g, 42.45 mmol), then *m*-cyanobenzyl bromide (6.935 g, 35.37 mmol). The mixture was stirred overnight at rt then the mixture was diluted with 300 mL EtOAc and washed with 2 x 200 mL 1:1 H₂O:brine. The organic extracts were dried (Na₂SO₄) and concentrated in vacuo. Purification by CombiFlash (120 g column; dry load; 0:100 EtOAc/heptane over 32 minutes) provided 7.60 g (95%) of the title compound as a white solid.

### Example I

### Synthesis of 3-((4-amino-1H-pyrazol-1-yl)methyl)benzonitrile

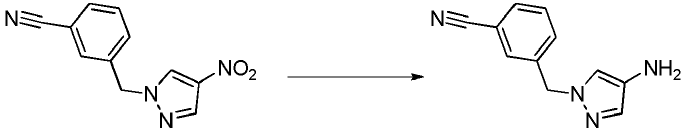

To a solution of 3-((4-nitro-1H-pyrazol-1-yl)methyl)benzonitrile (1.38 g, 6.06 mmol) in ethanol (40 mL) was added ammonium chloride (1.62 g, 30.3 mmol) as a saturated solution in water then iron (1.69 g, 30.3 mmol). The mixture was heated to 80 °C for 60 minutes, then cooled to rt. The mixture was diluted with 150 mL EtOAc and washed with 100 mL sat. NaHCO₃(aq) and 100 mL brine. The organic extracts were dried (Na₂SO₄) and concentrated in vacuo. The unpurified residue (1.20 g; quant.) was used directly without further purification.

### Example J

### Synthesis of 6-Bromo-1-(2-trimethylsilanyl-ethoxymethyl)-1H-indazole-3-carboxylic acid [1-(3-cyano-benzyl)-1H-pyrazol-4-yl]-amide

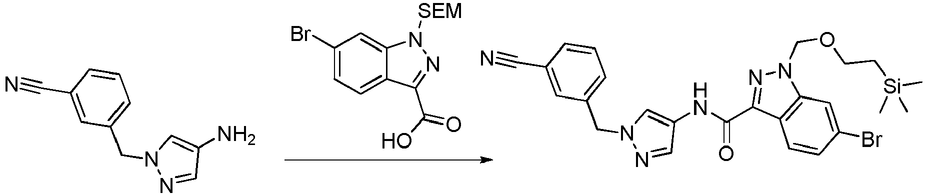

To a solution of 3-((4-amino-1H-pyrazol-1-yl)methyl)benzonitrile (1.20 g, 6.06 mmol) and 6-Bromo-1-(2-trimethylsilanyl-ethoxymethyl)-1H-indazole-3-carboxylic acid (1.50 g, 4.04 mmol) in N,N-dimethylformamide (25.0 mL) was added HATU (1.843 g, 4.848 mmol) and N,N-diisopropylethylamine (1.056 mL, 6.060 mmol) and the mixture was stirred for 36 hours. The mixture was diluted with 200 mL EtOAc and washed with 200 mL sat. NaHCO₃(aq) and 3 x 200 mL 1:1 H₂O:brine. The aqueous fractions were further extracted with 100 mL EtOAc, then the combined organic extracts were dried (Na₂SO₄) and concentrated in vacuo. Purification by CombiFlash (80 g column; dry load; 80:20 to 40:60 heptane:EtOAc over 30 minutes) provided 911 mg (41%) of the title compound as a white solid.

### Example K

### 5-Bromo-N-(1-(3-cyanobenzyl)-1H-pyrazol-4-yl)-1-((2-(trimethylsilyl)ethoxy)methyl)-1H-indazole-3-carboxamide

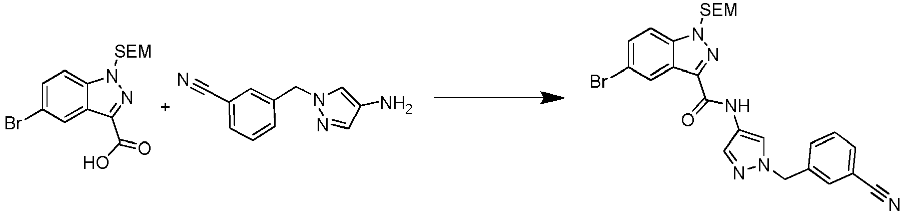

Prepared in an analogous manner to 6-Bromo-1-(2-trimethylsilanyl-ethoxymethyl)-1H-indazole-3-carboxylic acid [1-(3-cyano-benzyl)-1H-pyrazol-4-yl]-amide, substituting 5-bromo-1-((2-(trimethylsilyl)ethoxy)methyl)-1*H*-indazole-3-carboxylic acid for 6-Bromo-1-(2-trimethylsilanyl-ethoxymethyl)-1H-indazole-3-carboxylic acid.

### Example L

### 6-[1-(Tetrahydro-pyran-2-yl)-1H-pyrazol-4-yl]-1-(2-trimethylsilanyl-ethoxymethyl)-1H-indazole-3-carboxylic acid (1H-pyrazol-4-yl)-amide

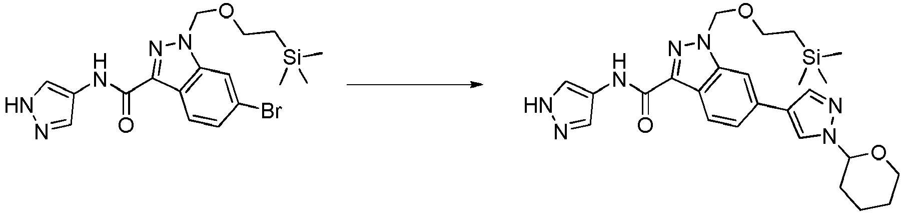

To a suspension of 6-Bromo-1-(2-trimethylsilanyl-ethoxymethyl)-1H-indazole-3-carboxylic acid **(1H-pyrazol-4-yl)-amide** (1.86 g, 4.26 mmol) and potassium 1-(tetrahydro-2H-pyran-2-yl)-1H-pyrazole-4-trifluoroborate (2.20 g, 8.52 mmol) in acetonitrile (20 mL) was added 1,1'-bis(diphenylphosphino)ferrocenepalladium (II) chloride (0.348 g, 0.426 mmol) and sodium carbonate (1.13 g, 10.6 mmol) as a 1.0 M solution in H₂O, and the mixture was heated to 120 °C in the microwave for 30 minutes. After cooling to rt, the mixture was diluted with 100 mL EtOAc and washed with 100 mL brine. The aqueous phase was further extracted with 100 mL EtOAc, then the combined organic extracts were dried (Na₂SO₄) and concentrated in vacuo. Purification by CombiFlash (40 g column; dry load; 50:50 to 0:100 heptane:EtOAc over 30 minutes) provided 1.71 g (79%) of the title compound as a light pink solid.

### Example M

### N-(1H-pyrazol-4-yl)-6-(3-pyridyl)-1-(2-trimethylsilylethoxymethyl)indazole-3-carboxamide

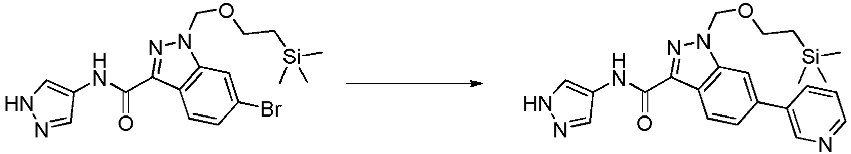

Prepared in an analogous manner to the synthesis of 6-[1-(Tetrahydro-pyran-2-yl)-1H-pyrazol-4-yl]-1-(2-trimethylsilanyl-ethoxymethyl)-1H-indazole-3-carboxylic acid (1H-pyrazol-4-yl)-amide, substituting 3-pyridinylboronic acid for potassium 1-(tetrahydro-2H-pyran-2-yl)-1H-pyrazole-4-trifluoroborate.

### Example N

### 6-[1-(Tetrahydro-pyran-2-yl)-1H-pyrazol-4-yl]-1-(2-trimethylsilanyl-ethoxymethyl)-1H-indazole-3-carboxylic acid [1-(3-bromo-benzyl)-1H-pyrazol-4-yl]-amide

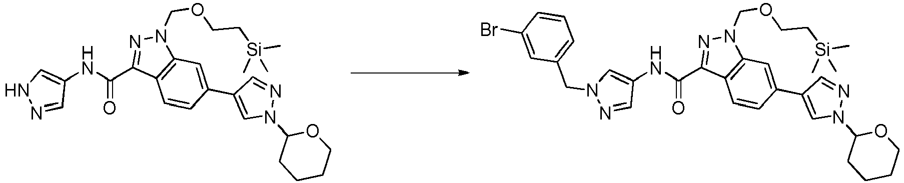

To a solution of 6-[1-(Tetrahydro-pyran-2-yl)-1H-pyrazol-4-yl]-1-(2-trimethylsilanyl-ethoxymethyl)-1H-indazole-3-carboxylic acid **(1H-pyrazol-4-yl)-amide** (200.0 mg, 0.3940 mmol) in N,N-Dimethylformamide (1 mL) was added 3-bromobenzyl bromide (128.0 mg, 0.5122 mmol) and Cesium Carbonate (166.9 mg, 0.5122 mmol) and the mixture was stirred overnight at rt. The mixture was diluted with 50 mL EtOAc and washed with 2 x 50 mL 1:1 H₂O:brine. The organic extracts were dried (Na₂SO₄) and concentrated in vacuo. Purification by CombiFlash (12 g; dry load; 70:30 to 30:70 heptane:EtOAc over 16 minutes; 2 mixed fractions at start of product peak not saved) provided 173 mg (0.256 mmol; 65%) of the desired product as a white solid.

### Example O

### N-[1-[(2-chlorothiazol-4-yl)methyl]pyrazol-4-yl]-6-(1-tetrahydropyran-2-ylpyrazol-4-yl)-1-(2-trimethylsilylethoxymethyl)indazole-3-carboxamide

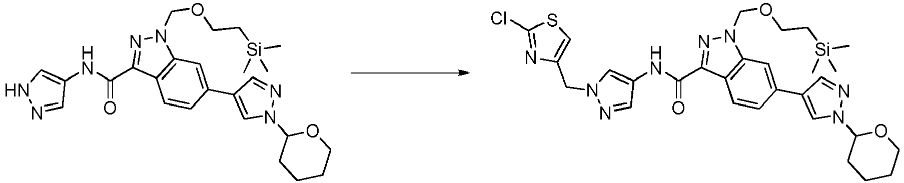

Prepared in an analogous manner to 6-[1-(Tetrahydro-pyran-2-yl)-1H-pyrazol-4-yl]-1-(2-trimethylsilanyl-ethoxymethyl)-1H-indazole-3-carboxylic acid [1-(3-bromo-benzyl)-1H-pyrazol-4-yl]-amide, substituting 2-chloro-4-(chloromethyl)thiazole for 3-bromobenzyl bromide.

### Example P

### 6-Bromo-1-(2-trimethylsilanyl-ethoxymethyl)-1H-indazole-3-carboxylic acid (1-thiazol-4-ylmethyl-1H-pyrazol-4-yl)-amide

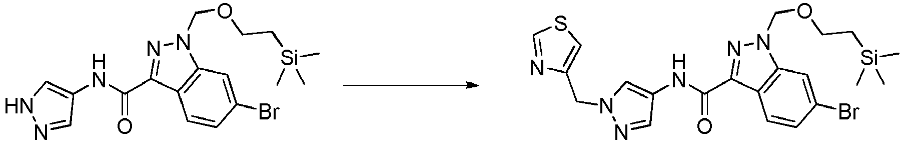

To a solution of 6-Bromo-1-(2-trimethylsilanyl-ethoxymethyl)-1H-indazole-3-carboxylic acid **(1H-pyrazol-4-yl)-amide** (251 mg, 0.575 mmol) in N,N-Dimethylformamide (2 mL) was added 4-(chloromethyl)thiazole hydrochloride (147 mg, 0.863 mmol) and cesium carbonate (562 mg, 1.72 mmol) and the mixture was stirred for 72 hours. LCMS showed >80% conversion, so an additional 0.5 equiv. of chloride and 1.0 equiv. of base were added, and the mixture was stirred for an additional 20 hours at rt and 12 hours at 50 °C. After cooling to rt, the mixture was diluted with 50 mL EtOAc and washed with 2 x 50 mL 1:1 H₂0:brine. The organic extracts were dried (Na₂SO₄) and concentrated in vacuo. Purification by CombiFlash (12 g; dry load; 50:50 to 0:100 heptane:EtOAc over 16 minutes) provided the title compound (146 mg; 0.274 mmol; 48%).

### Example Q N-(1-benzyl-1H-pyrazol-4-yl)-6-bromo-1-((2-(trimethylsilyl)ethoxy)methyl)-1H-indazole-3-carboxamide

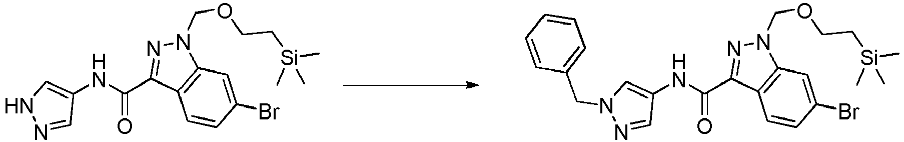

Prepared in an analogous manner to 6-Bromo-1-(2-trimethylsilanyl-ethoxymethyl)-1H-indazole-3-carboxylic acid (1-thiazol-4-ylmethyl-1H-pyrazol-4-yl)-amide, substituting benzyl bromide for 4-(chloromethyl)thiazole hydrochloride, and reducing number of equivalents of base by 1.0.

### Example R

### N-(1-(4-formylbenzyl)-1H-pyrazol-4-yl)-1-trityl-1H-indazole-3-carboxamide

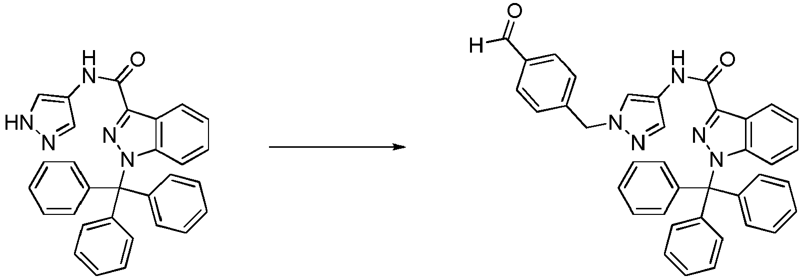

Prepared in an analogous manner to 6-[1-(Tetrahydro-pyran-2-yl)-1H-pyrazol-4-yl]-1-(2-trimethylsilanyl-ethoxymethyl)-1H-indazole-3-carboxylic acid [1-(3-bromo-benzyl)-1H-pyrazol-4-yl]-amide, substituting N-(1H-pyrazol-4-yl)-1-trityl-1H-indazole-3-carboxamide for 6-[1-(Tetrahydro-pyran-2-yl)-1H-pyrazol-4-yl]-1-(2-trimethylsilanyl-ethoxymethyl)-1H-indazole-3-carboxylic acid **(1H-pyrazol-4-yl)-amide** and 4-(bromomethyl)benzaldehyde for 3-bromobenzyl bromide.

### Example S

### N-(1-(3-formylbenzyl)-1H-pyrazol-4-yl)-1-trityl-1H-indazole-3-carboxamide

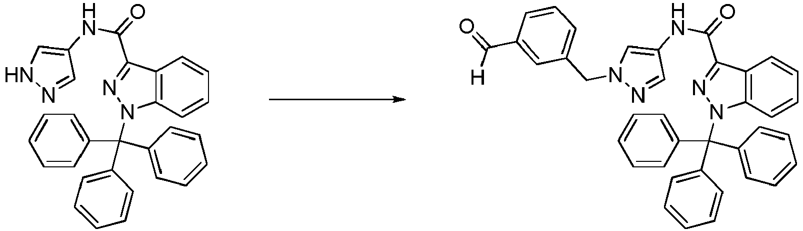

Prepared in an analogous manner to 6-[1-(Tetrahydro-pyran-2-yl)-1H-pyrazol-4-yl]-1-(2-trimethylsilanyl-ethoxymethyl)-1H-indazole-3-carboxylic acid [1-(3-bromo-benzyl)-1H-pyrazol-4-yl]-amide, substituting N-(1H-pyrazol-4-yl)-1-trityl-1H-indazole-3-carboxamide for 6-[1-(Tetrahydro-pyran-2-yl)-1H-pyrazol-4-yl]-1-(2-trimethylsilanyl-ethoxymethyl)-1H-indazole-3-carboxylic acid **(1H-pyrazol-4-yl)-amide** and 3-(bromomethyl)benzaldehyde for 3-bromobenzyl bromide.

### Example T

### 1-(3-Bromopropyl)-4-nitro-1H-pyrazole

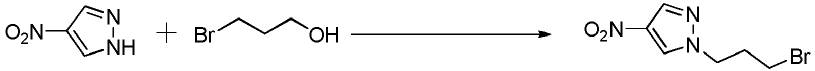

DIAD (5.4 g, 0.03 mol) was added slowly to a solution of 4-nitro-1*H*-pyrazole (2.0 g, 0.02 mol), 3-bromopropan-1-ol (3.6 g, 0.03 mol) and PPh₃ (7.0 g, 0.03 mol) in THF (200 mL). After the mixture was stirred at r.t. for 3 h. It was concentrated, and purified by silica gel chromatography (PE : EtOAc = 2:1) to give the titled compound (crude, purity = 50%) as a white solid (4.1 g, 44%).

### Example U

### 1-(2-Bromoethyl)-4-nitro-1H-pyrazole

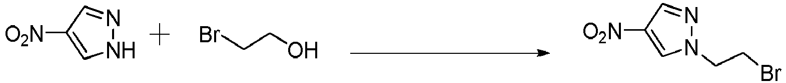

Prepared in an analogous manner to 1-(3-Bromopropyl)-4-nitro-1*H*-pyrazole, substituting 2-bromoethanol for 3-bromopropan-1-ol.

### Example V

### Ethyl 4-(4-nitro-1H-pyrazol-1-yl)butanoate

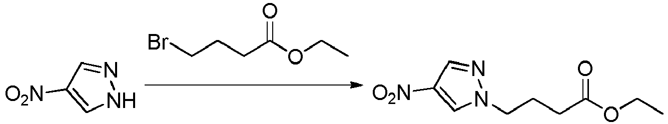

To a solution of 4-nitro-1*H*-pyrazole (5.0 g, 44.3 mmol) in THF (100 mL) was added NaH (2.3 g, 57.5 mmol) at 0 °C. After the mixture was stirred at r.t. for 1 h, 4-bromo-butyric acid ethyl ester (12.9 g, 66.4 mmol) was added. After the reaction mixture was stirred at r.t. overnight, it was poured into water, concentrated and extracted with EtOAc. It was dried, concentrated, and purified by column chromatography (hexanes: EtOAc = 10:1) to give the titled compound (10 g, 99%).

### Example W

### 4-(4-Nitro-1H-pyrazol-1-yl)butan-1-ol

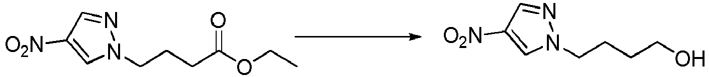

To a solution of LAH (0.60 g, 17.6 mmol) in THF was added 4-(4-nitro-pyrazol-1-yl)-butyric acid ethyl ester (2.0 g, 8.8 mmol) in THF (10 mL), and the mixture was stirred at 0 °C for 1 h. After TLC showed that the starting material was consumed, EtOAc (100 mL) and water (1 mL) were added. After 10 min, the mixture was filtered though a Celite pad and concentrated. It was purified by column chromatography (hexanes:EtOAc = 6:1) to give the titled compound (0.50 g, 30.3%).

### Example X

### 1-(4-Chlorobutyl)-4-nitro-1H-pyrazole

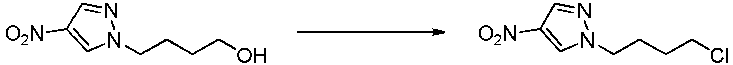

4-(4-Nitro-1*H-*pyrazol-1-yl)butan-1-ol (0.50 g, 2.7 mmol) was heated with SOCl₂ at reflux for 3 h. After it was concentrated, it was purified by silica gel chromatography (PE:EtOAc = 3:1) to give the titled compound (0.30 g, 54.7 %).

### Example Y

### 1-(3-(4-Nitro-1H-pyrazol-1-yl)propyl)piperidin-3-ol

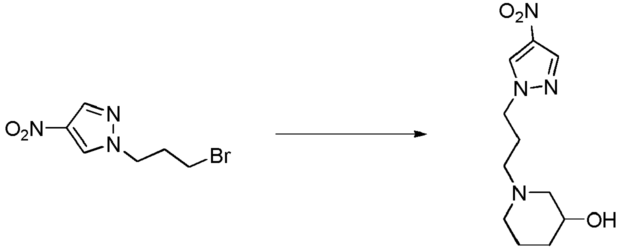

A mixture of 1-(3-bromopropyl)-4-nitro-1*H*-pyrazole (2.2 g, 0.01 mol), piperidin-3-ol (13.0 g, 0.1 mol) in DMF/H₂O (1:1) was mixed with Cs₂CO₃ (31.0 g, 0.1 mol) and stirred at r.t. for 12 h. LC-MS indicated that the reaction was completed. The mixture was extracted with EtOAc 3 times. The organic layers was combined, concentrated, and purified by silica gel chromatography (DCM : MeOH = 10:1) to give the titled compound (2.4 g, 95%).

### Example Z

### 1-(2-(4-Nitro-1H-pyrazol-1-yl)ethyl)piperidin-3-ol.

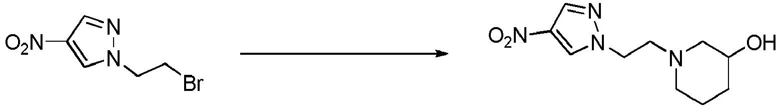

Prepared in an analogous manner to 1-(3-(4-Nitro-1*H*-pyrazol-1-yl)propyl)piperidin-3-ol, substituing 1-(2-bromoethyl)-4-nitro-1*H*-pyrazole for 1-(3-bromopropyl)-4-nitro-1*H*-pyrazole.

### Example AA

### 1-(4-(4-Nitro-1H-pyrazol-1-yl)butyl)piperidin-3-ol

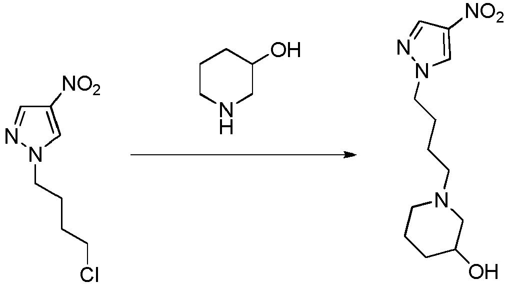

Prepared in an analogous manner to 1-(3-(4-Nitro-1*H*-pyrazol-1-yl)propyl)piperidin-3-ol, substituing 1-(4-Chlorobutyl)-4-nitro-1*H*-pyrazole for 1-(3-bromopropyl)-4-nitro-1*H*-pyrazole and increasing temperature to 80 °C.

### Example AB

### 1-(3-(4-Amino-1H-pyrazol-1-yl)propyl)piperidin-3-ol

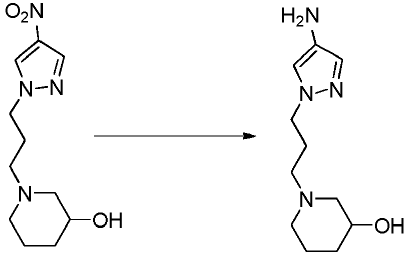

After a mixture of 1-(3-(4-nitro-1*H*-pyrazol-1-yl)propyl)piperidin-3-ol (2.4 g, 0.01 mol) in MeOH (100 mL) and Raney Ni was stirred at r.t. for 1 h, Raney Ni was removed by filtration and the solution was concentrated to give the crude product which was used directly in the next step without further purification.

### Example AC

### 1-(2-(4-Amino-1H-pyrazol-1-yl)ethyl)piperidin-3-ol

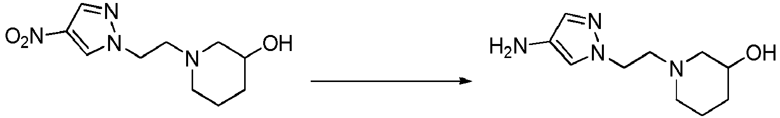

Prepared in an analogous manner to 1-(3-(4-Amino-1*H*-pyrazol-1-yl)propyl)piperidin-3-ol, substituting 1-(2-(4-nitro-1*H-*pyrazol-1-yl)ethyl)piperidin-3-ol for 1-(3-(4-nitro-1*H*-pyrazol-1-yl)propyl)piperidin-3-ol.

### Example AD

### 1-(4-(4-Amino-1H-pyrazol-1-yl)butyl)piperidin-3-ol

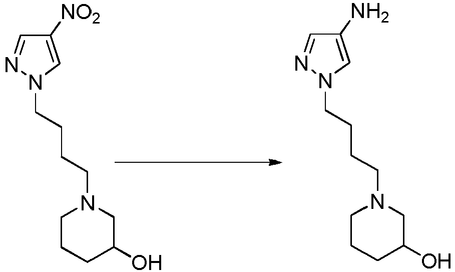

Prepared in an analogous manner to 1-(3-(4-Amino-1*H*-pyrazol-1-yl)propyl)piperidin-3-ol, substituting 1-(4-(4-nitro-1*H*- pyrazol-1-yl)butyl)piperidin-3-ol for 1-(3-(4-nitro- 1*H*-pyrazol-1-yl)propyl)piperidin-3-ol.

### Example AE

### 3-(Prop-1-en-2-yl)benzonitrile

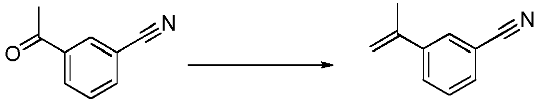

A solution of *n*-BuLi (20.0 mL, 2.5 M, 50.0 mmol) was added to a solution of PPh₃MeI (20.2 g, 50.0 mmol) in THF (200 mL) at -10 °C. After the mixture was stirred at -10 °C for 1 h, 3-acetyl-benzonitrile (4.85 g, 33.4 mmol) was added. The mixture was allowed to warm up to r.t. and stirred at r.t. for 3 h. Water (400 mL) was added to the reaction mixture and it was extracted with CH₂Cl₂ (200 mL × 2). It was dried over anhydrous sodium sulfate, and purified by column chromatography (PE: EtOAc = 50:1) to give the titled compound (3.4 g, 79%).

### Example AF

### 3-(2-(4-Nitro-1H-pyrazol-1-yl)propan-2-yl)benzonitrile

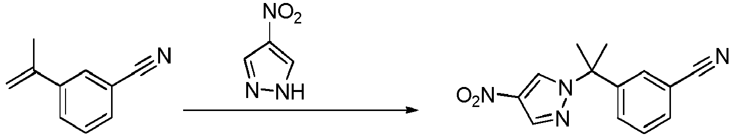

To a solution of iodine (89 mg, 0.35 mmol) and 3-isopropenyl-benzonitrile (0.50 g, 3.5 mmol) in toluene (10 mL) was added 4-nitro-1*H*-pyrazole (0.40 g, 3.5 mmol). After the mixture was stirred at 110°C for 24 h, it was cooled to r.t. and quenched with saturated sodium sulfite solution, extracted with EtOAc, dried over anhydrous sodium sulfate, and purified by column chromatography (PE:EtOAc = 4:1) to give the titled compound (0.10 g, 12%).

### Example AG

### 4-Nitro-1-(2-phenylpropan-2-yl)-1H-pyrazole

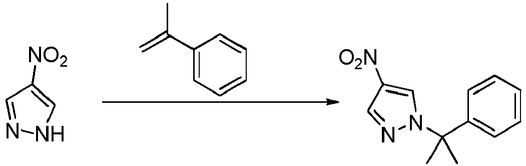

A solution of 4-nitro-1*H*-pyrazole (1.0 g, 8.8 mmol) and prop-1-en-2-ylbenzene (1.0 g, 8.8 mmol) in DCE (100 mL) was mixed with FeCl₃ and heated at 80 °C for 20 h. It was concentrated and purified by silica gel chromatography to give the titled compound (90 mg, 4.4%).

### Example AH

### 3-(2-(4-Amino-1H-pyrazol-1-yl)propan-2-yl)benzonitrile

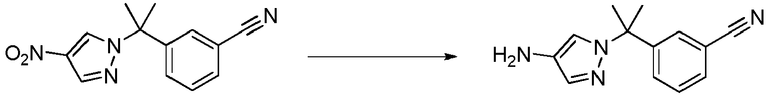

To a solution of 3-(2-(4-nitro-1*H*-pyrazol-1-yl)propan-2-yl)benzonitrile (1.1 g, 4.3 mmol) and NH₄Cl (2.3 g, 43 mmol) in EtOH (50 mL) and H₂O (5 mL) was added Fe (2.4 g, 43 mmol). After the mixture was stirred at 70 °C for 1 h, it was cooled to r.t., filtered, and the filtrate was purified by column chromatography (CH₂Cl₂: MeOH = 10:1) to give the titled compound (0.84 g, 87%).

### Example AI

### 1-(2-Phenylpropan-2-yl)-1H-pyrazol-4-amine

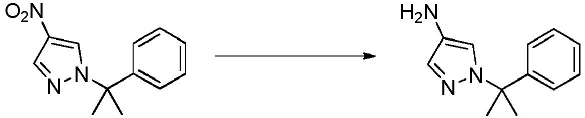

Prepared in an analogous manner to 3-(2-(4-Amino-1*H*-pyrazol-1-yl)propan-2-yl)benzonitrile, substituting 4-nitro-1-(2-phenylpropan-2-yl)-1*H*-pyrazole for 3-(2-(4-nitro-1*H*-pyrazol-1-yl)propan-2-yl)benzonitrile.

### Example AJ

### (S)-6-bromo-N-(1-(1-phenylpropyl)-1H-pyrazol-4-yl)-1-((2-(trimethylsilyl)ethoxy)methyl)-1H-indazole-3-carboxamide

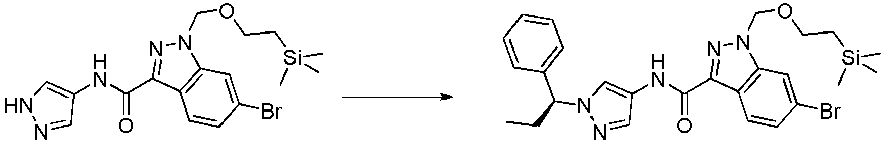

To a solution of 6-bromo-N-(1H-pyrazol-4-yl)-1-((2-(trimethylsilyl)ethoxy)methyl)-1H-indazole-3-carboxamide (1 mmol) in THF (5 mL) was added tributylphosphine (3.3 mmol), (R)-1-phenylpropan-1-ol (3.3 mmol), and diamide (3.3 mmol). The mixture was stirred for one hour at 70 °C then the mixture was diluted with 300 mL EtOAc and washed with 2 x 200 mL 1:1 H₂O:brine. The organic extracts were dried (Na₂SO₄) and concentrated in vacuo. Purification by CombiFlash (40 g column; 50:50 to 0:100 heptane:EtOAc over 15 minutes) provided the titled compound.

### Product Examples

### Example 1: 5-chloro-N-(1-(3-cyanobenzyl)-1H-pyrazol-4-yl)-1H-indazole-3-carboxamide

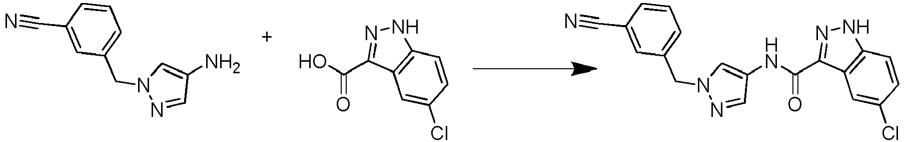

5-chloro-1H-indazole-3-carboxylic acid (49.1 mg, 0.25 mmol) and crude 3-((4-amino-1H-pyrazol-1-yl)methyl)benzonitrile (59.5 mg, 0.30 mmol) were weighed into a reaction vial, and then dichloromethane (0.50 mL) and MF (0.5 mL) were added. HOBt hydrate (57.4 mg, 0.375 mmol) was then added followed by EDCI (71.9 mg, 0.375 mmol). The reaction was stirred at room temperature until no further reaction was observed in LC-MS. The mixture was dissolved in enough DMF and then purified by reverse-phase HPLC to give the desired product (17.3 mg, 18.4%). ¹H NMR (400 MHz, DMSO) δ 13.93 (s, 1H), 10.71 (s, 1H), 8.26 (s, 1H), 8.19 (d, *J*= 1.6 Hz, 1H), 7.83 - 7.76 (m, 1H), 7.75 (s, 1H), 7.73 - 7.68 (m, *J*= 8.4 Hz, 2H), 7.60 - 7.54 (m, *J* = 4.9, 1.9 Hz, 2H), 7.46 (dd, *J*= 8.9, 2.0 Hz, 1H), 5.40 (s, 2H). MS: *m*/*z* = 377.0 (M+H)⁺.

### Example 2: N-(1-(3-cyanobenzyl)-1H-pyrazol-4-yl)-5-methyl-1H-indazole-3-carboxamide

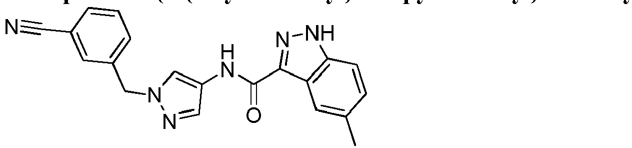

The title compound was synthesized according to Example 1 by substituting 5-chloro-1H-indazole-3-carboxylic acid with 5-methyl-1H-indazole-3-carboxylic acid. ¹H NMR (400 MHz, DMSO) δ 13.58 (s, 1H), 10.54 (s, 1H), 8.24 (s, 1H), 8.00 (s, 1H), 7.83 - 7.73 (m, 2H), 7.71 (s, 1H), 7.62 - 7.55 (m, 2H), 7.53 (d, *J*= 8.5 Hz, 1H), 7.27 (d, *J=* 8.7 Hz, 1H), 5.40 (s, 2H), 2.44 (s, 3H). MS: *m*/*z =* 357.1 (M+H)⁺.

### Example 3: N-(1-(3-cyanobenzyl)-1H-pyrazol-4-yl)-6-fluoro-1H-indazole-3-carboxamide

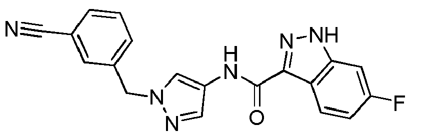

The title compound was synthesized according to Example 1 by substituting 5-chloro-1H-indazole-3-carboxylic acid with 6-fluoro-1H-indazole-3-carboxylic acid. ¹H NMR (400 MHz, DMSO) δ 13.75 (s, 1H), 10.65 (s, 1H), 8.26 (s, 1H), 8.21 (dd, *J*= 8.9, 5.4 Hz, 1H), 7.78 (dd, *J =* 6.4, 2.3 Hz, 1H), 7.75 (s, 1H), 7.70 (s, 1H), 7.58 (dd, *J=* 9.3, 4.4 Hz, 2H), 7.44 (dd, *J*= 9.4, 1.9 Hz, 1H), 7.16 (td, *J=* 9.4, 2.2 Hz, 1H), 5.40 (s, 2H). MS: *m*/*z* = 361.1 (M+H)⁺.

### Example 4: N-(1-(3-cyanobenzyl)-1H-pyrazol-4-yl)-4-methoxy-1H-indazole-3-carboxamide

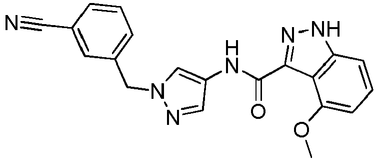

The title compound was synthesized according to Example 1 by substituting 5-chloro-1H-indazole-3-carboxylic acid with 4-methoxy-1H-indazole-3-carboxylic acid. ¹H NMR (400 MHz, DMSO) δ 13.52 (s, 1H), 10.47 (s, 1H), 8.26 (s, 1H), 7.78 (s, 1H), 7.72 (s, 1H), 7.66 (s, 1H), 7.58 (d, *J=* 4.0 Hz, 2H), 7.34 (t, *J =* 8.0 Hz, 1H), 7.17 (d, *J* = 8.3 Hz, 1H), 6.68 (*d*, *J* = 7.6 Hz, 1H), 5.40 (s, 2H), 3.92 (s, 3H). MS: *m*/*z =* 373.1 (M+H)⁺.

### Example 5: N-(1-(3-cyanobenzyl)-1H-pyrazol-4-yl)-7-methoxy-1H-indazole-3-carboxamide

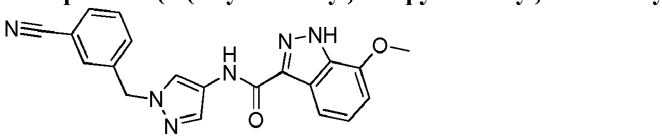

The title compound was synthesized according to Example 1 by substituting 5-chloro-1H-indazole-3-carboxylic acid with 7-methoxy-1H-indazole-3-carboxylic acid. ¹H NMR (400 MHz, DMSO) δ 13.90 (s, 1H), 10.56 (s, 1H), 8.25 (s, 1H), 7.83 - 7.67 (m, 4H), 7.60 - 7.54 (m, *J*= 5.2 Hz, 2H), 7.22 - 7.11 (m, *J*= 7.8 Hz, 1H), 6.95 - 6.84 (m, *J*= 7.4 Hz, 1H), 5.39 (s, 2H), 3.99 (s, 3H). MS: *m*/*z* = 373.1 (M+H)⁺.

### Example 6: N-(1-(3-cyanobenzyl)-1H-pyrazol-4-yl)-6-methoxy-1H-indazole-3-carboxamide

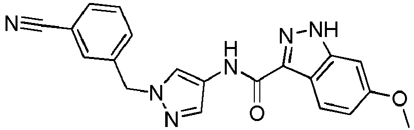

The title compound was synthesized according to Example 1 by substituting 5-chloro-1H-indazole-3-carboxylic acid with 6-methoxy-1H-indazole-3-carboxylic acid. ¹H NMR (400 MHz, DMSO) δ 13.43 (s, 1H), 10.52 (s, 1H), 8.25 (s, 1H), 8.04 (d, *J*= 8.9 Hz, 1H), 7.81 - 7.75 (m, *J =* 4.2 Hz, 1H), 7.74 (s, 1H), 7.70 (s, 1H), 7.61 - 7.53 (m, 2H), 6.99 (d, *J*= 1.9 Hz, 1H), 6.90 (dd, *J* = 8.9, 2.1 Hz, 1H), 5.39 (s, 2H), 3.85 (s, 3H). MS: *m*/*z =* 373.1 (M+H)⁺.

### Example 7: N-(1-(3-cyanobenzyl)-1H-pyrazol-4-yl)-5-fluoro-1H-indazole-3-carboxamide

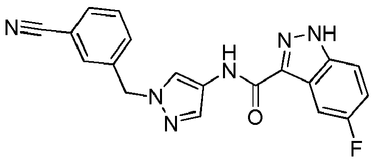

The title compound was synthesized according to Example 1 by substituting 5-chloro-1H-indazole-3-carboxylic acid with 5-fluoro-1H-indazole-3-carboxylic acid. ¹H NMR (400 MHz, DMSO) δ 14.24 - 13.37 (m, 1H), 10.61 (s, 1H), 8.25 (s, 1H), 7.84 (d, *J*= 9.0 Hz, 1H), 7.81 - 7.76 (m, *J*= 4.0 Hz, 1H), 7.75 (s, 1H), 7.73 - 7.66 (m, 2H), 7.58 (d, *J*= 6.3 Hz, 2H), 7.34 (t, *J =* 9.1 Hz, 1H), 5.40 (s, 2H). MS: *m*/*z =* 361.0 (M+H)⁺.

### Example 8: N-(1-(3-cyanobenzyl)-1H-pyrazol-4-yl)-5-methoxy-1H-indazole-3-carboxamide

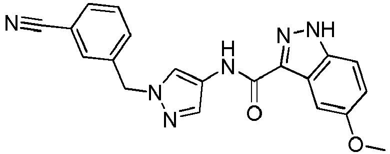

The title compound was synthesized according to Example 1 by substituting 5-chloro-1H-indazole-3-carboxylic acid with 5-methoxy-1H-indazole-3-carboxylic acid. ¹H NMR (400 MHz, DMSO) δ 13.57 (s, 1H), 10.51 (s, 1H), 8.25 (s, 1H), 7.82 - 7.76 (m, *J=* 4.5 Hz, 1H), 7.75 (s, 1H), 7.71 (s, 1H), 7.61 (s, 1H), 7.58 (d, *J=* 4.7 Hz, 2H), 7.55 (d, *J*= 9.0 Hz, 1H), 7.09 (d, *J*= 9.0 Hz, 1H), 5.39 (s, 2H), 3.82 (s, 3H). MS: *m*/*z =* 373.0 (M+H)⁺.

### Example 9: 6-(1H-Pyrazol-4-yl)-1H-indazole-3-carboxylic acid (1-quinolin-8-ylmethyl-1H-pyrazol-4-yl)-amide

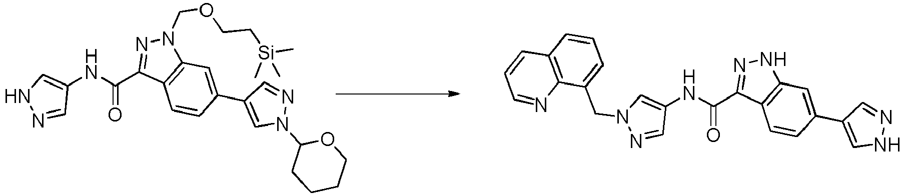

To a solution of 6-[1-(Tetrahydro-pyran-2-yl)-1H-pyrazol-4-yl]-1-(2-trimethylsilanyl-ethoxymethyl)-1H-indazole-3-carboxylic acid **(1H-pyrazol-4-yl)-amide** (40.0 mg, 0.0788 mmol) in N,N-Dimethylformamide (1 mL) was added 8-Bromomethyl-quinoline (20.0 mg, 0.0900 mmol) and Cesium Carbonate (30.8 mg, 0.0946 mmol) and the mixture was stirred for 24 hours. The mixture was diluted with 5 mL CH₂Cl₂ and 5 mL brine, filtered through a phase separator to remove the aqueous layer and concentrated in vacuo. The residue was diluted with 1.0 mL trifluoroacetic acid, then triisopropylsilane (80.9 µL, 0.394 mmol) and a few drops of CH₂Cl₂ were added. The mixture was stirred for 2 hours at rt. The mixture was concentrated in vacuo, then purified by automated reverse-phase HPLC, which provided 11 mg (32%) of the title compound. ¹H NMR (400 MHz, DMSO) δ 13.59 (s, 1H), 12.98 (s, 1H), 10.53 (s, 1H), 9.03 (d, *J* = 4.1 Hz, 1H), 8.43 (d, *J* = 8.3 Hz, 1H), 8.21 (s, 1H), 8.14 (d, *J* = 8.4 Hz, 1H), 7.97 (d, *J* = 8.1 Hz, 1H), 7.84 (t*, J*=7.2 Hz, 2H), 7.77 (s, 1H), 7.75 (s, 1H), 7.65-7.52 (m, 4H), 7.34 (d, *J*= 7.0 Hz, 1H), 5.96 (s, 2H). MS: *m*/*z =* 435.2 (M+H)⁺.

### Example 10: 3-(4-{[6-(1H-Pyrazol-4-yl)-1H-indazole-3-carbonyl]-amino}-pyrazol-1-ylmethyl)-benzoic acid methyl ester

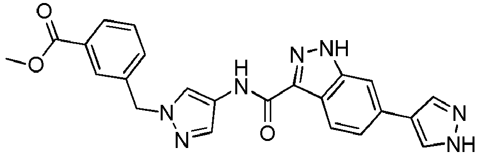

The title compound was synthesized according to Example 9, substituting Methyl 3-(bromomethyl)benzoate for 8-Bromomethyl-quinoline. ¹H NMR (400 MHz, DMSO) δ 13.60 (s, 1H), 12.99 (s, 1H), 10.56 (s, 1H), 8.24 (s, 1H), 8.21-8.11 (m, 3H), 7.92 - 7.85 (m, 2H), 7.76 (s, 1H), 7.74 (s, 1H), 7.58-7.49 (m, 3H), 5.41 (s, 2H), 3.85 (s, 3H). MS: *m*/*z* = 442.2 (M+H)⁺.

### Example 11: 6-(1H-Pyrazol-4-yl)-1H-indazole-3-carboxylic acid (1-phenethyl-1H-pyrazol-4-yl)-amide

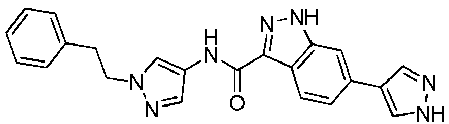

The title compound was synthesized according to Example 9, substituting 1-Bromo-2-phenylethane for 8-Bromomethyl-quinoline. ¹H NMR (400 MHz, DMSO) δ 13.59 (s, 1H), 12.99 (s, 1H), 10.48 (s, 1H), 8.31 (s, 1H), 8.15 (*d, J*= 8.5 Hz, 1H), 8.10-7.98 (m, 2H), 7.75 (s, 1H), 7.71 (s, 1H), 7.56 (d, *J=* 8.5 Hz, 1H), 7.32 - 7.25 (m, 2H), 7.23 - 7.16 (m, 3H), 4.33 (t, *J=* 7.3 Hz, 2H), 3.11 (t, *J*= 7.3 Hz, 2H). MS: *m*/*z* = 398.2 (M+H)⁺.

### Example 12: 6-(1H-Pyrazol-4-yl)-1H-indazole-3-carboxylic acid [1-(4-carbamoyl-benzyl)-1H-pyrazol-4-yl]-amide

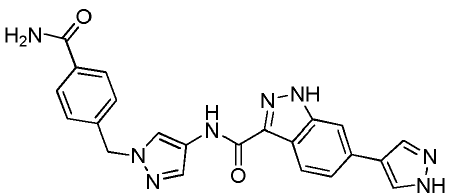

The title compound was synthesized according to Example 9, substituting 4-Bromomethyl-benzamide for 8-Bromomethyl-quinoline. MS: *m*/*z* = 427.1 (M+H)⁺.

### Example 13: 6-(1H-Pyrazol-4-yl)-1H-indazole-3-carboxylic acid {1-[2-(3,5-dimethyl-1H-pyrazol-4-yl)-ethyl]-1H-pyrazol-4-yl}-amide

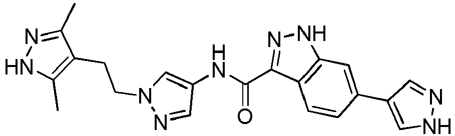

The title compound was synthesized according to Example 9, substituting 4-(2-Bromo-ethyl)-3,5-dimethyl-1H-pyrazole for 8-Bromomethyl-quinoline. MS: *m*/*z* = 416.2 (M+H)⁺.

### Example 14: 6-(1H-Pyrazol-4-yl)-1H-indazole-3-carboxylic acid (1-1,2-benzisoxazol-3-ylmethyl-1H-pyrazol-4-yl)-amide

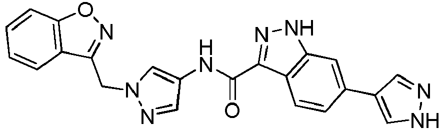

The title compound was synthesized according to Example 9, substituting 3-Bromomethyl-1,2-benzisoxazole for 8-Bromomethyl-quinoline. ¹H NMR (400 MHz, DMSO) δ 12.99 (s, 1H), 10.60 (s, 1H), 8.37 (s, 1H), 8.20-8.13 (m, 3H), 7.79-7.74 (m, 3H), 7.65 (t, *J=* 7.8 Hz, 1H), 7.59 (d, *J=* 8.0 Hz, 1H), 7.56 (d, *J=* 8.5 Hz, 1H), 7.37 (t, *J=* 7.5 Hz, 1H), 5.84 (s, 2H). MS: *m*/*z =* 425.1 (M+H)⁺.

### Example 15: 6-(1H-Pyrazol-4-yl)-1H-indazole-3-carboxylic acid [1-(5-methylcarbamoyl-furan-2-ylmethyl)-1H-pyrazol-4-yl]-amide

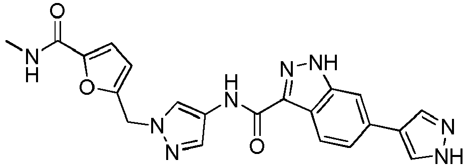

The title compound was synthesized according to Example 9, substituting 5-Chloromethyl-furan-2-carboxylic acid methylamide for 8-Bromomethyl-quinoline. ¹H NMR (400 MHz, DMSO) δ 13.61 (s, 1H), 10.56 (s, 1H), 8.28 - 8.11 (m, 5H), 7.76 (s, 1H), 7.73 (s, 1H), 7.56 (*d*, *J*= 8.5 Hz, 1H), 7.03 (d, *J*= 3.4 Hz, 1H), 6.56 (d, *J*= 3.4 Hz, 1H), 5.38 (s, 2H), 2.72 (d, *J*= 4.6 Hz, 3H). MS: *m*/*z* = 431.2 (M+H)⁺.

### Example 16: 6-(1H-Pyrazol-4-yl)-1H-indazole-3-carboxylic acid [1-(5-phenyl-isoxazol-3-ylmethyl)-1H-pyrazol-4-yl]-amide

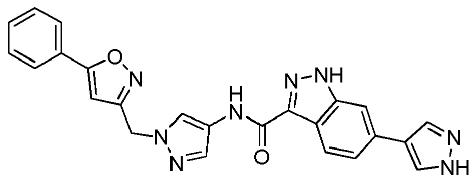

The title compound was synthesized according to Example 9, substituting 4-(2-Bromo-ethyl)-3,5-dimethyl-1H-pyrazole for 8-Bromomethyl-quinoline. ¹H NMR (400 MHz, DMSO) δ 13.63 (s, 1H), 13.00 (s, 1H), 10.60 (s, 1H), 8.36-8.22 (m, 2H), 8.17 (d, *J* = 8.5 Hz, 1H), 8.05 (s, 1H), 7.87 (d, *J* = 5.9 Hz, 2H), 7.77 (d, *J* = 8.9 Hz, 2H), 7.57 (d, *J* = 8.7 Hz, 1H), 7.54-7.47 (m, 3H), 6.90 (s, 1H), 5.49 (s, 2H). MS: *m*/*z* = 451.2 (M+H)⁺.

### Example 17: 6-(1H-Pyrazol-4-yl)-1H-indazole-3-carboxylic acid (1-benzothiazol-2-ylmethyl-1H-pyrazol-4-yl)-amide

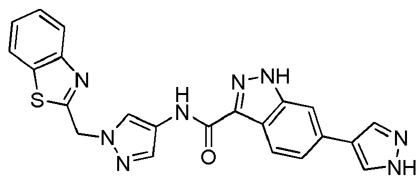

The title compound was synthesized according to Example 9, substituting 2-Bromomethyl-benzothiazole for 8-Bromomethyl-quinoline. ¹H NMR (400 MHz, DMSO) δ 13.64 (s, 1H), 13.00 (s, 1H), 10.65 (s, 1H), 8.37 (s, 1H), 8.29 (s, 1H), 8.16 (d, *J* = 8.5 Hz, 1H), 8.06 (d, *J* = 7.9 Hz, 1H), 8.01 (d, *J* = 8.1 Hz, 1H), 7.85 (s, 1H), 7.77 (s, 1H), 7.60 - 7.50 (m, 2H), 7.44 (t, *J* = 7.6 Hz, 1H), 5.85 (s, 2H). MS: *m*/*z* = 441.2 (M+H)⁺.

### Example 18: 6-(1H-Pyrazol-4-yl)-1H-indazole-3-carboxylic acid [1-(5-morpholin-4-ylmethyl-isoxazol-3-ylmethyl)-1H-pyrazol-4-yl]-amide

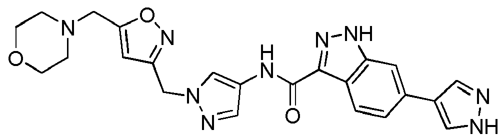

The title compound was synthesized according to Example 9, substituting 4-(3-Chloromethyl-isoxazol-5-ylmethyl)-morpholine for 8-Bromomethyl-quinoline. MS: *m*/*z* = 474.2 (M+H)⁺.

### Example 19: 6-(1H-Pyrazol-4-yl)-1H-indazole-3-carboxylic acid (1-imidazo[1,2-a]pyridin-2-ylmethyl-1H-pyrazol-4-yl)-amide

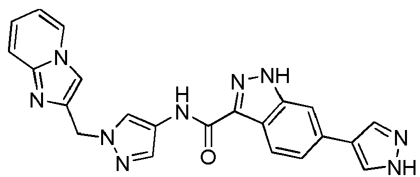

The title compound was synthesized according to Example 9, substituting 4-(3-Chloromethyl-isoxazol-5-ylmethyl)-morpholine for 8-Bromomethyl-quinoline. ¹H NMR (400 MHz, DMSO) δ 13.60 (s, 1H), 12.98 (s, 1H), 10.52 (s, 1H), 8.52 (d, *J* = 6.8 Hz, 1H), 8.23 - 8.11 (m, 4H), 7.82 (s, 1H), 7.75 (s, 1H), 7.72 (s, 1H), 7.55 (d, *J* = 9.6 Hz, 1H), 7.52 (d, *J* = 9.2 Hz, 1H), 7.27 - 7.20 (m, 1H), 6.88 (t, *J* = 6.7 Hz, 1H), 5.41 (s, 2H). MS: *m*/*z* = 424.2 (M+H)⁺.

### Example 20: 6-(1H-Pyrazol-4-yl)-1H-indazole-3-carboxylic acid [1-(2-phenyl-thiazol-4-ylmethyl)-1H-pyrazol-4-yl]-amide

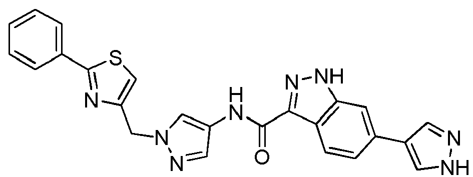

The title compound was synthesized according to Example 9, substituting 4-Chloromethyl-2-phenyl-thiazole for 8-Bromomethyl-quinoline. ¹H NMR (400 MHz, DMSO) δ 13.59 (s, 1H), 12.99 (s, 1H), 10.56 (s, 1H), 8.23 (s, 1H), 8.21-8.10 (m, 3H), 7.96-7.91 (m, 2H), 7.76 (s, 1H), 7.75 (s, 1H),7.58 - 7.46 (m, 5H), 5.47 (s, 2H). MS: *m*/*z* = 467.1 (M+H)⁺.

### Example 21: 6-(1H-Pyrazol-4-yl)-1H-indazole-3-carboxylic acid [1-(2-isopropyl-thiazol-4-ylmethyl)-1H-pyrazol-4-yl]-amide

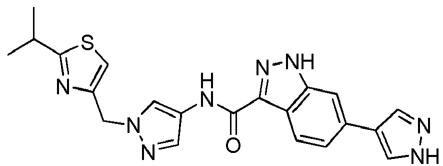

The title compound was synthesized according to Example 9, substituting 4-Chloromethyl-2-isopropyl-thiazole for 8-Bromomethyl-quinoline. ¹H NMR (400 MHz, DMSO) δ 13.60 (s, 1H), 12.99 (s, 1H), 10.54 (s, 1H), 8.31 (s, 1H), 8.17-8.13 (m, 2H), 8.04 (s, 1H), 7.76 (s, 1H), 7.74 (s, 1H), 7.56 (d, *J* = 8.5 Hz, 1H), 7.29 (s, 1H), 5.36 (s, 2H), 3.27 - 3.23 (m, 1H), 1.32 (d, *J* = 6.9 Hz, 6H). MS: *m*/*z* = 433.2 (M+H)⁺.

### Example 22: 6-(1H-Pyrazol-4-yl)-1H-indazole-3-carboxylic acid {1-[3-(4-hydroxy-piperidine-1-carbonyl)-benzyl]-1H-pyrazol-4-yl}-amide

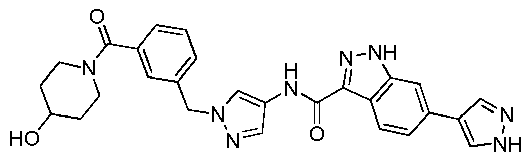

The title compound was synthesized according to Example 9, substituting (3-Chloromethylphenyl)-(4-hydroxy-piperidin-1-yl)-methanone for 8-Bromomethyl-quinoline. ¹H NMR (400 MHz, DMSO) δ 13.60 (s, 1H), 12.99 (s, 1H), 10.55 (s, 1H), 8.31 (s, 1H), 8.22 (s, 1H), 8.15 (d, *J* = 8.5 Hz, 1H), 8.04 (s, 1H), 7.76 (s, 1H), 7.74 (s, 1H), 7.56 (d, *J* = 8.5 Hz, 1H), 7.47 - 7.39 (m, 2H), 7.35-7.27 (m, 2H), 7.24 (s, 1H), 5.37 (s, 2H), 4.80 (s, 1H), 4.75 (dd, *J* = 8.7, 4.0 Hz, 1H), 3.99 (s, 1H), 3.79 - 3.67 (m, 1H), 3.46 (s, 1H), 3.28-3.01 (m, 2H), 1.85-1.60 (m, 2H), 1.45-1.25 (m, 2H). MS: *m*/*z* = 511.2 (M+H)⁺.

### Example 23: 6-(1H-Pyrazol-4-yl)-1H-indazole-3-carboxylic acid [1-(2-thiophen-2-yl-thiazol-4-ylmethyl)-1H-pyrazol-4-yl]-amide

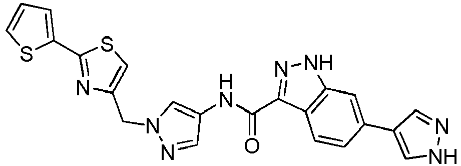

The title compound was synthesized according to Example 9, substituting 4-Chloromethyl-2-thiophen-2-yl-thiazole for 8-Bromomethyl-quinoline. ¹H NMR (400 MHz, DMSO) δ 13.60 (s, 1H), 13.00 (s, 1H), 10.57 (s, 1H), 8.29 (s, 1H), 8.21 (s, 1H), 8.16 (d, *J* = 8.5 Hz, 1H), 8.05 (s, 1H), 7.76 (s, 2H), 7.72 (d, *J* = 5.0 Hz, 1H), 7.67 (d, *J* = 3.7 Hz, 1H), 7.56 (d, *J* = 8.5 Hz, 1H), 7.43 (s, 1H), 7.18 - 7.15 (m, 1H), 5.43 (s, 2H). MS: *m*/*z* = 473.1 (M+H)⁺.

### Example 24: 6-(1H-pyrazol-4-yl)-N-[1-[[2-(2-pyridyl)thiazol-4-yl]methyl]pyrazol-4-yl]-1H-indazole-3-carboxamide

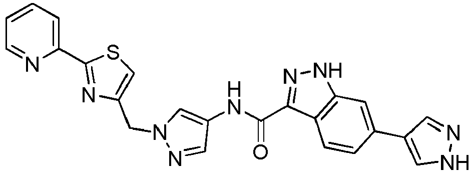

The title compound was synthesized according to Example 9, substituting 4-(3-Chloromethyl-isoxazol-5-ylmethyl)-morpholine for 8-Bromomethyl-quinoline. ¹H NMR (400 MHz, DMSO) δ 13.60 (s, 1H), 12.99 (s, 1H), 10.56 (s, 1H), 8.63 (d, *J* = 4.7 Hz, 1H), 8.31 (s, 1H), 8.23 (s, 1H), 8.16 (d, *J* = 8.5 Hz, 1H), 8.10 (d, *J=* 7.9 Hz, 1H), 8.03 (s, 1H), 7.96 (t, *J* = 7.7 Hz, 1H), 7.76 (s, 2H), 7.62 (s, 1H), 7.56 (d, *J* = 8.5 Hz, 1H), 7.52 - 7.47 (m, 1H), 5.49 (s, 2H). MS: *m*/*z* = 468.1 (M+H)⁺.

### Example 25: N-[1-[(3-ethynylphenyl)methyl]pyrazol-4-yl]-6-(1H-pyrazol-4-yl)-1H-indazole-3-carboxamide

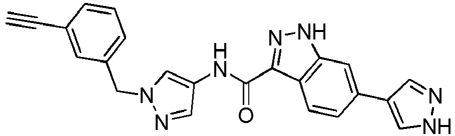

The title compound was synthesized according to Example 9, substituting Methanesulfonic acid 3-ethynyl-benzyl ester for 8-Bromomethyl-quinoline. ¹H NMR (400 MHz, DMSO) δ 13.61 (s, 1H), 12.99 (s, 1H), 10.55 (s, 1H), 8.22 (s, 1H), 8.16 (d, *J* = 8.5 Hz, 1H), 7.76 (s, 1H), 7.74 (s, 1H), 7.56 (d, *J* = 8.5 Hz, 1H), 7.42-7.26 (m, 4H), 5.33 (s, 2H), 4.18 (s, 1H). MS: *m*/*z* = 408.1 (M+H)⁺.

### Example 26: 6-(1H-Pyrazol-4-yl)-1H-indazole-3-carboxylic acid (1-thiazol-4-ylmethyl-1H-pyrazol-4-yl)-amide

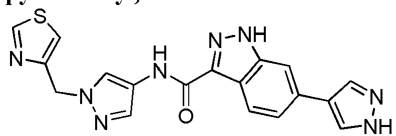

The title compound was synthesized according to Example 9, substituting 4-(chloromethyl)thiazole hydrochloride for 8-Bromomethyl-quinoline, and adding an additional equivalent of cesium carbonate. ¹H NMR (400 MHz, DMSO) δ 13.61 (s, 1H), 12.99 (s, 1H), 10.54 (s, 1H), 9.10 (d, *J* = 1.9 Hz, 1H), 8.36 - 7.96 (m, 4H), 7.76 (s, 1H), 7.72 (s, 1H), 7.58-7.54 (m, 2H), 5.45 (s, 2H). MS: *m*/*z* = 391.2 (M+H)⁺.

### Example 27: 6-(1H-Pyrazol-4-yl)-1H-indazole-3-carboxylic acid [1-(5-cyano-thiophen-2-ylmethyl)-1H-pyrazol-4-yl]-amide

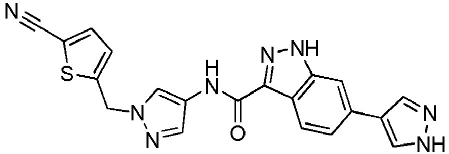

The title compound was synthesized according to Example 9, substituting 5-Bromomethylthiophene-2-carbonitrile for 8-Bromomethyl-quinoline. ¹H NMR (400 MHz, DMSO) δ 13.61 (s, 1H), 12.99 (s, 1H), 10.59 (s, 1H), 8.28 (s, 1H), 8.17 (s, 1H), 8.15 (d, *J* = 8.5 Hz, 1H), 7.85 (d, *J* = 3.8 Hz, 1H), 7.77 (s, 1H), 7.76 (s, 1H), 7.56 (d, *J* = 8.5 Hz, 1H), 7.25 (d, *J* = 3.8 Hz, 1H), 5.64 (s, 2H). MS: *m*/*z* = 415.2 (M+H)⁺.

### Example 28: 6-(1H-Pyrazol-4-yl)-1H-indazole-3-carboxylic acid [1-(2-amino-pyridin-4-ylmethyl)-1H-pyrazol-4-yl]-amide

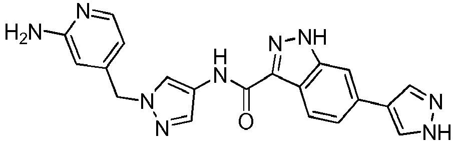

The title compound was synthesized according to Example 9, substituting (4-Bromomethyl-pyridin-2-yl)-carbamic acid tert-butyl ester for 8-Bromomethyl-quinoline. ¹H NMR (400 MHz, DMSO) δ 10.58 (s, 1H), 8.28 (s, 1H), 8.18 (s, 1H), 8.16 (d, *J* = 8.5 Hz, 1H), 7.84 (d, *J*= 5.3 Hz, 1H), 7.76 (s, 2H), 7.56 (d, *J* = 8.5 Hz, 1H), 6.31 (d, *J* = 5.3 Hz, 1H), 6.15 (s, 1H), 5.89 (s, 2H), 5.19 (s, 2H). MS: *mlz =* 400.3 (M+H)⁺.

### Example 29: 6-(1H-Pyrazol-4-yl)-1H-indazole-3-carboxylic acid [1-(3-carbamoyl-benzyl)-1H-pyrazol-4-yl]-amide

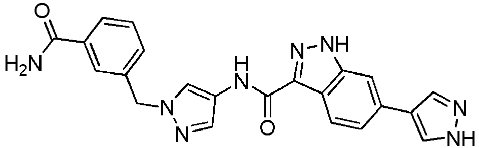

The title compound was synthesized according to Example 9, substituting 3-(chloromethyl)benzamide for 8-Bromomethyl-quinoline. ¹H NMR (400 MHz, DMSO) δ 12.96 (s, 1H), 10.51 (s, 1H), 8.20 (s, 1H), 8.15 (s, 1H), 8.13 (d, *J* = 8.6 Hz, 1H), 7.96 (s, 1H), 7.81 (s, 1H), 7.80 (d, *J* = 9.0 Hz, 1H), 7.75 (s, 1H), 7.73 (s, 1H), 7.52 (d, *J* = 8.4 Hz, 1H), 7.46 - 7.37 (m, 2H), 7.34 (s, 1H), 5.36 (s, 2H). MS: *m*/*z =* 400.3 (M+H)⁺.

### Example 30: 6-(1H-Pyrazol-4-yl)-1H-indazole-3-carboxylic acid [1-(3-cyano-benzyl)-1H-pyrazol-4-yl]-amide

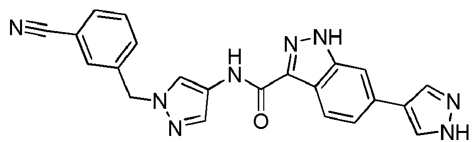

The title compound was synthesized according to Example 9, substituting m-Cyanobenzyl bromide for 8-Bromomethyl-quinoline. ¹H NMR (400 MHz, DMSO) δ 13.61 (s, 1H), 12.99 (s, 1H), 10.57 (s, 1H), 8.36 - 8.23 (m, 2H), 8.16 (d, *J* = 8.5 Hz, 1H), 8.05 (s, 1H), 7.81 - 7.74 (m, 3H), 7.71 (s, 1H), 7.61 - 7.53 (m, 3H), 5.40 (s, 2H). MS: *m*/*z =* 409.3 (M+H)⁺.

### Example 31: N-[1-[(3-carbamoylphenyl)methyl]pyrazol-4-yl]-6-(3-pyridyl)-1H-indazole-3-carboxamide

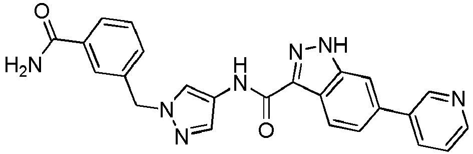

The title compound was synthesized according to Example 9, substituting 3-(chloromethyl)benzamide for 8-Bromomethyl-quinoline and N-(1H-pyrazol-4-yl)-6-(3-pyridyl)-1-(2-trimethylsilylethoxymethyl) indazole-3-carboxamide for 6-[1-(Tetrahydro-pyran-2-yl)-1H-pyrazol-4-yl]-1-(2-trimethylsilanyl-ethoxymethyl)-1H-indazole-3-carboxylic acid (1H-pyrazol-4-yl)-amide. ¹H NMR (400 MHz, DMSO) δ 13.87 (s, 1H), 10.63 (s, 1H), 8.99 (d, J = 2.1 Hz, 1H), 8.61 (d, J = 4.7 Hz, 1H), 8.31 (d, J = 8.5 Hz, 1H), 8.22 (s, 1H), 8.18 (d, J = 8.0 Hz, 1H), 7.96 (s, 1H), 7.91 (s, 1H), 7.80 (m, 2H), 7.75 (s, 1H), 7.62 (d, J = 8.5 Hz, 1H), 7.53 (dd, J = 7.9, 4.8 Hz, 1H), 7.46 - 7.37 (m, 2H), 7.34 (s, 1H), 5.37 (s, 2H). MS: *m*/*z* = 438.3 (M+H)⁺.

### Example 32: N-[1-[(2-amino-4-pyridyl)methyl]pyrazol-4-yl]-6-(3-pyridyl)-1H-indazole-3-carboxamide

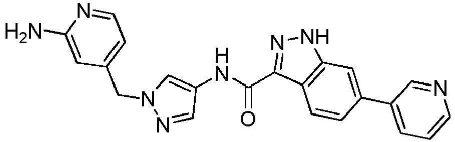

The title compound was synthesized according to Example 9, substituting tert-butyl N-[4-(bromomethyl)-2-pyridyl]carbamate and N-(1H-pyrazol-4-yl)-6-(3-pyridyl)-1-(2-trimethylsilylethoxymethyl) indazole-3-carboxamide for 6-[1-(Tetrahydro-pyran-2-yl)-1H-pyrazol-4-yl]-1-(2-trimethylsilanyl-ethoxymethyl)-1H-indazole-3-carboxylic acid (1H-pyrazol-4-yl)-amide. ¹H NMR (400 MHz, DMSO) δ 13.89 (s, 1H), 10.67 (s, 1H), 8.99 (s, 1H), 8.62 (d, J = 4.7 Hz, 1H), 8.32 (d, J = 8.5 Hz, 1H), 8.19 (m, 2H), 7.92 (s, 1H), 7.84 (d, J = 5.2 Hz, 1H), 7.77 (s, 1H), 7.63 (d, J = 8.5 Hz, 1H), 7.53 (dd, J = 7.9, 4.8 Hz, 1H), 6.31 (d, J = 5.3 Hz, 1H), 6.15 (s, 1H), 5.89 (s, 2H), 5.20 (s, 2H). MS: *m*/*z* = 411.3 (M+H)⁺.

### Example 33: N-[1-[[5-(methylcarbamoyl)-2-furyl]methyl]pyrazol-4-yl]-6-(3-pyridyl)-1H-indazole-3-carboxamide

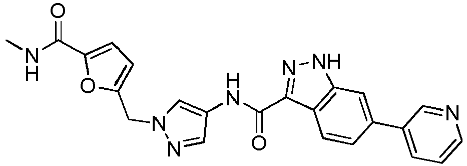

The title compound was synthesized according to Example 9, substituting 3-(chloromethyl)benzamide for 8-Bromomethyl-quinoline and N-(1H-pyrazol-4-yl)-6-(3-pyridyl)-1-(2-trimethylsilylethoxymethyl) indazole-3-carboxamide for 6-[1-(Tetrahydro-pyran-2-yl)-1H-pyrazol-4-yl]-1-(2-trimethylsilanyl-ethoxymethyl)-1H-indazole-3-carboxylic acid (1H-pyrazol-4-yl)-amide. ¹H NMR (400 MHz, DMSO) δ 13.87 (s, 1H), 10.64 (s, 1H), 8.99 (d, J = 2.1 Hz, 1H), 8.62 (d, J = 4.7 Hz, 1H), 8.32 (d, J = 8.5 Hz, 1H), 8.27-8.20 (m, 1H), 8.20-8.15 (m, 2H), 7.92 (s, 1H), 7.74 (s, 1H), 7.63 (d, J = 8.5 Hz, 1H), 7.53 (dd, J = 7.9, 4.8 Hz, 1H), 7.03 (d, J = 3.4 Hz, 1H), 6.56 (d, J = 3.4 Hz, 1H), 5.39 (s, 2H), 2.70 (s, 3H). MS: *m*/*z =* 442.3 (M+H)⁺.

### Example 34: 6-(3-pyridyl)-N-[1-(thiazol-4-ylmethyl)pyrazol-4-yl]-1H-indazole-3-carboxamide

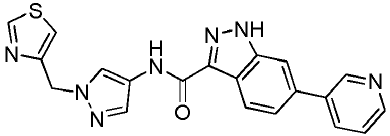

The title compound was synthesized according to Example 9, substituting 4-(chloromethyl)thiazole hydrochloride, adding an additional equivalent of cesium carbonate and N-(1H-pyrazol-4-yl)-6-(3-pyridyl)-1-(2-trimethylsilylethoxymethyl) indazole-3-carboxamide for 6-[1-(Tetrahydro-pyran-2-yl)-1H-pyrazol-4-yl]-1-(2-trimethylsilanyl-ethoxymethyl)-1H-indazole-3-carboxylic acid (1H-pyrazol-4-yl)-amide. ¹H NMR (400 MHz, DMSO) δ 10.60 (s, 1H), 9.10 (d, J = 1.7 Hz, 1H), 8.99 (d, J = 2.0 Hz, 1H), 8.61 (d, J = 4.6 Hz, 1H), 8.30 (d, J = 8.5 Hz, 1H), 8.20-8.15 (m, 2H), 7.91 (s, 1H), 7.73 (s, 1H), 7.61 (d, J = 8.5 Hz, 1H), 7.56 (s, 1H), 7.53 (dd, J = 7.9, 4.7 Hz, 1H), 5.46 (s, 2H). MS: *m*/*z =* 402.2 (M+H)⁺.

### Example 35: 5-(4-{[6-(1H-Pyrazol-4-yl)-1H-indazole-3-carbonyl]-amino}-pyrazol-1-ylmethyl)-furan-2-carboxylic acid

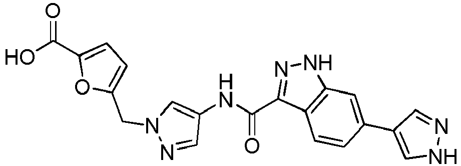

The title compound was synthesized according to Example 9, substituting 5-Chloromethyl-furan-2-carboxylic acid ethyl ester for 8-Bromomethyl-quinoline, and introducing a basic hydrolysis step (LiOH, THF/MeOH) prior to the acidic deprotection.¹H NMR (400 MHz, DMSO) δ 13.90-12.55 (m, 2H), 10.56 (s, 1H), 8.24-8.14 (m, 4H), 7.76 (s, 1H), 7.73 (s, 1H), 7.56 (d, *J* = 8.5 Hz, 1H), 6.93 (s, 1H), 6.51 (d, *J* = 3.1 Hz, 1H), 5.37 (s, 2H). MS: *m*/*z* = 418.1 (M+H)⁺.

### Example 36: 6-(1H-Pyrazol-4-yl)-1H-indazole-3-carboxylic acid [1-(3-pyridin-3-yl-benzyl)-1H-pyrazol-4-yl]-amide

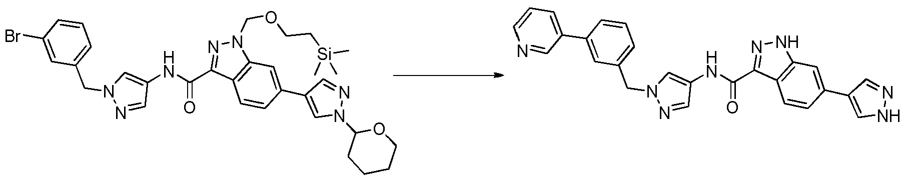

To a suspension of 6-[1-(Tetrahydro-pyran-2-yl)-1H-pyrazol-4-yl]-1-(2-trimethylsilanyl-ethoxymethyl)-1H-indazole-3-carboxylic acid [1-(3-bromo-benzyl)-1H-pyrazol-4-yl]-amide (30.0 mg, 0.0443 mmol) and 3-Pyridylboronic acid (0.0109 g, 0.0887 mmol) in acetonitrile (0.5 mL, 10 mmol) was added 1,1'-Bis(diphenylphosphino)ferrocenepalladium (II) chloride (0.00362 g, 0.00443 mmol) and Sodium carbonate (0.00940 g, 0.0887 mmol) as a solution in 1.0 M in H₂O. The mixture was heated to 120 °C for 30 minutes in the microwave, then cooled to rt. The mixture was diluted with 5 mL CH₂Cl₂ and 5 mL brine, then filtered through a phase separator and concentrated in vacuo. The residue was diluted with trifluoroacetic acid (1 mL), and triisopropylsilane (45.5 µL, 0.222 mmol) and a few drops of CH₂Cl₂ to homogenize were added. The mixture was stirred for 90 minutes at rt, then concentrated in vacuo. Purification by automated reverse phase HPLC provided 9.7 mg of the title compound. ¹H NMR (400 MHz, DMSO) δ 13.60 (s, 1H), 12.98 (s, 1H), 10.54 (s, 1H), 8.87 (d, *J* = 2.1 Hz, 1H), 8.58 (d, *J* = 4.8 Hz, 1H), 8.30 (s, 1H), 8.24 (s, 1H), 8.15 (d, *J* = 8.5 Hz, 1H), 8.10-8.01 (m, 2H), 7.75 (s, 1H), 7.74 (s, 1H), 7.68 (s, 1H), 7.67 (d, *J* = 6.7 Hz, 1H), 7.56 (d, *J* = 8.5 Hz, 1H), 7.53 - 7.46 (m, 2H), 7.30 (d, *J* = 7.6 Hz, 1H), 5.41 (s, 2H). MS: *m*/*z* = 461.2 (M+H)⁺.

### Example 37: 6-(1H-Pyrazol-4-yl)-1H-indazole-3-carboxylic acid [1-(3-pyridin-4-yl-benzyl)-1H-pyrazol-4-yl]-amide

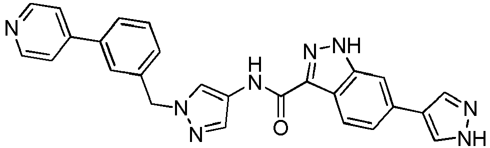

The title compound was synthesized according to Example 36, substituting 4-pyridylboronic acid for 3-pyridylboronic acid. ¹H NMR (400 MHz, DMSO) δ 13.62 (s, 1H), 13.00 (s, 1H), 10.55 (s, 1H), 8.65 (s, 1H), 8.64 (s, 1H), 8.30-8.02 (m, 4H), 7.77-7.72 (m, 4H), 7.69 (s, 1H), 7.67 (s, 1H), 7.58 - 7.46 (m, 2H), 7.35 (d, *J* = 7.5 Hz, 1H), 6.56 (s, 1H), 5.41 (s, 2H). MS: *m*/*z* = 461.2 (M+H)⁺.

### Example 38: 6-(1H-Pyrazol-4-yl)-1H-indazole-3-carboxylic acid [1-(3-pyridin-2-yl-benzyl)-1H-pyrazol-4-yl]-amide

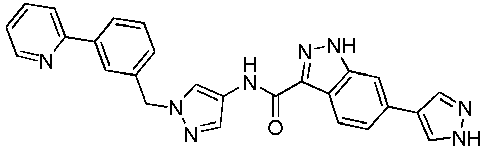

The title compound was synthesized according to Example 36, substituting 2-pyridylboronic acid for 3-pyridylboronic acid. ¹H NMR (400 MHz, DMSO) δ 13.65 (s, 1H), 13.01 (s, 1H), 10.55 (s, 1H), 8.67 (d, *J* = 4.5 Hz, 1H), 8.50 (s, 1H), 8.23 (s, 1H), 8.15 (d, *J* = 8.5 Hz, 1H), 8.05 (s, 1H), 8.00 (d, *J* = 7.8 Hz, 1H), 7.96 - 7.84 (m, 2H), 7.76 (s, 1H), 7.75 (s, 1H), 7.55 (d, *J* = 8.5 Hz, 1H), 7.48 (t, *J* = 7.7 Hz, 1H), 7.39-7.31 (m, 2H), 6.83 (s, 1H), 5.41 (s, 2H). MS: *m*/*z* = 461.2 (M+H)⁺.

### Example 39: N-[1-[[3-(4-methylpiperazin-1-yl)phenyl]methyl]pyrazol-4-yl]-6-(1H-pyrazol-4-yl)-1H-indazole-3-carboxamide

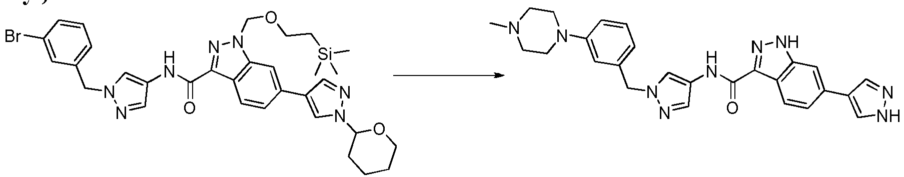

To a solution of N-[1-[(3-bromophenyl)methyl]pyrazol-4-yl]-6-(1-tetrahydropyran-2-ylpyrazol-4-yl)-1-(2-trimethylsilylethoxymethyl)indazole-3-carboxamide (50.0 mg; 0.0739 mmol) in THF (0.5 mL) was added 1-methylpiperazine (2 equiv.; 0.148 mmol; 0.0166 mL), RUPHOS (0.1 equiv.; 0.00739 mmol; 3.52 mg) and (RUPHOS) Palladium(II) phenethylamine chloride (0.1 equiv.; 0.00739 mmol; 5.38 mg), then LiHMDS in (1 M; 2 equiv.; 0.148 mmol; 0.148 mL). The mixture was heated to 85 °C for 3 hours, at which point the solution became deep brown and LCMS showed clean and complete conversion. The mixture was cooled to rt, then diluted with 5 mL CH₂Cl₂ and 5 mL brine, then filtered through a phase separator. The organic fraction was concentrated in vacuo. The residue was diluted with TFA (1 mL) and triisopropylsilane (5 equiv.; 0.369 mmol; 0.0764 mL) and a few drops of CH₂Cl₂ to homogenize, then the mixture was stirred at rt for 90 minutes. The mixture was concentrated in vacuo, and the residue was purified by automated reverse phase HPLC, which provided the title compound (14 mg; 0.02907 mmol; 39.4%). ¹H NMR (400 MHz, DMSO) δ 13.55 (s, 1H), 12.98 (s, 1H), 10.50 (s, 1H), 8.14 (m, 4H), 7.75 (s, 1H), 7.71 (s, 1H), 7.55 (d, J = 8.5 Hz, 1H), 7.17 (t, J = 7.9 Hz, 1H), 6.90 (s, 1H), 6.86 (d, J = 8.2 Hz, 1H), 6.65 (d, J = 7.5 Hz, 1H), 5.22 (s, 2H), 3.15 - 3.07 (m, 4H), 2.47 - 2.40 (m, 4H), 2.21 (s, 3H). MS: *m*/*z* = 482.3 (M+H)⁺.

### Example 40: N-[1-[(3-morpholinophenyl)methyl]pyrazol-4-yl]-6-(1H-pyrazol-4-yl)-1H-indazole-3-carboxamide

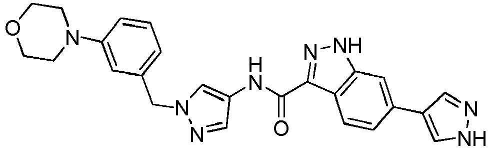

The title compound was synthesized according to Example 39, substituting morpholine for 1-methylpiperazine. ¹H NMR (400 MHz, DMSO) δ 13.58 (s, 1H), 12.98 (s, 1H), 10.51 (s, 1H), 8.14 (d, J = 8.1 Hz, 4H), 7.75 (s, 1H), 7.71 (s, 1H), 7.55 (d, J = 8.5 Hz, 1H), 7.20 (t, J = 7.8 Hz, 1H), 6.90 (s, 1H), 6.87 (d, J = 8.2 Hz, 1H), 6.68 (d, J = 7.5 Hz, 1H), 5.23 (s, 2H), 3.80 - 3.58 (m, 4H), 3.15 - 3.00 (m, 4H). MS: *m*/*z* = 469.3 (M+H)⁺.

### Example 41: N-[1-[(2-morpholinothiazol-4-yl)methyl]pyrazol-4-yl]-6-(1H-pyrazol-4-yl)-1H-indazole-3-carboxamide

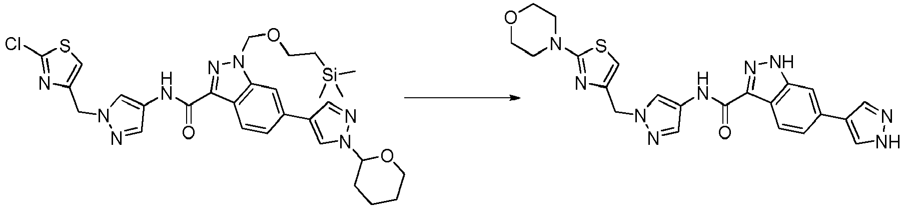

To a solution of N-[1-[(2-chlorothiazol-4-yl)methyl]pyrazol-4-yl]-6-(1-tetrahydropyran-2-ylpyrazol-4-yl)-1-(2-trimethylsilylethoxymethyl)indazole-3-carboxamide (50 mg; 0.07822 mmol; 50 mg) in DMSO (0.5 mL) was added morpholine ((5 equiv.; 0.3911 mmol; 0.0342 mL) and the mixture was heated to 140 °C for 60 minutes in the microwave. After cooling to rt, the mixture was diluted with 50 mL EtOAc and washed with 2 x 50 mL 1:1 H₂O:brine. The organic extracts were dried (Na₂SO₄) and concentrated in vacuo.

The residue was diluted with TFA (1.0 mL) and triisoproylsilane (5 equiv.; 0.3841 mmol; 0.0795 mL) and a few drops of CH₂Cl₂ to homogenize, and the mixture was stirred at rt for 90 minutes. After in vacuo concentration, the residue was purified by automated HPLC, which provided the title compound (22.7 mg; 0.0477 mmol; 62.1% Yield). ¹H NMR (400 MHz, DMSO) δ 13.57 (s, 1H), 12.99 (s, 1H), 10.53 (s, 1H), 8.31 (s, 1H), 8.16 (d, J = 8.5 Hz, 1H), 8.11 (s, 1H), 8.04 (s, 1H), 7.76 (s, 1H), 7.72 (s, 1H), 7.56 (d, J = 8.5 Hz, 1H), 6.64 (s, 1H), 5.15 (s, 2H), 3.72 - 3.66 (m, 4H), 3.38 - 3.32 (m, 4H). MS: *m*/*z* = 476.3 (M+H)⁺.

### Example 42: N-[1-[[2-(4-methylpiperazin-1-yl)thiazol-4-yl]methyl]pyrazol-4-yl]-6-(1H-pyrazol-4-yl)-1H-indazole-3-carboxamide

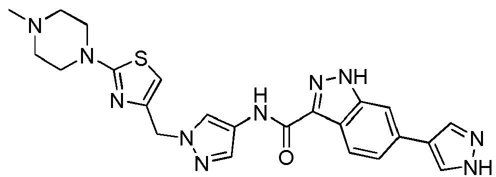

The title compound was synthesized according to Example 41, substituting N-methylpiperazine for morpholine. ¹H NMR (400 MHz, DMSO) δ 13.60 (s, 1H), 12.99 (s, 1H), 10.53 (s, 1H), 8.38 - 7.91 (m, 4H), 7.76 (s, 1H), 7.72 (s, 1H), 7.56 (d, J = 8.5 Hz, 1H), 6.61 (s, 1H), 5.14 (s, 2H), 3.39 (s, 4H), 2.25 (s, 3H). MS: *m*/*z* = 489.1 (M+H)⁺.

### Example 43: 6-(1H-Pyrazol-3-yl)-1H-indazole-3-carboxylic acid (1-thiazol-4-ylmethyl-1H-pyrazol-4-yl)-amide

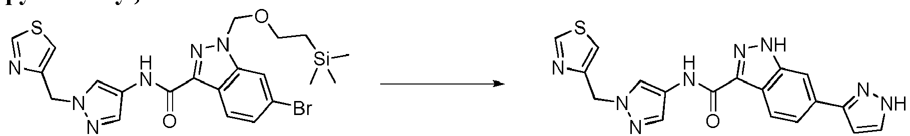

To a solution of 6-Bromo-1-(2-trimethylsilanyl-ethoxymethyl)-1H-indazole-3-carboxylic acid (1-thiazol-4-ylmethyl-1H-pyrazol-4-yl)-amide (49.0 mg, 0.0918 mmol) in acetonitrile (1 mL) was added 1H-pyrazole-2-boronic acid (20.6 mg, 0.184 mmol), 1,1'-Bis(diphenylphosphino)ferrocenepalladium (II) chloride (7.50 mg, 0.00918 mmol) and sodium carbonate (29.2 mg, 0.276 mmol) as a 1.0 M solution in water. The mixture was heated to 110 °C for 20 minutes in the microwave, then cooled to rt. The mixture was diluted with 5 mL CH₂Cl₂ and 5 mL brine and filtered through a phase separator. After in vacuo concentration, the residue was diluted with TFA (1 mL), triisopropylsilane (93 µL, 0.45 mmol) and a few drops of CH₂Cl₂ to homogenize, and the mixture was stirred at 90 minutes at rt. After in vacuo concentration, the residue was purified by automated reverse phase HPLC to provide the title compound (12 mg; 0.0297 mmol; 33%). ¹H NMR (400 MHz, DMSO) δ 13.68 (s, 1H), 12.95 (s, 1H), 10.57 (s, 1H), 9.10 (d, *J* = 1.9 Hz, 1H), 8.21 (d, *J* = 8.5 Hz, 1H), 8.18 (s, 1H), 7.99 (s, 1H), 7.79 (s, 1H), 7.73 (s, 1H), 7.56 (d, *J* = 1.7 Hz, 1H), 6.83 (s, 1H), 5.45 (s, 2H). MS: *m*/*z* = 391.1 (M+H)⁺.

### Example 44: 6-(5-Cyano-pyridin-3-yl)-1H-indazole-3-carboxylic acid (1-thiazol-4-ylmethyl-1H-pyrazol-4-yl)-amide

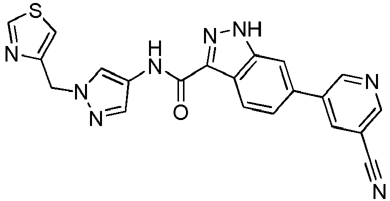

The title compound was synthesized according to example 43, substituting 3-cyanopyridine-5-boronic acid pinacol ester for 1H-pyrazole-2-boronic acid.¹H NMR (400 MHz, DMSO) δ 13.97 (s, 1H), 10.65 (s, 1H), 9.29 (d, *J* = 2.2 Hz, 1H), 9.10 (d, *J* = 1.9 Hz, 1H), 9.05 (d, *J* = 1.8 Hz, 1H), 8.76 (t, *J* = 2.0 Hz, 1H), 8.34 (d, *J* = 8.5 Hz, 1H), 8.19 (s, 1H), 8.05 (s, 1H), 7.73 (s, 1H), 7.71 (d, *J* = 8.5 Hz, 1H), 7.57 (d, *J* = 1.7 Hz, 1H), 5.46 (s, 2H). MS: *m*/*z* = 427.1 (M+H)⁺.

### Example 45: 6-(6-Amino-pyridin-3-yl)-1H-indazole-3-carboxylic acid (1-thiazol-4-ylmethyl-1H-pyrazol-4-yl)-amide

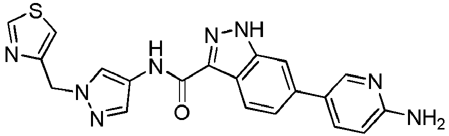

The title compound was synthesized according to example 43, substituting 2-aminopyridine-5-boronic acid, pinacol ester for 1H-pyrazole-2-boronic acid. ¹H NMR (400 MHz, DMSO) δ 13.66 (s, 1H), 10.56 (s, 1H), 9.09 (d, *J* = 1.8 Hz, 1H), 8.33 (d, *J* = 2.3 Hz, 1H), 8.21 (d, *J* = 8.5 Hz, 1H), 8.18 (s, 1H), 7.79 (dd, *J* = 8.6, 2.5 Hz, 1H), 7.72 (s, 1H), 7.70 (s, 1H), 7.56 (s, 1H), 7.50 (d, *J* = 8.6 Hz, 1H), 6.57 (d, *J* = 8.6 Hz, 1H), 6.09 (s, 2H), 5.45 (s, 2H). MS: *m*/*z* = 417.1 (M+H)⁺.

### Example 46: 3-(4-{[6-(1H-Pyrazol-4-yl)-1H-indazole-3-carbonyl]-amino}-pyrazol-1-ylmethyl)-benzoic acid

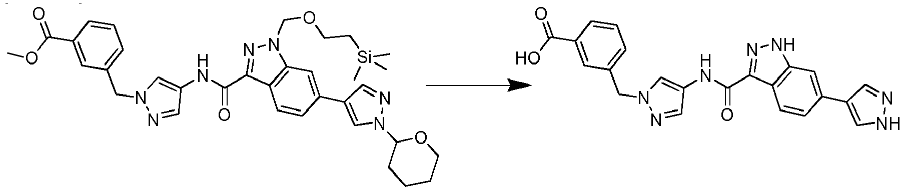

To a solution of 3-(4-{[6-[1-(Tetrahydro-pyran-2-yl)-1H-pyrazol-4-yl]-1-(2-trimethylsilanyl-ethoxymethyl)-1H-indazole-3-carbonyl]-amino}-pyrazol-1-ylmethyl)-benzoic acid methyl ester (39 mg, 0.059 mmol) in methanol (1 mL) and THF (1 mL) was added Lithium hydroxide (0.0043 g, 0.18 mmol) as a 1.0 M solution in H₂O. The mixture was heated to 50 °C for 60 minutes in the microwave, then concentrated in vacuo. The residue was diluted with TFA (1 mL) and triisopropylsilane (61 µL, 0.30 mmol) and a few drops of CH₂Cl₂ to homogenize the mixture. After stirring for 30 minutes at rt, the mixture was concentrated in vacuo. Purification by automated reverse phase HPLC provided the title compound (11.6 mg; 0.0271 mmol; 46% yield). ¹H NMR (400 MHz, DMSO) δ 10.55 (s, 1H), 8.22 - 8.10 (m, 4H), 7.85-7.79 (m, 2H), 7.76 (s, 1H), 7.74 (s, 1H), 7.56 (d, *J* = 8.6 Hz, 1H), 7.42-7.39 (m, 2H), 5.37 (s, 2H). MS: *m*/*z* = 428.1 (M+H)⁺.

### Example 47: 6-(1H-Pyrazol-4-yl)-1H-indazole-3-carboxylic acid {1-[3-(1-hydroxy-1-methyl-ethyl)-benzyl]-1H-pyrazol-4-yl}-amide

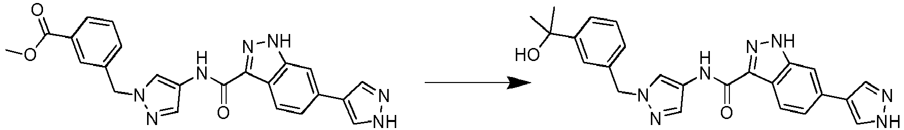

A solution of 3-(4-{[6-(1H-Pyrazol-4-yl)-1H-indazole-3-carbonyl]-amino}-pyrazol-1-ylmethyl)-benzoic acid methyl ester (Example 10, 36 mg, 0.082 mmol) in THF (1.0 mL, 12 mmol) was cooled to 0 °C, then methylmagnesium chloride (0.0610 g, 0.816 mmol) was added as a 3.0 M solution in THF. The mixture was allowed to warm to room temperature, and after stirring for 30 minutes at rt the reaction was quenched by the addition of ∼1 mL sat. NH₄Cl(aq), then the mixture was diluted with 50 mL EtOAc and washed with 50 mL brine. The organic extracts were dried (Na₂SO₄), concentrated in vacuo, and the residue purified by automated reverse phase HPLC, which provided the titled compound (11.9 mg, 0.0269 mmol, 33%). ¹H NMR (400 MHz, DMSO) δ 13.61 (s, 1H), 12.99 (s, 1H), 10.53 (s, 1H), 8.24-8.11 (m, 4H), 7.75 (s, 1H), 7.72 (s, 1H), 7.55 (d, *J* = 8.5 Hz, 1H), 7.45 (s, 1H), 7.38 (d, *J* = 7.9 Hz, 1H), 7.27 (t, *J* = 7.7 Hz, 1H), 7.06 (d, *J* = 7.5 Hz, 1H), 5.30 (s, 2H), 4.99 (s, 1H), 1.41 (s, 6H). MS: *m*/*z* = 442.3 (M+H)⁺.

### Example 48: 6-(1H-Pyrazol-4-yl)-1H-indazole-3-carboxylic acid {1-[3-(2H-tetrazol-5-yl)-benzyl]-1H-pyrazol-4-yl}-amide

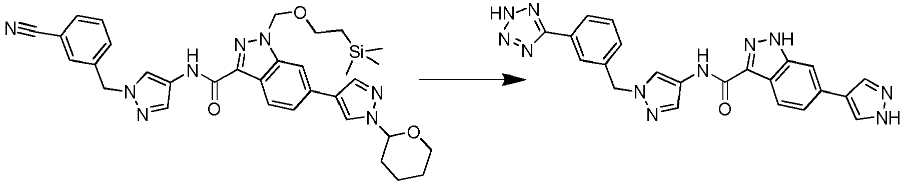

To a solution of N-(1-(3-cyanobenzyl)-1H-pyrazol-4-yl)-6-(1-(tetrahydro-2H-pyran-2-yl)-1H-pyrazol-4-yl)-1-((2-(trimethylsilyl)ethoxy)methyl)-1H-indazole-3-carboxamide (45 mg, 0.072 mmol) in toluene (0.5 mL) was added azidotributyltin(IV) (0.030 mL, 0.11 mmol) and the mixture was heated to 110 °C for four days. The mixture was concentrated in vacuo and then diluted with methylene chloride (1.0 mL), TFA (1.0 mL) and triisopropylsilane (0.074 mL, 0.36 mmol). The mixture was stirred at rt for 90 minutes, then concentrated in vacuo. The residue was purified by reverse phase HPLC, which provided the title compound (19.4 mg, 0.042 mmol, 58%). ¹H NMR (400 MHz, DMSO) δ 13.60 (s, 1H), 12.98 (s, 1H), 10.56 (s, 1H), 8.37 - 7.99 (m, 4H), 7.98 - 7.91 (m, 2H), 7.76 (s, 2H), 7.56 (d, *J* = 7.7 Hz, 1H), 7.52 (d, *J* = 7.6 Hz, 1H), 7.38 (d, *J* = 7.7 Hz, 1H), 5.42 (s, 2H). MS: *m*/*z =* 452.2 (M+H)⁺.

### Example 49: N-(1-(3-chlorobenzyl)-1H-pyrazol-4-yl)-1H-indazole-3-carboxamide

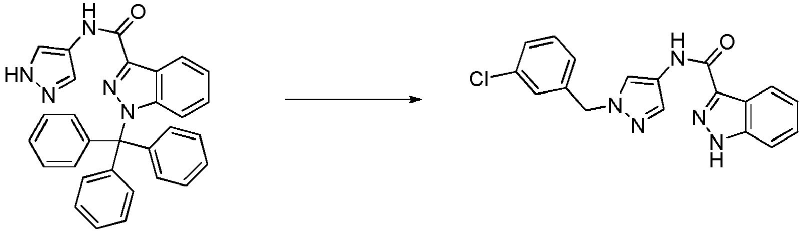

To a solution of N-(1H-pyrazol-4-yl)-1-trityl-1H-indazole-3-carboxamide (75 m g, 0.16 mmol) in THF (1 mL) was added tributylphosphine (107 mg, 0.48 mmol), m-chlorobenzylalcohol ( 68 mg, 0.48 mmol), and diamide (84 mg, 0.48 mmol). The mixture was stirred for one hour at rt then the mixture was diluted with 10 mL EtOAc and washed with 2 x 10 mL 1:1 H₂O:brine. The organic extracts were dried (Na₂SO₄) and concentrated in vacuo. Purification by CombiFlash (12 g column; 1-5% MeOH in DCM over 15 minutes) provided 95 mg (95%) of N-(1-(2-chlorobenzyl)-1H-pyrazol-4-yl)-1-trityl-1H-indazole-3-carboxamide. The residue was diluted with 1.0 mL trifluoroacetic acid, then triisopropylsilane (80.9 µL, 0.394 mmol) and a few drops of CH₂Cl₂ were added. The mixture was stirred for 2 hours at rt. The mixture was concentrated in vacuo, then purified by automated reverse-phase HPLC, which provided 35 mg (32%) of the title compound.¹H NMR (400 MHz, DMSO) δ 13.70 (s, 1H), 10.59 (s, 1H), 8.22 (d, *J=* 10.1 Hz, 2H), 7.74 (s, 1H), 7.64 (d, *J* = 8.3 Hz, 1H), 7.33 (ddd, *J* = 35.7, 22.2, 7.0 Hz, 7H), 5.34 (s, 2H). MS: *m*/*z* = 352.0 (M+H)⁺.

### Example 50: N-(1-(4-cyanobenzyl)-1H-pyrazol-4-yl)-1H-indazole-3-carboxamide

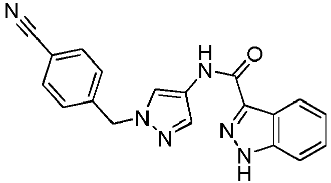

The title compound was synthesized according to example 49, substituting p-cyanobenzyl alcohol for m-chlorobenzyl alcohol. ¹H NMR (400 MHz, DMSO) δ 13.71 (s, 1H), 10.63 (s, 1H), 8.42 - 8.07 (m, 2H), 7.99 - 7.72 (m, 2H), 7.65 (d, *J* = 8.5 Hz, 1H), 7.55 - 7.13 (m, 3H), 5.45 (s, 2H). MS: *m*/*z* = 343.1 (M+H)⁺.

### Example 51: N-(1-benzyl-1H-pyrazol-4-yl)-1H-indazole-3-carboxamide

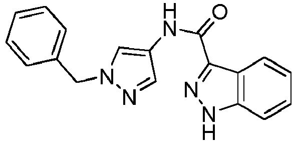

The title compound was synthesized according to example 49, substituting benzyl alcohol for m-chlorobenzyl alcohol.¹H NMR (400 MHz, DMSO) δ 13.69 (s, 1H), 10.58 (s, 1H), 8.21 (d, *J =* 8.3 Hz, 1H), 8.18 (s, 1H), 7.72 (s, 1H), 7.64 (d, *J* = 8.4 Hz, 1H), 7.47 - 7.41 (m, 1H), 7.39 - 7.23 (m, 6H), 5.32 (s, 2H). MS: *m*/*z* = 318.0 (M+H)⁺.

### Example 52: N-(1-(pyridin-4-ylmethyl)-1H-pyrazol-4-yl)-1H-indazole-3-carboxamide

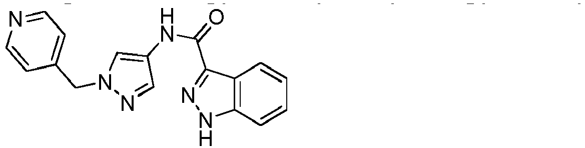

The title compound was synthesized according to example 49, substituting pyridin-4-ylmethanol for m-chlorobenzyl alcohol. MS: *m*/*z =* 319.0 (M+H)⁺.

### Example 53: N-(1-(4-methylbenzyl)-1H-pyrazol-4-yl)-1H-indazole-3-carboxamide

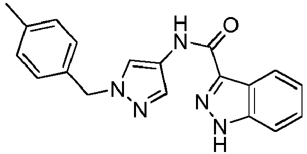

The title compound was synthesized according to example 49, substituting p-methylbenzyl alcohol for m-chlorobenzyl alcohol. ¹H NMR (400 MHz, DMSO) δ 13.69 (s, 1H), 10.57 (s, 1H), 8.32 - 8.07 (m, 2H), 7.67 (dd, J = 28.1, 6.6 Hz, 2H), 7.50 - 7.34 (m, 1H), 7.16 (s, 4H), 5.25 (s, 2H), 2.28 (s, 3H). MS: *m*/*z =* 332.1 (M+H)⁺.

### Example 54: N-(1-(pyridin-3-ylmethyl)-1H-pyrazol-4-yl)-1H-indazole-3-carboxamide

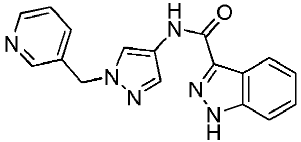

The title compound was synthesized according to example 49, substituting pyridin-2-ylmethanol for m-chlorobenzyl alcohol. ¹H NMR (400 MHz, DMSO) δ 13.71 (s, 1H), 10.61 (s, 1H), 8.75 - 8.40 (m, 2H), 8.21 (dd, *J* = 20.6, 12.5 Hz, 2H), 7.74 (s, 1H), 7.65 (t, *J* = 7.3 Hz, 1H), 7.50 - 7.20 (m, 2H), 5.38 (s, 2H). MS: *m*/*z* = 319.0 (M+H)⁺.

### Example 55: N-(1-(3-methylbenzyl)-1H-pyrazol-4-yl)-1H-indazole-3-carboxamide

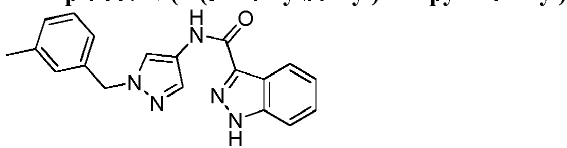

The title compound was synthesized according to example 49, substituting m-methylbenzyl alcohol for m-chlorobenzyl alcohol. ¹H NMR (400 MHz, DMSO) δ 13.69 (s, 1H), 10.58 (s, 1H), 8.38 - 8.09 (m, 2H), 7.71 (s, 1H), 7.64 (d, J = 8.4 Hz, 1H), 7.53 - 7.36 (m, 1H), 7.36 - 7.15 (m, 2H), 7.15 - 6.91 (m, 2H), 5.27 (s, 2H), 2.29 (s, 3H). MS: *m*/*z =* 332.1 (M+H)⁺.

### Example 56: N-(1-(2-methylbenzyl)-1H-pyrazol-4-yl)-1H-indazole-3-carboxamide

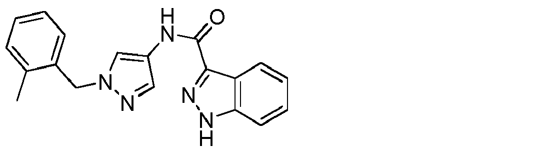

The title compound was synthesized according to example 49, substituting o-methylbenzyl alcohol for m-chlorobenzyl alcohol.¹H NMR (400 MHz, DMSO) δ 13.70 (s, 1H), 10.58 (s, 1H), 8.20 (d, *J* = 8.2 Hz, 1H), 8.07 (s, 1H), 7.73 (s, 1H), 7.63 (s, 1H), 7.55 - 7.34 (m, 1H), 7.34 - 7.10 (m, 3H), 7.00 (d, *J* = 7.4 Hz, 1H), 5.33 (s, 2H), 2.30 (s, 3H). MS: *m*/*z =* 332.1 (M+H)⁺.

### Example 57: N-(1-(3,5-dichlorobenzyl)-1H-pyrazol-4-yl)-1H-indazole-3-carboxamide

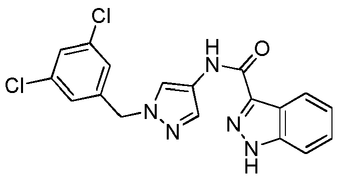

The title compound was synthesized according to example 49, substituting 3,5-dichlorobenzyl alcohol for m-chlorobenzyl alcohol. ¹H NMR (400 MHz, DMSO) δ 13.72 (s, 1H), 10.61 (s, 1H), 8.34 - 8.11 (m, 2H), 7.54 (ddd, J = 39.3, 34.0, 30.9 Hz, 4H), 7.29 (s, 3H), 5.36 (s, 2H). MS: *m*/*z* = 386.0 (M+H)⁺.

### Example 58: N-(1-(4-chlorobenzyl)-1H-pyrazol-4-yl)-1H-indazole-3-carboxamide

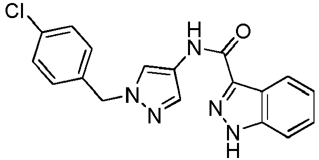

The title compound was synthesized according to example 49, substituting p-chlorobenzyl alcohol for m-chlorobenzyl alcohol. MS: *m*/*z* = 352.0 (M+H)⁺.

### Example 59: N-(1-(3-(trifluoromethyl)benzyl)-1H-pyrazol-4-yl)-1H-indazole-3-carboxamide

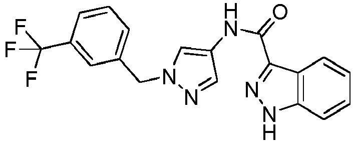

The title compound was synthesized according to example 49, substituting 3-(trifluoromethyl)benzyl alcohol for m-chlorobenzyl alcohol. ¹H NMR (400 MHz, DMSO) δ 13.69 (s, 1H), 10.60 (s, 1H), 8.47 - 8.07 (m, 2H), 8.01 - 7.51 (m, 7H), 7.44 (t, J = 7.4 Hz, 1H), 7.27 (t, J = 7.4 Hz, 1H), 5.44 (s, 2H). MS: *m*/*z* = 386.1 (M+H)⁺.

### Example 60: N-(1-(2-(trifluoromethyl)benzyl)-1H-pyrazol-4-yl)-1H-indazole-3-carboxamide

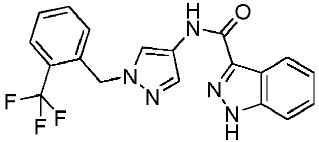

The title compound was synthesized according to example 49, substituting 2-(trifluoromethyl)benzyl alcohol for m-chlorobenzyl alcohol. MS: *m*/*z* = 386.1 (M+H)⁺.

### Example 61: N-(1-(2,6-dichlorobenzyl)-1H-pyrazol-4-yl)-1H-indazole-3-carboxamide

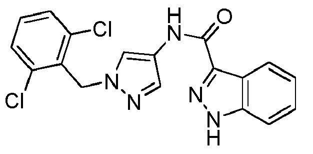

The title compound was synthesized according to example 49, substituting 2,6-dichlorobenzyl alcohol for m-chlorobenzyl alcohol. ¹H NMR (400 MHz, DMSO) δ 13.68 (s, 1H), 10.55 (s, 1H), 8.19 (dd, J = 40.6, 32.8 Hz, 2H), 7.87 - 7.54 (m, 4H), 7.54 - 7.31 (m, 2H), 7.27 (d, J = 7.1 Hz, 1H), 5.55 (s, 2H). MS: *m*/*z* = 386.0 (M+H)⁺.

### Example 62: N-(1-(4-(trifluoromethyl)benzyl)-1H-pyrazol-4-yl)-1H-indazole-3-carboxamide

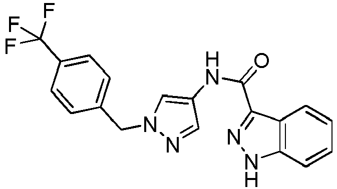

The title compound was synthesized according to example 49, substituting 4-(trifluoromethyl)benzyl alcohol for m-chlorobenzyl alcohol. ¹H NMR (400 MHz, DMSO) δ 13.69 (s, 1H), 10.61 (s, 1H), 8.40 - 8.04 (m, 2H), 7.91 - 7.54 (m, 4H), 7.44 (d, J = 7.4 Hz, 3H), 7.27 (t, J = 7.4 Hz, 1H), 5.45 (s, 2H). MS: *m*/*z* = 386.1 (M+H)⁺.

### Example 63: N-(1-(3-fluorobenzyl)-1H-pyrazol-4-yl)-1H-indazole-3-carboxamide

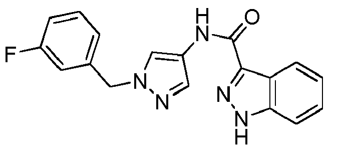

The title compound was synthesized according to example 49, substituting m-fluorobenzyl alcohol for m-chlorobenzyl alcohol.¹H NMR (400 MHz, DMSO) δ 13.71 (s, 1H), 10.58 (s, 1H), 8.21 (d, *J* = 7.7 Hz, 2H), 7.74 (s, 1H), 7.64 (d, *J* = 8.3 Hz, 1H), 7.43 (dd, *J* = 17.3, 8.5 Hz, 2H), 7.27 (t, *J* = 7.5 Hz, 1H), 7.09 (dd, *J* = 21.9, 12.2 Hz, 3H), 6.62 (s, 1H), 5.35 (s, 2H). MS: *m*/*z* = 336.1 (M+H)⁺.

### Example 64: N-(1-(2-methoxybenzyl)-1H-pyrazol-4-yl)-1H-indazole-3-carboxamide

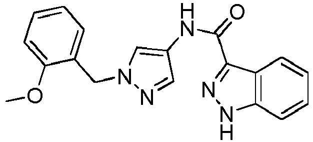

The title compound was synthesized according to example 49, substituting 2-methoxybenzyl alcohol for m-chlorobenzyl alcohol.¹H NMR (400 MHz, DMSO) δ 13.69 (s, 1H), 10.54 (s, 1H), 8.21 (d, *J* = 8.1 Hz, 1H), 8.07 (s, 1H), 7.72 (s, 1H), 7.64 (d, *J* = 8.3 Hz, 1H), 7.43 (t, *J* = 7.5 Hz, 1H), 7.37 - 7.12 (m, 2H), 7.05 (d, *J* = 8.2 Hz, 1H), 6.93 (d, *J* = 10.4 Hz, 2H), 5.26 (s, 2H). MS: *m*/*z* = 348.1 (M+H)⁺.

### Example 65: N-(1-(2-fluorobenzyl)-1H-pyrazol-4-yl)-1H-indazole-3-carboxamide

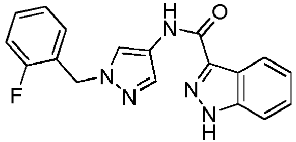

The title compound was synthesized according to example 49, substituting o-fluorobenzyl alcohol for m-chlorobenzyl alcohol.¹H NMR (400 MHz, DMSO) δ 13.72 (s, 1H), 10.64 (s, 1H), 8.65 - 8.42 (m, 2H), 8.24 (d, *J* = 25.0 Hz, 2H), 7.78 (s, 1H), 7.65 (d, *J* = 8.4 Hz, 1H), 7.53 - 7.39 (m, 1H), 7.34 - 7.22 (m, 1H), 7.14 (d, *J =* 5.7 Hz, 2H), 5.40 (s, 3H). MS: *m*/*z* = 336.1 (M+H)⁺.

### Example 66: N-(1-(4-methoxybenzyl)-1H-pyrazol-4-yl)-1H-indazole-3-carboxamide

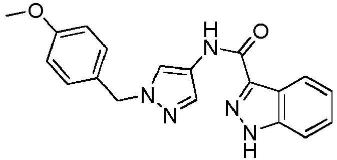

The title compound was synthesized according to example 49, substituting 4-methoxybenzyl alcohol for m-chlorobenzyl alcohol.¹H NMR (400 MHz, DMSO) δ 13.67 (s, 1H), 10.53 (s, 1H), 8.38 - 8.02 (m, 3H), 7.88 - 7.57 (m, 3H), 7.43 (s, 1H), 7.24 (s, 4H), 6.91 (d, *J* = 6.8 Hz, 3H), 5.22 (s, 2H), 3.74 (s, 3H). MS: *m*/*z* = 348.1 (M+H)⁺.

### Example 67: N-(1-(3-methoxybenzyl)-1H-pyrazol-4-yl)-1H-indazole-3-carboxamide

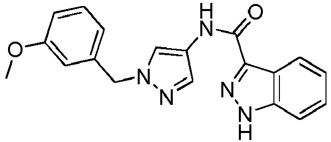

The title compound was synthesized according to example 49, substituting 3-methoxybenzyl alcohol for m-chlorobenzyl alcohol.¹H NMR (400 MHz, DMSO) δ 13.70 (s, 1H), 10.56 (s, 1H), 8.30 - 8.08 (m, 2H), 7.72 (s, 1H), 7.64 (d, *J* = 8.3 Hz, 1H), 7.44 (t, *J* = 7.6 Hz, 1H), 7.27 (t, *J* = 7.6 Hz, 2H), 6.84 (t, *J* = 14.5 Hz, 3H), 5.28 (s, 2H), 3.74 (s, 3H). MS: *m*/*z* = 348.1 (M+H)⁺.

### Example 68: N-(1-(3,4-difluorobenzyl)-1H-pyrazol-4-yl)-1H-indazole-3-carboxamide

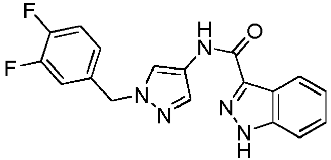

The title compound was synthesized according to example 49, substituting 3,4-difluorobenzyl alcohol for m-chlorobenzyl alcohol. ¹H NMR (400 MHz, DMSO) δ 13.69 (s, 1H), 10.59 (s, 1H), 8.22 (d, J = 10.8 Hz, 2H), 7.87 - 7.54 (m, 2H), 7.51 - 6.94 (m, 5H), 5.32 (s, 2H). MS: *m*/*z =* 354.0 (M+H)⁺.

### Example 69: N-(1-(2,5-difluorobenzyl)-1H-pyrazol-4-yl)-1H-indazole-3-carboxamide

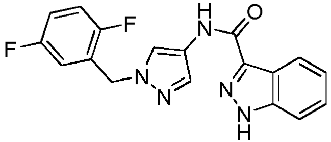

The title compound was synthesized according to example 49, substituting 2,5-difluorobenzyl alcohol for m-chlorobenzyl alcohol.¹H NMR (400 MHz, DMSO) δ 13.41 (s, 1H), 10.59 (s, 1H), 8.22 (d, J = 8.9 Hz, 2H), 7.81 - 7.54 (m, 2H), 7.33 (ddd, J = 20.2, 13.6, 5.8 Hz, 4H), 5.38 (s, 2H). MS: *m*/*z* = 354.0 (M+H)⁺.

### Example 70: N-(1-(3,5-dimethoxybenzyl)-1H-pyrazol-4-yl)-1H-indazole-3-carboxamide

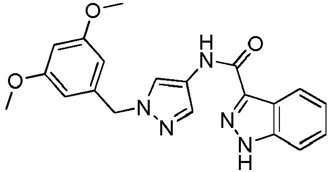

The title compound was synthesized according to example 49, substituting 3,5-dimethoxybenzyl alcohol for m-chlorobenzyl alcohol. ¹H NMR (400 MHz, DMSO) δ 13.88 - 13.28 (m, 1H), 10.55 (s, 1H), 8.37 - 8.00 (m, 2H), 7.84 - 7.57 (m, 2H), 7.51 - 7.34 (m, 1H), 7.26 (t, J = 7.5 Hz, 1H), 6.42 (dd, J = 6.3, 2.1 Hz, 3H), 5.23 (s, 2H). MS: *m*/*z* = 378.1 (M+H)⁺.

### Example 71: N-(1-(2,4-difluorobenzyl)-1H-pyrazol-4-yl)-1H-indazole-3-carboxamide

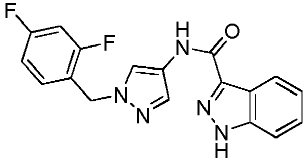

The title compound was synthesized according to example 49, substituting 2,4-difluorobenzyl alcohol for m-chlorobenzyl alcohol. ¹H NMR (400 MHz, DMSO) δ 13.66 (s, 1H), 10.57 (s, 1H), 8.40 - 8.07 (m, 3H), 7.84 - 7.54 (m, 3H), 7.35 (ddd, J = 21.4, 13.4, 7.1 Hz, 5H), 7.10 (t, J = 8.4 Hz, 1H), 5.35 (s, 3H). MS: *m*/*z =* 354.0 (M+H)⁺.

### Example 72: N-(1-(4-(trifluoromethoxy)benzyl)-1H-pyrazol-4-yl)-1H-indazole-3-carboxamide

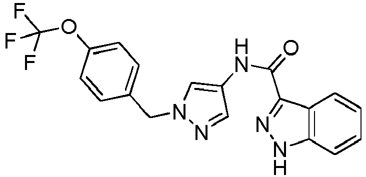

The title compound was synthesized according to example 49, substituting 4-(trifluoromethoxy)benzyl alcohol for m-chlorobenzyl alcohol. ¹H NMR (400 MHz, DMSO) δ 13.88 - 13.28 (m, 1H), 10.55 (s, 1H), 8.37 - 8.00 (m, 2H), 7.84 - 7.57 (m, 2H), 7.51 - 7.34 (m, 1H), 7.26 (t, J = 7.5 Hz, 1H), 6.42 (dd, J = 6.3, 2.1 Hz, 3H), 5.23 (s, 2H). MS: *m*/*z =* 402.0 (M+H)⁺.

### Example 73: N-(1-(2,4-dichlorobenzyl)-1H-pyrazol-4-yl)-1H-indazole-3-carboxamide

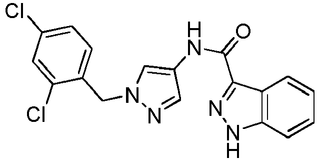

The title compound was synthesized according to example 49, substituting 2,4-dichlorobenzyl alcohol for m-chlorobenzyl alcohol. 1H NMR (400 MHz, DMSO) δ 13.88 - 13.28 (m, 1H), 10.55 (s, 1H), 8.37 - 8.00 (m, 2H), 7.84 - 7.57 (m, 2H), 7.51 - 7.34 (m, 1H), 7.26 (t, J = 7.5 Hz, 1H), 6.42 (dd, J = 6.3, 2.1 Hz, 3H), 5.23 (s, 2H). MS: *m*/*z* = 386.0 (M+H)⁺.

### Example 74: N-(1-(3-(3-Hydroxypiperidin-1-yl)propyl)-1H-pyrazol-4-yl)-1H-indazole-3-carboxamide

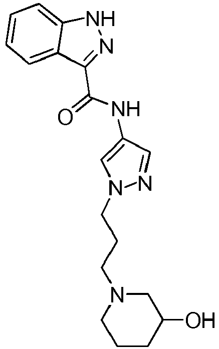

To a solution of 1-(3-(4-amino-1*H-*pyrazol-1-yl) propyl)piperidin-3-ol (0.20 g, 0.89 mmol) and 1*H-*indazole-3-carboxylic acid (1.4 g, 8.6 mmol) in THF (150 mL) was added HATU (5.0 g, 0.015 mol) and DIPEA (2.2 g, 0.020 mol). After the mixture was stirred at r.t. overnight, it was concentrated and part of the residue was purified by automated reverse-phase HPLC to give title compound (34 mg, 10%).¹H NMR (MeOD, 400 MHz) δ 8.26 (d, *J* = 8.4 Hz, 1H), 8.13 (s, 1H), 7.79 (s, 1H), 7.62 (t, *J* = 8.4 Hz, 1H), 7.44 (t, *J* = 7.6 Hz, 1H), 7.28 (d, *J* = 7.6 Hz, 1H), 4.29 (t, *J* = 6.0 Hz, 2H), 4.07 (s, 1H), 3.30-2.96 (m, 6H), 2.24-2.0 (m, 2H), 2.20-2.05 (m, 1H), 1.84-1.65 (m, 3H). MS: *m*/*z* = 368.9 (M+H)⁺.

### Example 75: N-(1-(2-(3-Hydroxypiperidin-1-yl)ethyl)-1H-pyrazol-4-yl)-1H-indazole-3-carboxamide

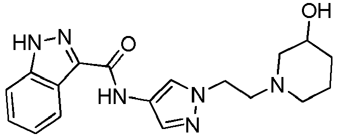

The title compound was synthesized according to Example 74, substituting 1-(2-(4-amino-1*H-*pyrazol-1-yl)ethyl)piperidin-3-ol for 1-(3-(4-amino-1*H-*pyrazol-1-yl) propyl)piperidin-3-ol.¹H NMR (MeOD, 400 MHz): δ 8.27 (d, *J* = 8.0 Hz, 1H), 8.22 (s, 1H), 7.82 (s, 1H), 7.61 (d, *J* = 8.4 Hz, 1H), 7.47-7.43 (m, 1H), 7.31-7.27 (m, 1H), 4.62-4.59 (m, 2H), 4.19 (s, 1H), 3.64-3.45 (m, 4H), 3.19-3.00 (m, 2H), 2.30-2.20 (m, 1 H), 1.95-1.70 (m, 3H). MS: *m*/*z* = 355.1 (M+H)⁺.

### Example 76: N-(1-(4-(3-Hydroxypiperidin-1-yl)butyl)-1H-pyrazol-4-yl)-1H-indazole-3-carboxamide

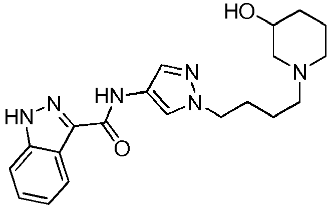

The title compound was synthesized according to Example 74, substituting 1-(4-(4-amino-1*H-*pyrazol-1-yl)butyl)piperidin-3-ol for 1-(3-(4-amino-1*H-*pyrazol-1-yl) propyl)piperidin-3-ol. ¹H NMR (MeOD, 400 MHz): δ 8.32 (d, *J* = 7.6 Hz, 1H), 8.27 (d, *J* = 8.4 Hz, 1H), 7.99-7.96 (m, 1H), 7.62 (d, *J* = 8.4 Hz, 1H), 7.47-7.43 (m, 1H), 7.29 (td, *J* = 8.0, 0.8 Hz, 1H), 4.34-4.31 (m, 2H), 4.16 (s, 1H), 3.41 (m, 2H), 3.10-3.03 (m, 4 H), 2.87 (s, 1H), 2.0-1.67 (m, 8H). MS: *m*/*z* = 382.9 (M+H)⁺.

### Example 77: N-(1-(2-(3-Cyanophenyl)propan-2-yl)-1H-pyrazol-4-yl)-1H-indazole-3-carboxamide

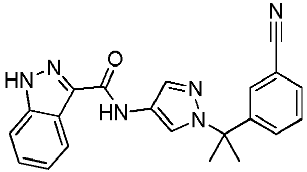

The title compound was synthesized according to Example 74, substituting 3-(2-(4-amino-1*H-*pyrazol-1-yl)propan-2-yl)benzonitrile for 1-(3-(4-amino-1*H*-pyrazol-1-yl) propyl)piperidin-3-ol. ¹H NMR (DMSO-*d*₆, 400 MHz): δ 13.74 (s, 1H), 10.67 (s, 1H), 8.210 (m, 2H), 7.82 (s, 1H), 7.72 (m, 1H), 7.63 (m, 1H), 7.49 (m, 2H), 7.44 (m, 1H), 7.22 (m, 2H), 1.95 (s, 6H). MS: *m*/*z* = 371.1 (M+H)⁺.

### Example 78: N-(1-(2-Phenylpropan-2-yl)-1H-pyrazol-4-yl)-1H-indazole-3-carboxamide

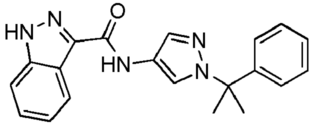

The title compound was synthesized according to Example 74, substituting 1-(2-phenylpropan-2-yl)-1*H*-pyrazol-4-amine for 1-(3-(4-amino-1*H*-pyrazol-1-yl) propyl)piperidin-3-01. ¹H NMR (MeOD, 400 MHz): δ 8.25 (d, *J=* 8.4 Hz, 1H), 8.22 (s, 1H), 7.80 (s, 1H), 7.60 (d, *J=* 8.8 Hz, 1H), 7.46-7.42 (m, 1H), 7.33-7.22 (m, 4H), 7.10-7.08 (m, 2H), 2.0 (s, 6H). MS: *mlz =* 367.9 (M+Na)⁺.

### Example 79: N-(1-(3-Cyanobenzyl)-1H-pyrazol-4-yl)-5-phenyl-1H-indazole-3-carboxamide

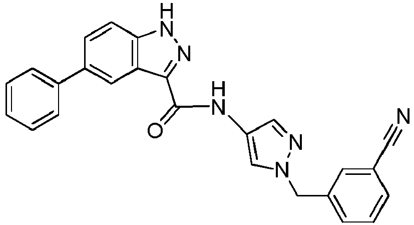

The title compound was synthesized according to Example 74, substituting 3-((4-amino-1*H-*pyrazol-1-yl)methyl)benzonitrile for 1-(3-(4-amino-1*H*-pyrazol-1-yl) propyl)piperidin-3-ol and 5-phenyl-1*H*-indazole-3-carboxylic acid for 1*H*-indazole-3-carboxylic acid. ¹H NMR (DMSO-*d6*, 400 MHz): δ 10.72 (s, 1H), 8.44 (s, 1H), 8.29 (s, 1H), 7.76-7.75 (m, 6H), 7.71 (m, 2 H), 7.58-7.57 (m, 2H), 7.52-7.48 (m, 2H), 7.38 (m, 1H), 5.40 (s, 1H). MS: *m*/*z =* 419.0 (M+H)⁺.

### Example 80: N-(1-(3-Cyanobenzyl)-1H-pyrazol-4-yl)-5-(1H-pyrazol-3-yl)-1H-indazole-3-carboxamide

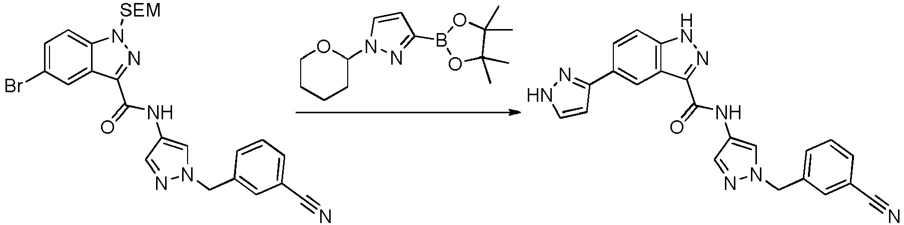

The title compound was synthesized according to Example 43, substituting 5-Bromo-*N*-(1-(3-cyanobenzyl)-1*H*-pyrazol-4-yl)-1-((2-(trimethylsilyl)ethoxy)methyl)-1*H*-indazole-3-carboxamide for 6-Bromo-1-(2-trimethylsilanyl-ethoxymethyl)-1*H*-indazole-3-carboxylic acid (1-thiazol-4-ylmethyl-1H-pyrazol-4-yl)-amide and 1-(tetrahydro-2*H*-pyran-2-yl)-3-(4,4,5,5-tetramethyl-1,3,2- dioxaborolan-2-yl)-1*H*-pyrazole for 1H-pyrazole-2-boronic acid. ¹H NMR (CDCl₃, 400 MHz): δ 8.59 (s, 1H), 8.14 (s, 1H), 7.85 *(d, J=* 8.8 Hz, 1H), 7.61 (m, 4H), 7.45 (m, 3H), 6.67 (s, 1H), 5.29 (s, 2H). MS: *m*/*z =* 409.1 (M+H)⁺.

### Example 81: N-(1-(3-Cyanobenzyl)-1H-pyrazol-4-yl)-5-(1H-pyrazol-3-yl)-1H-indazole-3-carboxamide

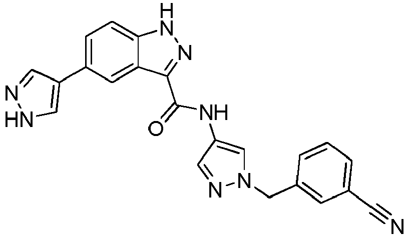

The title compound was synthesized according to Example 80, substituting 4-(4,4,5,5-tetramethyl-1,3,2-dioxaborolan-2-yl)-1-((2-(trimethylsilyl)ethoxy)methyl)-1*H*-pyrazole for 1-(tetrahydro-2*H*-pyran-2-yl)-3-(4,4,5,5-tetramethyl-1,3,2- dioxaborolan-2-yl)-1*H*-pyrazole. ¹H NMR (MeOD, 400 MHz): δ 8.46 (s, 1H), 8.24 (s, 1H), 8.07 (s, 2H), 7.80 (d, *J*= 0.8 Hz, 1H), 7.71 (m, 2H), 7.63 (m, 2H), 7.57 (m, 2H), 5.43 (s, 2H). MS: *m*/*z =* 409.1 (M+H)⁺.

### Example 82:

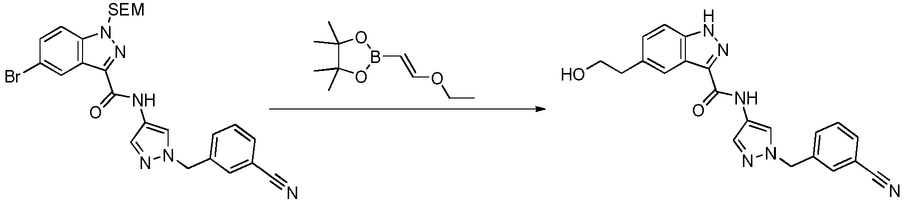

To a solution of 5-bromo-*N*-(1- (3-cyanobenzyl)-1*H*-pyrazol-4-yl)-1-((2-(trimethylsilyl)ethoxy)methyl)-1*H*-indazole-3-carboxamide (1.0 g, 1.8 mmol) in dioxane (100 mL) was added (*E*)-2-(2-ethoxyvinyl)- 4,4,5,5-tetramethyl-1,3,2-dioxaborolane (435 mg, 2.18 mmol), Pd(PPh₃)₂Cl₂ (165 mg, 0.18 mmol), and K₂CO₃ (497 mg, 3.6 mmol). After the mixture was stirred at 100 °C under N₂ overnight, the starting material was consumed as indicated by TLC. It was cooled to r.t., filtered, concentrated and purified by column chromatography (PE : EtOAc = 1:1 to 1:2) to give (*E*)-*N*-(1-(3-cyanobenzyl)-1*H*-pyrazol-4-yl)-5-(2-ethoxyvinyl)-1-((2-(trimethylsilyl)ethoxy)methyl)-1*H*-indazole-3-carboxamide (700 mg, 72%). The enol ether was diluted in EtOH (50 mL) and conc. HCl (3.0 mL) was added. After the reaction mixture was stirred at r.t. for 3 h, starting material was consumed as indicated by TLC. Saturated NaHCO₃ solution was added until pH = 8-9. It was extracted with EtOAc (50 mL × 3) and concentrated to give *N*-(1-(3-cyanobenzyl) -1*H*-pyrazol-4-yl)-5-(2-oxoethyl)-1-((2-(trimethylsilyl)ethoxy)methyl)-1*H*-indazole-3-carboxamide which was used without further purification (400 mg, 60%). To a solution of this aldehyde in MeOH (30 mL), was added NaBH₄ (45 mg, 1.1 mmol) and the mixture was stirred at r.t. overnight. After majority of the starting material was consumed as indicated by TLC, saturated NaHCO₃ (30 mL) was added and it was extracted with EtOAc (50 mL × 2) and concentrated to give *N*-(1-(3-cyanobenzyl)-1*H*-pyrazol-4-yl)-5-(2-hydroxyethyl) -1-((2-(trimethylsilyl)ethoxy)methyl)-1*H*-indazolc-3-carboxamide without further purification (280 mg, 69%). To a solution of this alcohol in EtOH (20 mL) was added conc. HCl (2.0 mL). After the mixture was heated at reflux for 5 h, starting material was consumed as indicated by TLC. It was concentrated and purified by automated reverse-phase HPLC to give the titled compound as white solid (11.5 mg, 5.5%). ¹H NMR (MeOD, 400 MHz) δ 8.21 (s, 1H), 8.11 (s, 1H), 7.78 (s, 1H), 7.68 -7.66 (m, 1H), 7.62, (s, 1H), 7.55 (m, 2H), 7.52 (d, *J=* 8.4 Hz, 1 H), 7.34 (dd, *J=* 8.4, 1.6 Hz, 1H), 5.41 (s, 2H), 3.81 (t, *J=* 7.2 Hz, 2H), 2.98 (t, *J=* 7.2 Hz, 2H). MS: *m*/*z* = 387.1 (M+H)⁺.

### Example 83: N-(1-ethyl-1H-pyrazol-4-yl)-6-(1H-pyrazol-4-yl)-1H-indazole-3-carboxamide

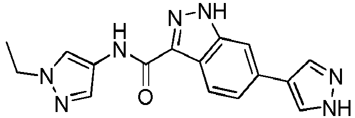

The title compound was synthesized according to Example 9, substituting bromoethane for 8-Bromomethyl-quinoline. ¹H NMR (400 MHz, DMSO) δ 13.01 (s, 2H), 10.47 (s, 1H), 8.26 - 8.11 (m, *J=* 8.5 Hz, 3H), 8.09 (s, 1H), 7.76 (s, 1H), 7.68 (s, 1H), 7.55 (d, *J=* 8.5 Hz, 1H), 4.12 (q, *J=* 7.3 Hz, 2H), 1.37 (t, *J*= 7.3 Hz, 4H). MS: *m*/*z* = 322.0 (M+H)⁺.

### Example 84: N-(1-benzyl-1H-pyrazol-4-yl)-6-(5-cyanopyridin-3-yl)-1H-indazole-3-carboxamide

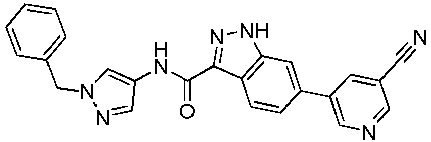

The title compound was synthesized according to Example 43, substituting N-(1-benzyl-1H-pyrazol-4-yl)-6-bromo-1-((2-(trimethylsilyl)ethoxy)methyl)-1H-indazole-3-carboxamide for 6-Bromo-1-(2-trimethylsilanyl-ethoxymethyl)-1H-indazole-3-carboxylic acid (1-thiazol-4-ylmethyl-1H-pyrazol-4-yl)-amide and 3-cyanopyridine-5-boronic acid pinacol ester for 1H-pyrazole-2-boronic acid. ¹H NMR (400 MHz, DMSO) δ 10.63 (s, 1H), 9.28 (d, *J*= 1.9 Hz, 1H), 9.04 (s, 1H), 8.75 (s, 1H), 8.32 (d, *J*= 8.5 Hz, 1H), 8.19 (s, 1H), 8.05 (s, 1H), 7.76 - 7.66 (m, 2H), 7.40 - 7.22 (m, 6H), 5.32 (s, 2H). MS: *m*/*z =* 420.3 (M+H)⁺.

### Example 85: N-(1-benzyl-1H-pyrazol-4-yl)-6-(5-methoxypyridin-3-yl)-1H-indazole-3-carboxamide

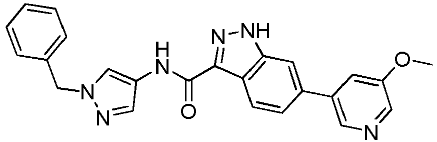

The title compound was synthesized according to Example 43, substituting N-(1-benzyl-1H-pyrazol-4-yl)-6-bromo-1-((2-(trimethylsilyl)ethoxy)methyl)-1H-indazole-3-carboxamide for 6-Bromo-1-(2-trimethylsilanyl-ethoxymethyl)-1H-indazole-3-carboxylic acid (1-thiazol-4-ylmethyl-1H-pyrazol-4-yl)-amide and 5-methoxy-3-pyridineboronic acid pinacol ester for 1H-pyrazole-2-boronic acid. ¹H NMR (400 MHz, DMSO) δ 10.61 (s, 1H), 8.57 (s, 1H), 8.31 (dd, *J=* 12.6, 5.5 Hz, 2H), 8.19 (s, 1H), 7.93 (s, 1H), 7.73 (d, *J =* 6.2 Hz, 2H), 7.64 (d, *J =* 8.6 Hz, 1H), 7.44 - 7.19 (m, 5H), 5.32 (s, 2H), 3.94 (s, 3H). MS: *m*/*z =* 425.0 (M+H)⁺.

### Example 86: N-(1-(3-cyanobenzyl)-1H-pyrazol-4-yl)-6-(pyrrolidin-1-yl)-1H-indazole-3-carboxamide

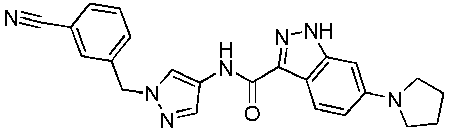

The title compound was synthesized according to Example 1, substituting 6-(pyrrolidin-1-yl)-1H-indazole-3-carboxylic acid for 5-chloro-1H-indazole-3-carboxylic acid. ¹H NMR (400 MHz, DMSO) δ 12.97 (s, 1H), 10.41 (s, 1H), 8.23 (s, 1H), 7.94 (d, *J=* 8.9 Hz, 1H), 7.77 (d, *J*= 5.4 Hz, 1H), 7.73 (s, 1H), 7.70 (s, 1H), 7.61 - 7.53 (m, 2H), 6.73 (d, *J*= 9.0 Hz, 1H), 6.39 (s, 1H), 5.39 (s, 2H), 1.99 (t, *J*= 6.3 Hz, 4H). MS: *m*/*z =* 412.1 (M+H)⁺.

### Example 87: N-(1-benzyl-1H-pyrazol-4-yl)-6-(1-methyl-1H-imidazol-4-yl)-1H-indazole-3-carboxamide

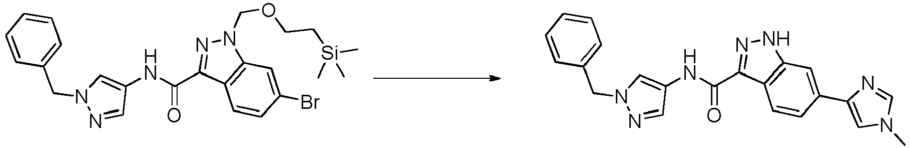

A vial was charged with 6-Bromo-1-(2-trimethylsilanyl-ethoxymethyl)-1H-indazole-3-carboxylic acid (1-benzyl-1H-pyrazol-4-yl)-amide (0.202 g, 0.383 mmol) and 4-(butyldipentylstannyl)-1-methyl-1H-imidazole (0.344 g, 1.15 mmol). The reaction mixture was purged with N₂ and Bis(di-tert-butyl(4-dimethylaminophenyl)phosphine)dichloropalladium(II) (0.0271 g, 0.0383 mmol) and Acetonitrile (2.00 mL) was added. The vial was sealed and thermally heated at 80 °C for 2 hours. The cooled reaction mixture was diluted with a large volume of EtOAc and the organic was washed with water, saline and dried (Na₂SO₄), then conc. in *vacuo* to a solid. This residue was purified by CombiFlash (12 g; dry load; 90:10 to 0:100 heptane:EtOAc) to provide 96 mg (>100%) of N-(1-benzyl-1H-pyrazol-4-yl)-6-(1-methyl-1H-imidazol-4-yl)-1-((2-(trimethylsilyl)ethoxy)methyl)-1H-indazole-3-carboxamide. The solid was diluted with TFA (1 mL) and triisopropylsilane (5.0 equiv.) and a few drops of CH₂Cl₂. The resulting solution was stirred for 90 minutes, then concentrated in vacuo. The residue was purified by automated reverse phase HPLC to provide the title compound. ¹H NMR (400 MHz, DMSO) δ 10.60 (s, 1H), 8.25 (d, *J=* 8.4 Hz, 1H), 8.18 (s, 1H), 7.74 (d, *J=* 6.8 Hz, 2H), 7.68 (s, 1H), 7.45 - 7.21 (m, 6H), 7.16 (s, 1H), 5.32 (s, 2H), 3.74 (s, 3H). MS: *m*/*z* = 398.0 (M+H)⁺.

### Example 88: N-(1-(3-cyanobenzyl)-1H-pyrazol-4-yl)-6-(3-hydroxypyrrolidin-1-yl)-1H-indazole-3-carboxamide

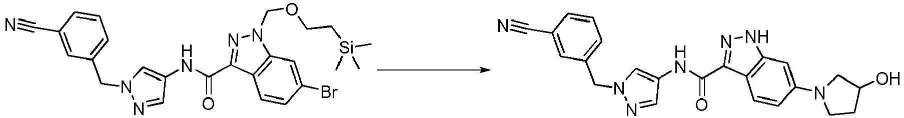

To a solution of 6-bromo-N-[1-[(3-cyanophenyl)methyl]pyrazol-4-yl]-1-(2-trimethylsilylethoxymethyl) indazole-3-carboxamide in t-BuOH in a vial was added cesium carbonate, (RuPhos)Palladium(II) phenethylamine chloride, RuPhos (ligand) and 3-hydroxypyrrolidine. After sealing the reaction vial, the mixture was degassed by bubbling N₂ through the slurry for several minutes The sealed vial was then heated at 80 °C for 2 hours. The reaction mixture was diluted with EtOAc and then washed with water, saline, dried (Na₂SO₄), then concentrated to a solid. Purification by CombiFlash (12 g; dry load; 90:10 to 10:90 heptane:EtOAc) provided 170 mg of N-(1-(3-cyanobenzyl)-1H-pyrazol-4-yl)-6-(3-hydroxypyrrolidin-1-yl)-1-((2-(trimethylsilyl)ethoxy)methyl)-1H-indazole-3-carboxamide. he solid was diluted with TFA (1 mL) and triisopropylsilane (5.0 equiv.) and a few drops of CH₂Cl₂. The resulting solution was stirred for 90 minutes, then concentrated in vacuo. The residue was purified by automated reverse phase HPLC to provide the title compound. ¹H NMR (400 MHz, DMSO) δ 12.97 (s, 1H), 10.41 (s, 1H), 8.23 (s, 1H), 7.94 *(d, J=* 8.9 Hz, 1H), 7.74 (dd, *J=* 23.2, 9.3 Hz, 3H), 7.63 - 7.47 (m, 2H), 6.70 (d, *J=* 8.9 Hz, 1H), 6.37 (s, 1H), 5.39 (s, 2H), 4.96 (s, 1H), 4.43 (s, 1H). MS: *m*/*z =* 429.1 (M+H)⁺.

### Example 89: N-(1-(3-((dimethylamino)methyl)benzyl)-1H-pyrazol-4-yl)-1H-indazole-3-carboxamide

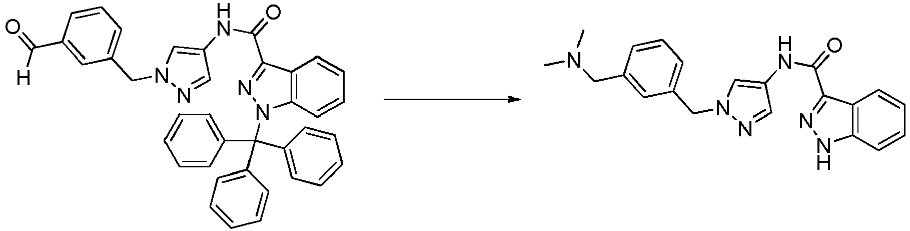

To a solution of N-(1-(3-formylbenzyl)-1H-pyrazol-4-yl)-1-trityl-1H-indazole-3-carboxamide (115 mg, 0.20 mmol) in methanol (3 mL) was added dimethylamine HCl (160 mg, 2.0 mmol), then sodium cyanoborohydride (291 mg, 1.4 mmol). The mixture was stirred for one hour at rt then the mixture was diluted with 20 mL EtOAc and washed with 2 x 200 mL 1:1 H₂O:brine. The organic extracts were dried (Na₂SO₄) and concentrated in vacuo. The residue was diluted with 1.0 mL trifluoroacetic acid, then triisopropylsilane (80.9 µL, 0.394 mmol) and a few drops of CH₂Cl₂ were added. The mixture was stirred for 2 hours at rt. The mixture was concentrated in vacuo, then purified by automated reverse-phase HPLC, which provided 25 mg (32%) of the title compound. ¹H NMR (400 MHz, DMSO) δ 13.68 (s, 1H), 10.56 (s, 1H), 8.21 (dd, *J=* 20.4, 12.2 Hz, 2H), 7.64 (dd, *J*= 34.7, 26.2 Hz, 2H), 7.44 *(t, J=* 7.6 Hz, 1H), 7.22 (ddd, *J*= 37.0, 18.7, 5.6 Hz, 7H), 5.30 (s, 3H), 3.34 (d,*J*= 16.7 Hz, 6H), 2.13 (s, 8H). MS: *m*/*z*= 375.2 (M+H)⁺.

### Example 90: N-(1-(3-((2-hydroxy-2-methylpropylamino)methyl)benzyl)-1H-pyrazol-4-yl)-1H-indazole-3-carboxamide

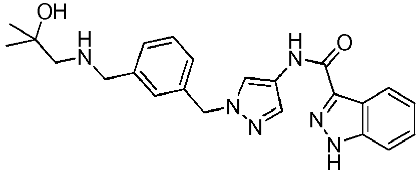

The title compound was synthesized according to Example 89, substituting 1-amino-2-methylpropan-2-ol for dimethylamine HCl. ¹H NMR (400 MHz, DMSO) δ 13.67 (s, 1H), 10.55 (s, 1H), 8.21 (d, *J* = 8.1 Hz, 1H), 8.16 (s, 1H), 7.71 (s, 1H), 7.64 (d, *J* = 8.4 Hz, 1H), 7.43 (t, *J =* 7.6 Hz, 1H), 7.32 - 7.23 (m, 4H), 7.10 (d, *J=* 7.0 Hz, 1H), 5.29 (s, 2H), 4.13 (s, 1H), 3.71 (s, 2H), 2.36 (s, 2H), 1.08 (s, 7H). MS: *m*/*z* = 419.2 (M+H)⁺.

### Example 91: N-(1-(3-(azetidin-1-ylmethyl)benzyl)-1H-pyrazol-4-yl)-1H-indazole-3-carboxamide

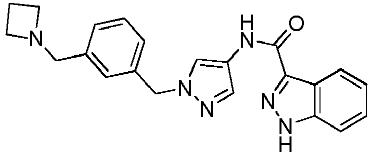

The title compound was synthesized according to Example 89, substituting azetedine for dimethylamine HCl. ¹H NMR (400 MHz, DMSO) δ 13.71 (s, 1H), 10.56 (s, 1H), 8.21 (d, *J=* 8.1 Hz, 1H), 8.16 (s, 1H), 7.72 (s, 1H), 7.64 (d, *J* = 8.4 Hz, 1H), 7.43 (t, *J =* 7.6 Hz, 1H), 7.27 (dt, *J* = 7.4, 2.7 Hz, 2H), 7.20 - 7.15 (m, 2H), 7.10 (d, *J=* 7.6 Hz, 1H), 5.29 (s, 2H), 3.48 (s, 2H), 3.09 (t, *J*= 6.9 Hz, 5H), 1.95 (p, *J*= 6.9 Hz, 2H). MS: *m*/*z* = 387.1 (M+H)⁺.

### Example 92: N-(1-(3-((2,2-difluoroethylamino)methyl)benzyl)-1H-pyrazol-4-yl)-1H-indazole-3-carboxamide

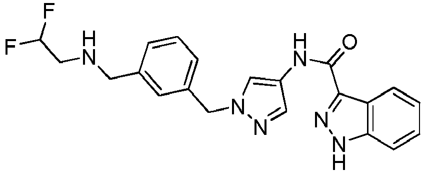

The title compound was synthesized according to Example 89, substituting 2,2-difluoroethanamine for dimethylamine HCl. MS: *m*/*z* = 411.1 (M+H)⁺.

### Example 93: N-(1-(3-((2-hydroxypropylamino)methyl)benzyl)-1H-pyrazol-4-yl)-1H-indazole-3-carboxamide

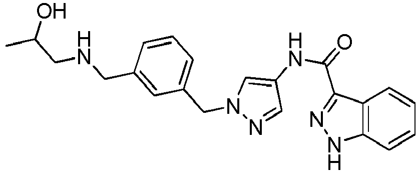

The title compound was synthesized according to Example 89, substituting 1-aminopropan-2-ol for dimethylamine HCl. ¹H NMR (400 MHz, DMSO) δ 13.67 (s, 1H), 10.55 (s, 1H), 8.21 (d, *J*= 8.1 Hz, 1H), 8.16 (s, 1H), 7.72 (s, 1H), 7.64 (d, *J* = 8.4 Hz, 1H), 7.43 (t, *J* = 7.6 Hz, 1H), 7.32-7.23 (m, 4H), 7.10 (d, *J=* 7.0 Hz, 1H), 5.29 (s, 2H), 4.41 (d, *J=* 4.2 Hz, 1H), 3.68 (s, 2H), 2.40 (d, *J=* 5.9 Hz, 2H), 1.98 (s, 1H), 1.02 (d, *J=* 6.2 Hz, 3H). MS: *m*/*z* = 405.2 (M+H)⁺.

### Example 94: N-(1-(3-((cyclobutylamino)methyl)benzyl)-1H-pyrazol-4-yl)-1H-indazole-3-carboxamide

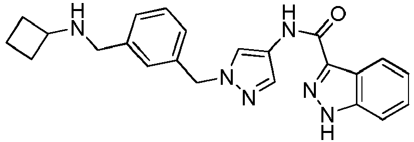

The title compound was synthesized according to Example 89, substituting cyclobutylamine for dimethylamine HCl. ¹H NMR (400 MHz, DMSO) δ 10.54 (s, 1H), 8.20 (d, *J=* 8.1 Hz, 1H), 8.14 (s, 1H), 7.71 (s, 1H), 7.63 (d, *J =* 8.4 Hz, 1H), 7.42 *(t, J =* 7.6 Hz, 1H), 7.31 - 7.23 (m, 3H), 7.20 (s, 1H), 7.18 (s, 1H), 5.27 (s, 2H), 3.57 (s, 2H), 3.16 - 3.06 (m, 1H), 2.09 - 2.00 (m, 2H), 1.72-1.43 (m, 4H). MS: *m*/*z* = 401.2 (M+H)⁺.

### Example 95: N-(1-(3-((cyclopropylmethylamino)methyl)benzyl)-1H-pyrazol-4-yl)-1H-indazole-3-carboxamide

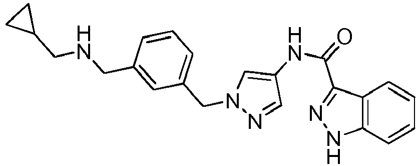

The title compound was synthesized according to Example 89, substituting cyclopropylmethylamine for dimethylamine HCl. ¹H NMR (400 MHz, DMSO) δ 13.70 (s, 1H), 10.56 (s, 1H), 8.21 (d, *J* = 8.1 Hz, 1H), 8.16 (s, 1H), 7.72 (s, 1H), 7.64 (d, *J* = 8.4 Hz, 1H), 7.44 (t, *J =* 7.6 Hz, 1H), 7.32 - 7.24 (m, 4H), 7.12 (d, *J =* 6.8 Hz, 1H), 5.29 (s, 2H), 3.72 (s, 2H), 2.38 (d, *J =* 6.7 Hz, 2H), 0.95 - 0.82 (m, 1H), 0.43 - 0.34 (m, 2H), 0.12 - 0.05 (m, 2H). MS: *m*/*z* = 401.2 (M+H)⁺.

### Example 96: N-(1-(3-((2-hydroxyethylamino)methyl)benzyl)-1H-pyrazol-4-yl)-1H-indazole-3-carboxamide

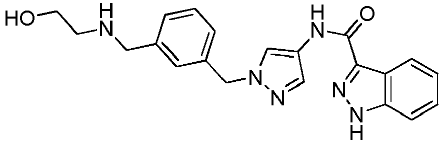

The title compound was synthesized according to Example 89, substituting ethanolamine for dimethylamine HCl. ¹H NMR (400 MHz, DMSO) δ 13.69 (s, 1H), 10.56 (s, 1H), 8.21 (d, *J=* 8.2 Hz, 1H), 8.16 (s, 1H), 7.72 (s, 1H), 7.64 (d, *J* = 8.4 Hz, 1H), 7.44 (t, *J* = 7.6 Hz, 1H), 7.32 - 7.23 (m, 4H), 7.11 (d, *J=* 7.0 Hz, 1H), 5.29 (s, 2H), 4.44 (s, 1H), 3.70 (s, 2H), 3.46 (t, *J=* 5.7 Hz, 2H), 2.57 (t, *J=* 5.8 Hz, 2H). MS: *m*/*z* = 391.1 (M+H)⁺.

### Example 97: N-(1-(3-((propylamino)methyl)benzyl)-1H-pyrazol-4-yl)-1H-indazole-3-carboxamide

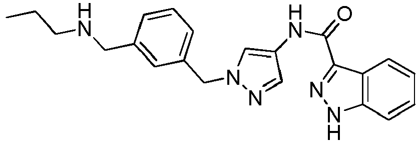

The title compound was synthesized according to Example 89, substituting propylamine for dimethylamine HCl. ¹H NMR (400 MHz, DMSO) δ 13.69 (s, 1H), 10.56 (s, 1H), 8.21 (d, *J=* 8.2 Hz, 1H), 8.16 (s, 1H), 7.72 (s, 1H), 7.64 (d, *J* = 8.4 Hz, 1H), 7.44 (t, *J* = 7.6 Hz, 1H), 7.32 - 7.23 (m, 4H), 7.11 (d, *J=* 6.9 Hz, 1H), 5.29 (s, 2H), 3.68 (s, 2H), 2.45 (t, *J*= 7.1 Hz, 2H), 1.48 - 1.36 (m, 2H), 0.85 (t, *J*= 7.4 Hz, 3H). MS: *m*/*z* = 389.2 (M+H)⁺.

### Example 98: N-(1-(3-((cyclopropylamino)methyl)benzyl)-1H-pyrazol-4-yl)-1H-indazole-3-carboxamide

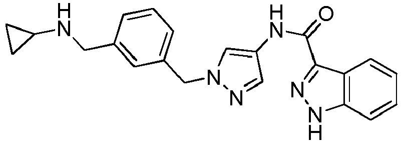

The title compound was synthesized according to Example 89, substituting cyclopropylamine for dimethylamine HCl. MS: *m*/*z* = 387.2 (M+H)⁺.

### Example 99: N-(1-(3-((ethylamino)methyl)benzyl)-1H-pyrazol-4-yl)-1H-indazole-3-carboxamide

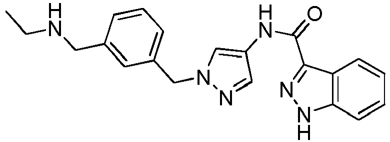

The title compound was synthesized according to Example 89, substituting ethylamine for dimethylamine HCl. ¹H NMR (400 MHz, DMSO) δ 13.69 (s, 1H), 10.56 (s, 1H), 8.21 (d, *J=* 8.1 Hz, 1H), 8.16 (s, 1H), 7.72 (s, 1H), 7.64 (d, *J* = 8.4 Hz, 1H), 7.44 (t, *J* = 7.6 Hz, 1H), 7.31 - 7.22 (m, 4H), 7.10 (d, *J=* 6.9 Hz, 1H), 5.29 (s, 2H), 3.66 (s, 2H), 2.53 (q, *J=* 7.1 Hz, 2H), 1.01 (t, *J*= 7.1 Hz, 3H). MS: *m*/*z* = 375.2 (M+H)⁺.

### Example 100: N-(1-(3-(((2-cyanoethyl)(methyl)amino)methyl)benzyl)-1H-pyrazol-4-yl)-1H-indazole-3-carboxamide

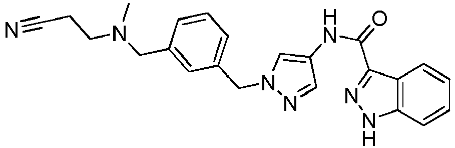

The title compound was synthesized according to Example 89, substituting 3-(methylamino)propanenitrile for dimethylamine HCl. ¹H NMR (400 MHz, DMSO) δ 13.67 (s, 1H), 10.5 (s, 1H), 8.21 (d, *J* = 8.2 Hz, 1H), 8.16 (s, 1H), 7.72 (s, 1H), 7.64 (d, *J* = 8.4 Hz, 1H), 7.43 (t, *J=* 7.6 Hz, 1H), 7.34 - 7.23 (m, 4H), 7.12 (d, *J=* 7.4 Hz, 1H), 5.30 (s, 2H), 3.52 (s, 2H), 2.71 - 2.65 (m, 2H), 2.64 - 2.58 (m, 2H), 2.16 (s, 3H). MS: *m*/*z =* 414.2 (M+H)⁺.

### Example 101: N-(1-(3-((methylamino)methyl)benzyl)-1H-pyrazol-4-yl)-1H-indazole-3-carboxamide

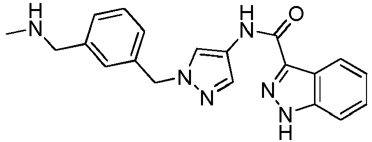

The title compound was synthesized according to Example 89, substituting methylamine HCl for dimethylamine HCl. ¹H NMR (400 MHz, DMSO) δ 10.56 (s, 1H), 8.21 (d, *J=* 8.1 Hz, 1H), 8.16 (s, 1H), 7.72 (s, 1H), 7.64 (d, *J* = 8.4 Hz, 1H), 7.43 (t, *J* = 7.6 Hz, 1H), 7.32 - 7.21 (m, 4H), 7.11 *(d, J=* 7.3 Hz, 1H), 5.29 (s, 2H), 3.61 (s, 2H), 2.25 (s, 3H). MS: *m*/*z* = 361.1 (M+H)⁺.

### Example 102: N-(1-(3-((2-(dimethylamino)ethylamino)methyl)benzyl)-1H-pyrazol-4-yl)-1H-indazole-3-carboxamide

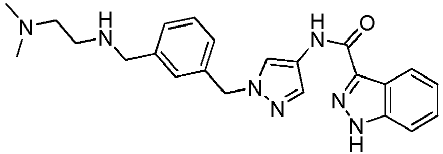

The title compound was synthesized according to Example 89, substituting N1,N1-dimethylethane-1,2-diamine for dimethylamine HCl. ¹H NMR (400 MHz, DMSO) δ 10.54 (s, 1H), 8.20 (d, *J* = 8.1 Hz, 1H), 8.15 (s, 1H), 7.71 (s, 1H), 7.63 (d, *J* = 8.4 Hz, 1H), 7.42 (t, *J =* 7.6 Hz, 1H), 7.31 - 7.21 (m, 4H), 7.11 (d, *J*= 7.4 Hz, 1H), 5.29 (s, 2H), 3.67 (s, 2H), 2.54 (t, *J=* 6.4 Hz, 2H), 2.29 (t, *J*= 6.4 Hz, 2H), 2.08 (s, 6H). MS: *m*/*z* = 418.2 (M+H)⁺.

### Example 103: N-(1-(4-(azetidin-1-ylmethyl)benzyl)-1H-pyrazol-4-yl)-1H-indazole-3-carboxamide

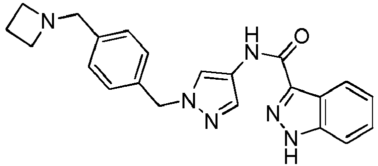

The title compound was synthesized according to Example 89, substituting azetidine for dimethylamine HCl and N-(1-(4-formylbenzyl)-1H-pyrazol-4-yl)-1-trityl-1H-indazole-3-carboxamide for N-(1-(3-formylbenzyl)-1H-pyrazol-4-yl)-1-trityl-1H-indazole-3-carboxamide. ¹H NMR (400 MHz, DMSO) δ 13.68 (s, 1H), 10.56 (s, 1H), 8.21 (d, *J* = 8.1 Hz, 1H), 8.15 (s, 1H), 7.71 (s, 1H), 7.64 (d, *J* = 8.4 Hz, 1H), 7.44 (t, *J* = 7.6 Hz, 1H), 7.27 (d, *J* = 7.6 Hz, 1H), 7.25 - 7.14 (m, 5H), 5.27 (s, 3H), 3.48 (s, 2H), 3.09 (t, *J* = 6.9 Hz, 3H), 1.95 (p, *J* = 6.9 Hz, 2H). MS: *m*/*z* = 387.2 (M+H)⁺.

### Example 104: N-(1-(4-((2-(dimethylamino)ethylamino)methyl)benzyl)-1H-pyrazol-4-yl)-1H-indazole-3-carboxamide

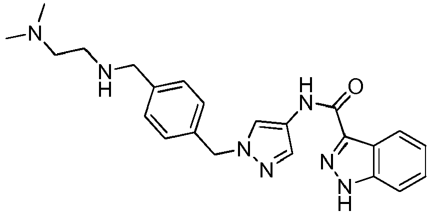

The title compound was synthesized according to Example 103, substituting N1,N1-dimethylethane-1,2-diamine for azetidine. ¹H NMR (400 MHz, DMSO) δ 10.54 (s, 1H), 8.20 (d, *J* = 8.2 Hz, 1H), 8.14 (s, 1H), 7.71 (s, 1H), 7.64 (d, *J* = 8.4 Hz, 1H), 7.43 (t, *J* = 7.6 Hz, 1H), 7.32 - 7.17 (m, 6H), 5.28 (s, 2H), 3.67 (s, 2H), 2.53 (t, *J*= 5.1 Hz, 2H), 2.29 (t, *J*= 6.4 Hz, 2H), 2.09 (s, 6H). MS: *m*/*z* = 418.2 (M+H)⁺.

### Example 105: N-(1-(4-((2,2-difluoroethylamino)methyl)benzyl)-1H-pyrazol-4-yl)-1H-indazole-3-carboxamide

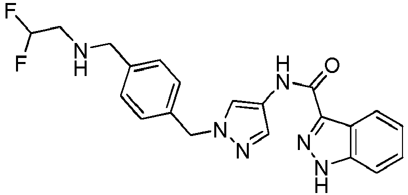

The title compound was synthesized according to Example 103, substituting 2,2-difluoroethanamine for azetidine. ¹H NMR (400 MHz, DMSO) δ 13.67 (s, 1H), 10.55 (s, 1H), 8.21 (d, *J* = 8.1 Hz, 1H), 8.15 (s, 1H), 7.71 (s, 1H), 7.64 (d, *J* = 8.4 Hz, 1H), 7.43 (t, *J =* 7.6 Hz, 1H), 7.33 - 7.19 (m, 5H), 5.98 (tt, *J=* 56.4, 4.2 Hz, 1H), 5.29 (s, 2H), 3.72 (s, 2H), 2.82 (td, *J=* 15.9, 4.2 Hz, 2H). MS: *m*/*z* = 411.1 (M+H)⁺.

### Example 106: N-(1-(4-((2-hydroxypropylamino)methyl)benzyl)-1H-pyrazol-4-yl)-1H-indazole-3-carboxamide

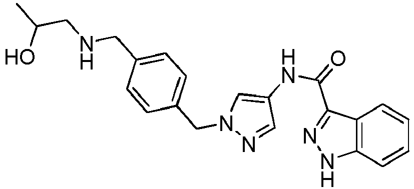

The title compound was synthesized according to Example 103, substituting 1-aminopropan-2-ol for azetidine. ¹H NMR (400 MHz, DMSO) δ 10.54 (s, 1H), 8.20 (d, *J=* 8.1 Hz, 1H), 8.15 (s, 1H), 7.71 (s, 1H), 7.63 (d, *J* = 8.4 Hz, 1H), 7.43 (t, *J* = 7.6 Hz, 1H), 7.32 - 7.18 (m, 5H), 5.28 (s, 2H), 4.39 (d, *J=* 3.9 Hz, 1H), 3.72 - 3.62 (m, 3H), 2.38 (d, *J*= 5.9 Hz, 2H), 1.02 (d, *J=* 6.2 Hz, 3H). MS: *m*/*z* = 405.2 (M+H)⁺.

### Example 107: N-(1-(4-((cyclobutylamino)methyl)benzyl)-1H-pyrazol-4-yl)-1H-indazole-3-carboxamide

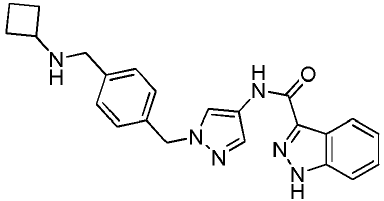

The title compound was synthesized according to Example 103, substituting cyclobutylamine for azetidine. ¹H NMR (400 MHz, DMSO) δ 10.54 (s, 1H), 8.20 *(d, J=* 8.1 Hz, 1H), 8.14 (s, 1H), 7.71 (s, 1H), 7.63 (d, *J* = 8.4 Hz, 1H), 7.42 *(t, J =* 7.6 Hz, 1H), 7.31 - 7.16 (m, 5H), 5.27 (s, 2H), 3.57 (s, 2H), 3.16 - 3.06 (m, 1H), 2.09 - 2.00 (m, 2H), 1.72 - 1.44 (m, 4H). MS: *m*/*z =* 401.2 (M+H)⁺.

### Example 108: N-(1-(4-((cyclopropylmethylamino)methyl)benzyl)-1H-pyrazol-4-yl)-1H-indazole-3-carboxamide

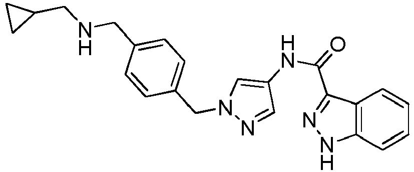

The title compound was synthesized according to Example 103, substituting cyclopropylmethylamine for azetidine. ¹H NMR (400 MHz, DMSO) δ 13.70 (s, 1H), 10.56 (s, 1H), 8.21 (d, *J* = 8.1 Hz, 1H), 8.15 (s, 1H), 7.71 (s, 1H), 7.64 (d, *J* = 8.4 Hz, 1H), 7.44 (t, *J =* 7.6 Hz, 1H), 7.31 (d, *J=* 8.0 Hz, 2H), 7.26 (t, *J=* 7.5 Hz, 1H), 7.21 (d, *J=* 8.0 Hz, 2H), 5.28 (s, 2H), 3.73 (s, 2H), 2.39 (d, *J=* 6.7 Hz, 2H), 0.96 - 0.82 (m, 1H), 0.43 - 0.36 (m, 2H), 0.12 - 0.05 (m, 2H). MS: *m*/*z* = 401.2 (M+H)⁺.

### Example 109: N-(1-(4-((propylamino)methyl)benzyl)-1H-pyrazol-4-yl)-1H-indazole-3-carboxamide

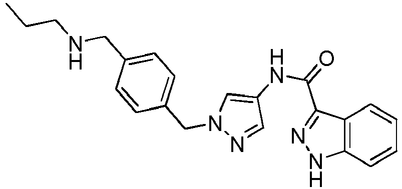

The title compound was synthesized according to Example 103, substituting propylamine for azetidine. ¹H NMR (400 MHz, DMSO) δ 13.69 (s, 1H), 10.55 (s, 1H), 8.21 (d, *J*= 8.1 Hz, 1H), 8.15 (s, 1H), 7.71 (s, 1H), 7.64 (d, *J* = 8.4 Hz, 1H), 7.44 (t, *J =* 7.6 Hz, 1H), 7.31 (d, *J* = 8.0 Hz, 2H), 7.26 *(t, J=* 7.5 Hz, 1H), 7.21 (d, *J=* 8.0 Hz, 2H), 5.28 (s, 2H), 3.69 (s, 3H), 2.46 (t, *J=* 7.2 Hz, 2H), 1.49 - 1.37 (m, 2H), 0.85 *(t, J=* 7.4 Hz, 3H). MS: *m*/*z* = 389.2 (M+H)⁺.

### Example 110: N-(1-(4-((ethylamino)methyl)benzyl)-1H-pyrazol-4-yl)-1H-indazole-3-carboxamide

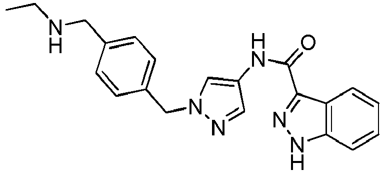

The title compound was synthesized according to Example 103, substituting ethylamine for azetidine. ¹H NMR (400 MHz, DMSO) δ 13.69 (s, 1H), 10.55 (s, 1H), 8.21 (d, *J*= 8.1 Hz, 1H), 8.15 (s, 1H), 7.71 (s, 1H), 7.64 (d, *J* = 8.4 Hz, 1H), 7.44 (t, *J =* 7.6 Hz, 1H), 7.32 - 7.18 (m, 5H), 5.29 (d, *J*= 11.0 Hz, 2H), 3.67 (s, 2H), 2.53 (q, *J=* 7.1 Hz, 2H), 1.01 (t, *J=* 7.1 Hz, 3H). MS: *m*/*z* = 375.2 (M+H)⁺.

### Example 111: N-(1-(4-(((2-cyanoethyl)(methyl)amino)methyl)benzyl)-1H-pyrazol-4-yl)-1H-indazole-3-carboxamide

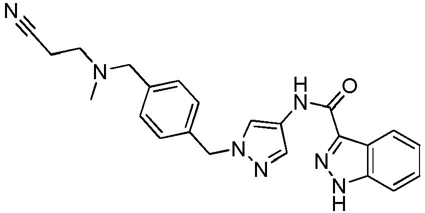

The title compound was synthesized according to Example 103, substituting 3-(methylamino)propanenitrile for azetidine. ¹H NMR (400 MHz, DMSO) δ 13.68 (s, 1H), 10.56 (s, 1H), 8.21 (d, *J* = 8.1 Hz, 1H), 8.17 (s, 1H), 7.72 (s, 1H), 7.64 (d, *J* = 8.4 Hz, 1H), 7.43 (t, *J* =7.6 Hz, 1H), 7.32 - 7.19 (m, 5H), 5.30 (s, 2H), 3.51 (s, 2H), 2.71 - 2.65 (m, 2H), 2.62 - 2.57 (m, 2H), 2.15 (s, 3H). MS: *m*/*z* = 414.2 (M+H)⁺.

### Example 112: N-(1-(4-((methylamino)methyl)benzyl)-1H-pyrazol-4-yl)-1H-indazole-3-carboxamide

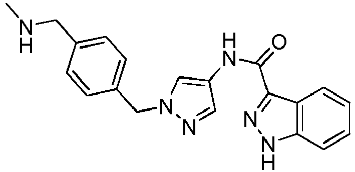

The title compound was synthesized according to Example 103, substituting methylamine HCl for azetidine. ¹H NMR (400 MHz, DMSO) δ 13.66 (s, 1H), 10.56 (s, 1H), 8.21 (d, *J=* 8.1 Hz, 1H), 8.15 (s, 1H), 7.71 (s, 1H), 7.64 (d, *J* = 8.4 Hz, 1H), 7.44 *(t, J =* 7.6 Hz, 1H), 7.32 - 7.18 (m, 5H), 5.28 (s, 2H), 3.63 (s, 2H), 2.25 (s, 3H). MS: *m*/*z* = 361.1 (M+H)⁺.

### Example 113: N-(1-(4-((2-hydroxy-2-methylpropylamino)methyl)benzyl)-1H-pyrazol-4-yl)-1H-indazole-3-carboxamide

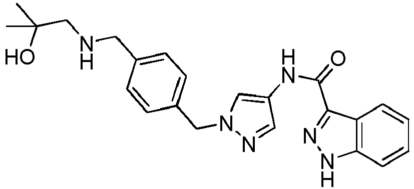

The title compound was synthesized according to Example 103, substituting 1-amino-2-methylpropan-2-ol for azetidine. ¹H NMR (400 MHz, DMSO) δ 13.75 (s, 1H), 10.56 (s, 1H), 8.21 (d, *J =* 8.1 Hz, 1H), 8.16 (s, 1H), 7.71 (s, 1H), 7.64 (d, *J* = 8.4 Hz, 1H), 7.44 (t, *J =* 7.6 Hz, 1H), 7.32 (d, *J=* 7.9 Hz, 2H), 7.26 *(t, J=* 7.5 Hz, 1H), 7.21 (d, *J=* 7.9 Hz, 2H), 5.28 (s, 2H), 3.72 (s, 2H), 2.37 (s, 2H), 1.08 (s, 6H). MS: *m*/*z* = 419.2 (M+H)⁺.

### Example 114: N-(1-(4-((3-hydroxypyrrolidin-1-yl)methyl)benzyl)-1H-pyrazol-4-yl)-1H-indazole-3-carboxamide

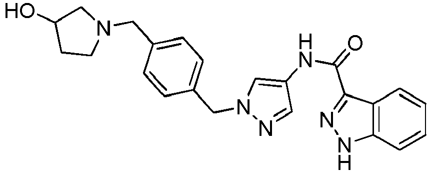

The title compound was synthesized according to Example 103, substituting 3-hydroxypyrrolidine for azetidine. ¹H NMR (400 MHz, DMSO) δ 13.70 (s, 1H), 10.56 (s, 1H), 8.21 (d, *J =* 5.0 Hz, 2H), 8.16 (s, 1H), 7.72 (s, 1H), 7.64 (d, *J* = 8.4 Hz, 1H), 7.44 (t, *J =* 7.6 Hz, 1H), 7.26 *(t, J=* 7.3 Hz, 3H), 7.20 (d, *J=* 8.0 Hz, 2H), 5.29 (s, 2H), 3.8 - 3.1 (bm, 5H). MS: *m*/*z* = 417.2 (M+H)⁺.

### Example 115: N-(1-(4-((dimethylamino)methyl)benzyl)-1H-pyrazol-4-yl)-1H-indazole-3-carboxamide

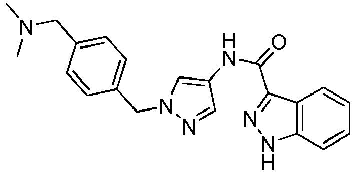

The title compound was synthesized according to Example 103, substituting dimethylamine HCl for azetidine. ¹H NMR (400 MHz, DMSO) δ 13.69 (s, 1H), 10.56 (s, 1H), 8.21 (d, *J*= 8.2 Hz, 1H), 8.16 (s, 1H), 7.72 (s, 1H), 7.64 (d, *J* = 8.4 Hz, 1H), 7.44 (t, *J* = 7.3 Hz, 1H), 7.30 - 7.18 (m, 5H), 5.29 (s, 2H), 3.35 (s, 2H), 2.12 (s, 6H). MS: *m*/*z* = 375.2 (M+H)⁺.

### Example 116: N-(1-(3-cyanobenzyl)-1H-pyrazol-4-yl)-6-(pyridin-4-yl)-1H-indazole-3-carboxamide

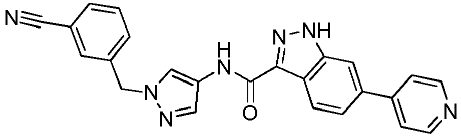

The title compound was synthesized according to Example 43, substituting 4-pyridinylboronic acid for 1H-pyrazole-2-boronic acid and 6-Bromo-1-(2-trimethylsilanyl-ethoxymethyl)-1H-in dazole-3-carboxylic acid [1-(3-cyano-benzyl)-1H-pyrazol-4-yl]-amide for 6-Bromo-1-(2-trimethylsilanyl-ethoxymethyl)-1H-indazole-3-carboxylic acid (1-thiazol-4-ylmethyl-1H-pyrazol-4-yl)-amide. ¹H NMR (400 MHz, DMSO) δ 10.69 (s, 1H), 10.69 (s, 1H), 8.68 (d, J = 5.9 Hz, 1H), 8.42 - 8.21 (m, 1H), 8.01 (s, 1H), 7.93 - 7.43 (m, 5H), 5.41 (s, 2H). MS: *m*/*z =* 420.1 (M+H)⁺.

### Example 117: N-(1-(3-cyanobenzyl)-1H-pyrazol-4-yl)-6-(6-methoxypyridin-3-yl)-1H-indazole-3-carboxamide

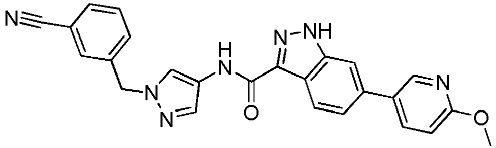

The title compound was synthesized according to Example 116, substituting 4-methoxy-3-pyridinylboronic acid for 4-pyridinylboronic acid. ¹H NMR (400 MHz, DMSO) δ 14.02 - 13.50 (m, 1H), 10.65 (s, 1H), 8.58 (d, J = 2.4 Hz, 1H), 8.19 (dd, J = 65.5, 6.6 Hz, 3H), 7.93 - 7.43 (m, 5H), 6.96 (d, J = 8.6 Hz, 1H), 5.41 (s, 2H), 3.92 (s, 3H). MS: *m*/*z =* 450.1 (M+H)⁺.

### Example 118: N-(1-(3-cyanobenzyl)-1H-pyrazol-4-yl)-6-(2-methylpyridin-3-yl)-1H-indazole-3-carboxamide

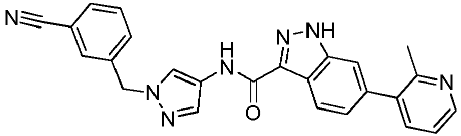

The title compound was synthesized according to Example 116, substituting 2-methylpyridine-3-boronic acid for 4-pyridinylboronic acid. ¹H NMR (400 MHz, DMSO) δ 10.63 (s, 1H), 8.84 (s, 1H), 8.27 (d, *J*= 6.7 Hz, 2H), 8.06 (d, *J*= 7.5 Hz, 1H), 7.95 - 7.46 (m, 5H), 7.38 (d, *J*= 8.0 Hz, 1H), 5.41 (s, 2H), 2.54 (s, 3H). MS: *m*/*z =* 434.1 (M+H)⁺.

### Example 119: N-(1-(3-cyanobenzyl)-1H-pyrazol-4-yl)-6-phenyl-1H-indazole-3-carboxamide

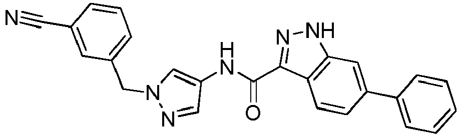

The title compound was synthesized according to Example 116, substituting phenylboronic acid for 4-pyridinylboronic acid. ¹H NMR (400 MHz, DMSO) δ 10.65 (s, 1H), 8.30 - 8.25 (m, 2H), 7.82 (s, 1H), 7.81 - 7.74 (m, 4H), 7.72 (s, 1H), 7.62 - 7.56 (m, 3H), 7.51 (t, *J=* 7.6 Hz, 2H), 7.41 (t, *J=* 7.3 Hz, 1H), 5.41 (s, 2H). MS: *m*/*z =* 419.1 (M+H)⁺.

### Example 120: N-(1-(3-cyanobenzyl)-1H-pyrazol-4-yl)-6-(6-methylpyridin-3-yl)-1H-indazole-3-carboxamide

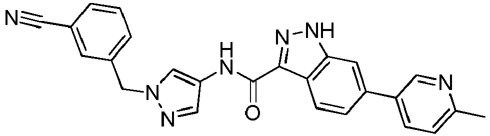

The title compound was synthesized according to Example 116, substituting 2-picoline-5-boronic acid for 4-pyridinylboronic acid. ¹H NMR (400 MHz, DMSO) δ 10.67 (s, 1H), 8.50 (dd, *J* = 4.8, 1.6 Hz, 1H), 8.29 (s, 1H), 8.27 (d, *J* = 8.3 Hz, 1H), 7.80 - 7.76 (m, 2H), 7.73 - 7.68 (m, 2H), 7.62 - 7.57 (m, 3H), 7.34 (dd, *J*= 7.7, 4.9 Hz, 1H), 7.31 - 7.27 (m, 1H), 5.41 (s, 2H), 2.46 (s, 3H). MS: *m*/*z* = 434.1 (M+H)⁺.

### Example 121: N-(1-(3-cyanobenzyl)-1H-pyrazol-4-yl)-6-(1-methyl-1H-pyrazol-4-yl)-1H-indazole-3-carboxamide

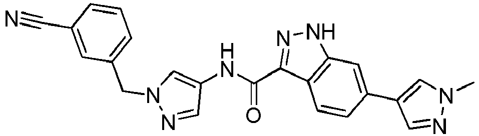

The title compound was synthesized according to Example 116, substituting 1-methylpyrazole-4-boronic acid pinacol ester for 4-pyridinylboronic acid. ¹H NMR (400 MHz, DMSO) δ 10.60 (s, 1H), 8.27 (s, 2H), 8.15 (d, *J*= 8.5 Hz, 1H), 7.98 (s, 1H), 7.81 - 7.74 (m, 2H), 7.72 (s, 2H), 7.62-7.55 (m, 2H), 7.51 (d, *J*= 8.4 Hz, 1H), 5.40 (s, 2H), 3.89 (s, 3H). MS: *m*/*z* = 423.1 (M+H)⁺.

### Example 122: 6-(6-aminopyridin-3-yl)-N-(1-(3-cyanobenzyl)-1H-pyrazol-4-yl)-1H-indazole-3-carboxamide

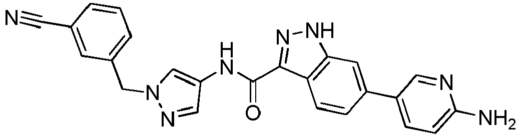

The title compound was synthesized according to Example 116, substituting 5-(4,4,5,5-tetramethyl-1,3,2-dioxaborolan-2-yl)pyridin-2-amine for 4-pyridinylboronic acid. ¹H NMR (400 MHz, DMSO) δ 13.68 (s, 1H), 10.62 (s, 1H), 8.34 (s, 1H), 8.28 (s, 1H), 8.21 *(d, J=* 8.7 Hz, 1H), 7.83 - 7.75 (m, 3H), 7.71 (d, *J=* 4.9 Hz, 2H), 7.61 - 7.56 (m, 2H), 7.51 (d, *J=* 9.0 Hz, 1H), 6.56 (d, *J*= 8.6 Hz, 1H), 6.11 (s, 2H), 5.40 (s, 2H). MS: *m*/*z* = 435.1 (M+H)⁺.

### Example 123: N-(1-(3-cyanobenzyl)-1H-pyrazol-4-yl)-6-(pyridin-3-yl)-1H-indazole-3-carboxamide

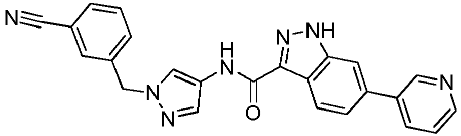

The title compound was synthesized according to Example 116, substituting 3-pyridinylboronic acid for 4-pyridinylboronic acid. ¹H NMR (400 MHz, DMSO) δ 13.88 (s, 1H), 10.69 (s, 1H), 9.00 (s, 1H), 8.62 (d, *J* = 4.7 Hz, 1H), 8.32 (d, *J* = 8.4 Hz, 1H), 8.29 (s, 1H), 8.18 (d, *J* = 7.9 Hz, 1H), 7.92 (s, 1H), 7.81 - 7.76 (m, 2H), 7.72 (s, 1H), 7.64 (d, *J =* 8.5 Hz, 1H), 7.60 - 7.57 (m, 2H), 7.54 (dd, *J*= 7.9, 4.7 Hz, 1H), 5.41 (s, 2H). MS: *m*/*z* = 420.1 (M+H)⁺.

### Example 124: N-(1-(3-cyanobenzyl)-1H-pyrazol-4-yl)-6-(1H-pyrazol-3-yl)-1H-indazole-3-carboxamide

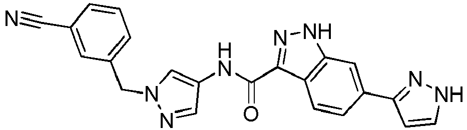

The title compound was synthesized according to Example 116, substituting 1H-pyrazol-3-ylboronic acid for 4-pyridinylboronic acid. ¹H NMR (400 MHz, DMSO) δ 13.67 (s, 1H), 12.95 (s, 1H), 10.60 (s, 1H), 8.27 (s, 1H), 8.20 (d, *J =* 8.5 Hz, 1H), 8.00 (s, 1H), 7.85 - 7.75 (m, 4H), 7.71 (s, 1H), 7.60 - 7.55 (m, 2H), 6.84 (s, 1H), 5.40 (s, 2H). MS: *m*/*z =* 409.1 (M+H)⁺.

### Example 125: N-(1-(3-(dimethylamino)-1-phenylpropyl)-1H-pyrazol-4-yl)-6-(1H-pyrazol-4-yl)-1H-indazole-3-carboxamide

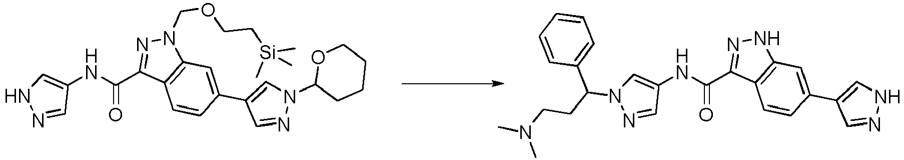

To a solution of N-(1H-pyrazol-4-yl)-6-(1-(tetrahydro-2H-pyran-2-yl)-1H-pyrazol-3-yl)-1-((2-(trimethylsilyl)ethoxy)methyl)-1H-indazole-3-carboxamide (561 mg, 1.13 mmol) in THF (5 mL) was added tributylphosphine (752 mg, 3.3 mmol), 3-(dimethylamino)-1-phenylpropan-1-ol (600 mg,3.3 mmol), and diamide (588 mg, 3.3 mmol). The mixture was stirred for one hour at 70 °C then the mixture was diluted with 300 mL EtOAc and washed with 2 x 200 mL 1:1 H₂O:brine. The organic extracts were dried (Na₂SO₄) and concentrated in vacuo. Purification by CombiFlash (40 g column; 1% - 5% MeOH in DCM over 15 minutes) provided 525 mg (75%) of N-(1-(3-(dimethylamino)-1-phenylpropyl)-1H-pyrazol-4-yl)-6-(1H-pyrazol-3-yl)-1H-indazole-3-carboxamide. The residue was diluted with 1.0 mL trifluoroacetic acid, then triisopropylsilane (80.9 uL, 0.394 mmol) and a few drops of CH₂Cl₂ were added. The mixture was stirred for 2 hours at rt. The mixture was concentrated in vacuo, then purified by automated reverse-phase HPLC, which provided 300 mg (66%) of the title compound. ¹H NMR (400 MHz, DMSO) δ 13.59 (s, 1H), 12.99 (s, 1H), 10.51 (s, 1H), 8.31 (s, 1H), 8.18 (s, 1H), 8.15 (d, *J*= 8.5 Hz, 1H), 8.03 (s, 1H), 7.75 (s, 1H), 7.74 (s, 1H), 7.56 (d, *J*= 8.5 Hz, 1H), 7.40 - 7.24 (m, 5H), 5.47 (dd, *J*= 9.0, 5.7 Hz, 1H), 2.25 - 2.13 (m, 2H), 2.12 (s, 6H), 2.11 - 2.01 (m, 2H). MS: *m*/*z* = 455.2 (M+H)⁺.

### Example 126: (S)-N-(1-(3-(dimethylamino)-1-phenylpropyl)-1H-pyrazol-4-yl)-6-(1H-pyrazol-4-yl)-1H-indazole-3-carboxamide

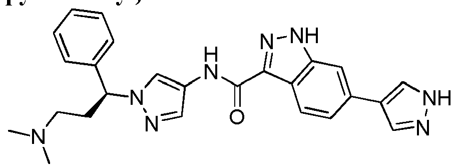

The title compound was separated from its racemate (N-(1-(3-(dimethylamino)-1-phenylpropyl)-1H-pyrazol-4-yl)-6-(1H-pyrazol-4-yl)-1H-indazole-3-carboxamide; Example 125) using automated SFC chromatography using a chiral stationary phase. ¹H NMR (400 MHz, DMSO) δ 13.59 (s, 1H), 12.99 (s, 1H), 10.51 (s, 1H), 8.31 (s, 1H), 8.18 (s, 1H), 8.15 (d, *J=* 8.5 Hz, 1H), 8.03 (s, 1H), 7.75 (s, 1H), 7.74 (s, 1H), 7.56 (d, *J*= 8.5 Hz, 1H), 7.40 - 7.24 (m, 5H), 5.47 (dd, *J* = 9.0, 5.7 Hz, 1H), 2.25 - 2.13 (m, 2H), 2.12 (s, 6H), 2.11 - 2.01 (m, 2H). MS: *m*/*z* = 455.2 (M+H)⁺.

### Example 127: (R)-N-(1-(3-(dimethylamino)-1-phenylpropyl)-1H-pyrazol-4-yl)-6-(1H-pyrazol-4-yl)-1H-indazole-3-carboxamide

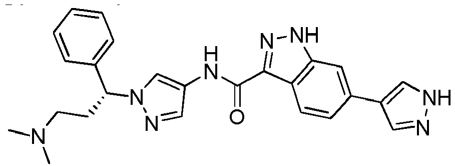

The title compound was separated from its racemate (N-(1-(3-(dimethylamino)-1-phenylpropyl)-1H-pyrazol-4-yl)-6-(1H-pyrazol-4-yl)-1H-indazole-3-carboxamide; Example 125) using automated SFC chromatography using a chiral stationary phase. ¹H NMR (400 MHz, DMSO) δ 13.59 (s, 1H), 12.99 (s, 1H), 10.51 (s, 1H), 8.31 (s, 1H), 8.18 (s, 1H), 8.15 (d, *J*= 8.5 Hz, 1H), 8.03 (s, 1H), 7.75 (s, 1H), 7.74 (s, 1H), 7.56 (d, *J*= 8.5 Hz, 1H), 7.40 - 7.24 (m, 5H), 5.47 (dd, *J* = 9.0, 5.7 Hz, 1H), 2.25 - 2.13 (m, 2H), 2.12 (s, 6H), 2.11 - 2.01 (m, 2H). MS: *m*/*z* = 455.2 (M+H)⁺.

### Example 128: (S)-N-(1-(1-phenylpropyl)-1H-pyrazol-4-yl)-6-(1H-pyrazol-4-yl)-1H-indazole-3-carboxamide

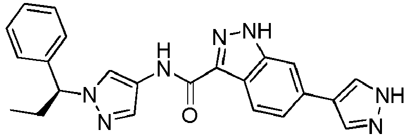

The title compound was synthesized according to Example 125, substituting (R)-1-phenylpropan-1-ol for 3-(dimethylamino)-1-phenylpropan-1-ol. ¹H NMR (400 MHz, DMSO) δ 13.59 (s, 1H), 10.51 (s, 1H), 8.22 - 8.12 (m, 4H), 7.75 (s, 1H), 7.74 (s, 1H), 7.56 *(d, J=* 8.5 Hz, 1H), 7.38 - 7.31 (m, 4H), 7.30 - 7.24 (m, 1H), 5.30 (dd, *J*= 9.2, 6.2 Hz, 1H), 2.42 - 2.30 (m, 1H), 2.18 - 2.06 (m, 1H), 0.84 (t, *J=* 7.2 Hz, 3H). MS: *m*/*z =* 412.2 (M+H)⁺.

### Example 129: (S)-N-(1-(1-phenylpropyl)-1H-pyrazol-4-yl)-1H-indazole-3-carboxamide

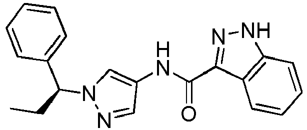

The title compound was synthesized according to Example 125, substituting (R)-1-phenylpropan-1-ol for 3-(dimethylamino)-1-phenylpropan-1-oland N-(1H-pyrazol-4-yl)-1-trityl-1H-indazole-3-carboxamide for N-(1H-pyrazol-4-yl)-6-(1-(tetrahydro-2H-pyran-2-yl)-1H-pyrazol-3-yl)-1-((2-(trimethylsilyl)ethoxy)methyl)-1H-indazole-3-carboxamide. ¹H NMR (400 MHz, DMSO) δ 13.67 (s, 1H), 10.54 (s, 1H), 8.24 - 8.16 (m, 2H), 7.73 (s, 1H), 7.64 *(d, J=* 8.3 Hz, 1H), 7.44 (t, *J=* 7.6 Hz, 1H), 7.39 - 7.23 (m, 7H), 5.30 (t, *J*= 7.5 Hz, 1H), 2.43 - 2.30 (m, 1H), 2.18 - 2.07 (m, 1H), 0.84 (t, *J*= 7.1 Hz, 3H). MS: *m*/*z =* 346.1 (M+H)⁺.

### Example 130: (R)-N-(1-(1-phenylpropyl)-1H-pyrazol-4-yl)-1H-indazole-3-carboxamide

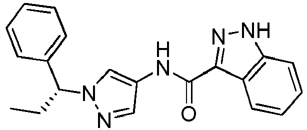

The title compound was synthesized according to Example 125, substituting (S)-1-phenylpropan-1-ol for 3-(dimethylamino)-1-phenylpropan-1-oland N-(1H-pyrazol-4-yl)-1-trityl-1H-indazole-3-carboxamide for N-(1H-pyrazol-4-yl)-6-(-(tetrahydro-2H-pyran-2-yl)-1H-pyrazol-3-yl)-1-((2-(trimethylsilyl)ethoxy)methyl)-1H-indazole-3-carboxamide. ¹H NMR (400 MHz, DMSO) δ 13.67 (s, 1H), 10.54 (s, 1H), 8.24 - 8.16 (m, 2H), 7.73 (s, 1H), 7.64 (d, *J*= 8.3 Hz, 1H), 7.44 (t, *J* = 7.6 Hz, 1H), 7.39 - 7.23 (m, 7H), 5.30 (t, *J =* 7.5 Hz, 1H), 2.43 - 2.30 (m, 1H), 2.18 - 2.07 (m, 1H), 0.84 *(t, J=* 7.1 Hz, 3H). MS: *m*/*z =* 455.2 (M+H)⁺.

### Example 131: N-(1-(1-phenylethyl)-1H-pyrazol-4-yl)-1H-indazole-3-carboxamide

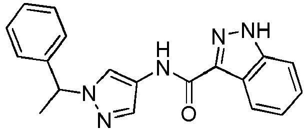

The title compound was synthesized according to Example 125, substituting 1-phenylethanol for 3-(dimethylamino)-1-phenylpropan-1-ol and N-(1H-pyrazol-4-yl)-1-trityl-1H-indazole-3-carboxamide for N-(1H-pyrazol-4-yl)-6-(1-(tetrahydro-2H-pyran-2-yl)-1H-pyrazol-3-yl)-1-((2-(trimethylsilyl)ethoxy)methyl)-1H-indazole-3-carboxamide. MS: *m*/*z* = 332.1 (M+H)⁺.

### Example 132: N-(1-(2-hydroxy-1-phenylethyl)-1H-pyrazol-4-yl)-6-(1H-pyrazol-4-yl)-1H-indazole-3-carboxamide

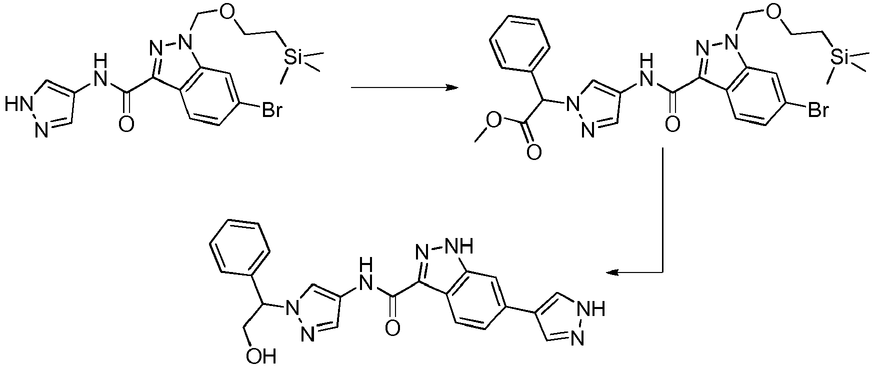

To a solution of N-(1H-pyrazol-4-yl)-6-(1-(tetrahydro-2H-pyran-2-yl)-1H-pyrazol-3-yl)-1-((2-(trimethylsilyl)ethoxy)methyl)-1H-indazole-3-carboxamide (561 mg, 1.13 mmol) in THF (5 mL) was added tributylphosphine (752 mg, 3.3 mmol), 3-(dimethylamino)-1-phenylpropan-1-ol (600 mg,3.3 mmol), and diamide (588 mg, 3.3 mmol). The mixture was stirred for one hour at 70 °C then the mixture was diluted with 300 mL EtOAc and washed with 2 x 200 mL 1:1 H₂O:brine. The organic extracts were dried (Na₂SO₄) and concentrated in vacuo. Purification by CombiFlash (40 g; 50:50 to 0:100 heptane:EtOAc) provided methyl 2-(4-(6-bromo-1-((2-(trimethylsilyl)ethoxy)methyl)-1H-indazole-3-carboxamido)-1H-pyrazol-1-yl)-2-phenylacetate. A solution of methyl 2-(4-(6-bromo-1-((2-(trimethylsilyl)ethoxy)methyl)-1H-indazole-3-carboxamido)-1H-pyrazol-1-yl)-2-phenylacetate (650 mg, 1.1 mmol) in THF (5 mL) was cooled to 0 °C and charged with Lithium Aluminum Hydride (3.3 mmol). The mixture was then stirred at 0 °C for 2 hours, followed by quenching with water and 3 mL of 1M HCl. The mixture was then diluted with Ethyl Acetate and water, the layers were separated, and the aqueous was discarded. The organic was then dried over Na₂SO₄, filtered, and concentrated in vacuo to afford crude 6-bromo-N-(1-(2-hydroxy-1-phenylethyl)-1H-pyrazol-4-yl)-1-((2-(trimethylsilyl)ethoxy)methyl)-1H-indazole-3-carboxamide. This material was dissolved in acetonitrile (10 mL) and potassium 1-(tetrahydro-2H-pyran-2-yl)-1H-pyrazole-4-trifluoroborate (2.5 mmol), 1,1'-bis(diphenylphosphino)ferrocenepalladium (II) chloride (0.1 mmol) and sodium carbonate (3 mmol) as a 1.0 M solution in H₂O, and the mixture was heated to 120 °C overnight. After cooling to rt, the mixture was diluted with 100 mL EtOAc and washed with 100 mL brine. The aqueous phase was further extracted with 100 mL EtOAc, then the combined organic extracts were dried (Na₂SO₄) and concentrated in vacuo. The residue was diluted with 5 mL TFA, and triisopropylsilane (5 mmol) and a few drops of CH₂Cl₂ were added. After stirring for 90 minutes at rt, the mixture was concentrated in vacuo, and the residue purified by automated reverse phase HPLC to provide the title compound. ¹H NMR (400 MHz, DMSO) δ 13.59 (s, 1H), 12.99 (s, 1H), 10.52 (s, 1H), 8.31 (s, 1H), 8.24 (s, 1H), 8.16 (d, *J*= 8.5 Hz, 1H), 8.04 (s, 1H), 7.75 (s, 2H), 7.56 (d, *J*= 8.5 Hz, 1H), 7.37 - 7.25 (m, 5H), 5.44 (dd, *J*= 8.3, 5.2 Hz, 1H), 5.09 (t, *J*= 5.3 Hz, 1H), 4.22 (ddd, *J*= 13.9, 8.4, 5.7 Hz, 1H), 4.01 - 3.92 (m, 1H). MS: *m*/*z =* 414.2 (M+H)⁺.

### Example 133: (S)-N-(1-(2-hydroxy-1-phenylethyl)-1H-pyrazol-4-yl)-6-(1H-pyrazol-4-yl)-1H-indazole-3-carboxamide

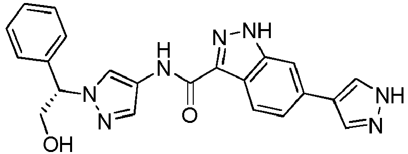

The title compound was separated from its racemate (N-(1-(2-hydroxy-1-phenylethyl)-1H-pyrazol-4-yl)-6-(1H-pyrazol-4-yl)-1H-indazole-3-carboxamide; Example 132) using automated SFC chromatography using a chiral stationary phase. ¹H NMR (400 MHz, DMSO) δ 13.59 (s, 1H), 12.99 (s, 1H), 10.52 (s, 1H), 8.31 (s, 1H), 8.24 (s, 1H), 8.16 (d, *J*= 8.5 Hz, 1H), 8.04 (s, 1H), 7.75 (s, 2H), 7.56 (d, *J*= 8.5 Hz, 1H), 7.37 - 7.25 (m, 5H), 5.44 (dd, *J*= 8.3, 5.2 Hz, 1H), 5.09 (t, *J* = 5.3 Hz, 1H), 4.22 (ddd, *J=* 13.9, 8.4, 5.7 Hz, 1H), 4.01 - 3.92 (m, 1H). MS: *m*/*z =* 414.2 (M+H)⁺.

### Example 134: (R)-N-(1-(2-hydroxy-1-phenylethyl)-1H-pyrazol-4-yl)-6-(1H-pyrazol-4-yl)-1H-indazole-3-carboxamide

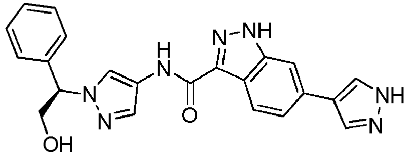

The title compound was separated from its racemate (N-(1-(2-hydroxy-1-phenylethyl)-1H-pyrazol-4-yl)-6-(1H-pyrazol-4-yl)-1H-indazole-3-carboxamide; Example 132) using automated SFC chromatography using a chiral stationary phase. ¹H NMR (400 MHz, DMSO) δ 13.59 (s, 1H), 12.99 (s, 1H), 10.52 (s, 1H), 8.31 (s, 1H), 8.24 (s, 1H), 8.16 (d, *J=* 8.5 Hz, 1H), 8.04 (s, 1H), 7.75 (s, 2H), 7.56 (d, *J* = 8.5 Hz, 1H), 7.37 - 7.25 (m, 5H), 5.44 (dd, *J* = 8.3, 5.2 Hz, 1H), 5.09 (t, *J* = 5.3 Hz, 1H), 4.22 (ddd, *J=* 13.9, 8.4, 5.7 Hz, 1H), 4.01 - 3.92 (m, 1H). MS: *m*/*z* = 414.2 (M+H)⁺.

### Example 135: N-(1-(2-(dimethylamino)-1-phenylethyl)-1H-pyrazol-4-yl)-6-(1H-pyrazol-4-yl)-1H-indazole-3-carboxamide

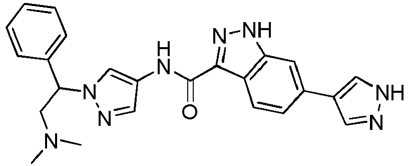

The title compound was synthesized according to Example 125, substituting 2-(dimethylamino)-1-phenylethanol for 3-(dimethylamino)-1-phenylpropan-1-ol. ¹H NMR (400 MHz, DMSO) δ 10.50 (s, 1H), 8.25 (s, 1H), 8.16 (s, 2H), 7.76 (s, 1H), 7.71 (s, 1H), 7.56 (d, *J* = 8.5 Hz, 1H), 7.41 - 7.25 (m, 5H), 5.60 (dd, *J* = 9.1, 5.7 Hz, 1H), 3.27 (dd, *J=* 12.9, 9.2 Hz, 1H), 2.82 (dd, *J=* 12.9, 5.7 Hz, 1H), 2.20 (s, 6H). MS: *m*/*z* = 441.2 (M+H)⁺.

### Example 136: (S)-6-(6-aminopyridin-3-yl)-N-(1-(1-phenylpropyl)-1H-pyrazol-4-yl)-1H-indazole-3-carboxamide

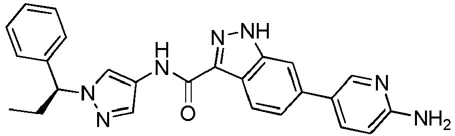

The title compound was synthesized according to Example 116, substituting 5-(4,4,5,5-tetramethyl-1,3,2-dioxaborolan-2-yl)pyridin-2-amine for 4-pyridinylboronic acid and (S)-6-bromo-N-(1-(-phenylpropyl)-1H-pyrazol-4-yl)-1-((2-(trimethylsilyl)ethoxy)methyl)-1H-indazole-3-carboxamide for 6-Bromo-1-(2-trimethylsilanyl-ethoxymethyl)-1H-indazole-3-carboxylic acid [1-(3-cyano-benzyl)-1H-pyrazol-4-yl]-amide. ¹H NMR (400 MHz, DMSO) δ 13.83 (s, 1H), 10.59 (s, 1H), 8.37 (d, *J* = 1.9 Hz, 1H), 8.32 - 8.25 (m, 2H), 8.19 (s, 1H), 7.95 (s, 1H), 7.82 (s, 1H), 7.75 (s, 1H), 7.55 (d, *J* = 8.5 Hz, 1H), 7.39 - 7.31 (m, 4H), 7.30 - 7.25 (m, 1H), 7.01 (d, *J* = 9.1 Hz, 1H), 5.31 (dd, *J* = 9.2, 6.2 Hz, 1H), 3.17 (s, 2H), 2.42 - 2.30 (m, 1H), 2.19 - 2.08 (m, 1H), 0.84 (t, *J* = 7.2 Hz, 3H). MS: *m*/*z* = 438.2 (M+H)⁺.

### Example 137: (S)-N-(1-(1-phenylpropyl)-1H-pyrazol-4-yl)-6-(1H-pyrazol-3-yl)-1H-indazole-3-carboxamide

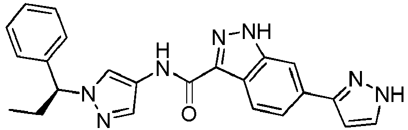

The title compound was prepared according to Example 136, substituting 1H-pyrazol-3-ylboronic acid for 5-(4,4,5,5-tetramethyl-1,3,2-dioxaborolan-2-yl)pyridin-2-amine. ¹H NMR (400 MHz, DMSO) δ 13.69 (s, 1H), 10.57 (d, *J* = 20.5 Hz, 1H), 8.23 - 8.17 (m, 2H), 7.99 (s, 1H), 7.95 (s, 1H), 7.81 - 7.71 (m, 3H), 7.39 - 7.32 (m, 4H), 7.30 - 7.24 (m, 1H), 6.83 (d, *J* = 2.0 Hz, 1H), 5.30 (dd, *J=* 9.1, 6.2 Hz, 1H), 2.42 - 2.30 (m, 1H), 2.18 - 2.05 (m, 1H), 0.84 (t, *J=* 7.2 Hz, 3H). MS: *m*/*z* = 412.2 (M+H)⁺.

### Example 138: (S)-N-(1-(1-phenylpropyl)-1H-pyrazol-4-yl)-6-(pyrimidin-5-yl)-1H-indazole-3-carboxamide

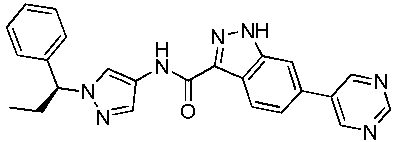

The title compound was prepared according to Example 136, substituting pyrimidine-5-boronic acid for 5-(4,4,5,5-tetramethyl-1,3,2-dioxaborolan-2-yl)pyridin-2-amine. ¹H NMR (400 MHz, DMSO) δ 13.94 (s, 1H), 10.62 (s, 1H), 9.24 (s, 2H), 9.23 (s, 1H), 8.34 (d, *J* = 8.5 Hz, 1H), 8.21 (s, 1H), 8.03 (s, 1H), 7.75 (s, 1H), 7.69 (d, *J* = 8.5 Hz, 1H), 7.39 - 7.31 (m, 4H), 7.30 - 7.24 (m, 1H), 5.31 (dd, *J* = 9.2, 6.2 Hz, 1H), 2.43 - 2.30 (m, 1H), 2.18 - 2.05 (m, 1H), 0.85 (t, *J* = 7.2 Hz, 3H). MS: *m*/*z* = 424.2 (M+H)⁺.

### Example 139:

### (S)-N-(1-(1-phenylpropyl)-1H-pyrazol-4-yl)-6-(pyridin-3-yl)-1H-indazole-3-carboxamide

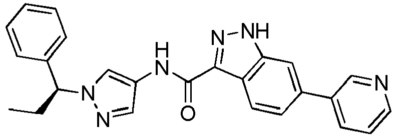

The title compound was prepared according to Example 136, substituting pyridine-3-boronic acid for 5-(4,4,5,5-tetramethyl-1,3,2-dioxaborolan-2-yl)pyridin-2-amine. ¹H NMR (400 MHz, DMSO) δ 13.85 (s, 1H), 10.60 (s, 1H), 8.99 (d, *J* = 2.2 Hz, 1H), 8.62 (dd, *J* = 7.6, 2.9 Hz, 1H), 8.31 (d, *J=* 8.5 Hz, 1H), 8.25 - 8.15 (m, 2H), 7.91 (s, 1H), 7.75 (s, 1H), 7.63 (d, *J=* 8.5 Hz, 1H), 7.53 (dd, *J=* 7.9, 4.8 Hz, 1H), 7.40 - 7.31 (m, 3H), 7.31 - 7.24 (m, 1H), 5.31 (dd, *J=* 9.2, 6.2 Hz, 1H), 2.44 - 2.30 (m, 1H), 2.21 - 2.05 (m, 1H), 0.85 (t, *J=* 7.2, 3H). MS: *m*/*z* = 423.2 (M+H)⁺.

### Example 140:

### (S)-N-(1-(1-phenylpropyl)-1H-pyrazol-4-yl)-6-(pyridin-4-yl)-1H-indazole-3-carboxamide

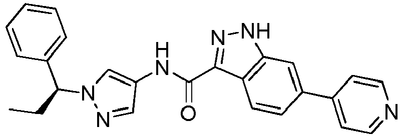

The title compound was prepared according to Example 136, substituting 4-pyridinylboronic acid for 5-(4,4,5,5-tetramethyl-1,3,2-dioxaborolan-2-yl)pyridin-2-amine. ¹H NMR (400 MHz, DMSO) δ 13.99 (s, 1H), 10.64 (s, 1H), 8.82 - 8.75 (m, 2H), 8.36 (d, *J* = 8.5 Hz, 1H), 8.21 (d, *J* = 4.6 Hz, 1H), 8.09 (s, 1H), 8.05 (d, *J* = 5.5 Hz, 2H), 7.78 - 7.73 (m, 2H), 7.39 - 7.31 (m, 4H), 7.31 - 7.25 (m, 1H), 5.31 (dd, *J* = 9.2, 6.2 Hz, 1H), 2.43 - 2.31 (m, 1H), 2.18 - 2.06 (m, 1H), 0.84 (t, *J* = 7.2 Hz, 3H). MS: *m*/*z* = 423.2 (M+H)⁺.

The following examples in Table 1 were made according to the above general procedures.

**Table 1**

| Ex. | Structure | Name | MS | ITK Ki (µm) |
|---|---|---|---|---|
| 141 | 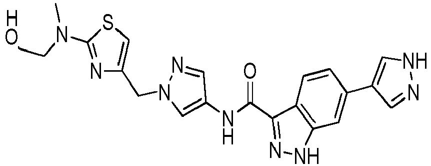 | N-[1-[[2-[2-hydroxyethyl(met hyl)amino]thiazol -4-yl]methyl]pyrazol -4-yl]-6-(1H-pyrazol-4-yl)-1H-indazole-3-carboxamide | 464.2 | 0.0028 |
| 142 | 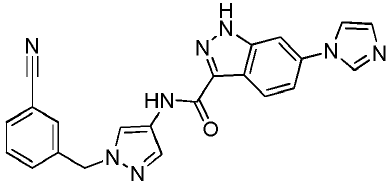 | N-[1-[(3-cyanophenyl)met hyl]pyrazol-4-yl]-6-imidazol-1-yl-1H-indazole-3-carboxamide | 409.0 | 0.0324 |
| 143 | 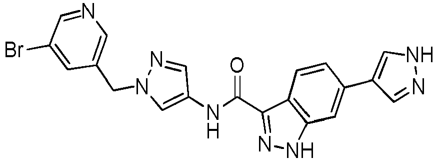 | N-[1-[(5-bromo-3-pyridyl)methyl]py razol-4-yl]-6-(1H-pyrazol-4-yl)-1H-indazole-3-carboxamide | 465.0 | 0.000614 |
| 144 | 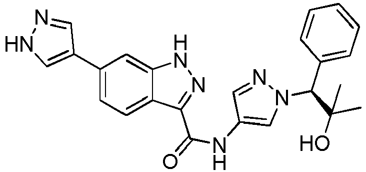 | N-[1-[(1S)-2-hydroxy-2-methyl-1-phenyl-propyl]pyrazol-4-yl]-6-(1H-pyrazol-4-yl)-1H-indazole-3-carboxamide | 442.2 | 0.000235 |
| 145 | 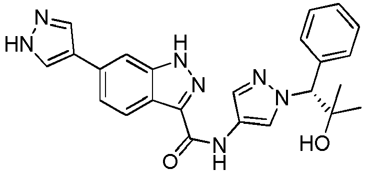 | N-[1-[(1R)-2-hydroxy-2-methyl-1-phenyl-propyl]pyrazol-4-yl]-6-(1H-pyrazol-4-yl)-1H-indazole-3-carboxamide | 442.2 | 0.000812 |
| 146 | 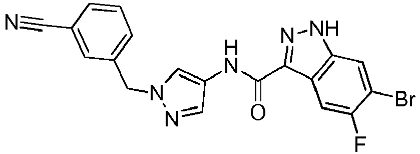 | 6-bromo-N-[1-[(3-cyanophenyl)met hyl]pyrazol-4-yl]-5-fluoro-1H-indazole-3-carboxamide | 439.0 | 0.111 |
| 147 | 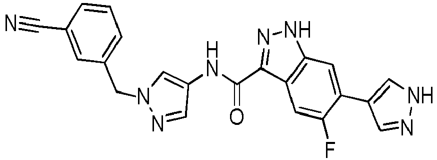 | N-[1-[(3-cyanophenyl)met hyl]pyrazol-4-yl]-5-fluoro-6-(1H-pyrazol-4-yl)-1H-indazole-3-carboxamide | 427.2 | 0.00779 |
| 148 | 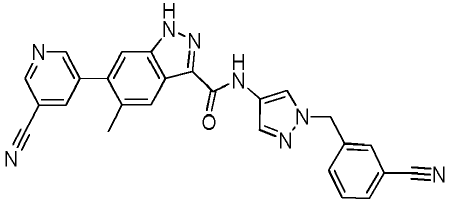 | N-[1-[(3-cyanophenyl)met hyl]pyrazol-4-yl]-6-(5-cyano-3-pyridyl)-5-methyl-1H-indazole-3-carboxamide | 459.0 | 0.0572 |
| 149 | 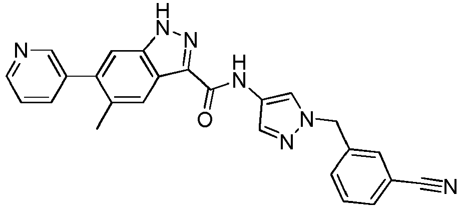 | N-[1-[(3-cyanophenyl)met hyl]pyrazol-4-yl]-5-methyl-6-(3-pyridyl)-1H-indazole-3-carboxamide | 433.9 | 0.0711 |
| 150 | 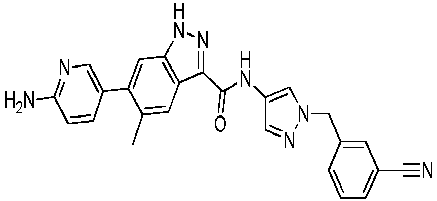 | 6-(6-amino-3-pyridyl)-N-[1-[(3-cyanophenyl)met hyl]pyrazol-4-yl]-5-methyl-1H-indazole-3-carboxamide | 449.0 | 0.0201 |
| 151 | 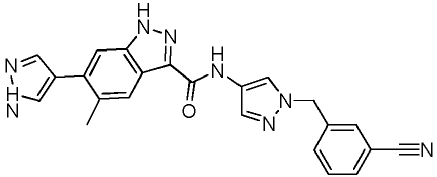 | N-[1-[(3-cyanophenyl)met hyl]pyrazol-4-yl]-5-methyl-6-(1H-pyrazol-4-yl)-1H-indazole-3-carboxamide | 423.0 | 0.0164 |
| 152 | 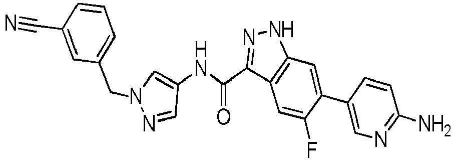 | 6-(6-amino-3-pyridyl)-N-[1-[(3-cyanophenyl)met hyl]pyrazol-4-yl]-5-fluoro-1H-indazole-3-carboxamide | 453.2 | 0.0128 |
| 153 | 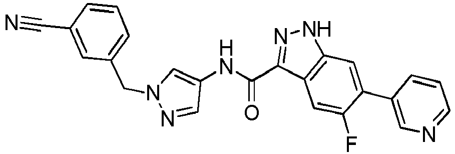 | N-[1-[(3-cyanophenyl)met hyl]pyrazol-4-yl]-5-fluoro-6-(3-pyridyl)-1H-indazole-3-carboxamide | 438.1 | 0.0575 |
| 154 | 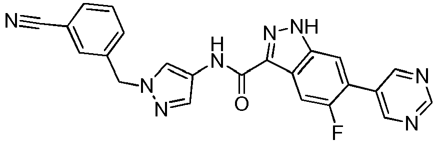 | N-[1-[(3-cyanophenyl)met hyl]pyrazol-4-yl]-5-fluoro-6-pyrimidin-5-yl-1H-indazole-3-carboxamide | 439.2 | 0.112 |
| 155 | 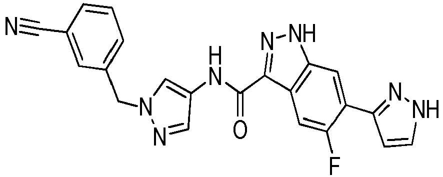 | N-[1-[(3-cyanophenyl)met hyl]pyrazol-4-yl]-5-fluoro-6-(1H-pyrazol-3-yl)-1H-indazole-3-carboxamide | 427.1 | 0.0999 |
| 156 | 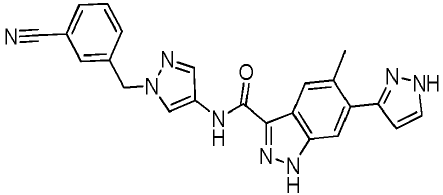 | N-[1-[(3-cyanophenyl)met hyl]pyrazol-4-yl]-5-methyl-6-(1H-pyrazol-3-yl)-1H-indazole-3-carboxamide | 423.0 | 0.0808 |
| 157 | 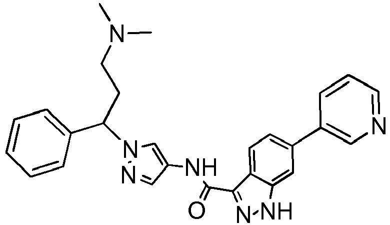 | N-[1-[3-(dimethylamino)-1-phenylpropyl]pyrazol-4-yl]-6-(3-pyridyl)-1H-indazole-3-carboxamide | 466.2 | 0.00781 |
| 158 | 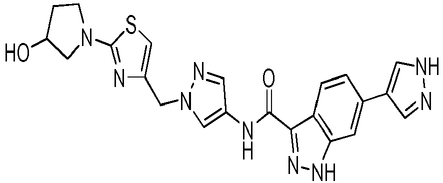 | N-[1-[[2-(3-hydroxypyrrolidin -1-yl)thiazol-4-yl]methyl]pyrazol -4-yl]-6-(1H-pyrazol-4-yl)-1H-indazole-3-carboxamide | 476.2 | 0.00393 |
| 159 | 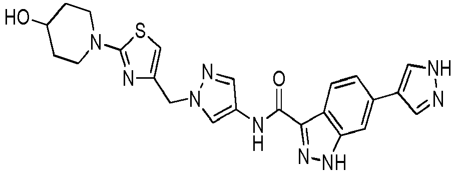 | N-[1-[[2-(4-hydroxy-1-piperidyl)thiazol-4-yl]methyl]pyrazol -4-yl]-6-(1H-pyrazol-4-yl)-1H-indazole-3-carboxamide | 490.2 | 0.00311 |
| 160 | 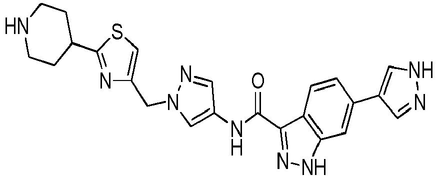 | N-[1-[[2-(4-piperidyl)thiazol-4-yl]methyl]pyrazol -4-yl]-6-(1H-pyrazol-4-yl)-1H-indazole-3-carboxamide | 474.2 | 0.00465 |
| 161 | 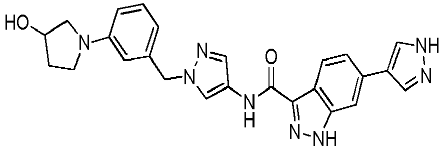 | N-[1-[[3-(3-hydroxypyrrolidin -1-yl)phenyl] methyl] pyrazol-4-yl]-6-(1H-pyrazol-4-yl)-1H-indazole-3-carboxamide | 469.2 | 0.00115 |
| 162 | 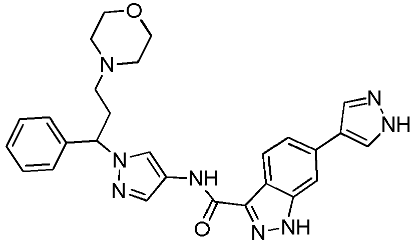 | N-[1-(3-morpholino-1-phenylpropyl)pyrazol-4-yl]-6-(1H-pyrazol-4-yl)-1H-indazole-3-carboxamide | 497.3 | 0.00028 |
| 163 | 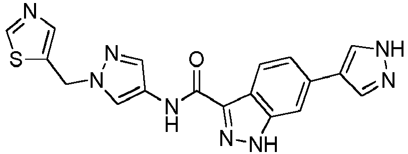 | 6-(1H-pyrazol-4-yl)-N-[1-(thiazol-5-ylmethyl)pyrazo 1-4-yl]-1H-indazole-3-carboxamide | 391.1 | 0.00116 |
| 164 | 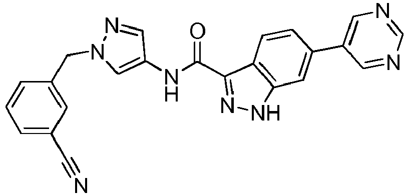 | N-[1-[(3-cyanophenyl)met hyl]pyrazol-4-yl]-6-pyrimidin-5-yl-1H-indazole-3-carboxamide | 421.8 | 0.0169 |
| 165 | 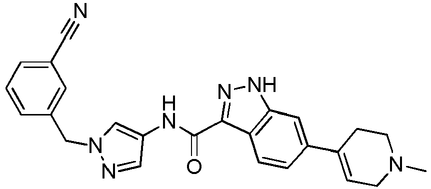 | N-[1-[(3-cyanophenyl)met hyl]pyrazol-4-yl]-6-(1-methyl-3,6-dihydro-2H-pyridin-4-yl)-1H-indazole-3-carboxamide | 438.1 | 0.654 |
| 166 | 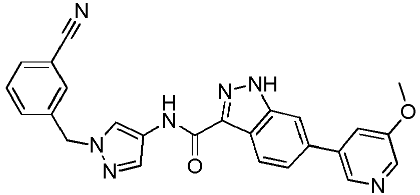 | N-[1-[(3-cyanophenyl)met hyl]pyrazol-4-yl]-6-(5-methoxy-3-pyridyl)-1H-indazole-3-carboxamide | 450.1 | 0.00774 |
| 167 | 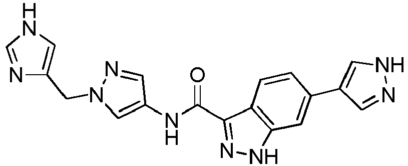 | N-[1-(1H-imidazol-4-ylmethyl)pyrazo 1-4-yl]-6-(1H-pyrazol-4-yl)-1H-indazole-3-carboxamide | 374.2 | 0.00768 |
| 168 | 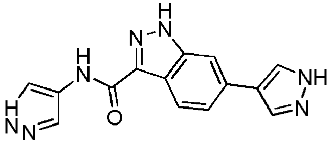 | N,6-bis(1H-pyrazol-4-yl)-1H-indazole-3-carboxamide | 294.0 | 0.0216 |
| 169 | 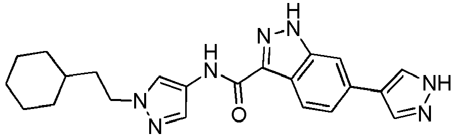 | N-[1-(2-cyc lo hexylethyl)p yrazol-4-yl]-6-(1H-pyrazol-4-yl)-1H-indazole-3-carboxamide | 404.1 | 0.00765 |
| 170 | 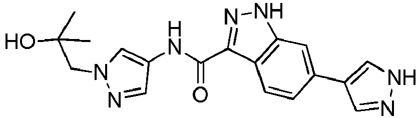 | N-[1-(2-hydroxy-2-methylpropyl)pyrazol-4-yl]-6-(1H-pyrazol-4-yl)-1H-indazole-3-carboxamide | 366.1 | 0.00874 |
| 171 | 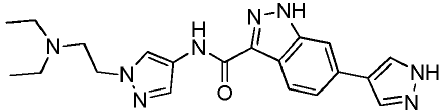 | N-[1-[2-(diethylamino)eth yl]pyrazol-4-yl]-6-(1H-pyrazol-4-yl)-1H-indazole-3-carboxamide | 393.1 | 0.00799 |
| 172 | 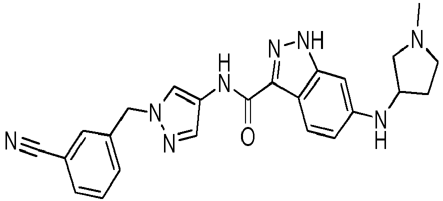 | N-[1-[(3-cyanophenyl)met hyl]pyrazol-4-yl]-6-[(1-methylpyrro lidin-3-yl)amino]-1H-indazole-3-carboxamide | 441.4 | 1.4 |
| 173 | 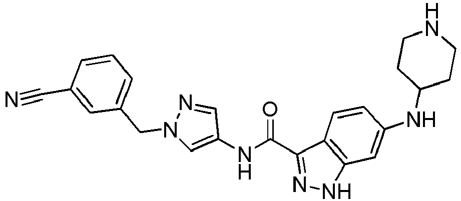 | N-[1-[(3-cyanophenyl)met hyl]pyrazol-4-yl]-6-(4-piperidylamino)-1H-indazole-3-carboxamide | 441.1 | 1.2 |
| 174 | 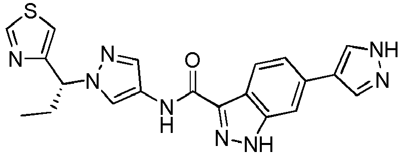 | 6-(1H-pyrazol-4-yl)-N-[1-[(1R)-1-thiazol-4-ylpropyl]pyrazol-4-yl]-1H-indazole-3-carboxamide | 419.1 | 0.00142 |
| 175 | 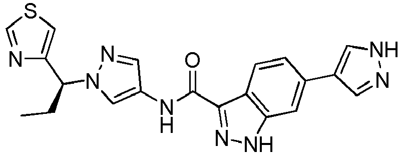 | 6-(1H-pyrazol-4-yl)-N-[1-[(1S)-1-thiazol-4-ylpropyl]pyrazol-4-yl]-1H-indazole-3-carboxamide | 419.0 | 0.00070 |
| 176 | 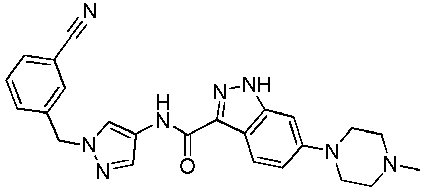 | N-[1-[(3-cyanophenyl)met hyl]pyrazol-4-yl]-6-(4-methylpiperazin-1-yl)-1H-indazole-3-carboxamide | 441.1 | 0.374 |
| 177 | 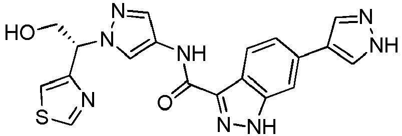 | N-[1-[(1R)-2-hydroxy-1-thiazol-4-yl-ethyl]pyrazol-4-yl]-6-(1H-pyrazol-4-yl)-1H-indazole-3-carboxamide | 421.0 | 0.0075 |
| 178 | 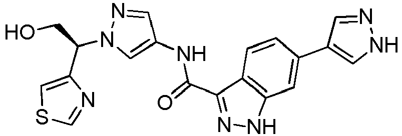 | N-[1-[(1S)-2-hydroxy-1-thiazol-4-yl-ethyl]pyrazol-4-yl]-6-(1H-pyrazol-4-yl)-1H-indazole-3-carboxamide | 421.0 | 0.00619 |
| 179 | 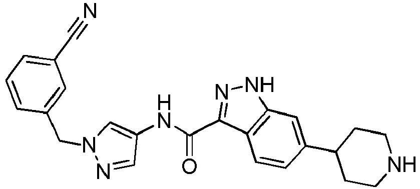 | N-[1-[(3-cyanophenyl)met hyl]pyrazol-4-yl]-6-(4-piperidyl)-1H-indazole-3-carboxamide | 426.0 | 0.154 |
| 180 | 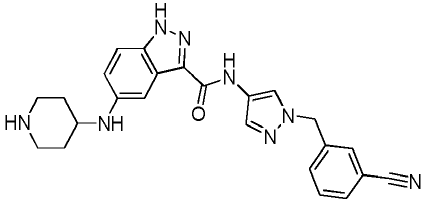 | N-[1-[(3-cyanophenyl)met hyl]pyrazol-4-yl]-5-(4-piperidylamino)-1H-indazole-3-carboxamide | 441.1 | 0.00271 |
| 181 | 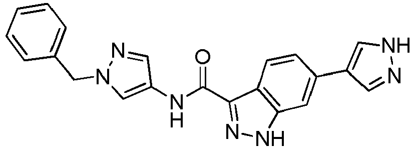 | N-(1-benzylpyrazol-4-yl)-6-(1H-pyrazol-4-yl)-1H-indazole-3-carboxamide | 384.0 | 0.00107 |
| 182 | 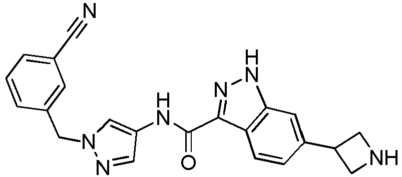 | 6-(azetidin-3-yl)-N-[1-[(3-cyanophenyl)met hyl]pyrazol-4-yl]-1H-indazole-3-carboxamide | 398.0 | 0.24 |
| 183 | 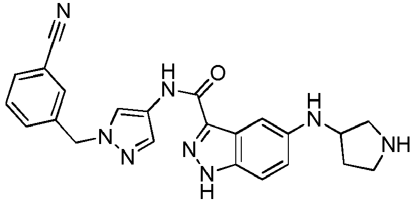 | N-[1-[(3-cyanophenyl)met hyl]pyrazol-4-yl]-5-(pyrrolidin-3-ylamino)-1H-indazole-3-carboxamide | 427.0 | 0.00752 |
| 184 | 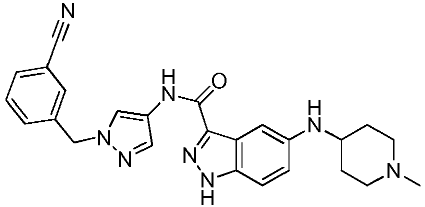 | N-[1-[(3-cyanophenyl)met hyl]pyrazol-4-yl]-5-[(1-methyl-4-piperidyl)amino]-1H-indazole-3-carboxamide | 455.1 | 0.00133 |
| 185 | 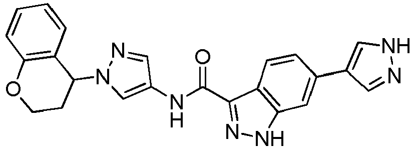 | N-(1-chroman-4-ylpyrazol-4-yl)-6-(1H-pyrazol-4-yl)-1H-indazole-3-carboxamide | 426.2 | 0.759 |
| 186 | 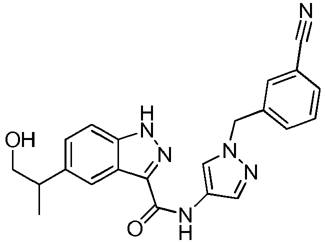 | N-[1-[(3-cyanophenyl)met hyl]pyrazol-4-yl]-5-(2-hydroxy-1-methyl-ethyl)-1H-indazole-3-carboxamide | 400.9 | 0.022 |
| 187 | 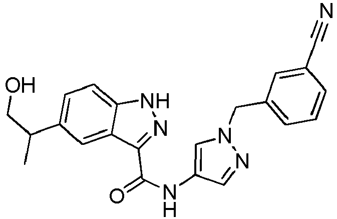 | N-[1-[(3-cyanophenyl)met hyl]pyrazol-4-yl]-5-(2-hydroxy-1-methyl-ethyl)-1H-indazole-3-carboxamide | 401.1 | 0.0078 |
| 188 | 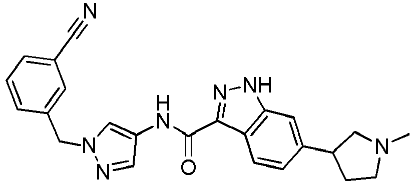 | N-[1-[(3-cyanophenyl)met hyl]pyrazol-4-yl]-6-(1-methylpyrro lidin-3-yl)-1H-indazole-3-carboxamide | 426.2 | 1.6 |
| 189 | 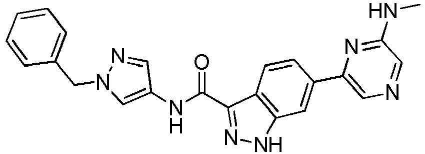 | N-(1-benzylpyrazol-4-yl)-6-[6-(methylamino)pyr azin-2-yl]-1H-indazole-3-carboxamide | 425.2 | 0.0248 |
| 190 | 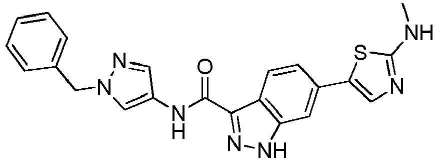 | N-(1-benzylpyrazol-4-yl)-6-[2-(methylamino)thi azol-5-yl]-1H-indazole-3-carboxamide | 430.1 | 0.0185 |
| 191 | 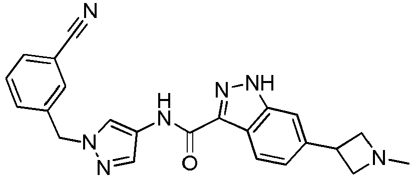 | N-[1-[(3-cyanophenyl)met hyl]pyrazol-4-yl]-6-(1-methylazetidin-3-yl)-1H-indazole-3-carboxamide | 412.2 | 2.4 |
| 192 | 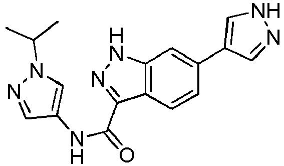 | N-(1-isopropylpyrazol-4-yl)-6-(1H-pyrazol-4-yl)-1H-indazole-3-carboxamide | 336.2 | 0.0102 |
| 193 | 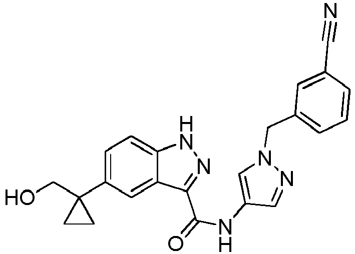 | N-[1-[(3-cyanophenyl)met hyl]pyrazol-4-yl]-5-[1-(hydroxymethyl)c yclopropyl]-1H-indazole-3-carboxamide | 413.0 | 0.0263 |
| 194 | 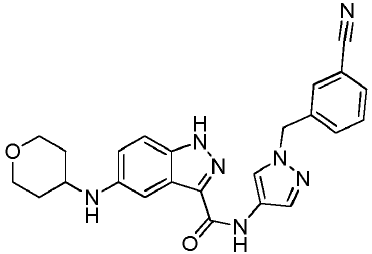 | N-[1-[(3-cyanophenyl)met hyl]pyrazol-4-yl]-5-(tetrahydropyran-4-ylamino)-1H-indazole-3-carboxamide | 442.0 | 0.106 |
| 195 | 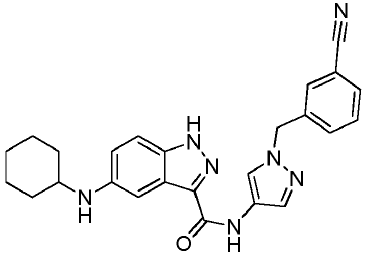 | N-[1-[(3-cyanophenyl)met hyl]pyrazol-4-yl]-5-(cyclohexylamino )-1H-indazole-3-carboxamide | 440.0 | 0.0424 |
| 196 | 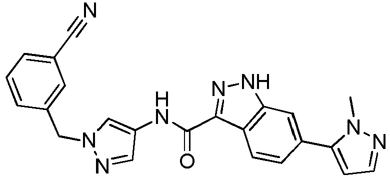 | N-[1-[(3-cyanophenyl)met hyl]pyrazol-4-yl]-6-(2-methylpyrazol-3-yl)-1H-indazole-3-carboxamide | 454.1 | 0.354 |
| 197 | 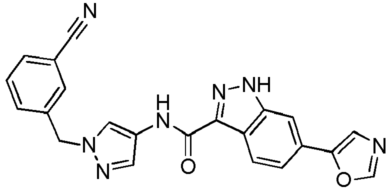 | N-[1-[(3-cyanophenyl)met hyl]pyrazol-4-yl]-6-oxazol-5-yl-1H-indazole-3-carboxamide | 410.1 | 0.00204 |
| 198 | 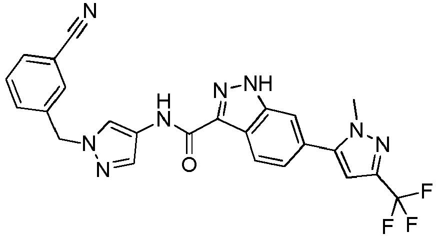 | N-[1-[(3-cyanophenyl)met hyl]pyrazol-4-yl]-6-[2-methyl-5-(trifluoromethyl)p yrazol-3-yl]-1H-indazole-3-carboxamide | 491.1 | 0.183 |
| 199 | 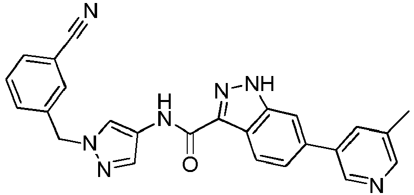 | N-[1-[(3-cyanophenyl)met hyl]pyrazol-4-yl]-6-(5-methyl-3-pyridyl)-1H-indazole-3-carboxamide | 434.2 | 0.00841 |
| 200 | 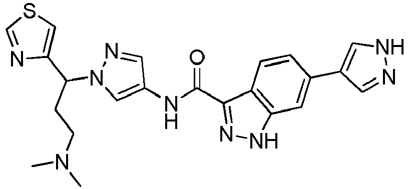 | N-[1-[3-(dimethylamino)-1-thiazol-4-yl-propyl]pyrazol-4-yl]-6-(1H-pyrazol-4-yl)-1H-indazole-3-carboxamide | 462.1 | 0.000727 |
| 201 | 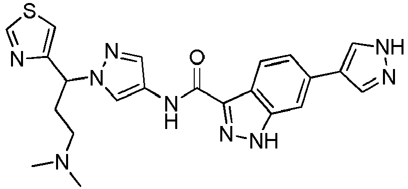 | N-[1-[3-(dimethylamino)-1-thiazol-4-yl-propyl]pyrazol-4-yl]-6-(1H-pyrazol-4-yl)-1H-indazole-3-carboxamide | 462.1 | 0.00289 |
| 202 | 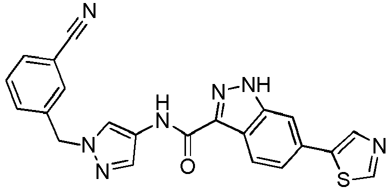 | N-[1-[(3-cyanophenyl)met hyl]pyrazol-4-yl]-6-thiazol-5-yl-1H-indazole-3-carboxamide | 426.1 | 0.00577 |
| 203 | 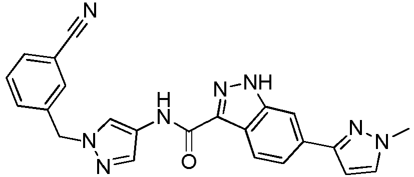 | N-[1-[(3-cyanophenyl)met hyl]pyrazol-4-yl]-6-(1-methylpyrazol-3-yl)-1H-indazole-3-carboxamide | 423.1 | 1.1 |
| 204 | 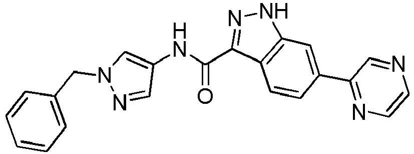 | N-(1-benzyl-1H-pyrazol-4-yl)-6-(pyrazin-2-yl)-1H-indazole-3-carboxamide | 396.1 | 0.0378 |
| 205 | 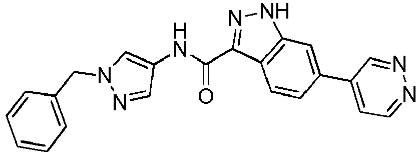 | N-(1-benzylpyrazol-4-yl)-6-pyridazin-4-yl-1H-indazole-3-carboxamide | 396.1 | 0.131 |
| 206 | 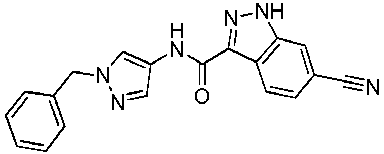 | N-(1-benzylpyrazol-4-yl)-6-cyano-1H-indazole-3-carboxamide | 343.1 | 0.129 |
| 207 | 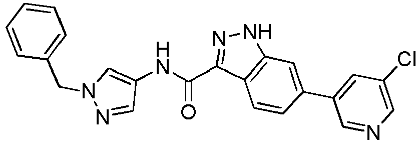 | N-(1-benzylpyrazol-4-yl)-6-(5-chloro-3-pyridyl)-1H-indazole-3-carboxamide | 429.1 | 0.00939 |
| 208 | 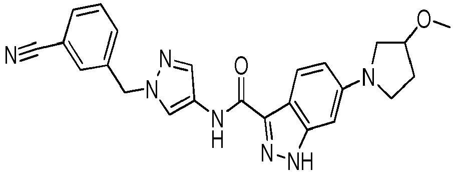 | N-[1-[(3-cyanophenyl)met hyl]pyrazol-4-yl]-6-(3-methoxypyrrolidi n-1-yl)-1H-indazole-3-carboxamide | 442.2 | 0.458 |
| 209 | 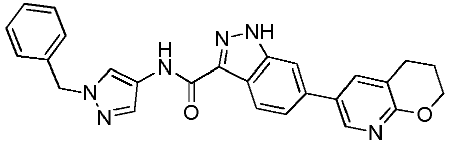 | N-(1-benzylpyrazol-4-yl)-6-(3,4-dihydro-2H-pyrano[2,3-b]pyridin-6-yl)-1H-indazole-3-carboxamide | 451.2 | 0.0662 |
| 210 | 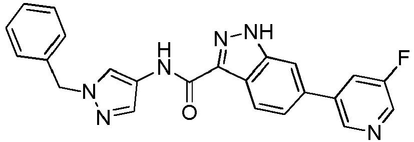 | N-(1-benzylpyrazol-4-yl)-6-(5-fluoro-3-pyridyl)-1H-indazole-3-carboxamide | 413.1 | 0.0050 |
| 211 | 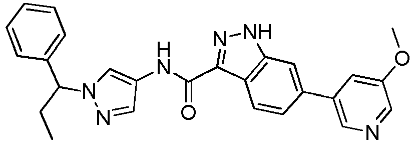 | 6-(5-methoxy-3-pyridyl)-N-[1-(1-phenylpropyl)pyr azol-4-yl]-1H-indazole-3-carboxamide | 453.2 | 0.0538 |
| 212 | 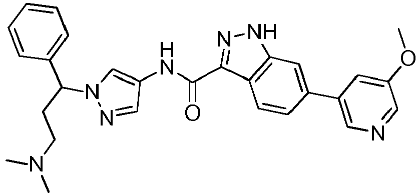 | N-[1-[3-(dimethylamino)-1-phenylpropyl]pyrazol-4-yl]-6-(5-methoxy-3-pyridyl)-1H-indazole-3-carboxamide | 496.2 | 0.00164 |
| 213 | 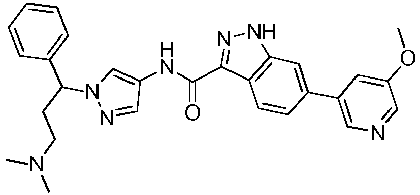 | N-[1-[3-(dimethylamino)-1-phenylpropyl]pyrazol-4-yl]-6-(5-methoxy-3-pyridyl)-1H-indazole-3-carboxamide | 496.2 | 0.00801 |
| 214 | 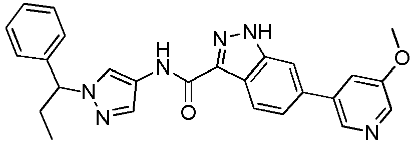 | 6-(5-methoxy-3-pyridyl)-N-[1-(1-phenylpropyl)pyr azol-4-yl]-1H-indazole-3-carboxamide | 453.2 | 0.00717 |
| 215 | 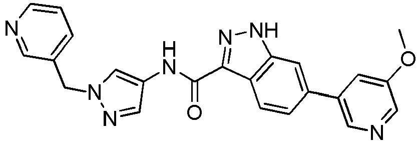 | 6-(5-methoxy-3-pyridyl)-N-[1-(3-pyridylmethyl)pyr azol-4-yl]-1H-indazole-3-carboxamide | 426.1 | 0.0172 |
| 216 | 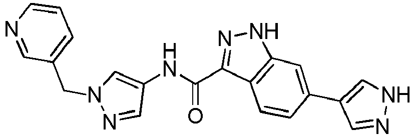 | 6-(1H-pyrazol-4-yl)-N-[1-(3-pyridylmethyl)pyr azol-4-yl]-1H-indazole-3-carboxamide | 385.1 | 0.00112 |
| 217 | 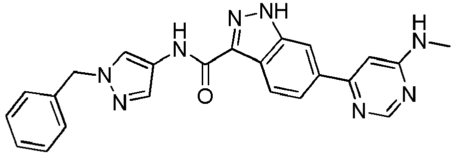 | N-(1-benzylpyrazol-4-yl)-6-[6-(methylamino)pyr imidin-4-yl]-1H-indazole-3-carboxamide | 425.2 | 0.404 |
| 218 | 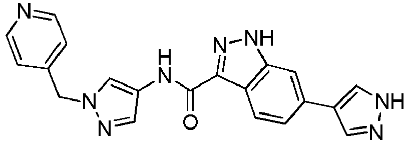 | 6-(1H-pyrazol-4-yl)-N-[1-(4-pyridylmethyl)pyr azol-4-yl]-1H-indazole-3-carboxamide | 385.1 | 0.00319 |
| 219 | 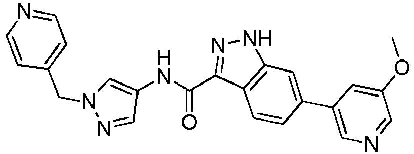 | 6-(5-methoxy-3-pyridyl)-N-[1-(4-pyridylmethyl)pyr azol-4-yl]-1H-indazole-3-carboxamide | 426.2 | 0.023 |
| 220 | 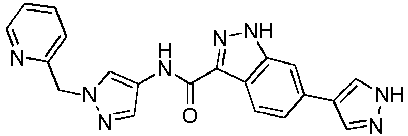 | 6-(1H-pyrazol-4-yl)-N-[1-(2-pyridylmethyl)pyr azol-4-yl]-1H-indazole-3-carboxamide | 385.1 | 0.00293 |
| 221 | 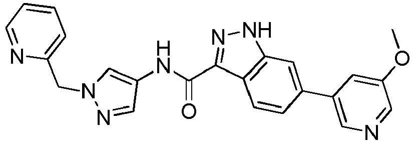 | 6-(5-methoxy-3-pyridyl)-N-[1-(2-pyridylmethyl)pyr azol-4-yl]-1H-indazole-3-carboxamide | 426.2 | 0.0514 |
| 222 | 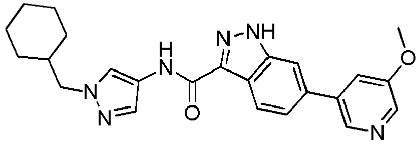 | N-[1-(cyclohexylmethy 1)pyrazol-4-yl]-6-(5-methoxy-3-pyridyl)-1H-indazole-3-carboxamide | 431.2 | 0.0753 |
| 223 | 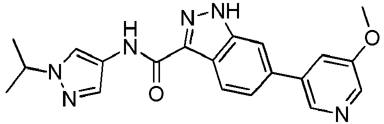 | N-(1-isopropylpyrazol-4-yl)-6-(5-methoxy-3-pyridyl)-1H-indazole-3-carboxamide | 377.0 | 0.090 |
| 224 | 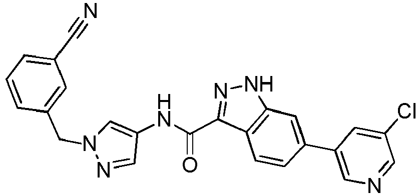 | 6-(5-chloro-3-pyridyl)-N-[1-[(3-cyanophenyl)met hyl]pyrazol-4-yl]-1H-indazole-3-carboxamide | 423.2 | 0.00518 |
| 225 | 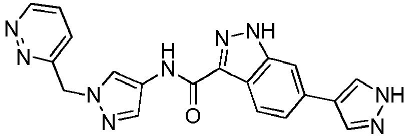 | 6-(1H-pyrazol-4-yl)-N-[1-(pyridazin-3-ylmethyl)pyrazo 1-4-yl]-1H-indazole-3-carboxamide | 386.0 | 0.00485 |
| 226 | 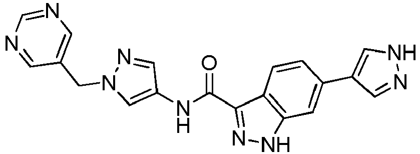 | 6-(1H-pyrazol-4-yl)-N-[1-(pyrimidin-5-ylmethyl)pyrazo 1-4-yl]-1H-indazole-3-carboxamide | 386.0 | 0.00112 |
| 227 | 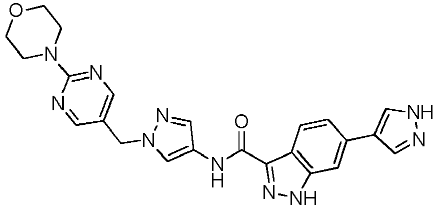 | N-[1-[(2-morpholinopyrimi din-5-yl)methyl]pyrazol -4-yl]-6-(1H-pyrazol-4-yl)-1H-indazole-3-carboxamide | 471.0 | 0.00236 |
| 228 | 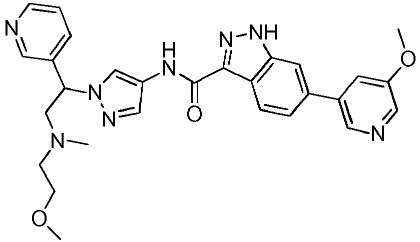 | N-[1-[2-[2-methoxyethyl(met hyl)amino]-1-(3-pyridyl)ethyl]pyra zol-4-yl]-6-(5-methoxy-3-pyridyl)-1H-indazole-3-carboxamide | 527.2 | 0.00554 |
| 229 | 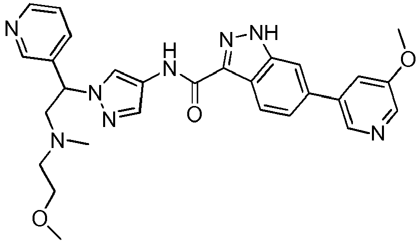 | N-[1-[2-[2-methoxyethyl(met hyl)amino]-1-(3-pyridyl)ethyl]pyra zol-4-yl]-6-(5-methoxy-3-pyridyl)-1H-indazole-3-carboxamide | 627.2 | 0.0324 |
| 230 | 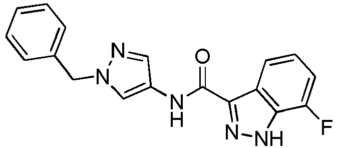 | N-(1-benzylpyrazol-4-yl)-7-fluoro-1H-indazole-3-carboxamide | 335.9 | 0.726 |
| 231 | 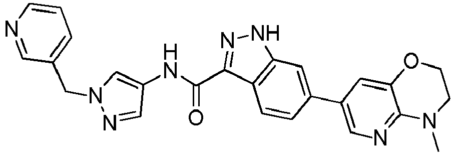 | 6-(4-methyl-2,3-dihydropyrido [3,2 -b][1,4]oxazin-7-yl)-N-[1-(3-pyridylmethyl)pyr azol-4-yl]-1H-indazole-3-carboxamide | 467.2 | 0.0408 |
| 232 | 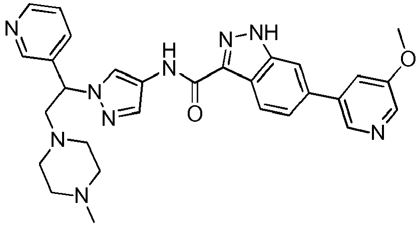 | 6-(5-methoxy-3-pyridyl)-N-[1-[2-(4-methylpiperazin-1-yl)-1-(3-pyridyl)ethyl]pyra zol-4-yl]-1H-indazole-3-carboxamide | 538.2 | 0.0314 |
| 233 | 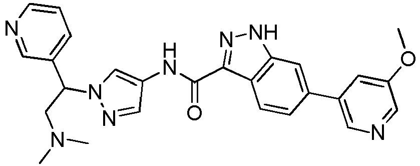 | N-[1-[2-(dimethylamino)-1-(3-pyridyl)ethyl]pyra zol-4-yl]-6-(5-methoxy-3-pyridyl)-1H-indazole-3-carboxamide | 483.2 | 0.051 |
| 234 | 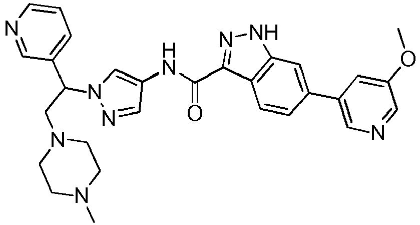 | 6-(5-methoxy-3-pyridyl)-N-[1-[2-(4-methylpiperazin-1-yl)-1-(3-pyridyl)ethyl]pyra zol-4-yl]-1H-indazole-3-carboxamide | 538.2 | 0.00538 |
| 235 | 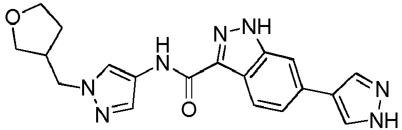 | 6-(1H-pyrazol-4-yl)-N-[1-(tetrahydro furan-3-ylmethyl)pyrazo 1-4-yl]-1H-indazole-3-carboxamide | 398.1 | 0.00464 |
| 236 | 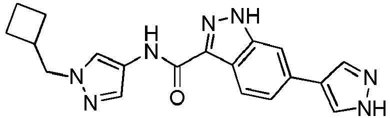 | N-[1-(cyclobutylmethyl )pyrazol-4-yl]-6-(1H-pyrazol-4-yl)-1H-indazole-3-carboxamide | 362.1 | 0.00609 |
| 237 | 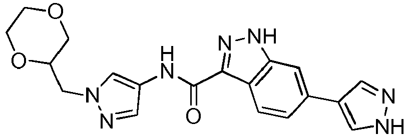 | N-[1-(1,4-dioxan-2-ylmethyl)pyrazo 1-4-yl]-6-(1H-pyrazol-4-yl)-1H-indazole-3-carboxamide | 394.2 | 0.0037 |
| 238 | 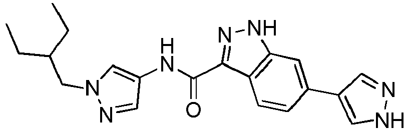 | N-[1-(2-ethylbutyl)pyrazo 1-4-yl]-6-(1H-pyrazol-4-yl)-1H-indazole-3-carboxamide | 378.2 | 0.00279 |
| 239 | 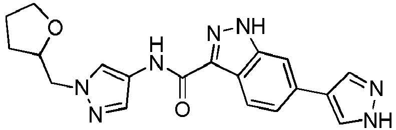 | 6-(1H-pyrazol-4-yl)-N-[1-(tetrahydro furan-2-ylmethyl)pyrazo 1-4-yl]-1H-indazole-3-carboxamide | 378.2 | 0.00658 |
| 240 | 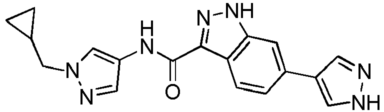 | N-[1-(cyclopropylmeth yl)pyrazol-4-yl]-6-(1H-pyrazol-4-yl)-1H-indazole-3-carboxamide | 348.1 | 0.00533 |
| 241 | 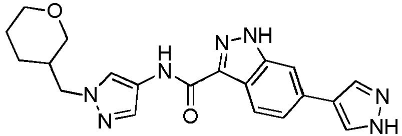 | 6-(1H-pyrazol-4-yl)-N-[1-(tetrahydropyran-3-ylmethyl)pyrazo 1-4-yl]-1H-indazole-3-carboxamide | 392.1 | 0.00279 |
| 242 | 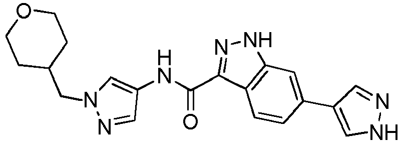 | 6-(1H-pyrazol-4-yl)-N-[1-(tetrahydropyran-4-ylmethyl)pyrazo 1-4-yl]-1H-indazole-3-carboxamide | 392.2 | 0.00247 |
| 243 | 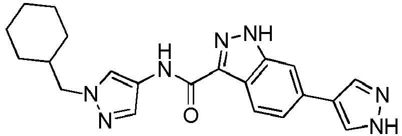 | N-[1-(cyclohexylmethy 1)pyrazol-4-yl]-6-(1H-pyrazol-4-yl)-1H-indazole-3-carboxamide | 390.1 | 0.00243 |
| 244 | 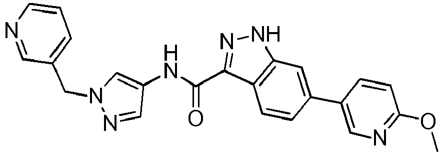 | 6-(6-methoxy-3-pyridyl)-N-[1-(3-pyridylmethyl)pyr azol-4-yl]-1H-indazole-3-carboxamide | 379.1 | 0.0323 |
| 245 | 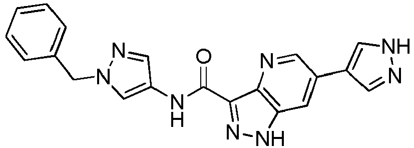 | N-(1-benzylpyrazol-4-yl)-6-(1H-pyrazol-4-yl)-1H-pyrazolo[4,3-b]pyridine-3-carboxamide | 385.0 | 0.0189 |
| 246 | 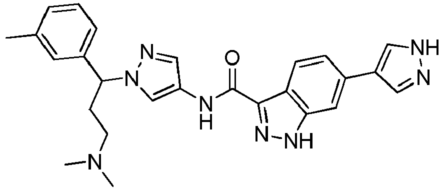 | N-[1-[3-(dimethylamino)-1-(m-tolyl)propyl]pyraz ol-4-yl]-6-(1H-pyrazol-4-yl)-1H-indazole-3-carboxamide | 469.3 | 0.0000734 |
| 247 | 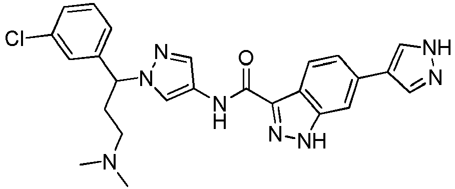 | N-[1-[1-(3-chlorophenyl)-3-(dimethylamino)p ropyl]pyrazol-4-yl]-6-(1H-pyrazol-4-yl)-1H-indazole-3-carboxamide | 489.0 | 0.000127 |
| 248 | 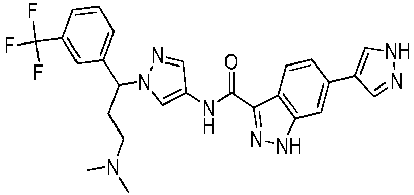 | N-[1-[3-(dimethylamino)-1-[3-(trifluoromethyl)p henyl]propyl]pyra zol-4-yl]-6-(1H-pyrazol-4-yl)-1H-indazole-3-carboxamide | 532.3 | 0.000128 |
| 249 | 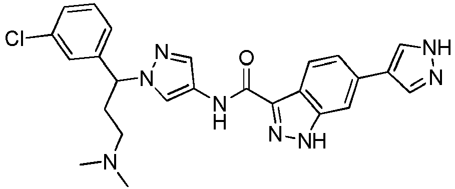 | N-[1-[1-(3-chlorophenyl)-3-(dimethylamino)p ropyl]pyrazol-4-yl]-6-(1H-pyrazol-4-yl)-1H-indazole-3-carboxamide | 489.2 | 0.000060 |
| 250 | 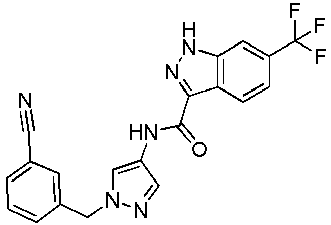 | N-[1-[(3-cyanophenyl)met hyl]pyrazol-4-yl]-6-(trifluoromethyl)-1H-indazole-3-carboxamide | 411.1 | 0.103 |
| 251 | 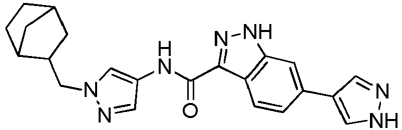 | N-[1-(norbornan-2-ylmethyl)pyrazo 1-4-yl]-6-(1H-pyrazol-4-yl)-1H-indazole-3-carboxamide | 402.2 | 0.0050 |
| 252 | 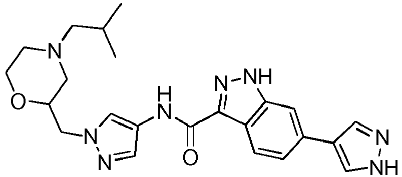 | N-[1-[(4-isobutylmorpholi n-2-yl)methyl]pyrazol -4-yl]-6-(1H-pyrazol-4-yl)-1H-indazole-3-carboxamide | 449.2 | 0.00358 |
| 253 | 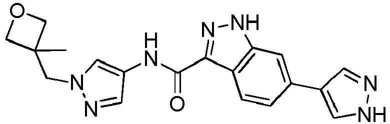 | N-[1-[(3-methyloxetan-3-yl)methyl]pyrazol -4-yl]-6-(1H-pyrazol-4-yl)-1H-indazole-3-carboxamide | 378.1 | 0.0367 |
| 254 | 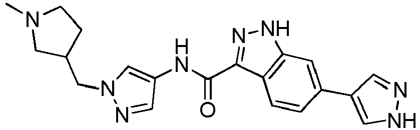 | N-[1-[(1-methylpyrro lidin-3-yl)methyl]pyrazol -4-yl]-6-(1H-pyrazol-4-yl)-1H-indazole-3-carboxamide | 391.2 | 0.0065 |
| 255 | 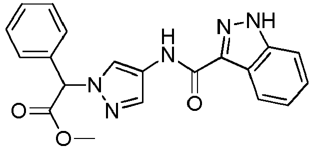 | methyl 2-[4-(1H-indazole-3-carbonylamino)py razol-1-yl]-2-phenyl-acetate | 376.1 | 0.0383 |
| 256 | 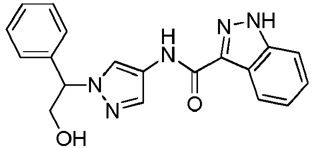 | N-[1-(2-hydroxy-1-phenylethyl)pyrazol-4-yl]-1H-indazole-3-carboxamide | 348.1 | 0.146 |
| 257 | 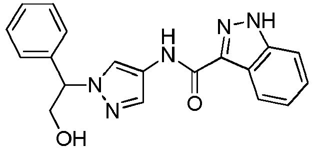 | N-[1-(2-hydroxy-1-phenylethyl)pyrazol-4-yl]-1H-indazole-3-carboxamide | 348.1 | 0.0355 |
| 258 | 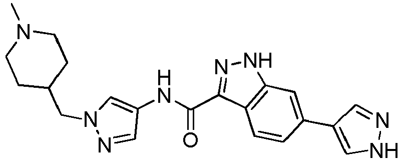 | N-[1-[(1-methyl-4-piperidyl)methyl] pyrazol-4-yl]-6-(1H-pyrazol-4-yl)-1H-indazole-3-carboxamide | 405.2 | 0.00568 |
| 259 | 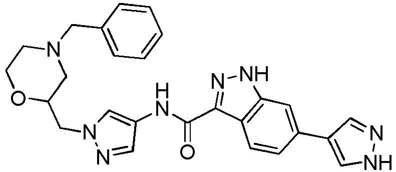 | N-[1-[(4-benzylmorpho lin-2-yl)methyl]pyrazol -4-yl]-6-(1H-pyrazol-4-yl)-1H-indazole-3-carboxamide | 483.2 | 0.00276 |
| 260 | 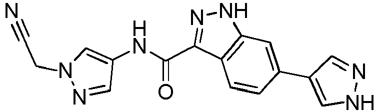 | N-[1-(cyanomethyl)pyr azol-4-yl]-6-(1H-pyrazol-4-yl)-1H-indazole-3-carboxamide | 333.1 | 0.0119 |
| 261 | 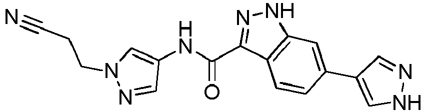 | N-[1-(2-cyano ethyl)pyraz ol-4-yl]-6-(1H-pyrazol1-4-yl)-1H-indazole-3-carboxamide | 347.1 | 0.00481 |
| 262 | 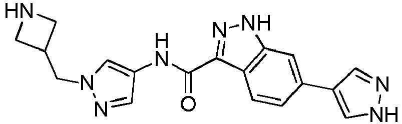 | N-[1-(azetidin-3-ylmethyl)pyrazo 1-4-yl]-6-(1H-pyrazol-4-yl)-1H-indazole-3-carboxamide | 363.1 | 0.00469 |
| 263 | 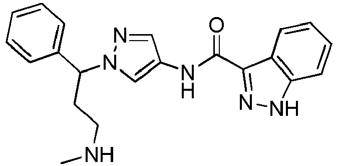 | N-[1-[3-(methylamino)-1-phenylpropyl]pyrazol-4-yl]-1H-indazole-3-carboxamide | 375.2 | 0.00514 |
| 264 | 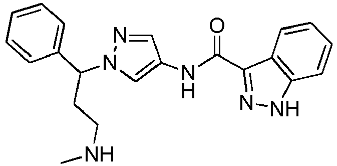 | N-[1-[3-(methylamino)-1-phenylpropyl]pyrazol-4-yl]-1H-indazole-3-carboxamide | 375.2 | 0.0555 |
| 265 | 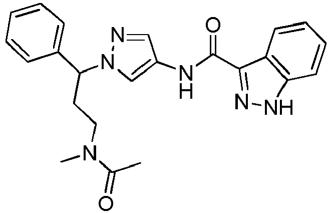 | N-[1-[3-[acetyl(methyl)a mino]-1-phenylpropyl]pyrazol-4-yl]-1H-indazole-3-carboxamide | 417.2 | 0.0238 |
| 266 | 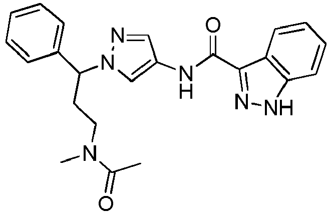 | N-[1-[3-[acetyl(methyl)a mino]-1-phenylpropyl]pyrazol-4-yl]-1H-indazole-3-carboxamide | 417.2 | 0.10 |
| 267 | 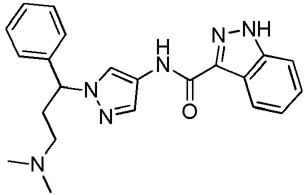 | N-[1-[3-(dimethylamino)-1-phenylpropyl]pyrazol-4-yl]-1H-indazole-3-carboxamide | 389.2 | 0.0769 |
| 268 | 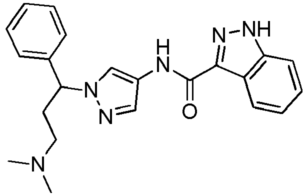 | N-[1-[3-(dimethylamino)-1-phenylpropyl]pyrazol-4-yl]-1H-indazole-3-carboxamide | 389.2 | 0.00598 |
| 269 | 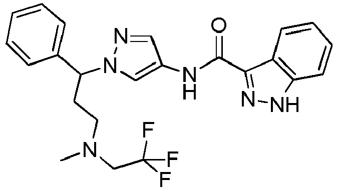 | N-[1-[3-[methyl(2,2,2-trifluoroethyl)ami no]-1-phenyl-propyl]pyrazol-4-yl]-1H-indazole-3-carboxamide | 457.2 | 0.105 |
| 270 | 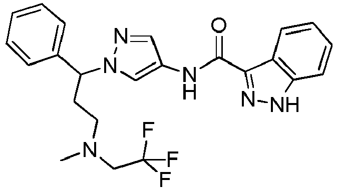 | N-[1-[3-[methyl(2,2,2-trifluoroethyl)ami no]-1-phenyl-propyl]pyrazol-4-yl]-1H-indazole-3-carboxamide | 457.2 | 0.00892 |
| 271 | 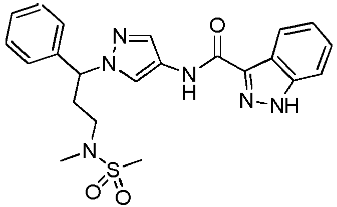 | N-[1-[3-[methyl(methylsu lfonyl)amino]-1-phenylpropyl]pyrazol-4-yl]-1H-indazole-3-carboxamide | no MS | 0.00692 |
| 272 | 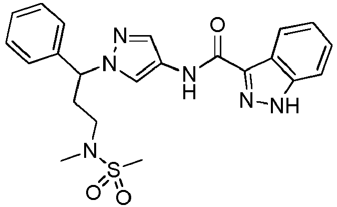 | N-[1-[3-[methyl(methylsu lfonyl)amino]-1-phenylpropyl]pyrazol-4-yl]-1H-indazole-3-carboxamide | no MS | 0.0416 |
| 273 | 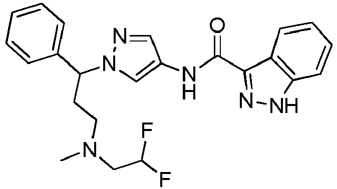 | N-[1-[3-[2,2-difluoroethyl(met hyl)amino]-1-phenylpropyl]pyrazol-4-yl]-1H-indazole-3-carboxamide | 439.2 | 0.0083 |
| 274 | 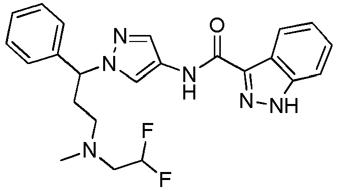 | N-[1-[3-[2,2-difluoroethyl(met hyl)amino]-1-phenylpropyl]pyrazol-4-yl]-1H-indazole-3-carboxamide | 439.2 | 0.0848 |
| 275 | 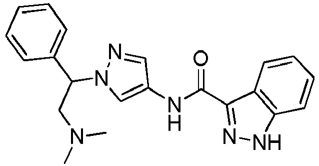 | N-[1-[2-(dimethylamino)-1-phenylethyl]pyrazol-4-yl]-1H-indazole-3-carboxamide | 375.2 | 0.00783 |
| 276 | 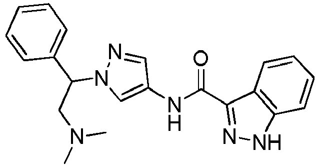 | N-[1-[2-(dimethylamino)-1-phenylethyl]pyrazol-4-yl]-1H-indazole-3-carboxamide | 375.2 | 0.152 |
| 277 | 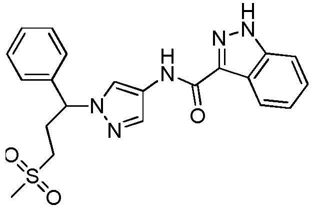 | N-[1-(3-methylsulfonyl-1-phenylpropyl)pyrazol-4-yl]-1H-indazole-3-carboxamide | 424.0 | 0.114 |
| 278 | 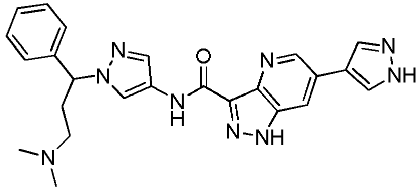 | N-[1-[3-(dimethylamino)-1-phenylpropyl]pyrazol-4-yl]-6-(1H-pyrazol-4-yl)-1 H-pyrazolo[4,3-b]pyridine-3-carboxamide | 456.0 | 0.00129 |
| 279 | 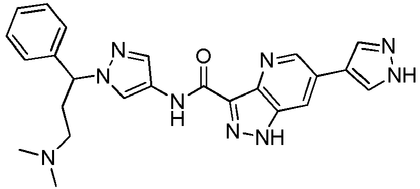 | N-[1-[3-(dimethylamino)-1-phenylpropyl]pyrazol-4-yl]-6-(1H-pyrazol-4-yl)-1H-pyrazolo[4,3-b]pyridine-3-carboxamide | 456.0 | 0.0367 |

### BIOLOGICAL EXAMPLES

### Example (i)

The ability of purified ITK (Invitrogen PV3875) to catalyze peptide phosphorylation is monitored using a Caliper LabChip 3000 microfluidic unit (Caliper assay) or by liquid chromatography-mass spectrometry (LCMS) using a Waters Acquity system (LCMS assay). In the Caliper assay, ITK is incubated at room temperature with test compounds for 45 minutes in 100 mM 2-[4-(2-hydroxyethyl)piperazin-1-yl]ethanesulfonic acid (HEPES) buffer (pH 7.2) containing 10 mM MgCl₂, 2 mM dithiothreitol (DTT), 20 µM adenosine-5'-triphosphate (ATP), 0.015% Brij 35, 2% dimethylsulfoxide (DMSO), and 2 µM (5-carboxyfluorescein)-EFPIYDFLPAKKK-NH₂ peptide substrate. Reactions are quenched by the addition of 2,2',2",2'''-(ethane-1,2-diyldinitrilo)tetraacetic acid (50 mM final). Non-phosphorylated substrate and phosphorylated product peptides are separated and quantified using a Caliper LabChip 3000 instrument. In the LCMS assay, ITK is incubated at room temperature with test compounds for 1 hour in 50 mM HEPES buffer (pH 7.2) containing 15 mM MgCl₂, 2 mM DTT, 20 µM ATP, 0.015% Brij 35, 2% DMSO, and 2 µM Acetyl-EFPIYDFLPAKKK-NH₂ peptide substrate. Reactions are quenched by the addition of trichloroacetic acid (5% v/v final). Non-phosphorylated substrate and phosphorylated product peptides are separated by ultra performance LC and detected by a coupled triple quadrupole MS device applying multiple reaction monitoring (MRM) for quantification. The area of the MRM-extracted mass signal is used to assess the inhibition by test compounds. Equilibrium dissociation constant (*K*ᵢ) values for ITK inhibitors are calculated from plots of activity vs. inhibitor concentration using Morrison's quadratic equation that accounts for the potential of tight binding, and by also applying the conversion factor that accounts for competitive inhibition and the concentration of ATP used in the assay relative to its apparent Michaelis constant (*K*ₘ,ₐₚₚ).

Examples 1-140 were tested in the above assay and the results of the tests are shown below in Table 2 (were + is 0.01-1 nM, ++ is 1-100 nM, and +++ is 100-3,500 nM or higher).

**Table 2**

| Example# | ITK Ki |
|---|---|
| 1 | ++ |
| 2 | ++ |
| 3 | ++ |
| 4 | +++ |
| 5 | +++ |
| 6 | ++ |
| 7 | ++ |
| 8 | ++ |
| 9 | ++ |
| 10 | ++ |
| 11 | ++ |
| 12 | + |
| 13 | ++ |
| 14 | ++ |
| 15 | ++ |
| 16 | ++ |
| 17 | ++ |
| 18 | ++ |
| 19 | ++ |
| 20 | ++ |
| 21 | ++ |
| 22 | ++ |
| 23 | ++ |
| 24 | + |
| 25 | ++ |
| 26 | ++ |
| 27 | + |
| 28 | ++ |
| 29 | + |
| 30 | + |
| 31 | ++ |
| 32 | ++ |
| 33 | ++ |
| 34 | ++ |
| 35 | ++ |
| 36 | + |
| 37 | + |
| 38 | ++ |
| 39 | + |
| 40 | + |
| 41 | ++ |
| 42 | ++ |
| 43 | ++ |
| 44 | ++ |
| 45 | ++ |
| 46 | + |
| 47 | ++ |
| 48 | + |
| 49 | ++ |
| 50 | ++ |
| 51 | ++ |
| 52 | ++ |
| 53 | ++ |
| 54 | ++ |
| 55 | ++ |
| 56 | ++ |
| 57 | ++ |
| 58 | ++ |
| 59 | ++ |
| 60 | +++ |
| 61 | +++ |
| 62 | ++ |
| 63 | ++ |
| 64 | +++ |
| 65 | ++ |
| 66 | ++ |
| 67 | ++ |
| 68 | ++ |
| 69 | ++ |
| 70 | ++ |
| 71 | ++ |
| 72 | +++ |
| 73 | +++ |
| 74 | +++ |
| 75 | +++ |
| 76 | +++ |
| 77 | +++ |
| 78 | +++ |
| 79 | +++ |
| 80 | ++ |
| 81 | ++ |
| 82 | ++ |
| 83 | ++ |
| 84 | ++ |
| 85 | ++ |
| 86 | ++ |
| 87 | ++ |
| 88 | ++ |
| 89 | ++ |
| 90 | ++ |
| 91 | ++ |
| 92 | ++ |
| 93 | ++ |
| 94 | ++ |
| 95 | ++ |
| 96 | ++ |
| 97 | ++ |
| 98 | ++ |
| 99 | ++ |
| 100 | ++ |
| 101 | ++ |
| 102 | ++ |
| 103 | ++ |
| 104 | ++ |
| 105 | ++ |
| 106 | ++ |
| 107 | ++ |
| 108 | ++ |
| 109 | ++ |
| 110 | ++ |
| 111 | ++ |
| 112 | ++ |
| 113 | ++ |
| 114 | ++ |
| 115 | ++ |
| 116 | ++ |
| 117 | ++ |
| 118 | +++ |
| 119 | +++ |
| 120 | ++ |
| 121 | ++ |
| 122 | ++ |
| 123 | ++ |
| 124 | ++ |
| 125 | + |
| 126 | + |
| 127 | + |
| 128 | ++ |
| 129 | ++ |
| 130 | ++ |
| 131 | ++ |
| 132 | + |
| 133 | ++ |
| 134 | + |
| 135 | + |
| 136 | ++ |
| 137 | ++ |
| 138 | ++ |
| 139 | ++ |
| 140 | ++ |

Specific values for certain examples tested in the above assay Example (i) are included in Table 3:

**Table 3**

| Example# | ITK Ki (µM) |
|---|---|
| 1 | 0.0377 |
| 2 | 0.0165 |
| 3 | 0.0185 |
| 4 | 1.3 |
| 5 | >3.5 |
| 9 | 0.00742 |
| 14 | 0.0020 |
| 16 | 0.0069 |
| 17 | 0.0018 |
| 19 | 0.0023 |
| 54 | 0.0532 |
| 58 | 0.0392 |
| 66 | 0.0377 |
| 75 | 0.428 |
| 77 | 0.307 |
| 96 | 0.0387 |
| 118 | 0.11 |
| 137 | 0.0099 |
| 138 | 0.0056 |

## Claims

1. A compound of formula I: 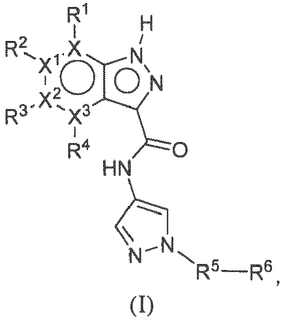 stereoisomers or a pharmaceutically acceptable salt thereof, wherein:
X, X¹, X² are C and X³ is C or N;
R¹, R², R³ and R⁴ are independently do not exist, hydrogen, C₁-C₁₂ alkyl, C₂-C₁₂ alkenyl, C₂-C₁₂ alkynyl, halogen, -CN, -OR⁷, -SR⁷, -NR⁷R⁸, -CF₃, -OCF₃, -NO₂, -C(O)R⁷, -C(O)OR⁷, -C(O)NR⁷R⁸, -NR⁷C(O)R⁸, -S(O)₁₋₂R⁷, -NR⁷S(O)₁₋₂R, -S(O)₁₋₂NR⁷R⁸, C₃-C₆ cycloalkyl, 3-10-membered heterocyclyl or 6-10 membered aryl, wherein R¹, R², R³ and R⁴ are independently optionally substituted by R⁹;
R⁵ is does not exist, C₁-C₆ alkylene, C₂-C₆ alkenylene, C₂-C₆ alkynylene, wherein said alkylene, alkenylene and alkynylene are independently optionally substituted by halogen, oxo, C₁-C₁₂ alkyl, C₂-C₁₂ alkenyl, C₂-C₁₂ alkynyl, -OR¹⁶, -SR¹⁶, -NR¹⁶R¹⁷, -CN, -C(O)R¹⁶,-C(O)OR¹⁶, -NR¹⁶C(O)R¹⁷, -NR¹⁶S(O)₁₋₂R¹⁷, -CF₃, -OCF₃, 3-10-membered heterocyclyl or 6-10 membered aryl, and wherein said alkyl, alkenyl, alkynyl, heterocyclyl and phenyl are independently optionally substituted with R⁹;
R⁶ is hydrogen, C₃-C₁₀ cycloalkyl, 3-10-membered heterocyclyl or 6-10-membered aryl, wherein R⁶ is independently optionally substituted by R⁹;
each R⁷ and R⁸ are independently hydrogen, C₁-C₆ alkyl, C₃-C₆ cycloalkyl, 3-6-membered heterocyclyl or phenyl, wherein said alkyl, cycloalkyl, heterocyclyl and phenyl are independently optionally substituted by halogen, -CN, -CF₃, -OCF₃, oxo or C₁-C₆ alkyl optionally substituted by halogen or oxo; or
R⁷ and R⁸ are independently taken together with the atom to which they are attached to form a 3-6 membered heterocyclyl optionally substituted by halogen, oxo or C₁-C₆ alkyl optionally substituted by halogen or oxo;
each R⁹ is independently hydrogen, oxo, C₁-C₁₂ alkyl, C₂-C₁₂ alkenyl, C₁-C₁₂ alkynyl, halogen, -(C₀-C₆ alkylene)CN, -(C₀-C₆ alkylene)OR¹⁰, -(C₀-C₆ alkylene)SR¹⁰, -(C₀-C₆ alkylene)NR¹⁰R¹¹, -(C₀-C₆ alkylene)CF₃, -(C₀-C₆ alkylene)NO₂, -(C₀-C₆ alkylene)C(O)R¹⁰,-(C₀-C₆ alkylene)C(O)OR¹⁰, -(C₀-C₆ alkylene)C(O)NR¹⁰R¹¹, -(C₀-C₆ alkylene)NR¹⁰C(O)R¹¹,-(C₀-C₆ alkylene)S(O)₁₋₂R¹⁰, -(C₀-C₆ alkylene)NR¹⁰S(O)₁₋₂R¹¹, -(C₀-C₆ alkylene)S(O)₁₋₂NR¹⁰R¹¹, -(C₀-C₆ alkylene)(C₃-C₆ cycloalkyl), -(C₀-C₆ alkylene)(3-10-membered heterocyclyl), -(C₀-C₆ alkylene)C(O)(3-10-membered heterocyclyl), or -(C₀-C₆ alkylene)(6-10 membered aryl), wherein each R⁹ is independently optionally substituted by halogen, oxo, -CF₃, -CN, -OR¹²,-SR¹², -NR¹²R¹³, -C(O)R¹², -S(O)₁₋₂R¹², C₁-C₆ alkyl optionally substituted by oxo or halogen, C₂-C₆ alkenyl optionally substituted by oxo or halogen, or C₂-C₆ alkynyl optionally substituted by oxo or halogen;
each R¹⁰ and R¹¹ are independently hydrogen, C₁-C₆ alkyl, C₂-C₆ alkenyl, C₂-C₆ alkynyl, 3-6-membered heterocyclyl, phenyl or C₃-C₆ cycloalkyl, wherein said alkyl, alkenyl, alkynyl, heterocyclyl, phenyl and cycloalkyl are independently optionally substituted by halogen, oxo,-CF₃, -OCF₃, -OR¹⁴, -SR¹⁴, -NR¹⁴R¹⁵, -CN, 3-6-membered heterocyclyl, phenyl, C₃-C₆ cycloalkyl or C₁-C₆ alkyl optionally substituted by halogen or oxo; or
R¹⁰ and R¹¹ are independently taken together with the atom to which they are attached to form a 3-6 membered heterocyclyl optionally substituted by halogen, oxo or C₁-C₆ alkyl optionally substituted by halogen or oxo;
each R¹² and R¹³ are independently hydrogen or C₁-C₆ alkyl optionally substituted by halogen or oxo; or
R¹² and R¹³ are independently taken together with the atom to which they are attached to form a 3-6 membered heterocyclyl optionally substituted by halogen, oxo or C₁-C₆ alkyl optionally substituted by halogen;
each R¹⁴ and R¹⁵ are independently hydrogen or C₁-C₆ alkyl optionally substituted by halogen or oxo; or
R¹⁴ and R¹⁵ are independently taken together with the atom to which they are attached to form a 3-6 membered heterocyclyl optionally substituted by halogen, oxo or C₁-C₆ alkyl optionally substituted by halogen;
each R¹⁶ and R¹⁷ are independently hydrogen, C₁-C₆ alkyl, C₂-C₆ alkenyl, C₂-C₆ alkynyl, 3-6-membered heterocyclyl, phenyl or C₃-C₆ cycloalkyl, wherein said alkyl, alkenyl, alkynyl, heterocyclyl, phenyl and cycloalkyl are independently optionally substituted by halogen, oxo,-CF₃, -OCF₃, -OR¹⁸, -SR¹⁸, -NR¹⁸R¹⁹, -CN, 3-6-membered heterocyclyl, phenyl, C₃-C₆ cycloalkyl or C₁-C₆ alkyl optionally substituted by halogen, -OR²⁰, -NR²⁰R²¹, or oxo; or
R¹⁶ and R¹⁷ are independently taken together with the atom to which they are attached to form a 3-6 membered heterocyclyl optionally substituted by halogen, oxo or C₁-C₆ alkyl optionally substituted by halogen or oxo;
each R¹⁸ and R¹⁹ are independently hydrogen or C₁-C₆ alkyl optionally substituted by halogen or oxo; or
R¹⁸ and R¹⁹ are independently taken together with the atom to which they are attached to form a 3-6 membered heterocyclyl optionally substituted by halogen, oxo or C₁-C₆ alkyl optionally substituted by halogen; and
each R²⁰ and R²¹ are independently hydrogen or C₁-C₆ alkyl optionally substituted by halogen or oxo; other than
N-(1-ethyl-1H-pyrazol-4-yl)-1H-indazole-3-carboxamide;
5-amino-N-(1-ethyl-1H-pyrazol-4-yl)-1H-indazole-3-carboxamide; 5-amino-N-(1-methyl-1H-pyrazol-4-yl)-1H-Indazole-3-carboxamide;
N-[1-(imidazo[1,2-a]pyridin-2-ylmethyl)-1H-pyrazol-4-yl]-1H-Indazole-3-carboxamide;
N-[1-[2-(diethylamino)ethyl]-1H-pyrazol-4-yl]-1H-Indazole-3-carboxamide;
N-(1-methyl-1H-pyrazol-4-yl)-5-nitro-1H-Indazole-3-carboxamide;
N-[1-[2-(3,4-dimethoxyphenyl)ethyl]-1H-pyrazol-4-yl]-1H-Indazole-3-carboxamide;
4-[(2H-indazol-3-ylcarbonyl)amino]-1H-Pyrazole-1-acetic acid;
N-[1-[2-(phenylmethoxy)ethyl]-1H-pyrazol-4-yl]-1H-Indazole-3-carboxamide;
N-[1-(4-cyanobutyl)-1H-pyrazol-4-yl]-1H-Indazole-3-carboxamide; or
N-[1-[(3-cyanophenyl)methyl]-1H-pyrazol-4-yl]-1H-Indazole-3-carboxamide..

2. The compound of claim 1, wherein X, X¹, X² and X³ are C.

3. The compound of claims 1-2, wherein R¹ and R⁴ are independently hydrogen, halogen or -OR⁷.

4. The compound of claims 1-3, wherein R² is hydrogen, halogen, -OR⁷, CF₃, CN or-NR⁷R⁸, dihydro-2H-pyrano[2,3-b]pyridinyl, pyridzainyl, oxazolyl, thiazolyl, pyrazinyl, pyrrolidinyl, azetidinyl, piperazinyl, 3,6-dihydropyridinyl, pyrazolyl, piperidinyl, 2,3-dihydropyrido[3,2-b][1,4]oxazin or pyrimidinyl, wherein R² is optionally substituted by R⁹.

5. The compound of claims 1-4, wherein R³ is hydrogen, halogen, -OR⁷, -NR⁷R⁸, C₁-C₁₂ alkyl, 3-10-membered heterocyclyl or 6-10 membered aryl, wherein R³ is optionally substituted by R⁹.

6. The compound of claims 1-5, wherein R⁵ is -CH₂-, -CH₂CH₂-, -CH₂CH₂CH₂-,-CH₂(CH₃)CH₂-, -CH₂CH(CH₃)₂-, -CH₂CH₂CH₂CH₂-, -CH₂CH(CH₂CH₃)CH₂CH₂-,-CH(CH₃)-, (*R*)-CH(CH₃)-, (S)-CH(CH₃)-, (*R*)-CH(CH(CH₃)₂)-, (*S*)-CH(CH(CH₃)₂)-,-CH(CH₂CH₃)-, -CH(CH₂CH₃)-, (*R*)-CH(CH₂CH₃)-, (*S*)-CH(CH₂CH₃)- or -C(CH₃)₂-, wherein R⁵ is independently optionally substituted by halogen, oxo, C₁-C₁₂ alkyl, C₂-C₁₂ alkenyl, C₂-C₁₂ alkynyl, -OR¹⁶, -SR¹⁶, -NR¹⁶R¹⁷, -CN, -CF₃, -OCF₃, -C(O)R¹⁶, -C(O)OR¹⁶, -NR¹⁶C(O)R¹⁷, NR¹⁶S(O)¹⁻²R¹⁷, 3-10-membered heterocyclyl or 6-10 membered aryl, and wherein said alkyl, alkenyl, alkynyl, heterocyclyl and phenyl are independently optionally substituted with R⁹.

7. The compound of claims 1-6, wherein R⁶ is hydrogen, cyclohexyl, cyclobutyl, norbornyl imidazolyl, pyrrolidinyl, azetidinyl, pyridazinyl, chromanyl, pyrimidinyl, tetrahydrofuranyl, tetrahydropyranyl, dioxanyl, morpholinyl, oxetanyl, phenyl, pyrazolyl, benzisoxazolyl, furanyl, isoxazolyl, benzothiazolyl, thiazolyl, thienyl, pyridinyl, piperidinyl, imidazo[1,2-a]pyridinyl or quinolinyl, wherein R⁶ is independently optionally substituted by R⁹.

8. The compound of claims 1-7, wherein R⁷ and R⁸ are independently hydrogen or C₁-C₆ alkyl, wherein said alkyl is independently optionally substituted by halogen, -CN, -CF₃, -OCF₃, oxo or C₁-C₆ alkyl optionally substituted by halogen or oxo; or R⁷ and R⁸ are independently taken together with the atom to which they are attached to form a 3-6 membered heterocyclyl optionally substituted by halogen, oxo or C₁-C₆ alkyl optionally substituted by halogen or oxo.

9. The compound of claims 1-8, wherein R⁹ is independently hydrogen, methyl, ethyl, propyl, -CN, -OCH₃, -OH, -C(O)OCH₃, -C(O)OH, -C(O)NH₂, -CF₃, -OCF₃, Br, Cl, F, -CH₃, -C(CH₃)₂OH, -NH₂, -CH₂N(CH₃)₂, -NH(CH₃), -N(CH₃)CH₂OH, -CH₂OH, morpholinyl,-C(O)piperidinyl, ethynyl, piperazinyl, pyridinyl, tetrazolyl, phenyl, -C(O)NH(CH₃),-CH₂morpholinyl, isopropyl, thienyl,

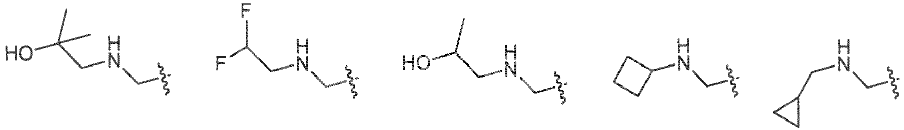

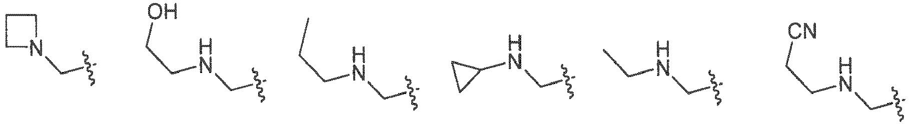

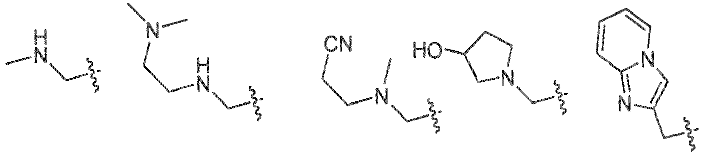

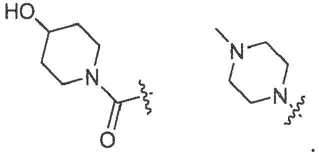

10. The compound of claims 1-9, wherein each R¹⁰ and R¹¹ is independently hydrogen or C₁-C₆ alkyl optionally substituted by halogen or oxo, wherein said alkyl is independently optionally substituted by halogen or oxo; or R¹⁰ and R¹¹ are independently taken together with the atom to which they are attached to form a 3-6 membered heterocyclyl optionally substituted by halogen, oxo or C₁-C₆ alkyl optionally substituted by halogen or oxo.

11. The compound of claims 1-10, wherein each R¹²⁻²¹ is independently hydrogen or methyl.

12. A pharmaceutical composition comprising a compound of claims 1-11 and a therapeutically inert carrier, diluent or excipient.

13. A compound of claims 1-11 for use in the treatment of an immunological or inflammatory disease.

14. A method of manufacturing a compound of claims 1-11, comprising reacting a compound of formula 1-3: 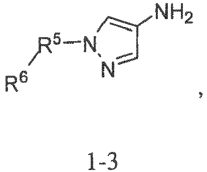 or a salt thereof, with a comopund of formula 1-4 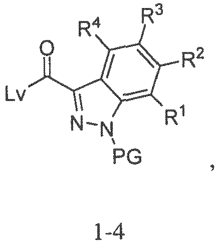 or a salt thereof, wherein PG is an amino protecting group and Lv is a leaving group.

## Patentansprüche

1. Eine Verbindung der Formel I: 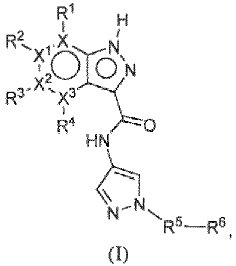 Stereoisomere oder ein pharmazeutisch verträgliches Salz davon, wobei:
X, X¹, X² gleich C sind und X³ gleich C oder N ist;
R¹, R², R³ und R⁴ unabhängig nicht existieren, Wasserstoff, C₁-C₁₂-Alkyl, C₂-C₁₂-Alkenyl, C₂-C₁₂-Alkinyl, Halogen, -CN, -OR⁷, -SR⁷, -NR⁷R⁸, -CF₃, -OCF₃, -NO₂, -C(O)R⁷, -C(O)OR⁷, -C(O)NR⁷R⁸, -NR⁷C(O)R⁸, -S(O)₁₋₂R⁷, -NR⁷S(O)₁₋₂R⁸, -S(O)₁₋₂NR⁷R⁸, C₃-C₆-Cycloalkyl, 3-10-gliedriges Heterocyclyl oder 6-10-gliedriges Aryl sind, wobei R¹, R², R³ und R⁴ unabhängig gegebenenfalls mit R⁹ substituiert sind;
R⁵ nicht existiert, C₁-C₆-Alkylen, C₂-C₆-Alkenylen, C₂-C₆-Alkinylen ist, wobei Alkylen, Alkenylen und Alkinylen unabhängig gegebenenfalls mit Halogen, Oxo, C₁-C₁₂-Alkyl, C₂-C₁₂-Alkenyl, C₂-C₁₂-Alkinyl, -OR¹⁶, -SR¹⁶, -NR¹⁶R¹⁷, -CN, -C(O)R¹⁶, -C(O)OR¹⁶, -NR¹⁶C(O)R¹⁷, -NR¹⁶S(O)₁₋₂R¹⁷, -CF₃, -OCF₃, 3-10-gliedrigem Heterocyclyl oder 6-10-gliedrigem Aryl substituiert sind und wobei das Alkyl, Alkenyl, Alkinyl, Heterocyclyl und Phenyl unabhängig gegebenenfalls mit R⁹ substituiert sind;
R⁶ Wasserstoff, C₃-C₁₀-Cycloalkyl, 3-10-gliedriges Heterocyclyl oder 6-10-gliedriges Aryl ist, wobei R⁶ unabhängig gegebenenfalls mit R⁹ substituiert ist;
jedes R⁷ und R⁸ unabhängig Wasserstoff, C₁-C₆-Alkyl, C₃-C₆-Cycloalkyl, 3-6-gliedriges Heterocyclyl oder Phenyl ist, wobei Alkyl, Cycloalkyl, Heterocyclyl und Phenyl unabhängig gegebenenfalls mit Halogen, -CN, -CF₃, -OCF₃, Oxo oder gegebenenfalls mit Halogen oder Oxo substituiertem C₁-C₆-Alkyl substituiert sind; oder
R⁷ und R⁸ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen oder Oxo substituiertem C₁-C₆-Alkyl substituiert ist;
jedes R⁹ unabhängig Wasserstoff, Oxo, C₁-C₁₂-Alkyl, C₂-C₁₂-Alkenyl, C₂-C₁₂-Alkinyl, Halogen, -(C₀-C₆-Alkylen)CN, -(C₀-C₆-Alkylen)OR¹⁰), -(C₀-C₆-Alkylen)SR¹⁰, -(C₀-C₆-Alkylen)NR¹⁰R¹¹, -(C₀-C₆-Alkylen)CF₃, -(C₀-C6-Alkylen)NO₂, -(C₀-C₆-Alkylen)C(O)R¹⁰, -(C₀-C₆-Alkylen)C(O)OR¹⁰, -(C₀-C₆-Alkylen)C(O)NR¹⁰R¹¹, -(C₀-C₆-Alkylen)NR¹⁰C(O)R¹¹, -(C₀-C₆-Alkylen)S(O)₁₋₂R¹⁰, -(C₀-C₆-Alkylen)NR¹⁰S(O)₁₋₂R¹¹, -(C₀-C₆-Alkylen)S(O)₁₋₂NR¹⁰R¹¹, -(C₀-C₆-Alkylen)(C₃-C₆-cycloalkyl), -(C₀-C₆-Alkylen)(3-10-gliedriges Heterocyclyl), -(C₀-C₆-Alkylen)C(O)(3-10-gliedriges Heterocyclyl) oder -(C₀-C₆-Alkylen)(6-10-gliedriges Aryl) ist, wobei jedes R⁹ unabhängig gegebenenfalls mit Halogen, Oxo, -CF₃, -CN, -OR¹², -SR¹², -NR¹²R¹³, -C(O)R¹², -S(O)₁₋₂R¹², gegebenenfalls mit Oxo oder Halogen substituiertem C₁-C₆-Alkyl, gegebenenfalls mit Oxo oder Halogen substituiertem C₂-C₆-Alkenyl oder gegebenenfalls mit Oxo oder Halogen substituiertem C₂-C₆-Alkinyl substituiert ist;
jedes R¹⁰ und R¹¹ unabhängig Wasserstoff, C₁-C₆-Alkyl, C₂-C₆-Alkenyl, C₂-C₆-Alkinyl, 3-6-gliedriges Heterocyclyl, Phenyl oder C₃-C₆-Cycloalkyl ist, wobei Alkyl, Alkenyl, Alkinyl, Heterocyclyl, Phenyl und Cycloalkyl unabhängig gegebenenfalls mit Halogen, Oxo, -CF₃, -OCF₃, -OR¹⁴, -SR¹⁴, -NR¹⁴R¹⁵, -CN, 3-6-gliedrigem Heterocyclyl, Phenyl,
C₃-C₆-Cycloalkyl oder gegebenenfalls mit Halogen oder Oxo substituiertem C₁-C₆-Alkyl substituiert sind; oder
R¹⁰ und R¹¹ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen oder Oxo substituiertem C₁-C₆-alkyl substituiert ist;
jedes R¹² und R¹³ unabhängig Wasserstoff oder gegebenenfalls mit Halogen oder Oxo substituiertes C₁-C₆-Alkyl ist; oder
R¹² und R¹³ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen substituiertem C₁-C₆-Alkyl substituiert ist;
jedes R¹⁴ und R¹⁵ unabhängig Wasserstoff oder gegebenenfalls mit Halogen oder Oxo substituiertes C₁-C₆-Alkyl ist; oder
R¹⁴ und R¹⁵ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen substituiertem C₁-C₆-Alkyl substituiert ist;
jedes R¹⁶ und R¹⁷ unabhängig Wasserstoff, C₁-C₆-Alkyl, C₂-C₆-Alkenyl, C₂-C₆-Alkinyl,
3-6-gliedriges Heterocyclyl, Phenyl oder C₃-C₆-Cycloalkyl ist, wobei Alkyl, Alkenyl, Alkinyl, Heterocyclyl, Phenyl und Cycloalkyl unabhängig gegebenenfalls mit Halogen, Oxo, -CF₃, -OCF₃, -OR¹⁸, -SR¹⁸, -NR¹⁸R¹⁹, -CN, 3-6-gliedrigem Heterocyclyl, Phenyl,
C₃-C₆-Cycloalkyl oder gegebenenfalls mit Halogen, -OR²⁰, -NR²⁰R²¹ oder Oxo substituiertem C₁-C₆-Alkyl substituiert sind; oder
R¹⁶ und R¹⁷ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen oder Oxo substituiertem C₁-C₆-Alkyl substituiert ist;
jedes R¹⁸ und R¹⁹ unabhängig Wasserstoff oder gegebenenfalls mit Halogen oder Oxo substituiertes C₁-C₆-Alkyl ist; oder
R¹⁸ und R¹⁹ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen substituiertem C₁-C₆-Alkyl substituiert ist; und
jedes R²⁰ und R²¹ unabhängig Wasserstoff oder gegebenenfalls mit Halogen oder Oxo substituiertes C₁-C₆-Alkyl ist; mit Ausnahme von
N-(1-Ethyl-1H-pyrazol-4-yl)-1H-indazol-3-carboxamid;
5-Amino-N-(1-ethyl-1H-pyrazol-4-yl)-1H-indazol-3-carboxamid;
5-Amino-N-(1-methyl-1H-pyrazol-4-yl)-1H-indazol-3-carboxamid;
N-[1-(Imidazo[1,2-a]pyridin-2-ylmethyl)-1H-pyrazol-4-yl]-1H-indazol-3-carboxamid;
N-[1-[2-(Diethylamino)ethyl]-1H-pyrazol-4-yl]-1H-indazol-3-carboxamid;
N-(1-Methyl-1H-pyrazol-4-yl)-5-nitro-1H-indazol-3-carboxamid;
N-[1-[2-(3,4-Dimethoxyphenyl)ethyl]-1H-pyrazol-4-yl]-1H-indazol-3-carboxamid;
4-[(2H-Indazol-3-ylcarbonyl)amino]-1H-pyrazol-1-essigsäure;
N-[1-[2-(Phenylmethoxy)ethyl]-1H-pyrazol-4-yl]-1H-indazol-3-carboxamid;
N-[1-(4-Cyanobutyl)-1H-pyrazol-4-yl]-1H-indazol-3-carboxamid; oder
N-[1-[(3-Cyanophenyl)methyl]-1H-pyrazol-4-yl]-1H-indazol-3-carboxamid.

2. Die Verbindung nach Anspruch 1, wobei X, X¹, X² und X³ gleich C sind.

3. Die Verbindung nach den Ansprüchen 1-2, wobei R¹ und R⁴ unabhängig Wasserstoff, Halogen oder -OR⁷ sind.

4. Die Verbindung nach den Ansprüchen 1-3, wobei R² Wasserstoff, Halogen, -R⁷, CF₃, CN oder -NR⁷R⁸, Dihydro-2H-pyrano[2,3-b]pyridinyl, Pyridazinyl, Oxazolyl, Thiazolyl, Pyrazinyl, Pyrrolidinyl, Azetidinyl, Piperazinyl, 3,6-Dihydropyridinyl, Pyrazolyl, Piperidinyl, 2,3-Dihydropyrido[3,2-b][1,4]oxazin oder Pyrimidinyl ist, wobei R² gegebenenfalls mit R⁹ substituiert ist.

5. Die Verbindung nach den Ansprüchen 1-4, wobei R³ Wasserstoff, Halogen, -OR⁷, -NR⁷R⁸, C₁-C₁₂-Alkyl, 3-10-gliedriges Heterocyclyl oder 6-10-gliedriges Aryl ist, wobei R³ gegebenenfalls mit R⁹ substituiert ist.

6. Die Verbindung nach den Ansprüchen 1-5, wobei R⁵ gleich -CH₂-, -CH₂CH₂-, -CH₂CH₂CH₂-, -CH₂(CH₃)CH₂-, -CH₂CH(CH₃)₂-, -CH₂CH₂CH₂CH₂-, -CH₂CH(CH₂CH₃)CH₂CH₂-, -CH(CH₃)-, (*R*)-CH(CH₃)-, (*S*)-CH(CH₃)-, (*R*)-CH(CH(CH₃)₂)-, (*S*)-CH(CH(CH₃)₂)-, -CH(CH₂CH₃)-, -CH(CH₂CH₃)-, (*R*)-CH(CH₂CH₃)-, (*S*)-CH(CH₂CH₃)- oder -C(CH₃)₂- ist, wobei R⁵ unabhängig gegebenenfalls mit Halogen, Oxo, C₁-C₁₂-Alkyl, C₂-C₁₂-Alkenyl, C₂-C₁₂-Alkinyl, -OR¹⁶, -SR¹⁶, -NR¹⁶R¹⁷, -CN, -CF₃, -OCF₃, -C(O)R¹⁶, -C(O)OR¹⁶, -NR¹⁶C(O)R¹⁷, -NR¹⁶S(O)₁₋₂R¹⁷, 3-10-gliedrigem Heterocyclyl oder 6-10-gliedrigem Aryl substituiert ist und wobei Alkyl, Alkenyl, Alkinyl, Heterocyclyl und Phenyl unabhängig gegebenenfalls mit R⁹ substituiert sind.

7. Die Verbindung nach den Ansprüchen 1-6, wobei R⁶ Wasserstoff, Cyclohexyl, Cyclobutyl, Norbornyl, Imidazolyl, Pyrrolidinyl, Azetidinyl, Pyridazinyl, Chromanyl, Pyrimidinyl, Tetrahydrofuranyl, Tetrahydropyranyl, Dioxanyl, Morpholinyl, Oxetanyl, Phenyl, Pyrazolyl, Benzisoxazolyl, Furanyl, Isoxazolyl, Benzothiazolyl, Thiazolyl, Thienyl, Pyridinyl, Piperidinyl, Imidazo[1,2-a]pyridinyl oder Chinolinyl ist, wobei R⁶ unabhängig gegebenenfalls mit R⁹ substituiert ist.

8. Die Verbindung nach den Ansprüchen 1-7, wobei R⁷ und R⁸ unabhängig Wasserstoff oder C₁-C₆-Alkyl sind, wobei das Alkyl unabhängig gegebenenfalls mit Halogen, -CN, -CF₃, -OCF₃, Oxo oder gegebenenfalls mit Halogen oder Oxo substituiertem C₁-C₆-Alkyl substituiert ist; oder R⁷ und R⁸ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen oder Oxo substituiertem C₁-C₆-Alkyl substituiert ist.

9. Die Verbindung nach den Ansprüchen 1-8, wobei R⁹ unabhängig Wasserstoff, Methyl, Ethyl, Propyl, -CN, -OCH₃, -OH, -C(O)OCH₃, -C(O)OH, -C(O)NH₂, -CF₃, -OCF₃, Br, Cl, F, -CH₃, -C(CH₃)₂OH, -NH₂, -CH₂N(CH₃)₂, -NH(CH₃), -N(CH₃)CH₂OH, -CH₂OH, Morpholinyl, -C(O)Piperidinyl, Ethinyl, Piperazinyl, Pyridinyl, Tetrazolyl, Phenyl, -C(O)NH(CH₃), -CH₂Morpholinyl, Isopropyl, Thienyl, 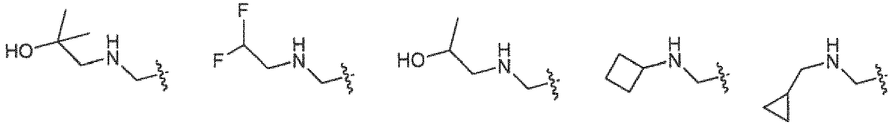 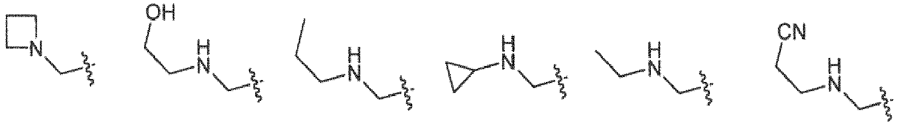 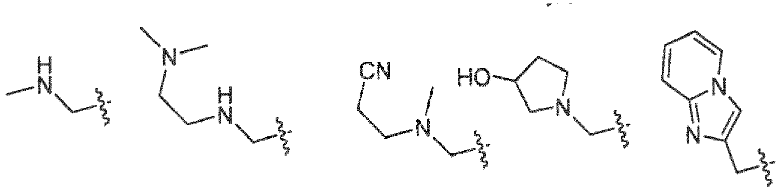 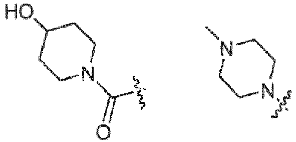 ist.

10. Die Verbindung nach den Ansprüchen 1-9, wobei jedes R¹⁰ und R¹¹ unabhängig Wasserstoff oder gegebenenfalls mit Halogen oder Oxo substituiertes C₁-C₆-Alkyl ist, wobei das Alkyl unabhängig gegebenenfalls mit Halogen oder Oxo substituiert ist; oder R¹⁰ und R¹¹ unabhängig mit dem Atom, an das sie gebunden sind, zusammengenommen sind, um ein 3-6-gliedriges Heterocyclyl zu bilden, das gegebenenfalls mit Halogen, Oxo oder gegebenenfalls mit Halogen oder Oxo substituiertem C₁-C₆-Alkyl substituiert ist.

11. Die Verbindung nach den Ansprüchen 1-10, wobei jedes R¹²⁻²¹ unabhängig Wasserstoff oder Methyl ist.

12. Ein Arzneimittel, umfassend eine Verbindung nach den Ansprüchen 1-11 und einen therapeutisch inerten Träger, Lösungsmittel oder Exzipienten.

13. Eine Verbindung nach den Ansprüchen 1-11 zur Verwendung bei der Behandlung einer immunologischen oder entzündlichen Erkrankung.

14. Ein Verfahren zur Herstellung einer Verbindung nach den Ansprüchen 1-11, umfassend Umsetzen einer Verbindung der Formel 1-3: 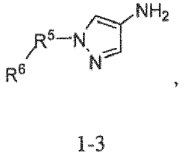 oder eines Salzes davon mit einer Verbindung der Formel 1-4 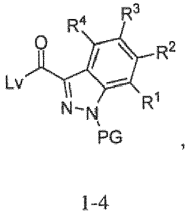 oder eines Salzes davon, wobei PG eine Aminoschutzgruppe ist und Lv eine Abgangsgruppe ist.

## Revendications

1. Composé de formule (I) : 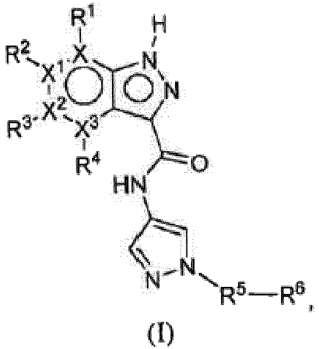 ses stéréoisomères ou un de leurs sels pharmaceutiquement acceptables, formule dans laquelle :
X, X¹ et X² représentent C et X³ représente C ou N ;
R¹, R², R³ ou R⁴, indépendamment, n'existent pas ou représentent un atome d'hydrogène, un groupe alkyle en C₁ à C₁₂, alcényle en C₂ à C₁₂, alcynyle en C₂ à C₁₂, halogéno, -CN, -OR⁷, -SR⁷, NR⁷R⁸, -CF₃, -OCF₃, -NO₂, -C(O)R⁷, -C(O)OR⁷, -C(O)NR⁷R⁸, -NR⁷C(O)R⁸, -S(O)₁₋₂R⁷, -NR⁷S(O)₁₋₂R⁸, -S(O)₁₋₂NR⁷R⁸, cycloalkyle en C₃ à C₆, hétérocyclyle tri- à décagonal ou aryle hexa- à décagonal, les groupes R¹, R², R³ et R⁴ étant facultativement substitués indépendamment avec R⁹ ;
R⁵ n'existe pas ou représente un groupe alkylène en C₁ à C₆, alcénylène en C₂ à C₆, alcynylène en C₂ à C₆, lesdits groupes alkylène, alcénylène et alcynylène étant facultativement substitués indépendamment avec des substituants halogéno, oxo, alkyle en C₁ à C₁₂, alcényle en C₂ à C₁₂, alcynyle en C₂ à C₁₂, -OR¹⁶, -SR¹⁶, -NR¹⁶R¹⁷, -CN, -C(O)R¹⁶, -C(O)OR¹⁶, -NR¹⁶C(O)R¹⁷, -NR¹⁶S(O)₁₋₂R¹⁷, -CF₃, -OCF₃, hétérocyclyle tri- à décagonal ou aryle hexa- à décagonal, lesdits groupes alkyle, alcényle, alcynyle, hétérocyclyle et phényle étant facultativement substitués indépendamment avec R⁹ ;
R⁶ représente un atome d'hydrogène, un groupe cycloalkyle en C₃ à C₁₀, hétérocyclyle tri- à décagonal ou aryle hexa- à décagonal, R⁶ étant facultativement substitué indépendamment avec R⁹ ;
chacun de R⁷ et R⁸ représente indépendamment un atome d'hydrogène, un groupe alkyle en C₁ à C₆, cycloalkyle en C₃ à C₆, hétérocyclyle tri- à hexagonal ou phényle, lesdits groupes alkyle, cycloalkyle, hétérocyclyle et phényle étant facultativement substitués indépendamment avec des substituants halogéno, -CN, -CF₃, -OCF₃, oxo ou alkyle en C₁ à C₆ facultativement substitué avec un substituant halogéno ou oxo ; ou bien
R⁷ et R⁸ sont pris indépendamment conjointement avec l'atome auquel ils sont fixés pour former un groupe hétérocyclyle tri- à hexagonal facultativement substitué avec un substituant halogéno, oxo ou alkyle en C₁ à C₆ facultativement substitué avec un substituant halogéno ou oxo ;
chaque groupe R⁹ représente indépendamment un atome d'hydrogène, un groupe oxo, alkyle en C₁ à C₁₂, alcényle en C₂ à C₁₂, alcynyle en C₂ à C₁₂, halogéno, - (alkylène en C₀ à C₆)CN, -(alkylène en C₀ à C₆)OR¹⁰, -(alkylène en C₀ à C₆) SR¹⁰, -(alkylène en C₀ à C₆)NR¹⁰R¹¹, -(alkylène en C0 à C₆CF₃, - (alkylène en C₀ à C₆) NO₂, -(alkylène en C₀ à C₆) C(O)R¹⁰, - (alkylène en C₀ à C₆)C(O)OR¹⁰, -(alkylène en C₀ à C₆) C(O)NR¹⁰R¹¹, (alkylène en C₀ à C₆) NR¹⁰C (O)R¹¹, - (alkylène en C₀ à C₆) S(O)₁₋₂R¹⁰, -(alkylene en C₀ à C₆) NR¹⁰S (O)₁₋₂R¹¹, - (alkylène en C₀ à C₆) S(O)₁₋₂NR¹⁰R¹¹, -(alkylène en C₀ à C₆) (cycloalkyle en C₃ à C₆), -(alkylène en C₀ à C₆) (hétérocyclyle tri- à décagonal), -(alkylène en C₀ à C₆)C(O) (hétérocyclyle tri- à décagonal) ou -(alkylène en C₀ à C₆)(aryle en C₆ à C₁₀), chaque groupe R⁹ étant facultativement substitué indépendamment avec un substituant halogéno, oxo, -CF₃, -CN, -OR¹², -SR¹², -NR¹²R¹³, -C(O)R¹², -S(O)₁₋₂R¹², alkyle en C₁ à C₆ facultativement substitué avec un substituant oxo ou halogéno, alcényle en C₂ à C₆ facultativement substitué avec un substituant oxo ou halogéno, ou alcynyle en C₂ à C₆ facultativement substitué avec un substituant oxo ou halogéno ;
chacun de R¹⁰ et R¹¹ représente indépendamment un atome d'hydrogène, un groupe alkyle en C₁ à C₆, alcényle en C₂ à C₆, alcynyle en C₂ à C₆, hétérocyclyle tri- à hexagonal, phényle ou cycloalkyle en C₃ à C₆, lesdits groupes alkyle, alcényle, alcynyle, hétérocyclyle, phényle et cycloalkyle étant facultativement substitués indépendamment avec des substituants oxo, -CF₃, -OCF₃, -OR¹⁴, -SR¹⁴, -NR¹⁴R¹⁵, -CN, hétérocyclyle tri- à hexagonal, phényle, cycloalkyle en C₃ à C₆ ou alkyle en C₁ à C₆ facultativement substitué avec un substituant halogéno ou oxo ; ou bien
R¹⁰ et R¹¹ sont pris indépendamment conjointement avec l'atome de carbone auquel ils sont fixés pour former un groupe hétérocyclyle tri- à hexagonal facultativement substitué avec un substituant halogéno, oxo ou alkyle en C₁ à C₆ facultativement substitué avec un substituant halogéno ou oxo ;
chacun de R¹² et R¹³ représente indépendamment un atome d'hydrogène ou un groupe alkyle en C₁ à C₆ facultativement substitué avec un substituant halogéno ou oxo ; ou bien
R¹² et R¹³ sont pris indépendamment conjointement avec l'atome auquel ils sont fixés pour former un groupe hétérocyclyle tri- à hexagonal facultativement substitué avec un substituant halogéno, oxo, ou alkyle en C₁ à C₆ facultativement substitué avec un substituant halogéno ;
chacun de R¹⁴ et R¹⁵ représente indépendamment un atome d'hydrogène ou un groupe alkyle en C₁ à C₆ facultativement substitué avec un substituant halogéno ou oxo ; ou bien
R¹⁴ et R¹⁵ sont pris indépendamment conjointement avec l'atome auquel ils sont fixés pour former un groupe hétérocyclyle tri- à hexagonal facultativement substitué avec un substituant halogéno, oxo, ou alkyle en C₁ à C₆ facultativement substitué avec un substituant halogéno ;
chacun de R¹⁶ et R¹⁷ représente indépendamment un atome d'hydrogène, un groupe alkyle en C₁ à C₆, alcényle en C₂ à C₆, alcynyle en C₂ à C₆, hétérocyclyle tri- à hexagonal, phényle ou cycloalkyle en C₃ à C₆, lesdits groupes alkyle, alcényle, alcynyle, hétérocyclyle, phényle et cycloalkyle étant facultativement substitués indépendamment avec des substituants halogéno, oxo, -CF₃, -OCF₃, -OR¹⁸, -SR¹⁸, -NR¹⁸R¹⁹, -CN, hétérocyclyle tri- à hexagonal, phényle, cycloalkyle en C₃ à C₆ ou alkyle en C₁ à C₆ facultativement substitué avec un substituant halogéno, -OR²⁰, -NR²⁰R²¹, ou oxo ; ou bien
R¹⁶ et R¹⁷ sont pris indépendamment conjointement avec l'atome auquel ils sont fixés pour former un groupe hétérocyclyle tri- à hexagonal facultativement substitué avec un substituant halogéno, oxo, ou alkyle en C₁ à C₆ facultativement substitué avec un substituant halogéno ou oxo ;
chacun de R¹⁸ et R¹⁹ représente indépendamment un atome d'hydrogène ou un groupe alkyle en C₁ à C₆ facultativement substitué avec un substituant halogéno ou oxo ; ou bien
R¹⁸ et R¹⁹ sont pris indépendamment conjointement avec l'atome auquel ils sont fixés pour former un groupe hétérocyclyle tri- à hexagonal facultativement substitué avec un substituant halogéno, oxo ou alkyle en C₁ à C₆ facultativement substitué avec un substituant halogéno ; et
chacun de R²⁰ et R²¹ représente indépendamment un atome d'hydrogène ou un groupe alkyle en C₁ à C₆ facultativement substitué avec un substituant halogéno ou oxo ; autre que
N-(1-éthyl-1H-pyrazole-4-yl)-1H-indazole-3-carboxamide;
5-amino-N-(1-éthyl-1H-pyrazole-4-yl)-1H-indazole-3-carboxamide ;
5-amino-N-(1-méthyl-1H-pyrazole-4-yl)-1H-indazole-3-carboxamide ;
N-[1-(imidazo[1,2-a]pyridine-2-ylméthyl)-1H-pyrazole-4-yl]-1H-indazole-3-carboxamide ;
N-[1-[2-(diéthylamino)éthyl]-1H-pyrazole-4-yl]-1H-indazole-3-carboxamide ;
N-(1-méthyl-1H-pyrazole-4-yl)-5-nitro-1H-indazole-3-carboxamide ;
N-[1-[2-(3,4-diméthoxyphényl)éthyl]-1H-pyrazole-4-yl]-1H-indazole-3-carboxamide;
acide 4-[(2H-indazole-3-ylcarbonyl)amino]-1H-pyrazole-1-acétique ;
N-[1-[2-(phénylméthoxy)éthyl]-1H-pyrazole-4-yl]-1H-indazole-3-carboxamide ;
N-[1-(4-cyanobutyl)-1H-pyrazole-4-yl]-1H-indazole-3-carboxamide ; ou
N-[1-[(3-cyanophényl)méthyl]-1H-pyrazole-4-yl]-1H-indazole-3-carboxamide.

2. Composé suivant la revendication 1, dans lequel X, X¹, X² et X³ représentent C.

3. Composé suivant les revendications 1 et 2, dans lequel R¹ et R⁴ représentent indépendamment un atome d'hydrogène, un groupe halogéno ou -OR⁷.

4. Composé suivant les revendications 1 à 3, dans lequel R² représente un atome d'hydrogène, un groupe halogéno, -OR⁷, CF₃, CN ou -NR⁷R⁸, dihydro-2H-pyrano[2,3-b]-pyridinyle, pyridazinyle, oxazolyle, thiazolyle, pyrazinyle, pyrrolidinyle, azétidinyle, pipérazinyle, 3,6-dihydro-pyridinyle, pyrazolyle, pipéridinyle, 2,3-dihydro-pyrido[3,2-b][1,4]oxazine ou pyrimidinyle, R² étant facultativement substitué avec R⁹.

5. Composé suivant les revendications 1 à 4, dans lequel R³ représente un atome d'hydrogène, un groupe halogéno, -OR⁷, -NR⁷R⁸, alkyle en C₁ à C₁₂, hétérocyclyle tri- à décagonal ou aryle hexa- à décagonal, R³ étant facultativement substitué avec R⁹.

6. Composé suivant les revendication 1 à 5, dans lequel R⁵ représente un groupe -CH₂-, -CH₂CH₂-, -CH₂CH₂CH₂-, -CH₂(CH₃)CH₂-, -CH₂CH(CH₃)₂-, -CH₂CH₂CH₂CH₂-, -CH₂CH(CH₂CH₃)CH₂CH₂-, -CH(CH₃)-, (*R*)-CH(CH₃) -, (*S*)-CH(CH₃) -, (*R*)-CH(CH(CH₃)₂) -, (*S*)-CH(CH(CH₃)₂) -, -CH(CH₂CH₃)-, -CH(CH₂CH₃)-, (*R*)-CH(CH₂CH₃)-, (*S*)-CH(CH₂CH₃) - ou -C(CH₃)₂-, R⁵ étant facultativement substitué indépendamment avec un substituant halogéno, oxo, alkyle en C₁ à C₁₂, alcényle en C₂ à C₁₂, alcynyle en C₂ à C₁₂, -OR¹⁶, -SR¹⁶, -NR¹⁶R¹⁷, -CN, -CF₃, -OCF₃, -C(O)R¹⁶, -C(O)OR¹⁶, -NR¹⁶C(O)R¹⁷, NR¹⁶S(O)₁₋₂R¹⁷, hétérocyclyle tri- à décagonalou aryle hexa- à décagonal, lesdits groupes alkyle, alcényle, alcynyle, hétérocyclyle et phényle étant facultativement substitués indépendamment avec R⁹.

7. Composé suivant les revendications 1 à 6, dans lequel R⁶ représente un atome d'hydrogène, un groupe cyclohexyle, cyclobutyle, norbornyle, imidazolyle, pyrrolidinyle, azétidinyle, pyridazinyle, chromanyle, pyrimidinyle, tétrahydrofuranyle, tétrahydropyranyle, dioxanyle, morpholinyle, oxétanyle, phényle, pyrazolyle, benzisoxazolyle, furanyle, isoxazolyle, benzothiazolyle, thiazolyle, thiényle, pyridinyle, pipéridinyle, imidazo[1,2-a]pyridinyle ou quinolinyle, R⁶ étant facultativement substitué indépendamment avec R⁹.

8. Composé suivant les revendications 1 à 7, dans lequel R⁷ et R⁸ représentent indépendamment un atome d'hydrogène ou un groupe alkyle en C₁ à C₆, ledit groupe alkyle étant facultativement substitué indépendamment avec un substituant halogéno, -CN, -CF₃, -OCF₃, oxo ou alkyle en C₁ à C₆ facultativement substitué avec un substituant halogéno ou oxo ; ou bien R⁷ et R⁸ sont pris indépendamment conjointement avec l'atome auquel ils sont fixés pour former un groupe hétérocyclyle tri- à hexagonal facultativement substitué avec un substituant halogéno, oxo, ou alkyle en C₁ à C₆ facultativement substitué avec un substituant halogéno ou oxo.

9. Composé suivant les revendications 1 à 8, dans lequel R⁹ représente indépendamment un atome d'hydrogène, un groupe méthyle, éthyle, propyle, -CN, -OCH₃, -OH, -C(O)OCH₃, -C(O)OH, -C(O)NH₂, -CF₃, -OCF₃, Br, Cl, F, -CH₃, -C(CH₃)₂OH, -NH₂, -CH₂N(CH₃)₂, -NH(CH₃), -N(CH₃)CH₂OH, -CH₂OH, morpholinyle, -C(O)pipéridinyle, éthynyle, pipérazinyle, pyridinyle, tétrazolyle, phényle, -C(O)NH(CH₃), -CH₂-morpholinyle, isopropyle, thiényle,

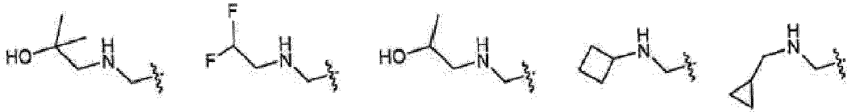

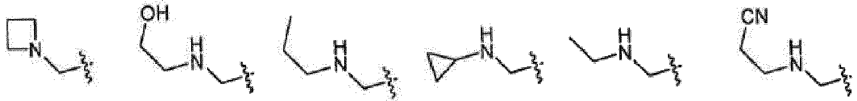

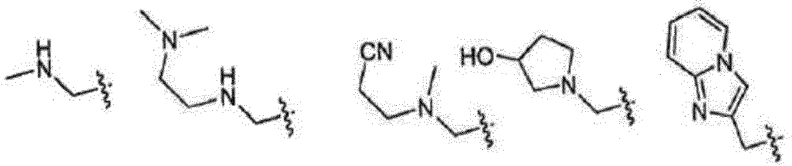

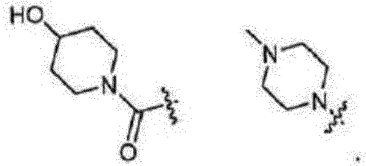

10. Composé suivant les revendications 1 à 9, dans lequel chacun de R¹⁰ et R¹¹ représente indépendamment un atome d'hydrogène ou un groupe alkyle en C₁ à C₆ facultativement substitué avec un substituant halogéno ou oxo, ledit groupe alkyle étant facultativement substitué indépendamment avec un substituant halogéno ou oxo ; ou bien R¹⁰ ou R¹¹ sont pris indépendamment conjointement avec l'atome auquel ils sont fixés pour former un groupe hétérocyclyle tri- à hexagonal facultativement substitué avec un substituant halogéno, oxo, ou alkyle en C₁ à C₆ facultativement substitué avec un substituant halogéno ou oxo.

11. Composé suivant les revendications 1 à 10, dans lequel chacun des groupes R¹² à R²¹ représente indépendamment un atome d'hydrogène ou un groupe méthyle.

12. Composition pharmaceutique comprenant un composé des revendications 1 à 11 et un support, diluant ou excipient thérapeutiquement inerte.

13. Composé suivant les revendications 1 à 11, pour une utilisation dans le traitement d'une maladie immunologique ou inflammatoire.

14. Procédé de production d'un composé des revendications 1 à 11, comprenant la réaction d'un composé de formule 1-3 : 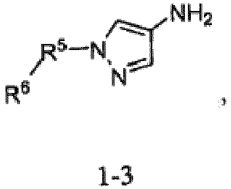 ou d'un de ses sels, avec un composé de formule 1-4 : 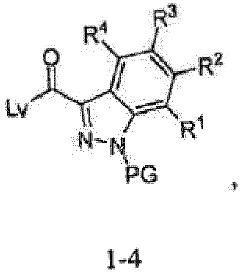 ou un de ses sels, où PG représente un groupe protecteur de la fonction amino et Lv représente un groupe partant.
